(19) Europäisches Patentamt / European Patent Office / Office européen des brevets

(11) EP 2 297 347 B1

(12) # EUROPEAN PATENT SPECIFICATION

(45) Date of publication and mention of the grant of the patent: **08.03.2017 Bulletin 2017/10**

(21) Application number: **09812449.8**

(22) Date of filing: **14.05.2009**

(51) Int Cl.: ***C12Q 1/68*** *(2006.01)*

(86) International application number: **PCT/US2009/002981**

(87) International publication number: **WO 2010/096036 (26.08.2010 Gazette 2010/34)**

(54) **METHODS AND KITS FOR MONITORING THE EFFECTS OF IMMUNOMODULATORS ON ADAPTIVE IMMUNITY**

VERFAHREN UND KITS ZUR ÜBERWACHUNG DER WIRKUNG VON IMMUNMODULATOREN AUF DIE ADAPTIVE IMMUNITÄT

MÉTHODES ET TROUSSES POUR SURVEILLER LES EFFETS D'IMMUNOMODULATEURS SUR L'IMMUNITÉ ADAPTATIVE

(84) Designated Contracting States: **AT BE BG CH CY CZ DE DK EE ES FI FR GB GR HR HU IE IS IT LI LT LU LV MC MK MT NL NO PL PT RO SE SI SK TR**

(30) Priority: **14.05.2008 US 127522 P**

(43) Date of publication of application: **23.03.2011 Bulletin 2011/12**

(73) Proprietor: **Millennium Pharmaceuticals, Inc.**
**Cambridge, MA 02139 (US)**

(72) Inventor: **FEDYK, Eric, R.**
**Acton, MA 01720 (US)**

(74) Representative: **Lau, Sarah Jane**
**Kilburn & Strode LLP**
**20 Red Lion Street**
**London WC1R 4PJ (GB)**

(56) References cited:

**WO-A1-99/18238** **WO-A2-02/18575**
**WO-A2-03/031934** **US-A1- 2004 137 539**

- **D. FRASCA ET AL: 'Mechanisms for Decreased Function of B Cells in Aged Mice and Humans' THE JOURNAL OF IMMUNOLOGY vol. 180, no. 5, 21 February 2008, pages 2741 - 2746, XP055187346 DOI: 10.4049/jimmunol.180.5.2741 ISSN: 0022-1767**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 297 347 B1

Printed by Jouve, 75001 PARIS (FR)

## Description

### Background of the Invention

Germinal centers are unique substructures of follicles within secondary lymphoid organs (*e.g.*, spleen, lymph node, gut-associated lymphoid tissue, *etc.*) and chronically inflamed tissues (*e.g.*, rheumatoid synovium). Functional activity within germinal centers is important for protective immunity of mammalian species. Biologic activities driven by germinal centers that are important for generating efficient antibody responses include clonal expansion of B lymphocytes, immunoglobulin isotype class switching and somatic mutation of immunoglobulin genes.

The germinal centers of secondary lymphoid organs generate high affinity antibody responses to antigens and provide protective immunity against pathogens. Germinal centers also generate autoantibodies that drive the pathogenesis of some autoimmune disease. Many immunosuppressive drugs decrease the activity of germinal centers (*i.e.*, cause atrophy) and inhibit antibody responses. These drugs thus could have efficacy for treating autoimmune disease and/or predispose subjects to infection, depending on the magnitude of immunosuppression. Conversely, stimulation of the immune system, for example, by vaccination, increases the activity of germinal centers and generates protective antibody responses. The convention for assessing the activity of germinal centers is assessment of their morphology in tissue sections from secondary lymphoid organs, by an anatomic pathologist. This procedure, which requires obtaining tissue from secondary lymphoid organs, is an invasive procedure that causes discomfort and risk to the patient. An alternative is a human vaccination study, within which subjects are immunized with a specific antigen and the antibody response to this antigen is monitored via serum samples. The major risk of this approach is the stimulation of a subject's immune system, which can cause adverse events ranging from minor (*i.e.*, fever and malaise) to severe (*i.e.*, death). A safer, less tedious and less invasive methodology of assessing immune competence is preferred by clinicians. A routine, noninvasive test for germinal center activity can identify patients at risk for developing adverse conditions and prompt prophylactic measures that prevent the condition, to avoid the morbidity and cost associated with treating the condition.

### Summary of the Invention

The invention relates to assessment of immunocompetence of the adaptive immune system by noninvasive measurement of follicular germinal center activity. The activity of germinal centers correlates positively with antibody responses to antigens in vaccination studies. The activity of germinal centers correlates inversely with adverse events caused by therapeutic agents. For example, splenic germinal center atrophy can lead to increased susceptibility to pathogens, *e.g.*, bacterial infection or cancer, *e.g.*, lymphoma. In another example, germinal center hyperplasia can indicate efficacy of vaccination to enhance immunity.

The present invention is as defined in the claims. In one aspect, the invention provides kits and the use of a probe useful in determination of germinal center activity. In another aspect, the invention provides methods for determining germinal center activity. In these aspects, biomarkers in patient samples are measured. In one embodiment, the activity is increased (*i.e.*, due to hyperplasia). In this embodiment an increase in the level of biomarker can be measured. In another embodiment, the activity is decreased (*i.e.*, due to atrophy). In this embodiment, a decrease in the level of biomarker can be measured.

In some embodiments of the disclosure, the biomarkers allow measurement of therapeutic activity of agents administered to treat pathogenic conditions and disease.

In other embodiments of the disclosure, the biomarkers allow measurement of the efficacy of vaccination, *e.g.*, to prevent disease.

In other embodiments of the disclosure, the biomarkers are predictive of drug-induced splenic germinal center atrophy.

In other embodiments of the disclosure, patient risk for detrimental immunosuppression can be monitored by analysis of differences or trends in germinal center activity over time. In another embodiment, the biomarkers allow the selection of treatment regimen for a patient.

In further embodiments, the disclosure relates to stimulation of germinal center activity and thus gain of immunity for protection from pathogenic disorders, *e.g.*, after vaccination.

Biomarkers preferably are measured in peripheral blood samples. Preferred biomarkers of the disclosure include germline transcript mu (GLT-$\mu$), activation-induced cytidine deaminase (AID), circular transcripts containing gamma 1 and 2 (CT-$\gamma$1&2), immunoglobulin heavy chain locus G1 isotype (IGHG1) and/or immunoglobulin heavy chain locus A1 isotype (IGHA1).

Preferred methods analyze nucleic acid transcripts of the biomarkers.

Other features and advantages of the invention will be apparent from the following detailed description, and from the claims.

**Brief Description of the Drawings**

Figure 1. Effect of ML120B on switch transcripts in mouse spleens after a single dose.

Figure 2. Effect of ML120B on switch transcripts in mouse spleens after a 6 hour treatment.

Figure 3. Effect of ML120B on switch transcripts in mouse spleens for 4 days of treatments.

Figure 4. Histopathology of secondary lymphoid organs (spleen, mandibular lymph node (Mand. LN), popliteal lymph node (pop. LN) and mesenteric lymph node (Mes. LN)) of cynomolgous monkeys in study using Test Agent A.

Figure 5. Frequency of IgG+ germinal centers in spleens of cynomolgous monkeys in study using Test Agent A.

Figures 6A-C. Flow cytometry of peripheral blood from cynomolgous monkeys in study using Test Agent A. 6A, Total B cells; 6B, percent change in number of post-germinal center B cells; 6C, percent change in number of pre-germinal center B cells.

Figures 7A-D. Fold change of GLT-μ (ST1) transcript in blood over time in cynomolgous monkey study using Test Agent A (TA). Figure 7A, vehicle-treated animals; Figure 7B, 40 mg/kg-treated animals; Figure 7C, 60 mg/kg-treated animals; Figure 7D, 100 mg/kg-treated animals.

Figures 8A-B. Measurement of ICS transcripts in blood from normal human volunteers. Figure 8A, level of GLT-μ; Figure 8B, level of circle transcripts CT-γ1&2.

Figure 9. Measurement of ICS transcripts (circle transcripts CT-γ1&2) in human blood samples, unmodified or modified to remove non-B cell populations.

Figure 10. Comparison of timecourse of treatment of cynomolgous monkeys with Test Agent A with timecourse of contracting bacterial infection.

Figures 11A-B. Fold change of transcripts in peripheral blood of cynomolgous monkeys during timecourse of study using Test Agent A. Figure 11A, animal No. 1005 (vehicle control); Figure 11B, animal No. 4005 (80 mg/kg Test Agent A for 13 weeks from Day 0).

Figures 12A-H. Correlation of fold change from baseline of level of transcripts with histological assessment of reactivity of germinal centers in study of using Test Agent A in cynomolgous monkeys. Figure 12A, correlation of measurement of GLT-μ; Figure 12B, correlation of measurement of CT-γ1&2; Figure 12C, correlation of measurement of AID; Figure 12D, correlation of measurement of 18S RNA; Figure 12E, correlation of measurement of IGHG1; Figure 12F, correlation of measurement of IGHA1; Figure 12G, correlation of measurement of IGL-κ; Figure 12H, correlation of measurement of IGL-λ.

**Detailed Description of the Invention**

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, preferred methods and materials are described herein.

The articles "a" and "an" are used herein to refer to one or to more than one (*i.e.* at least one) of the grammatical object of the article. By way of example, "an element" means at least one element and can include more than one element.

As used herein, the term "noninvasive" refers to a procedure which inflicts minimal harm to a subject. In the case of clinical applications, a noninvasive sampling procedure can be performed quickly, e.g., in a walk-in setting, typically without anaesthesia and/or without surgical implements or suturing. Examples of noninvasive samples include blood, serum, saliva, urine, buccal swabs, throat cultures, stool samples and cervical smears. Noninvasive diagnostic analyses include x-rays, magnetic resonance imaging.

The term "immunosuppressive agent", as used herein, refers to compounds which can inhibit an immune response.

A "marker" or "biomarker" is a gene whose altered level of expression in a tissue or cell from its expression level in untreated tissue or cell is associated with an affected or altered state of immunity, including disease states, such as immunosuppression, or lymphoproliferative disorder, or protective immunity. A "marker nucleic acid" is a nucleic acid (*e.g.*, mRNA, cDNA) encoded by or corresponding to a marker or biomarker of the invention. Such marker nucleic acids include DNA (*e.g.*, cDNA) comprising the entire or a partial sequence of any of the biomarkers or the complement of such a sequence. The marker nucleic acids also include RNA comprising the entire or a partial sequence of any biomarkers or the complement of such a sequence, wherein all thymidine residues are replaced with uridine residues, sense and anti-sense strands of genomic DNA (*i.e.* including any introns occurring therein), RNA generated by transcription of genomic DNA (*i.e.* prior to splicing), RNA generated by splicing of RNA transcribed from genomic DNA, and proteins generated by translation of spliced RNA (*i.e.* including proteins both before and after cleavage of normally cleaved regions such as transmembrane signal sequences). As used herein, a "marker" may also include a cDNA made by reverse transcription of an RNA generated by transcription of genomic DNA (including spliced RNA). A "marker protein" is a protein encoded by or corresponding to a marker of the invention. The terms "protein" and "polypeptide' are used interchangeably.

The term "probe" refers to any molecule which is capable of selectively binding to a specifically intended target molecule, for example a marker of the invention. Probes can be either synthesized by one skilled in the art, or derived from appropriate biological preparations. For purposes of detection of the target molecule, probes may be specifically designed to be labeled, as described herein. Examples of molecules that can be utilized as probes include, but are not limited to, RNA, DNA, proteins, antibodies, and organic molecules.

The "normal" level of expression of a marker is the level of expression of the marker in cells in a similar environment or response situation, in a subject with unaltered immunity, including one not afflicted with a disease state, such as immunosuppression, autoimmunity or a lymphoproliferative disorder or an unvaccinated subject, or a patient prior to treatment with the test agent. A normal level of expression of a marker may also refer to the level of expression of a "control sample", (*e.g.*, sample from a healthy subject not having the marker-associated disease state), preferably, the average expression level of the marker in several control samples. A control sample may be comprised of a control database. Alternatively, a "normal" level of expression of a marker is the level of expression of the marker in non-disease cells in a similar environment or response situation from the patient.

"Over-expression" and "under-expression" of a marker refer to expression of the marker of a patient at a greater or lesser level, respectively, than normal level of expression of the marker (*e.g.* more than one and a half-fold, at least two-fold, at least three-fold, greater or lesser level etc.) in a test sample that is greater than the standard error of the assay employed to assess expression. A "significant" expression level may refer to level which either meets or is above or below a pre-determined score for a biomarker set as determined by methods provided herein.

A "transcribed polynucleotide" or "nucleotide transcript" or "transcript" refers to a polynucleotide (*e.g.* an mRNA, hnRNA, a cDNA, or an analog of such RNA or cDNA) which is complementary to or homologous with all or a portion of a mature mRNA made by transcription of a marker of the invention and normal post-transcriptional processing (*e.g.* splicing), if any, of the RNA transcript, and reverse transcription of the RNA transcript.

"Complementary" refers to the broad concept of sequence complementarity between regions of two nucleic acid strands or between two regions of the same nucleic acid strand. It is known that an adenine residue of a first nucleic acid region is capable of forming specific hydrogen bonds ("base pairing") with a residue of a second nucleic acid region which is antiparallel to the first region if the residue is thymine or uracil. Similarly, it is known that a cytosine residue of a first nucleic acid strand is capable of base pairing with a residue of a second nucleic acid strand which is antiparallel to the first strand if the residue is guanine. A first region of a nucleic acid is complementary to a second region of the same or a different nucleic acid if, when the two regions are arranged in an antiparallel fashion, at least one nucleotide residue of the first region is capable of base pairing with a residue of the second region. Preferably, the first region comprises a first portion and the second region comprises a second portion, whereby, when the first and second portions are arranged in an antiparallel fashion, at least about 50%, and preferably at least about 75%, at least about 90%, or at least about 95% of the nucleotide residues of the first portion are capable of base pairing with nucleotide residues in the second portion. More preferably, all nucleotide residues of the first portion are capable of base pairing with nucleotide residues in the second portion.

"Homologous" as used herein, refers to nucleotide sequence similarity between two regions of the same nucleic acid strand or between regions of two different nucleic acid strands. When a nucleotide residue position in both regions is occupied by the same nucleotide residue, then the regions are homologous at that position. A first region is homologous to a second region if at least one nucleotide residue position of each region is occupied by the same residue. Homology between two regions is expressed in terms of the proportion of nucleotide residue positions of the two regions that are occupied by the same nucleotide residue. By way of example, a region having the nucleotide sequence 5'-ATTGCC-3' and a region having the nucleotide sequence 5'-TATGGC-3' share 50% homology. Preferably, the first region comprises a first portion and the second region comprises a second portion, whereby, at least about 50%, and preferably at least about 75%, at least about 90%, or at least about 95% of the nucleotide residue positions of each of the portions are occupied by the same nucleotide residue. More preferably, all nucleotide residue positions of each of the portions are occupied by the same nucleotide residue.

As used herein, the term "agent" is defined broadly as anything that a patient or isolated cells may be exposed to in a therapeutic or *in vitro* protocol. In the context of the present invention, such agents include, but are not limited to, immumodulatory agents, as well as chemotherapeutic agents as described in further detail herein.

Unless otherwise specified herewithin, the terms "antibody" and "antibodies" broadly encompass naturally-occurring forms of antibodies (*e.g.*, IgG, IgA, IgM, IgE) and recombinant antibodies such as single-chain antibodies, chimeric and humanized antibodies and multi-specific antibodies, as well as fragments and derivatives of all of the foregoing, which fragments and derivatives have at least an antigenic binding site. Antibody derivatives may comprise a protein or chemical moiety conjugated to an antibody.

A kit is any article of manufacture (*e.g.* a package or container) comprising at least one reagent, *e.g.* a probe, for specifically detecting a marker or marker set of the invention. The article of manufacture may be promoted, distributed, or sold as a unit for performing the methods of the present invention. The reagents included in such a kit comprise nucleic acid probes/primers and/or antibodies for use in biomarker expression. In addition, the kits of the present invention may preferably contain instructions which describe a suitable detection assay. Such kits can be conveniently used, *e.g.*, in clinical settings, to diagnose and evaluate patients at risk of exhibiting symptoms of a disease state, such as immunosuppression or a lymphoproliferative disorder, in particular patients exhibiting the possible presence of germinal center atrophy after treatment with an immunomodulator, including, e.g., patients being treated for chronic disease.

Described herein is the assessment of immunocompetence through measurement of the activity of germinal centers. Also described are assessing such capacity by noninvasive, convenient or low-cost means, for example, in blood samples. Typical methods to determine germinal center activity employ tissue sections (which typically are prepared from a biopsy of a tissue, an invasive procedure involving collecting tissue for morphological analysis; not practiced for many autoimmune indications), or cumbersome and inconvenient vaccination tests, e.g., T cell-dependent antibody responses (TDAR), or antigen exposure tests, e.g. a test for delayed-type hypersensitivity (e.g., the tuberculin test). The invention provides methods for determining, assessing, advising or providing an appropriate therapy regimen for treating a chronic disease in a patient. Monitoring a treatment using the kits and methods disclosed herein can identify the potential for adverse events and allow their prevention, and thus a savings in morbidity, mortality and treatment costs through adjustment in the therapeutic regimen, cessation of therapy or use of alternative therapy. The invention provides probes, kits and methods for measuring the activity of germinal centers and could be widely utilized as a noninvasive means of assessing germinal center activity and the immunocompetence of the adaptive immune system.

The kits, methods and use of a probe of the invention have the following uses, among others:

a) assessing the status of adaptive immunity in a human patient;
b) assessing the degree of immunosuppression in a human patient;
c) assessing the degree of immunoactivation in a patient;
d) assessing the immumodulatory potential of a test agent;
e) determining whether adaptive immunity has recovered after removal of immunomodulatory agent;
f) predicting the clinical outcome of a patient in disorders associated with changes in immunoglobulin isotype class switching;
g) assessing whether a patient is afflicted with a disease that perturbs immunoglobulin isotype class switching, such as the immunodefficiencies with hyper-IgM: HIGM1, HIGM 2, HIGM 3, HIGM 4 & HIGM 5 (*e.g.*, Common variable immunodeficiency (CVID) and Immunoglobulin-A deficiency);
h) assessing the histological type of neoplasm;
i) assessing effectiveness of treatment against Diffuse Large B-Cell Lymphoma;
j) assessing the presence of differentiating or mature B cells;
k) predicting whether a patient is at risk for contracting an infection;
l) predicting whether a patient is at risk for contracting lymphoma or leukemia;
m) assessing the efficacy of a immunomodulatory therapy in a patient;
n) monitoring the efficacy of a vaccination;
o) selecting a composition or therapy for treating chronic disease in a patient;
p) assessing the immunomodulatory potential of a test compound;
q) preventing the onset of opportunistic infection in a patient; and
r) assessing the immunomodulatory potential of an environmental toxin.

Immature lymphocytes, *e.g.*, immature B cells from blood, typically enter a secondary lymphoid organ, *e.g.*, spleen, lymph node or Peyer's patch, (and sometimes additional sites of chronic inflammation, *e.g.*, synovium if there is joint inflammation or mucosa if there is respiratory inflammation) where, upon direction by T cells, which had been stimulated by antigen and are secreting instructional cytokines, they undergo clonal expansion, differentiation, and affinity maturation and commence production of antibodies.

Clonal expansion is the proliferation of antigen-activated B cells and is a mechanism for increasing the magnitude of an antibody response. It can be monitored by measuring markers of proliferating cells and/or the number of B cells within samples. The expression of CD 19 and CD20 is unique to B lymphocytes and their expression does not appear to be regulated by IKKβ and NF-κB activity. Therefore, biomarkers for splenic germinal center atrophy can be CD19 or CD20, whose levels in peripheral blood can be measured to determine the level of B lymphocytes.

Immunoglobulin isotype class switching (ICS) is a differentiation activity whereby IgM, the isotype of the primary antibody response (i.e., what a B cell would produce without any differentiation instructions from a T cell), is switched to immunoglobulins type G, A or E (IgG, IgA, or IgE, respectively) during the secondary antibody response. Switching the isotype of an antibody response brings additional, alternative effector functions of the immune system to bear in combating a pathogen. Immunoglobulin ICS therefore can be monitored by measuring IgG, IgA, and IgE levels (Snapper et al. (1997) Immunity 6:217-23, Chaudhuri and Alt (2004) Nat. Rev. Immunol. 4:541-52, 655). Immunoglobulin ICS can be monitored by measuring IgG, IgA, and IgE levels in peripheral blood of laboratory animals housed in aseptic environments. These techniques are not effective in animals or subjects not housed in aseptic environments, as their immune systems have been stimulated previously and consequently harbor large reservoirs of Ig, precluding detection of the relatively small changes related specifically to germinal center activity. Due to the large pre-existing pool of immunoglobulins (Igs) that exhibit relatively long half-lives, it can be important to wait for a decrease in the level of pre-existing Igs before monitoring changes in production *de novo.* The half-life of serum IgG, for example, is 27 days.

Germinal center activity preferably is measured by quantifying evidence of differentiation and/or proliferation of B cells. In certain aspects, determining or confirming a value for a parameter related to a patient's germinal center activity comprises detection of mRNA. Such detection can be carried out by any relevant method, including e.g., PCR, northern, nucleotide array detection, *in vivo* imaging using probes capable of detection of the appropriate nucleic acid. In other aspects, determining a value for a parameter related to the expression of a biomarker in a sample comprises detection of protein. Such detection can be carried out using any relevant method for protein detection, including e.g., ELISA, western blot, immunoassay, protein array detection, *in vivo* imaging using probes capable of detection of the appropriate peptide. A standard way of measuring immunocompetence is a vaccination study, also referred to as the T cell-dependent antibody response (TDAR) in nonclinical investigations. In the TDAR test, the ability of the subject to mount a response to a new antigen is measured. It is performed by immunizing the subject with a foreign substance, e.g., keyhole limpet hemocyanin, and determining the amount of recall upon reintroduction. Vaccination studies in clinical trials or during routine therapeutic treatment are laborious, inconvenient, costly and can present risk of adverse reactions in subjects. Another way of testing is to identify the composition of B cells in peripheral blood by Flow Cytometry. However, Flow Cytometry cannot be utilized to assay samples at some clinical sites, worldwide (*i.e.*, Ph III trials, ambulatory clinics), as it uses expensive machinery and requires trained operators.

Assays that monitor transcription, *e.g.*, measure Ig transcripts, are preferable for early detection of atrophy because a) transcription is terminated prior to translation, b) Ig transcripts are less stable than proteins (they have shorter half-lives), and c) several meaningful transcripts, such as switch transcripts, are not translated into proteins. Switch transcripts are intermediary products unique to ICS that present a potential biomarker for splenic germinal center atrophy (Snapper et al. (1997) Immunity 6:217-23, Chaudhuri and Alt (2004) Nat. Rev. Immunol. 4:541-52; *erratum* pg. 655). Germline switch transcripts, *e.g.*, germline transcript mu (GLT-$\mu$) are generated immediately prior to switch recombination, when chromatin decondenses around an Ig gene locus, the substrate for the deoxyribonucleic acid (DNA) recombination reaction that characterizes ICS (Snapper *et al.*, *supra*, Chaudhuri and Alt, *supra*). Circle transcripts, *e.g.*, those containing gamma 1 and 2 (CT-$\gamma$1 and CT-$\gamma$2) are another type of switch transcript and are produced later in the ICS process. They are transcripts from the excised genomic DNA that are essentially the byproducts of DNA recombination during ICS (Snapper *et al.*, *supra*, Chaudhuri and Alt *supra*). Measuring levels of these transcripts permits identification of which stage of DNA recombination is occurring. Additional genes are required for DNA recombination during human ICS, such as activation-induced cytidine deaminase (AID, AICDA; Revy et al. (2000) Cell 102:565-75, Imai et al. (2003) Nature Immunol. 4:1023-28), uracil-DNA glycosylase (UNG, Imai *et al.*, *supra*, severe-combined immunodeficiency (SCID) gene product (Rolink et al. (1996) Immunity 5:319-30), Ku heterodimer (ku70/ku86, Manis et al. (1998) J. Exp. Med. 187:2081-9), or ku80 (Casellas et al. (1998) EMBO J. 17:2404-11) expression or nuclear localization or activity of DNA-dependent serine/threonine protein kinase (DNA-PK; p350) (Snapper *et al.*, *supra*; Zelazowski et al. (1997) J. Immunol. 159:2559-62) or recombination activating gene 1 (RAG1, Girschick et al. (2001) J. Immunol. 166:377-86).

Homeostasis entails the migration of B lymphocytes that participated in a germinal center reaction from secondary lymphoid organs to other organs (*e.g.*, the bone marrow) via the vasculature. Expression of AID is exclusively restricted to B lymphocytes that have recently participated in a germinal center reaction. Mutations in the AID gene prevent ICS and are characterized by the presence of germline, but not circle, transcripts, by hyperplastic (*i.e.*, giant) germinal centers, and by profound immunodeficiency (*e.g.*, hyper-immunoglobulin M types 2 and 5 (HIGM2 and HIGM5)). Both humans and mice express AID and germline switch transcripts indicative of early ICS activity, but not the circle transcripts resulting from DNA recombination indicative of late ICS activity (Revy *et al.*, *supra*, Imai *et al.*, *supra*). Measuring levels of switch transcripts and AICDA expression therefore provides information on what stage of ICS is affected, illustrating their potential utility in monitoring atrophy of germinal centers and immunosuppression in response to a test agent. Human immunodeficiences with hyper-immuglobulin (Ig) M, types 1 and 3 (HIGM1 and HIGM3, which abolish NF-$\kappa$B signaling in B cells, also are characterized by hypoplastic germinal centers, immune deficiency and recurrent bacterial infections (Ramesh et al. (1999) Primary Immunodeficiency Diseases: A Molecular and Genetic Approach, Oxford University Press, New York, pp. 233-49, Castigli et al. (1994) Proc. Natl. Acad. Sci. USA 91:12135-9, Ferrari et al. (2001) Proc. Natl. Acad. Sci. USA 98:12614-9). Expression of AID is unique to B lymphocytes, whereas UNG is ubiquitously expressed. Moreover, these switch transcripts exist in peripheral blood because homeostasis involves migration of memory B cells and plasmablasts from secondary lymphoid organs to the bone marrow via the vasculature (Kunkel and Butcher (2003) Nat. Rev. Immunol. 3: 882-9), Frasca et al., (2008), J. Immunology, 180, 2741-2746, describes the use of RNA markers for detecting the function of B Cells.

Alternatively, affinity maturation could be used as a biomarker for splenic germinal center atrophy by measuring somatic mutation of IgS. However, this is a labor-intensive and relatively expensive methodology.

Exemplary biomarkers to measure include markers of germline activation, including germline switch transcripts containing immunoglobulin chains, e.g., mu, delta, gamma, alpha or epsilon heavy chains (*e.g.*, germline transcript mu (GLT-$\mu$) *e.g.*, SEQ ID NO:1, in the region of bases 961381 to 967501 of GenBank Accession No. NG_001019 (Jan 10, 2006 version), or other sequences at the immunoglobulin heavy (IGH) locus on chromosome 14); circle switch transcripts, containing immunoglobulin chains, *e.g.*, mu, delta, gamma, alpha or epsilon heavy chains (*e.g.*, those containing gamma 1 and 2 (CT-$\gamma$1 and CT-$\gamma$2, *e.g.*, SEQ ID NO:2, in the region of bases 967201 to 1048261 of NG_001019 (Jan. 10, 2006 version)) or other sequences at the immunoglobulin heavy (IGH) locus on chromosome 14); circular DNA fragments containing mu, delta, gamma, alpha or epsilon heavy chains; and/or DNA recombination effectors (*e.g.*, activation-induced cytidine deaminase, AID or AIDCA, GenBank Accession No. NM_020661, SEQ ID NOs:3,4; UNG, GenBank Accession No. NM_080911, SEQ ID NOs:5,6; Ku70, GenBank Accession No. NM_001469, SEQ ID NOs:7,8; Ku80, GenBank Accession No. NM_021141, SEQ ID NOs:9,10; or RAG1, NM_000448, SEQ ID NOs:11,12) or light chains (e.g., immunoglobulin light chain kappa (IgL-$\kappa$) GenBank Accession No. BC070336, SEQ ID NOs:13,14; IgL-$\lambda$, BC012159, SEQ ID NOs:15,16). Preferable biomarkers have expression limited to germinal center B cells. Preferred biomarkers to detect as disclosed herein include GLT-$\mu$, AICDA (AID), CT-$\gamma$1&2, IGHG1, *e.g.*, in the region of bases 1080134-1081837 of NG_001019, *e.g.*, NC_000014, SEQ ID NO:20; IGHG2; IGHG3; IGHG4; IGHA1, *e.g.*, in the region of bases 1114540-1116066 of GenBank Accession No. NG_001019, SEQ ID NO:21; IGHA2 and/or IGHE. (The Entrez Gene database (National Center for Biotechnology Information, Bethesda, MD), as well as the supporting information provided in NG_001019, defines the regions for each biomarker not described herein by SEQ ID NO. One of skill in the art can review the database information and readily obtain the regions for the other biomarkers, *e.g.*, IGHG2, IGHG3, IGHG4, IGHA2, IGHE.) Most preferred biomarkers include GLT-$\mu$, AICDA (AID), CT-$\gamma$1&2, IGHG1 and/or IGHA2.

The choice of biomarker can be adjusted according to the pathway of action of the immumodulator. For example, activation of the tumor necrosis factor-alpha (TNF-$\alpha$) and NF-$\kappa$B pathways results in regulation of expression of genes which can be used as markers in this method. Levels of TNF-$\alpha$ and Interleukin-1 Beta (IL-1$\beta$) could be measured, since their expression is regulated by IKK$\beta$ and NF-$\kappa$B and, as a positive control for activity of such an immunomodulator, represent a pharmacodynamic (mechanistic) marker for its activity. However, these transcripts have a ubiquitous expression pattern, meaning they are expressed in many types of blood cells. Expression of IgL-$\kappa$, which also is regulated by NF-$\kappa$B, also can be measured as a potential mechanistic marker for activity of the immunomodulators which affect tumor necrosis factor-alpha (TNF-$\alpha$) or NF-$\kappa$B pathways. However, unlike TNF-$\alpha$ AND IL-1$\beta$, it is target cell-specific, in that it is exclusively expressed by B lymphocytes, so measuring IGL-$\kappa$ levels can allow monitoring of activity of such an immunomodulator specifically in the B cell compartment. Accordingly, if the immunomodulator has a mechanism of action that affects the TNF$\alpha$ or the NF-$\kappa$B pathway, a preferred biomarker is IGL-$\kappa$.

The term "biological sample" is intended to include tissues, cells, biological fluids and isolates thereof, isolated from a subject, as well as tissues, cells and fluids present within a subject. Based on general histological information on the frequency of various types of cells in the blood, post-germinal center B cells (e.g., plasmablasts), as a small subset of B cells, are a very low percentage of the cell population in whole blood. Surprisingly, transcripts representative of ICS are detectable in peripheral blood samples. Thus, preferable noninvasive samples, *e.g.*, for *in vitro* measurement of adaptive immunity, include peripheral blood samples. Accordingly, cells within peripheral blood can be tested for ICS. Blood collection containers preferably comprise an anti-coagulant, *e.g.*, heparin or ethylene-diaminetetraacetic acid (EDTA), sodium citrate or citrate solutions with additives to preserve blood integrity, such as dextrose or albumin or buffers, *e.g.*, phosphate. If the amount of biomarker is being measured by measuring the level of its RNA in the sample, an RNA stabilizer, *e.g.*, an agent that inhibits RNAse, can be added to the sample. If the amount of biomarker is being measured by measuring the level of its protein in the sample, protein stabilizer, *e.g.*, an agent that inhibits proteases, can be added to the sample. An example of a blood collection container is PAXGENE® tubes (PREANALYTIX, Valencia, CA), useful for RNA stabilization upon blood collection. Peripheral blood samples can be modified, *e.g.*, fractionated, sorted or concentrated (*e.g.*, to result in samples enriched with antibody-producing B cells). Examples of modified samples include plasmablasts, which can be collected by *e.g.*, negative selection, *e.g.*, separation of white blood cells from red blood cells (*e.g.*, differential centrifugation through a dense sugar or polymer solution (*e.g.*, FICOLL® solution (Amersham Biosciences division of GE healthcare, Piscataway, NJ) or HISTOPAQUE®-1077 solution, Sigma-Aldrich Biotechnology LP and Sigma-Aldrich Co., St. Louis, MO)) and/or positive selection by binding B cells to a selection agent (*e.g.*, a reagent which binds to a B cell marker, such as CD19, CD38, CD138, or CD30, for direct isolation (*e.g.*, the application of a magnetic field to solutions of cells comprising magnetic beads (*e.g.*, from Miltenyi Biotec, Auburn, CA) which bind to the B cell markers) or fluorescent-activated cell sorting). Alternatively, a B cell line, *e.g.*, B-cell lymphoma (*e.g.*, BC-3) can be assayed. A skilled artisan readily can select and obtain the appropriate cells (*e.g.*, from American Type Culture Collection (ATCC®), Manassas, VA) that are used in the present method. A sample modified to select for B cells can be useful to measure markers, *e.g.*, Ku70, RAG1, *etc.*, whose expression is a hallmark of ICS, but is not limited to germinal center B cells. If the compositions or methods are being used to predict capacity for adaptive immunity in a patient or monitor the effectiveness of a therapeutic protocol, then a tissue or blood sample from the patient being treated is a preferred source.

The sample, *e.g.*, blood or modified blood, can be subjected to a variety of well-known post-collection preparative and storage techniques (*e.g.*, nucleic acid and/or protein extraction, fixation, storage, freezing, ultrafiltration, concentration, evaporation, centrifugation, *etc.*) prior to assessing the amount of the marker in the sample.

In a particular embodiment, the level of mRNA corresponding to the marker can be determined both by *in situ* and by *in vitro* formats in a biological sample using methods known in the art. Many expression detection methods use isolated RNA. For *in vitro* methods, any RNA isolation technique that does not select against the isolation of mRNA can be utilized for the purification of RNA from tumor cells (see, *e.g.*, Ausubel et al., ed., Current Protocols in Molecular Biology, John Wiley & Sons, New York 1987-1999). Additionally, large numbers of tissue samples can readily be processed using techniques well known to those of skill in the art, such as, for example, the single-step RNA isolation process of Chomczynski (1989, U.S. Patent No. 4,843,155). RNA can be isolated using standard procedures (see *e.g.,* Chomczynski and Sacchi (1987) Anal. Biochem. 162:156-159), solutions (*e.g.*, trizol, TRI REAGENT® (Molecular Research Center, Inc., Cincinnati, OH; see U.S. Patent No. 5,346,994) or kits (*e.g.*, a QIAGEN® Group RNEASY® isolation kit (Valencia, CA) or LEUKOLOCK™ Total RNA Isolation System, Ambion division of Applied Biosystems, Austin, TX).

Additional steps may be employed to remove DNA. Cell lysis can be accomplished with a nonionic detergent, followed by microcentrifugation to remove the nuclei and hence the bulk of the cellular DNA. In one embodiment, RNA is extracted from cells of the various types of interest using guanidinium thiocyanate lysis followed by CsCl centrifugation to separate the RNA from DNA (Chirgwin et al. (1979) Biochemistry 18:5294-99). Poly(A)+RNA is selected by selection with oligo-dT cellulose (see Sambrook et al. (1989) Molecular Cloning--A Laboratory Manual (2nd ed.), Cold Spring Harbor Laboratory, Cold Spring Harbor, N.Y.). Alternatively, separation of RNA from DNA can be accomplished by organic extraction, for example, with hot phenol or phenol/chloroform/isoamyl alcohol. If desired, RNAse inhibitors may be added to the lysis buffer. Likewise, for certain cell types, it may be desirable to add a protein denaturation/digestion step to the protocol. For many applications, it is desirable to preferentially enrich mRNA with respect to other cellular RNAs, such as transfer RNA (tRNA) and ribosomal RNA (rRNA). Most mRNAs contain a poly(A) tail at their 3' end. This allows them to be enriched by affinity chromatography, for example, using oligo(dT) or poly(U) coupled to a solid support, such as cellulose or SEPHADEX.R™. medium (see Ausubel et al. (1994) Current Protocols In Molecular Biology, vol. 2, Current Protocols Publishing, New York). Once bound, poly(A)+mRNA is eluted from the affinity column using 2 mM EDTA/0.1% SDS.

The sample of RNA can comprise a plurality of different RNA molecules, each different RNA molecule having a different nucleotide sequence. In a specific embodiment, the RNA molecules in the RNA sample comprise at least 100 different nucleotide sequences. More preferably, the RNA molecules of the RNA sample comprise RNA molecules corresponding to each of the marker genes.

The level of germinal center activity, *e.g.*, the extent of isotype class switching or clonal expansion of B lymphocytes, can be measured using any suitable assay, for example, directly or indirectly. Expression of a marker of the invention may be assessed by any of a wide variety of well known methods for detecting expression of a transcribed nucleic acid and/or translated protein. Non-limiting examples of such methods include immunological methods for detection of secreted, cell-surface, cytoplasmic, or nuclear proteins, protein purification methods, protein function or activity assays, nucleic acid hybridization methods, nucleic acid reverse transcription methods, and nucleic acid amplification methods. These methods, include gene array/chip technology, RT-PCR, *in situ* hybridization, immunohistochemistry, immunoblotting, FISH (fluorescence *in situ* hybridization), FACS analyses, northern blot, southern blot or cytogenetic analyses. The detection methods of the invention can thus be used to detect RNA, mRNA, protein, cDNA, or genomic DNA, for example, in a biological sample *in vitro* as well as *in vivo.* Furthermore, *in vivo* techniques for detection of a polypeptide or nucleic acid corresponding to a marker of the invention include introducing into a subject a labeled probe to detect the biomarker, *e.g.*, a nucleic acid complementary to the transcript of a biomarker or a labeled antibody, Fc receptor or antigen directed against the polypeptide, *e.g.*, immunoglobulin or DNA recombination effector. For example, the antibody can be labeled with a radioactive marker whose presence and location in a subject can be detected by standard imaging techniques. These assays can be conducted in a variety of ways. A skilled artisan can select from these or other appropriate and available methods based on the nature of the marker(s), tissue sample and isotype in question. Some methods are described in more detail in later sections. Different methods or combinations of methods could be appropriate in different cases or, for instance in different chronic diseases or patient populations.

An exemplary method for detecting the presence or absence of nucleic acid corresponding to a biomarker of the invention in a biological sample involves obtaining a biological sample (*e.g.*, a blood sample) from a test subject and contacting the biological sample with a compound or an agent capable of detecting the nucleic acid (*e.g.*, RNA, mRNA, genomic DNA, or cDNA). For example, *in vitro* techniques for detection of mRNA include PCR, northern hybridizations, *in situ* hybridizations, nucleotide array detection, and TAQMAN® gene expression assays (Applied Biosystems, Foster City, CA), preferably under GLP approved laboratory conditions. *In vitro* techniques for detection of genomic DNA include Southern hybridizations.

In one embodiment, expression of a marker is assessed by preparing mRNA/cDNA (*i.e.*, a transcribed polynucleotide) from cells in a patient sample, and by hybridizing the mRNA/cDNA with a reference polynucleotide which is a complement of a marker nucleic acid, or a fragment thereof. cDNA can, optionally, be amplified using any of a variety of polymerase chain reaction methods prior to hybridization with the reference polynucleotide; preferably, it is not amplified. Expression of one or more markers likewise can be detected using quantitative PCR to assess the level of expression of the marker(s). Alternatively, any of the many known methods of detecting mutations or variants (*e.g.* single nucleotide polymorphisms, deletions, etc.) of a marker of the invention may be used to detect occurrence of a marker in a patient. For example, measurement of mutated forms of AICDA (AID) can indicate the level of immunocompetence (Revy et al. (2000) Cell 102:565-75).

*In vitro* techniques for detection of a polypeptide corresponding to a marker of the invention include enzyme linked immunosorbent assays (ELISAs), Western blots, protein array, immunoprecipitations and immunofluorescence. In such examples, expression of a marker is assessed using an antibody (*e.g.*, a radio-labeled, chromophore-labeled, fluorophore-labeled, or enzyme-labeled antibody), an antibody derivative (*e.g.*, an antibody conjugated with a substrate or with the protein or ligand of a protein-ligand pair (*e.g.*, biotin-streptavidin)), or an antibody fragment (*e.g.*, a single-chain antibody, an isolated antibody hypervariable domain, *etc*.) which binds specifically with a marker protein or fragment thereof, including a marker protein which has undergone all or a portion of its normal post-translational modification.

An example of direct measurement is quantification of transcripts. As used herein, the level or amount of expression refers to the absolute level of expression of an mRNA encoded by the marker or the absolute level of expression of the protein encoded by the marker. As an alternative to making determinations based on the absolute expression level of selected markers, determinations may be based on normalized expression levels. Expression levels are normalized by correcting the absolute expression level of a biomarker upon comparing its expression to the expression of a control marker that is not a biomarker, *e.g.*, in a housekeeping role that is constitutively expressed. Suitable markers for normalization also include housekeeping genes, such as the actin gene or beta-2 microglobulin. Reference biomarkers for data normalization purposes include markers which are ubiquitously expressed and/or whose expression is not regulated by immunomodulators. Preferred reference markers include 18S ribosomal RNA (18S, GenBank Accession No. X03205, SEQ ID NO:17), and transcripts of beta-2 microglobulin (B2M, GenBank Accession No. NM_004048, SEQ ID NOs:18,19). Constitutively expressed genes are known in the art and can be identified and selected according to the relevant tissue and/or situation of the patient and the analysis methods. Such normalization allows one to compare the expression level in one sample, to another sample, *e.g.*, between samples from different times or different subjects. Further, the expression level can be provided as a relative expression level. To determine a relative expression level of a marker or marker set, the level of expression of the biomarker or biomarker set is determined for at least 1, preferably 2, 3, 4, 5, or more samples, *e.g.*, 7, 10, 15, 20 or 50 or more samples in order to establish a baseline, prior to the determination of the expression level for the sample in question. To establish a baseline measurement, the mean expression level of each of the biomarkers or biomarker sets assayed in the larger number of samples is determined and this is used as a baseline expression level for the biomarkers or biomarker sets in question. The expression level of the biomarker or biomarker set determined for the test sample (absolute level of expression) is then divided by the baseline expression value obtained for that biomarker or biomarker set. This provides a relative expression level and aids in identifying extreme levels of germinal center activity.

Some markers, *e.g.*, AICDA (AID) and IGL-κ, are expressed as mRNAs (*e.g.*, are poly-adenylated) and ultimately translated as proteins and some markers, *e.g.*, GLT-μ and the CT's, are not. Thus, the detection methods and measurement of expression levels are selected accordingly. Choices including cDNA amplification methods and protein detection methods can be used for translated biomarkers; nucleic acid measurement and amplification methods are used for biomarkers which are not translated. Primers and nucleic acid probes can be optimized for the particular transcripts. Commercial assays for transcripts used in ICS are available for use with quantitative RT-PCR systems, e.g., Assay-On-Demand formats from Applied Biosystems, Inc., Foster City, CA. Modifications of the system can enable high throughput analyses of the samples, *e.g.*, the TAQMAN® low density array upgrade (Applied Biosystems, Foster City, CA).

Preferred primers or nucleic acid probes comprise a nucleotide sequence complementary to a specific allelic variant of a biomarker polymorphic region and of sufficient length to selectively hybridize with a biomarker gene. In a preferred embodiment, the primer or nucleic acid probe, *e.g.*, a substantially purified oligonucleotide, comprises a region having a nucleotide sequence which hybridizes under stringent conditions to about 6, 8, 10, or 12, preferably 15, 20, 25, 30, 40, 50, 60, 75, 100 or more consecutive nucleotides of a biomarker gene. In an even more preferred embodiment, the primer or nucleic acid probe is capable of hybridizing to a biomarker nucleotide sequence and comprises a nucleotide sequence of any sequence set forth in any of SEQ ID NOs:22-57, or a complement thereof. For example, a primer or nucleic acid probe comprising a nucleotide sequence of at least about 15 consecutive nucleotides, at least about 25 nucleotides or having from about 15 to about 20 nucleotides set forth in any of SEQ ID NOs:22-57 or a complement thereof are provided by the disclosure. Primers or nucleic acid probes having a sequence of more than about 25 nucleotides are also within the scope of the invention. In another embodiment of the disclosure, a primer or nucleic acid probe can have a sequence at least 70%, preferably 75%, 80% or 85%, more preferably, 90%, 95% or 97% identical to the nucleotide sequence of any sequence set forth in any of SEQ ID NOs:22-57, or a complement thereof. Nucleic acid analogs can be used as binding sites for hybridization. An example of a suitable nucleic acid analogue is peptide nucleic acid (see, *e.g.*, Egholm et al., Nature 363:566 568 (1993); U.S. Pat. No. 5,539,083). Primers or nucleic acid probes are preferably selected using an algorithm that takes into account binding energies, base composition, sequence complexity, cross-hybridization binding energies, and secondary structure (see Friend et al., International Patent Publication WO 01/05935, published Jan. 25, 2001; Hughes et al., Nat. Biotech. 19:342-7 (2001). Preferred primers or nucleic acid probes of the invention are primers that bind sequences which are unique for each transcript and can be used in PCR for amplifying and detecting only that particular transcript. One of skill in the art can design primers and nucleic acid probes for the biomarkers disclosed herein or related biomarkers with similar characteristics, *e.g.*, biomarkers having a role in ICS or germinal center activity, using the skill in the art, *e.g.*, adjusting the potential for primer or nucleic acid probe binding to standard sequences, mutants or allelic variants by manipulating degeneracy or GC content in the primer or nucleic acid probe. Computer programs that are well known in the art are useful in the design of primers with the required specificity and optimal amplification properties, such as Oligo version 5.0 (National Biosciences, Plymouth, MN). While perfectly complementary nucleic acid probes and primers are preferred for detecting the biomarkers described herein and polymorphisms or alleles thereof, departures from complete complementarity are contemplated where such departures do not prevent the molecule from specifically hybridizing to the target region. For example, an oligonucleotide primer may have a non-complementary fragment at its 5' end, with the remainder of the primer being complementary to the target region. Alternatively, non-complementary nucleotides may be interspersed into the nucleic acid probe or primer as long as the resulting probe or primer is still capable of specifically hybridizing to the target region.

Preferred sequences to detect for the measurement of GLT-$\mu$ (*e.g.*, to detect SEQ ID NO:1) comprise a fragment of at least 10, 15, 20, 25, 30, 35, 50, 75, or 100 or more nucleotides of the following sequence or complement thereof: SEQ ID NO:58 for 5' primer and/or SEQ ID NO:59, for the probe and 3' primer. Examples of 5' primers or complement thereof to use when detecting, amplifying and/or measuring GLT-$\mu$ are SEQ ID NOs:53, 55 or 34 (GLT1, GLT2 and GLT3, respectively). An example of a probe or complement thereof to use when detecting, amplifying and/or measuring GLT-$\mu$ is SEQ ID NO: 36. Examples of 3' primers, or reverse complement thereof to use when detecting, amplifying and/or measuring GLT-$\mu$ are SEQ ID NOs:35 or 54. Nucleic acid sequences comprising the above GLT-$\mu$ primers and probe (SEQ ID NOs:53, 55, 34, 36, 35 or 54), or complements thereof, can be used separately or together as a set for quantitative RT-PCR.

Preferred sequences to detect for the measurement of CT-$\gamma$1&2 (*e.g.*, to detect SEQ ID NO:2) comprise a fragment of at least 10, 15, 20, 25, 30, 35, 50, 75, or 100 or more nucleotides of the following sequences or complements thereof: SEQ ID NO:60 for 5' primer and/or SEQ ID NO:59 for the probe and 3' primer. Examples of 5' primer or complement thereof to use when detecting, amplifying and/or measuring CT-$\gamma$1&2 are SEQ ID NOs:37, 56, or 57 (CT1, CT2 and CT3, respectively). An example of a probe or complement thereof to use when detecting, amplifying and/or measuring CT-$\gamma$1&2 is SEQ ID NO:36. Examples of 3' primers, or reverse complement thereof to use when detecting, amplifying and/or measuring CT-$\gamma$1&2 are SEQ ID NOs:35 or 54. Nucleic acid sequences comprising the above CT-$\gamma$1&2 primers and probe (SEQ ID NOs:37, 56, 57, 36, 35, or 54), or complements thereof, can be used separately or together as a set for quantitative RT-PCR.

An indication of germinal center activity can be assessed by studying the level of 1 biomarker, 2 biomarkers, 3 biomarkers, 4 biomarkers, 5 biomarkers, 6 biomarkers, 7 biomarkers, 8 biomarkers, 9 biomarkers, 10 biomarkers, or more, *e.g.*, 15, 20 or 25 biomarkers. Biomarkers can be studied in combination with another measure of immune competence, *e.g.*, histological markers (*i.e.*, hypo- or hyperplasia of secondary lymphoid organs), frequency of subsets of leukocytes, level of cytokine, T cell-dependent antibody responses (TDAR).

Statistical methods can assist in the determination of germinal center activity upon measurement of the level of expression of biomarkers, *e.g.*, measurement of transcripts. The level of one transcript can be measured at multiple timepoints, *e.g.*, before treatment, during treatment, after treatment with an agent, *e.g.*, an immunomodulator. To determine the progression of change in expression of a marker from a baseline, *e.g.*, over time, the expression results can be analyzed by a repeated measures linear regression model (Littell, Miliken, Stroup, Wolfinger, Schabenberger (2006) SAS for Mixed Models, 2nd edition. SAS Institute, Inc., Cary, NC)):

Equation 1

$$Y_{ijk} - Y_{ij0} = Y_{ij0} + treatment_i + day_k + (treatment * day)_{ik} + \varepsilon_{ijk}$$

where $Y_{ijk}$ is the $\log_2$ transformed expression (normalized to the housekeeping genes) on the k$^{th}$ day of the j$^{th}$ animal in the i$^{th}$ treatment, $Y_{ij0}$ is the defined baseline $\log_2$ transformed expression (normalized to the housekeeping genes) of the j$^{th}$ animal in the i$^{th}$ treatment, $day_k$ is treated as a categorical variable, and $\varepsilon_{ijk}$ is the residual error term. A covariance matrix (e.g., first-order autoregressive, compound symmetry, spatial power law) can be specified to model the repeated measurements on each animal over time. Furthermore, each treatment time point can be compared back to the same time point in the vehicle group to test whether the treatment value was significantly different from vehicle.

A number of other methods can be used to analyze the data. For instance, the relative expression values could be analyzed instead of the cycle number. These values could be examined as either a fold change or as an absolute difference from baseline. Additionally, a repeated-measures analysis of variance (ANOVA) could be used if the variances are equal across all groups and time points. The observed change from baseline at the last (or other) time point could be analyzed using a paired t-test, or a Wilcoxon signed rank test if the data is not normally distributed, to compare whether a treated group was significantly different from the vehicle group.

A difference in expression from one timepoint to the next can indicate a change in germinal center activity. A baseline level can be determined by measuring expression at 1, 2, 3, 4, or more times prior to treatment, e.g., at time zero, one day, three days, one week and/or two weeks or more before treatment. Alternatively, a baseline level can be determined from a number of subjects, *e.g.*, normal subjects or patients with the same health status or disorder, who do not undergo or have not yet undergone the treatment, as discussed above. Alternatively, one can use expression values deposited with the Gene Expression Omnibus (GEO) program at the National Center for Biotechnology Information (NCBI, Bethesda, MD). To test the effect of the immunomodulator on immunocompetence, the expression of the biomarker can be measured at any time or multiple times after some treatment, e.g., after 1 day, 2 days, 3 days, 5 days, 1 week, 2 weeks, 3 weeks, 4 weeks, 1 month, 2 months, 3 months and/or 6 or more months of treatment. For example, the level of expression of a biomarker can be measured once after some treatment, or at multiple intervals, e.g., 1-week, 2-week, 4-week or 2-month, 3-month or longer intervals during treatment. Conversely, to determine whether there has been recovery or restoration of normal immunocompetence after stopping the administration of an immunomodulator, the expression of the biomarker can be measured at any time or multiple times after, *e.g.*, 1 day, 2 days, 3 days, 5 days, 1 week, 2 weeks, 3 weeks, 4 weeks, 1 month, 2 months, 3 months and/or 6 or more months after the last treatment. One of skill in the art would determine the timepoint or timepoints to assess the expression level of the biomarker depending on various factors, *e.g.*, the pharmacokinetics of the immunomodulator, the treatment duration, pharmacodynamics of the immunomodulator, age of the patient, the nature of the disorder or mechanism of action of the immunomodulator. A trend in the negative direction or a decrease in the amount relative to baseline or a pre-determined standard of expression of a biomarker of immune competence indicates a decrease in germinal center activity, *e.g.*, atrophy. A trend in the positive direction or an increase in the amount relative to the baseline or a pre-determined standard of expression of a biomarker of immune competence indicates an increase in germinal center activity, *e.g.*, hyperplasia or clonal expansion.

Because the kits, methods and uses of a probe of the invention rely on detection of a difference in expression levels of one or more markers of the invention, it is preferable that difference in the level of expression of the marker is significantly greater than the minimum detection limit of the method used to assess expression in at least one of baseline levels and treated levels. Preferably, changes in germinal center activity are measured by 2-, 2.5-, 3-, 3.5-, 4-, 4.5-, 5-, 7-,10- fold, or more differences of expression of the biomarker. For example, 2-, 2.5-, 3-, 3.5-, 4-, 4.5-, 5-, 7-,10- fold or more higher expression would indicate higher germinal center activity; 2- (*i.e.*, one-half), 2.5-, 3-, 3.5-, 4-, 4.5-, 5-, 7-,10- (*i.e.*, one tenth) fold or less lower expression would indicate lower germinal center activity. Any marker or combination of markers of the invention, as well as any known markers in combination with the markers of the invention, may be used in the kits, methods and use of a probe of the present invention. In general, it is preferable to use markers for which the difference between the level of expression of the marker in cells from atrophied or hyperplastic germinal centers and the level of expression of the same marker in cells from normal germinal centers is as great as possible. Although this difference can be as small as the limit of detection of the method for assessing expression of the marker, it is preferred that the difference be at least greater than the standard error of the assessment method, and preferably a difference of at least 2-, 3-, 4-, 5-, 6-, 7-, 8-, 9-, 10-, 15-, 20-, 25-, 100-, 500-, 1000-fold or greater than when the level of expression of the same marker is measured in samples comprising B cells which have not experienced a germinal center or which have not experienced the test agent. The difference can be qualified by a confidence level, *e.g.*, $p < 0.05$, preferably, $p < 0.02$, more preferably $p < 0.01$.

Measurement of more than one biomarker, *e.g.*, a set of 2, 3, 4, 5, 6, 7, 8, 9, 10, 12, 15, 20, or 25 or more biomarkers can provide an expression profile or a trend indicative of germinal center activity. In some embodiments, the predictive marker set comprises no more than 2, 3, 4, 5, 6, 7, 8, 9, 10, 12, 15, 20, or 25 biomarkers. In some embodiments, the predictive marker set includes a plurality of genes associated with isotype class switching, clonal expansion or maturation of B cells. Analysis of germinal center activity through assessing expression of biomarkers in a set can be accompanied by a statistical method, e.g., a weighted voting analysis which accounts for variables which can affect the contribution of the expression level of a marker in the set to the class or trend of germinal center activity, *e.g.*, the signal-to-noise ratio of the measurement or hybridization efficiency for each biomarker. A biomarker set, *e.g.*, a set of 2, 3, 4, 5, 6, 7, 8, 9, 10, 12, 15, 20, or 25 or more biomarkers, comprises a probe or probes to detect at least one biomarker described herein, *e.g.*, a marker indicative of ICS, (*e.g.*, 18S ribosomal RNA and/or beta-2 microglobulin (B2M (for data normalization purposes), and GLT-$\mu$, AICDA (AID), CT-$\gamma$1&2, IGL-$\kappa$, Ku70, RAG1, IGHG1, IGHG2, IGHG3, IGHG4, IGHA1, IGHA2 and/or IGHE. A preferred biomarker set, e.g., a set of 2, 3, 4, 5, 6, 7, 8, 9, 10, 12, 15, 20, or 25 or more biomarkers, comprises a probe or probes to detect at least one or at least two or more preferred biomarkers, *e.g.*, at least one or at least two of GLT-μ, AICDA (AID), CT-γ1, CT-γ2, IGHG1 and/or IGHA2. If the immunomodulator has a mechanism of action that targets TNFα or the NF-κB pathway, a preferred biomarker to include in a biomarker set is IGL-κ. Selected biomarker sets can be assembled from the biomarkers provided herein or selected from among germinal center activators, ICS expression products or ICS effectors using methods provided herein and analogous methods known in the art. A way to qualify a new marker for use in an assay of the invention is to correlate histological changes in germinal center activity with differences in expression (*e.g.*, fold-change from baseline) of an ICS biomarker. A useful way to judge the relationship is to calculate the coefficient of determination $r^2$, after solving for r, the Pearson product moment correlation coefficient and/or preparing a least squares plot, using standard statistical methods. A preferable correlation would analyze reactivity of germinal centers (inverse of atrophy) *versus* the level of ICS biomarker. Preferably, a gene product would be selected as a biomarker of ICS if the result of the correlation ($r^2$, *e.g.*, the linear slope of the data in this analysis), is at least 0.1-0.2, more preferably, at least 0.3-0.5, most preferably at least 0.6-0.8 or more, up to 1.0 (*i.e.*, perfect positive correlation). Some biomarkers can vary with a positive correlation to germinal center activity (*i.e.*, change expression levels in the same manner as germinal center activity levels, *e.g.*, decrease when germinal center activity is decreased) and some biomarkers can vary with a negative correlation to germinal center activity (*i.e.*, change expression levels in the opposite manner as germinal center activity levels, *e.g.*, increase when germinal center activity is decreased).

Another way to qualify a new marker for use in the assay would be to assay the expression of large numbers of germinal center activators, ICS expression products or ICS effectors in a number of subjects before and after treatment with a test agent. The expression results allow identification of the markers which show large changes in a given direction after treatment relative to the pre-treatment samples. One can build a repeated-measures linear regression model to identify the genes that show statistically significant changes. To then rank these significant genes, one can calculate the area under the change from baseline vs time curve. This can result in a list of genes that would show the largest statistically significant changes. A review of the function of the genes with large changes in expression could help qualify the suitability of the gene for assessing a trend, e.g. germinal center hyperplasia or atrophy (*e.g.*, predictive of immunosuppression), depending on the gene's role in germinal center activity or ICS. We could combine several genes together in a set by using such methods as principle component analysis, clustering methods (*e.g.*, k-means, hierarchical), multivariate analysis of variance (MANOVA), or linear regression techniques. To use such a gene (or group of genes) as a marker, genes which show 2-, 2.5-, 3-, 3.5-, 4-, 4.5-, 5-, 7-,10- fold, or more differences of expression from baseline would be included in the marker set. An expression profile, *e.g.*, a composite of the expression level differences from baseline or reference of the aggregate marker set would indicate at trend, *e.g.*, if a majority of markers show a particular result, *e.g.*, a significant difference from baseline or reference, preferably 60%, 70%, 80%, 90%, 95% or more markers; or more markers, *e.g.*, 10% more, 20% more, 30% more, 40% more, show a significant result in one direction than the other direction.

When the kits, methods and use of a probe of the invention are used for characterizing capacity of adaptive immunity, *e.g.*, immunosuppression or immunity in a patient, it is preferred that the biomarker or set of biomarkers of the invention is selected such that a significant result is obtained in at least about 20%, and preferably at least about 40%, 60%, or 80%, and more preferably in substantially all patients treated with the test agent. Preferably, the biomarker or set of biomarkers of the invention is selected such that a positive predictive value (PPV) of greater than about 10% is obtained for the general population (more preferably coupled with an assay specificity greater than 80%).

Thus, in one embodiment, *e.g.*, an embodiment using a nucleic acid array, the method of determining a particular adaptive immunity-related status of an individual comprises the steps of (1) hybridizing labeled target nucleic acid probes or primers from an individual to a plate, e.g., microarray containing one of the marker sets described herein or determined according to the parameters described herein; (2) hybridizing standard or control nucleic acid probes or primers to the microarray, wherein the standard or control molecules are differentially labeled from the target molecules; and (3) determining the ratio (or difference) of transcript levels between subject and control, reference or baseline, or simply the absolute transcript levels of the individual; and (4) comparing the results from (3) to the predefined standard, profiles, templates or trends, wherein said determining can be accomplished by means of the statistic as described herein, and wherein the difference, or lack thereof, determines the individual's adaptive immunity-related status.

The present disclosure pertains to the field of predictive medicine in which diagnostic assays, prognostic assays, pharmacogenomics, and monitoring clinical trails can be used for prognostic (predictive) purposes to thereby treat an individual prophylactically. Accordingly, one aspect of the present disclosure relates to diagnostic assays for determining the level of expression of one or more biomarker proteins or nucleic acids, in order to determine whether an individual is at risk of developing immunosuppression or whether a vaccination has succeeded at providing protective immunity. Such assays can be used for prognostic or predictive purposes to thereby prophylactically treat or adjust the treatment regimen of an individual prior to the onset of the condition.

Yet another aspect of the disclosure pertains to monitoring the influence of agents, *e.g.*, drugs or other compounds administered either to inhibit immunosuppression or to treat or prevent any other disorder *e.g.*, chronic disorder (*i.e.*, in order to understand any chronic effects that such treatment may have) on the expression or activity of a biomarker of the invention in clinical trials. The agents are described in further detail in the following sections.

The markers of the invention may serve as surrogate markers for one or more disorders or disease states or for conditions leading up to disease states, and in particular, prostate cancer. As used herein, a "surrogate marker" is an objective biochemical marker which correlates with the absence or presence of a disease or disorder, or with the progression of a disease or disorder (*e.g.*, with the presence or absence of a tumor). The presence or quantity of such markers is independent of the disease. Therefore, these markers may serve to indicate whether a particular course of treatment is effective in lessening a disease state or disorder. Surrogate markers are of particular use when the presence or extent of a disease state or disorder is difficult to assess through standard methodologies (*e.g.*, before pathogen exposure or early stage tumors), or when an assessment of capacity for adaptive immunity or disease progression is desired before a potentially dangerous clinical endpoint is reached (*e.g.*, use of the biomarkers described herein or an assessment of cardiovascular disease may be made using cholesterol levels as a surrogate marker, and an analysis of HIV infection may be made using HIV RNA levels as a surrogate marker, well in advance of the undesirable clinical outcomes of myocardial infarction or fully-developed AIDS). Examples of the use of surrogate markers in the art include: Koomen et al. (2000) J. Mass. Spectrom. 35: 258-264; and James (1994) AIDS Treatment News Archive 209.

The markers of the invention are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker may be indicative of the concentration of the drug in a biological tissue, in that the marker is either expressed or transcribed or not expressed or transcribed in that tissue in relationship to the level of the drug. In this fashion, the distribution or uptake of the drug may be monitored by the pharmacodynamic marker. Similarly, the presence or quantity of the pharmacodynamic marker may be related to the presence or quantity of the metabolic product of a drug, such that the presence or quantity of the marker is indicative of the relative breakdown rate of the drug *in vivo.* Pharmacodynamic markers are of particular use in increasing the sensitivity of detection of drug effects, particularly when the drug is administered in low doses. Since even a small amount of a drug may be sufficient to activate multiple rounds of marker transcription or expression, the amplified marker may be in a quantity which is more readily detectable than the drug itself. Also, the marker may be more easily detected due to the nature of the marker itself; for example, using the methods described herein, antibodies may be employed in an immune-based detection system for a protein marker, or marker-specific radiolabeled nucleic acid probes may be used to detect a RNA marker. Furthermore, the use of a pharmacodynamic marker may offer mechanism-based prediction of risk due to drug treatment beyond the range of possible direct observations. Examples of the use of pharmacodynamic markers in the art include: Matsuda et al. US 6,033,862; Hattis et al. (1991) Env. Health Perspect. 90: 229-238; Schentag (1999) Am. J. Health-Syst. Pharm. 56 Suppl. 3: S21-S24; and Nicolau (1999) Am, J. Health-Syst. Pharm. 56 Suppl. 3: S16-S20.

Monitoring the influence of agents (e.g., drug compounds) on the level of expression of a marker of the invention can be applied not only in basic drug screening, but also in clinical trials or during treatment. For example, the effectiveness of an agent to affect marker expression can be monitored in clinical trials or subjects receiving treatment for chronic conditions. In a preferred embodiment, the present disclosure provides a method for monitoring the effectiveness of treatment of a subject with an agent, *e.g.*, an immunomodulator (*e.g.*, an agonist, antagonist, peptidomimetic, protein, peptide, nucleic acid, small molecule, or other drug candidate) comprising the steps of (i) obtaining a pre-administration sample from a subject prior to administration of the agent; (ii) detecting the level of expression of one or more selected markers of the invention in the pre-administration sample; (iii) obtaining one or more during and/or post-administration samples from the subject; (iv) detecting the level of expression of the marker(s) in the during and/or post-administration samples; (v) comparing the level of expression of the marker(s) in the pre-administration sample with the level of expression of the marker(s) in the during and/or post-administration sample or samples; and (vi) altering the administration of the agent to the subject accordingly. For example, increased expression of the biomarker during the course of treatment may effective vaccination or hyperplasia. Conversely, decreased expression of the biomarker may indicate immunosuppression caused by treatment and the desirability of decreasing the dosage, e.g., administering a lower dose and/or a less frequent administration schedule, *e.g.*, at a dose statistically less than recommended for other patients receiving the treatment and/or at a dosing frequency less than recommended for patients receiving the treatment and optionally adding an agent which can treat the disorder by a different mechanism; or changing the therapeutic agent.

Many chronic conditions are treated with immunomodulators. There is risk of opportunistic infections for patients undergoing these treatments. Opportunistic infections are associated with significant morbidity and often are a challenge to treat. A simple, noninvasive test can monitor germinal center activity to identify risk of adverse events so treatment regimens can be adjusted accordingly.

Agents worth monitoring using this test include immunomodultors which include but are not limited to, anti-TNF agents, *e.g.*, ENBREL® etanercept (Immunex Corp., Thousand Oaks, CA), REMICADE® infliximab (Centocor Inc., Malvern, PA), sulfasalazine) HUMIRA® adalimumab (Abbott Laboratories, Abbott Park, IL), CIMZIA® certolizumab pegol (CDP870, UCB S.A. Corp., Brussels, Belgium, a PEGylated Fab' fragment of a humanized TNF inhibitor monoclonal antibody (its effects on B lymphocytes noted in Anolik et al. (2008) J. Immunol. 180:688-692)), CDP571; inhibitors of I kappa B kinase (IKK), *e.g.*, IKKβ inhibitors, (*e.g.*, beta-carbolines, *e.g.*, N-(6-chloro-9H-beta-carbolin-8-yl) nicotinamide (PS-1145), U.S. Patent Nos. 6,627,637, 7,026,331, N-(6-chloro-7-methoxy-9H-beta-carbolin-8-yl)-2-methyl-nicotinamide (ML120B, Nagashima et al. (2006) Blood 107:4266-73), U.S. Patent Application Publication No. 20040235839, Millennium Pharmaceuticals, Inc., Cambridge, MA, BMS-345541, 4(2'-aminoethyl)amino-1,8-dimethylimidazo(1,2-a)quinoxaline, Bristol Myers Squibb, Princeton, NJ); CD40 antagonists; CD40 ligands (*e.g.*, TNX-100 anti-CD40 antibody, 5D12, effects on B lymphocytes noted in deVos et al. (2004) Eur. J. Immunol. 34:3446-55), RITUXAN® rituximab (Idec Pharmaceuticals Corp., San Diego, CA); anti-CD20 antibody (its effects on B lymphocytes noted in Nyman et al. (2007) Blood 109:4930-5, Anolik et al. (2007) Arthritis Rheum. 56:3044-56); steroidal antiinflammatory compounds, *e.g.*, glucocorticoids (*e.g.*, prednisone, prednisolone, adrenocorticotrophic hormone (ACTH), dexamethasone, methylprednisolone, hydrocortisone, triamcinolone, effects on B lymphocytes noted in Sackstein and Borenstein (1995) J. Invest. Med. 43:68-77, Scheinman et al. (1995) Science 270:283-6); immunosuppressive agents (*e.g.*, azathioprene, 6-mercaptopurine, calcineurin inhibitors (*e.g.*, cyclosporin A, effects on B lymphocytes noted in Kuper et al. (2007) Toxicol. Pathol. 35:226-32, Gore et al. (2008) Toxicology 197:23-35)); and other immunomodulators (*e.g.*, thalidomide, interleukins (*e.g.*, recombinant human EL-10, recombinant human IL-11, IL-2 antagonists (*e.g.*, PROGRAF® tacrolimus, Apellas Pharma Inc., Tokyo, JP, FK-506; or antibodies against the IL-2 receptor alpha chain (CD25), *e.g.*, SIMULECT® basiliximab, Novartis Pharmaceuticals Corp. East Hanover, NJ, or ZENAPAX® daclizumab, Hoffman-LaRoche Corp. Nutley, NJ); CD-80 antagonists (*e.g.*, ORENCIA® abatacept, Bristol-Myers Squibb, Princeton, NJ); anti-IL-1 or other IL-1 antagonists (*e.g.*, KINERET® anakinra, Amgen Inc., Thousand Oaks, CA); and signal transduction inhibitors *(e.g.,* rapamycin, its effects on B lymphocytes noted in Woodland et al. (2008) Blood 111:750-60).

Additional agents whose effects, on germinal center activity can be tested or monitored include integrin antagonists, *e.g.*, of alpha4betal integrin ($\alpha4\beta1$ or VLA-4 antagonists, *e.g.*, TYSABRI® natalizumab, Biogen Idec and Elan Corp., Dublin, IE, Cambridge, MA or firategrast, SB-683699, GlaxoSmithKline, London UK); of alpha4beta7 integrin ($\alpha4\beta7$ antagonists, *e.g.*, vedolizumab, Millennium Pharmaceuticals, Inc., Cambridge, MA); of beta2 integrin ($\beta2$ or CD18, antagonists, *e.g.*, LFA-1 (CD11a/CD18) antagonists, *e.g.*, RAPTIVA® efalizumab, Genentech, Inc., South San Francisco, CA); and proteasome inhibitors, *e.g.*, peptidyl boronic acids, *e.g.*, VELCADE® bortezomib, Millennium Pharmaceuticals, Inc., Cambridge, MA; peptide aldehydes, *e.g.*, see U.S. Patent No. 5,693,617; peptidyl epoxy ketones, *e.g.*, see U.S. Patent No. 6,831,099; alpha-ketoamides, see *e.g.*, U.S. Patent Nos. 6,310,057; or lactacystin and salinosporamide and analogs thereof, *e.g.*, see U.S. Patent No. 5,756,764, international patent publication WO 05/002572 or U.S. Patent No. 7276530.

Examples of disorders being treated by the agents whose use can benefit from monitoring germinal center activity include autoimmune disorders (*e.g.*, systemic lupus erythematosis, immune-mediated glomerulonephritis, insulin-dependent diabetes, multiple sclerosis, Hashimoto's thyroiditis, Grave's disease, and arthritis (*e.g.*, juvenile rheumatoid arthritis, psoriatic arthritis, rheumatoid arthritis, ankylosing spondylitis, reactive arthritis, Lyme disease and osteoarthritis); disorders mediated by excess TNF$\alpha$; inflammatory bowel disease, *e.g.*, Crohn's disease and ulcerative colitis, sclerosing cholangitis, Celiac disease, pouchitis, eosinophilic gastroenteritis; skin disease, *e.g.*, contact dermatitis, psoriasis and hidradenitis suppurativa; graft rejection, graft versus host disease, sarcoidosis, respiratory inflammatory diseases and disorders, such as asthma, chronic obstructive pulmonary disease and allergic rhinitis; gastrointestinal allergies, including food allergies, eosinophilia, conjunctivitis, glomerular nephritis; certain pathogen susceptibilities such as helminthic (*e.g.*, leishmaniasis), certain viral infections, including HIV, and bacterial infections, including tuberculosis and lepromatous leprosy; cancers, *e.g.*, B cell neoplasms (*e.g.*, diffuse large B cell lymphoma, follicular lymphoma and B-cell prolymphocytic leukemia).

Opportunistic infections/diseases of which patients are at risk of acquiring: conditions caused by bacterial infection, *e.g.*, tuberculosis, listerosis, pneumonia, shigellosis, and salmonella; viral infection, *e.g.*, infection by herpesvirus, poxvirus, and adenovirus, cytomegalovirus, varicella zoster virus or Epstein-Barr virus. Certain disorders are associated with an increased number of surviving cells, which are produced and continue to survive or proliferate when apoptosis is inhibited or occurs at an undesirably low rate. These disorders include cancer (particularly follicular lymphomas, plasmablastic lymphoma, chronic myelogenous leukemia, multiple myeloma, mycosis fungoides, melanoma, colon cancer, lung carcinoma, carcinomas associated with mutations in p53, and hormone-dependent tumors such as breast cancer, prostate cancer, and ovarian cancer). Failure to remove autoimmune cells that arise during development or that develop as a result of somatic mutation during an immune response can result in autoimmune disease. Thus, an autoimmune disorder (*e.g.*, systemic lupus erythematosis, immune-mediated glomerulonephritis, and arthritis) can be caused by an undesirably low level of apoptosis.

The disclosure also includes a method of assessing the efficacy of a test compound for modulating adaptive immunity. As described above, differences in the level of expression of the markers of the invention correlate with the capacity of adaptive immunity. It is recognized that changes in the levels of expression of some of the biomarkers of the invention indicate isotype class switching. Thus, compounds which affect adaptive immunity in a patient, *e.g.*, one with immunosuppression or one who received a vaccine, will cause the level of expression of one or more of the biomarkers of the invention to change to a level different than the normal level of expression for that marker (*i.e.*, the level of expression for the marker in mature B cells or germline B cells, respectively).

This method thus comprises comparing the expression of a marker in a first sample comprising B cells and maintained in the presence of the test compound and expression of the marker in a second sample comprising B cells and maintained in the absence of the test compound. A significantly reduced expression of a marker of the invention in the presence of the test compound is an indication that the test compound inhibits B cell activity. The samples may, for example, be aliquots of a single sample comprising normal B cells obtained from a patient, pooled samples comprising normal B cells obtained from a patient, cells of a normal B cell line, aliquots of a single sample comprising B cells obtained from a patient, pooled samples comprising B cells obtained from a patient, cells of B cell line, or the like. In one embodiment, the samples comprising B cells are obtained from a patient and a plurality of compounds known to be effective for inhibiting various B cell activities are tested in order to identify the compound which is most likely to induce immunization or not to induce immunosuppression in the patient.

This method likewise may be used to assess the efficacy of a therapy for treating chronic disease in a patient or potential for the therapy to cause adverse effects. In this method, the level of expression of one or more markers of the invention in a pair of samples comprising B cells (one subjected to the therapy, the other not subjected to the therapy) is assessed. As with the method of assessing the efficacy of test compounds, if the therapy induces a significantly lower level of expression of a marker of the invention then the therapy can cause germinal center atrophy. As above, if samples from a selected patient are used in this method, then alternative therapies can be assessed *in vitro* in order to select a therapy most likely to be efficacious for modulating adaptive immunity in the patient. Conversely, if samples from a selected patient are used in this method, then alternative therapies can be assessed *in vitro* in order to select a therapeutic agent or dose least likely to cause detrimental immunosuppression.

Detection Methods

A general principle of such diagnostic and prognostic assays involves preparing a sample or reaction mixture that may contain a marker, and a probe, under appropriate conditions and for a time sufficient to allow the marker and probe to interact and bind, thus forming a complex that can be removed and/or detected in the reaction mixture. These assays can be conducted in a variety of ways.

For example, one method to conduct such an assay would involve anchoring the marker or probe onto a solid phase support, also referred to as a substrate, and detecting target marker/probe complexes anchored on the solid phase at the end of the reaction. In one embodiment of such a method, a sample from a subject, which is to be assayed for presence and/or concentration of marker, can be anchored onto a carrier or solid phase support. In another embodiment, the reverse situation is possible, in which the probe can be anchored to a solid phase and a sample from a subject can be allowed to react as an unanchored component of the assay. One example of such an embodiment includes use of an array or chip which contains a predictive marker or marker set anchored for expression analysis of the sample.

There are many established methods for anchoring assay components to a solid phase. These include, without limitation, marker or probe molecules which are immobilized through conjugation of biotin and streptavidin. Such biotinylated assay components can be prepared from biotin-NHS (*N*-hydroxy-succinimide) using techniques known in the art (*e.g.*, biotinylation kit, Pierce Chemicals, Rockford, IL), and immobilized in the wells of streptavidin-coated 96 well plates (Pierce Chemical). In certain embodiments, the surfaces with immobilized assay components can be prepared in advance and stored.

Other suitable carriers or solid phase supports for such assays include any material capable of binding the class of molecule to which the marker or probe belongs. Well-known supports or carriers include, but are not limited to, glass, polystyrene, nylon, polypropylene, nylon, polyethylene, dextran, amylases, natural and modified celluloses, polyacrylamides, gabbros, and magnetite. One skilled in the art will know many other suitable carriers for binding antibody or antigen, and will be able to adapt such support for use with the present invention. For example, protein isolated from blood cells can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

In order to conduct assays with the above mentioned approaches, the non-immobilized component is added to the solid phase upon which the second component is anchored. After the reaction is complete, uncomplexed components may be removed (e.g., by washing) under conditions such that any complexes formed will remain immobilized upon the solid phase. The detection of marker/probe complexes anchored to the solid phase can be accomplished in a number of methods outlined herein.

In a preferred embodiment, the probe, when it is the unanchored assay component, can be labeled for the purpose of detection and readout of the assay, either directly or indirectly, with detectable labels discussed herein and which are well-known to one skilled in the art. The term "labeled", with regard to the probe (*e.g.*, nucleic acid or antibody), is intended to encompass direct labeling of the probe by coupling (*i.e.*, physically linking) a detectable substance to the probe, as well as indirect labeling of the probe by reactivity with another reagent that is directly labeled. An example of indirect labeling includes detection of a primary antibody using a fluorescently labeled secondary antibody. It is also possible to directly detect marker/probe complex formation without further manipulation or labeling of either component (marker or probe), for example by utilizing the technique of fluorescence energy transfer (FET, see, for example, Lakowicz et al., U.S. Patent No. 5,631,169; Stavrianopoulos, et al., U.S. Patent No. 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (*e.g.*, using a fluorimeter).

In another embodiment, determination of the ability of a probe to recognize a marker can be accomplished without labeling either assay component (probe or marker) by utilizing a technology such as real-time Biomolecular Interaction Analysis (BIA) (see, *e.g.*, Sjolander, S. and Urbaniczky, C. (1991) Anal. Chem. 63:2338-2345 and Szabo et al. (1995) Curr. Opin. Struct. Biol. 5:699-705). As used herein, "BIA" or "surface plasmon resonance" is a technology for studying biospecific interactions in real time, without labeling any of the interactants (*e.g.*, BIACORE™). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, in another embodiment, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas, G., and Minton, A.P. (1993) Trends Biochem Sci. 18:284-7). Standard chromatographic techniques also can be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, *e.g.*, Heegaard, N.H. (1998) J. Mol. Recognit. 11:141-8; Hage, D.S., and Tweed, S.A. (1997) J. Chromatogr. B. Biomed. Sci. Appl. 699:499-525). Gel electrophoresis may also be employed to separate complexed assay components from unbound components (see, *e.g.*, Ausubel et al., ed., Current Protocols in Molecular Biology, John Wiley & Sons, New York, 1987-1999). In this technique, protein or nucleic acid complexes are separated based on size or charge, for example. In order to maintain the binding interaction during the electrophoretic process, non-denaturing gel matrix materials and conditions in the absence of reducing agent are typically preferred. Appropriate conditions to the particular assay and components thereof will be well known to one skilled in the art.

The isolated mRNA can be used in hybridization or amplification assays that include, but are not limited to, Southern or Northern analyses, polymerase chain reaction and TAQMAN® gene expression assays (Applied Biosystems, Foster City, CA) and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 20, 25, 30, 50, 75, 100, 125, 150, 175, 200, 250 or 500 or more consecutive nucleotides of the biomarker transcript and sufficient to specifically hybridize under stringent conditions to a mRNA or genomic DNA encoding a marker of the present invention. The exact length of the nucleic acid probe will depend on many factors that are routinely considered and practiced by the skilled artisan. Nucleic acid probes of the invention may be prepared by chemical synthesis using any suitable methodology known in the art, may be produced by recombinant technology, or may be derived from a biological sample, for example, by restriction digestion. Other suitable probes for use in the diagnostic assays of the invention are described herein. The probe can comprise a label group attached thereto, *e.g.*, a radioisotope, a fluorescent compound, an enzyme, an enzyme co-factor, a hapten, a sequence tag, a protein or an antibody. The nucleic acids can be modified at the base moiety, at the sugar moiety, or at the phosphate backbone. An example of a nucleic acid label is incorporated using SUPER™ Modified Base Technology (Nanogen, Bothell, WA, see U.S. Patent No. 7,045,610). The level of expression can be measured as general nucleic acid levels, *e.g.*, after measuring the amplified DNA levels (e.g. using a DNA intercalating dye, *e.g.*, the SYBR green dye (Qiagen Inc., Valencia, CA) or as specific nucleic acids, *e.g.*, using a probe based design, with the probes labeled. Preferable TAQMAN® assay formats use the probe-based design to increase specificity and signal-to-noise ratio.

Such probes can be used as part of a diagnostic test kit for identifying cells or tissues which express the protein, such as by measuring levels of a nucleic acid molecule transcribed during ICS or encoding a DNA recombination effector protein in a sample of cells from a subject, e.g., detecting transcript, mRNA levels or determining whether a gene encoding the protein has been mutated or deleted. Hybridization of an RNA or a cDNA with the nucleic acid probe indicates that the marker in question is being expressed. The invention further encompasses detecting nucleic acid molecules that differ, due to degeneracy of the genetic code, from the nucleotide sequence of nucleic acids encoding a marker protein (*e.g.*, protein having the sequence of the SEQ ID NOs:4, 6, 8, 10, 12, 14, or 16), and thus encode the same protein. It will be appreciated by those skilled in the art that DNA sequence polymorphisms that lead to changes in the amino acid sequence can exist within a population (*e.g.*, the human population). Such genetic polymorphisms can exist among individuals within a population due to natural allelic variation. An allele is one of a group of genes which occur alternatively at a given genetic locus. Such natural allelic variations can typically result in 1-5% variance in the nucleotide sequence of a given gene. Alternative alleles can be identified by sequencing the gene of interest in a number of different individuals. This can be readily carried out by using hybridization probes to identify the same genetic locus in a variety of individuals. Detecting any and all such nucleotide variations and resulting amino acid polymorphisms or variations that are the result of natural allelic variation and that do not alter the functional activity are intended to be within the scope of the invention. In addition, it will be appreciated that DNA polymorphisms that affect RNA expression levels can also exist that may affect the overall expression level of that gene (*e.g.*, by affecting regulation or degradation).

The nucleic acids described herein can be single stranded DNA (*e.g.*, an oligonucleotide), double stranded DNA (*e.g.*, double stranded oligonucleotide) or RNA. Preferred nucleic acids of the disclosure can be used as probes or primers. Primers of the disclosure refer to nucleic acids which hybridize to a nucleic acid sequence which is adjacent to the region of interest and is extended or which covers the region of interest. As used herein, the term "hybridizes" is intended to describe conditions for hybridization and washing under which nucleotide sequences that are significantly identical or homologous to each other remain hybridized to each other. Preferably, the conditions are such that sequences at least about 70%, more preferably at least about 80%, even more preferably at least about 85%, 90% or 95% identical to each other remain hybridized to each other for subsequent amplification and/or detection. Stringent conditions vary according to the length of the involved nucleotide sequence but are known to those skilled in the art and can be found or determined based on teachings in Current Protocols in Molecular Biology, Ausubel et al., eds., John Wiley & Sons, Inc. (1995), sections 2, 4 and 6. Additional stringent conditions and formulas for determining such conditions can be found in Molecular Cloning: A Laboratory Manual, Sambrook et al., Cold Spring Harbor Press, Cold Spring Harbor, NY (1989), chapters 7, 9 and 11. A preferred, non-limiting example of stringent hybridization conditions for hybrids that are at least 10 basepairs in length includes hybridization in 4X sodium chloride/sodium citrate (SSC), at about 65-70°C (or hybridization in 4X SSC plus 50% formamide at about 42-50°C) followed by one or more washes in 1X SSC, at about 65-70°C. A preferred, non-limiting example of highly stringent hybridization conditions for such hybrids includes hybridization in 1X SSC, at about 65-70°C (or hybridization in 1X SSC plus 50% formamide at about 42-50°C) followed by one or more washes in 0.3X SSC, at about 65-70°C. A preferred, non-limiting example of reduced stringency hybridization conditions for such hybrids includes hybridization in 4X SSC, at about 50-60°C (or alternatively hybridization in 6X SSC plus 50% formamide at about 40-45°C) followed by one or more washes in 2X SSC, at about 50-60°C. Ranges intermediate to the above-recited values, *e.g.*, at 65-70°C or at 42-50°C are also intended to be encompassed by the present invention. Another example of stringent hybridization conditions are hybridization in 6X sodium chloride/sodium citrate (SSC) at about 45°C, followed by one or more washes in 0.2X SSC, 0.1% SDS at 50-65°C. A further example of stringent hybridization buffer is hybridization in 1 M NaCl, 50 mM 2-(N-morpholino)ethanesulfonic acid (MES) buffer (pH 6.5), 0.5% sodium sarcosine and 30% formamide. SSPE (1xSSPE is 0.15M NaCl, 10mM $NaH_2PO_4$, and 1.25mM EDTA, pH 7.4) can be substituted for SSC (1xSSC is 0.15M NaCl and 15mM sodium citrate) in the hybridization and wash buffers; washes are performed for 15 minutes each after hybridization is complete The hybridization temperature for hybrids anticipated to be less than 50 base pairs in length should be 5-10°C less than the melting temperature ($T_m$) of the hybrid, where $T_m$ is determined according to the following equations. For hybrids less than 18 base pairs in length, $T_m$(°C) = 2(# of A + T bases) + 4(# of G + C bases). For hybrids between 18 and 49 base pairs in length, $T_m$(°C) = 81.5 + 16.6($\log_{10}[Na^+]$) + 0.41(%G+C) - (600/N), where N is the number of bases in the hybrid, and $[Na^+]$ is the concentration of sodium ions in the hybridization buffer ($[Na^+]$ for 1xSSC = 0.165 M). It will also be recognized by the skilled practitioner that additional reagents may be added to hybridization and/or wash buffers to decrease non-specific hybridization of nucleic acid molecules to membranes, for example, nitrocellulose or nylon membranes, including but not limited to blocking agents (*e.g.*, BSA or salmon or herring sperm carrier DNA), detergents (*e.g.*, SDS), chelating agents (*e.g.*, EDTA), Ficoll, polyvinylpyrrolidone (PVP) and the like. When using nylon membranes, in particular, an additional preferred, non-limiting example of stringent hybridization conditions is hybridization in 0.25-0.5M $NaH_2PO_4$, 7% SDS at about 65°C, followed by one or more washes at 0.02M $NaH_2PO_4$, 1% SDS at 65°C, see *e.g*., Church and Gilbert (1984) Proc. Natl. Acad. Sci. USA 81:1991-1995, (or alternatively 0.2X SSC, 1% SDS). A primer or nucleic acid probe can be used alone in a detection method, or a primer can be used together with at least one other primer or nucleic acid probe in a detection method. Primers can also be used to amplify at least a portion of a nucleic acid. Nucleic acid probes of the invention refer to nucleic acids which hybridize to the region of interest and which are not further extended. For example, a nucleic acid probe is a nucleic acid which specifically hybridizes to a polymorphic region of a biomarker, and which by hybridization or absence of hybridization to the DNA of a patient or the type of hybrid formed will be indicative of the identity of the allelic variant of the polymorphic region of the biomarker or the amount of germinal center activity.

In one format, the RNA is immobilized on a solid surface and contacted with a probe, for example by running the isolated RNA on an agarose gel and transferring the RNA from the gel to a membrane, such as nitrocellulose. In an alternative format, the nucleic acid probe(s) are immobilized on a solid surface and the RNA is contacted with the probe(s), for example, in an AFFYMETRIX® gene chip array (Santa Clara, CA) or customized array using a biomarker set comprising at least one biomarker indicative of ICS or B cell clonal expansion. A skilled artisan can readily adapt known RNA detection methods for use in detecting the level of RNA encoded by the markers of the present invention. For example, the high density microarray or branched DNA assay can benefit from a higher concentration of B cell in the sample, such as a sample which had been modified to isolate B cells as described in earlier sections. In a related embodiment, a mixture of transcribed polynucleotides obtained from the sample is contacted with a substrate having fixed thereto a polynucleotide complementary to or homologous with at least a portion (*e.g*., at least 7, 10, 15, 20, 25, 30, 40, 50, 100, 500, or more nucleotide residues) of a marker nucleic acid. If polynucleotides complementary to or homologous with the marker are differentially detectable on the substrate (*e.g*., detectable using different chromophores or fluorophores, or fixed to different selected positions), then the levels of expression of a plurality of markers can be assessed simultaneously using a single substrate (*e.g*., a "gene chip" microarray of polynucleotides fixed at selected positions). When a method of assessing marker expression is used which involves hybridization of one nucleic acid with another, it is preferred that the hybridization be performed under stringent hybridization conditions.

An alternative method for determining the level of RNA corresponding to a marker of the present invention in a sample involves the process of nucleic acid amplification, *e.g*., by RT-PCR (the experimental embodiment set forth in Mullis, 1987, U.S. Patent No. 4,683,202), ligase chain reaction (Barany, 1991, Proc. Natl. Acad. Sci. USA, 88:189-193), self sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technology 6:1197), rolling circle replication (Lizardi et al., U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to about 30 nucleotides in length and flank a region from about 50 to about 200 nucleotides in length. Under appropriate conditions and with appropriate reagents, such primers permit the amplification of a nucleic acid molecule comprising the nucleotide sequence flanked by the primers.

For *in situ* methods, RNA does not need to be isolated from the cells prior to detection. In such methods, a cell or tissue sample is prepared/processed using known histological methods. The sample is then immobilized on a support, typically a glass slide, and then contacted with a probe that can hybridize to RNA that encodes the marker.

In another embodiment of the present disclosure, a polypeptide corresponding to a marker is detected. A preferred agent for detecting a polypeptide of the disclosure is an antibody capable of binding to a polypeptide corresponding to a marker of the invention, preferably an antibody with a detectable label. Antibodies can be polyclonal, or more preferably, monoclonal. An intact antibody, or a fragment thereof (*e.g*., Fab or $F(ab')_2$) can be used.

A variety of formats can be employed to determine whether a sample contains a protein that binds to a given antibody. Examples of such formats include, but are not limited to, enzyme immunoassay (EIA), radioimmunoassay (RIA), Western blot analysis and enzyme linked immunoabsorbant assay (ELISA). A skilled artisan can readily adapt known protein/antibody detection methods for use in determining whether B cells express a marker of the present invention.

Another method for determining the level of a polypeptide corresponding to a marker is mass spectrometry. For example, intact proteins or peptides, *e.g*., tryptic peptides can be analyzed from a sample, *e.g*., a blood sample, a lymph sample or other sample, containing one or more polypeptide markers. The method can further include treating the sample to lower the amounts of abundant proteins, *e.g*., serum albumin, to increase the sensitivity of the method. For example, liquid chromatography can be used to fractionate the sample so portions of the sample can be analyzed separately by mass spectrometry. The steps can be performed in separate systems or in a combined liquid chromatography/mass spectrometry system (LC/MS, see for example, Liao, et al. (2004) Arthritis Rheum. 50:3792-3803). The mass spectrometry system also can be in tandem (MS/MS) mode. The charge state distribution of the protein or peptide mixture can be acquired over one or multiple scans and analyzed by statistical methods, e.g. using the retention time and mass-to-charge ratio (m/z) in the LC/MS system, to assess germinal center activity or capacity for adaptive immunity, including testing samples from patients responsive or non-responsive to proteasome inhibition and/or glucocorticoid therapy. Examples of mass spectrometers which can be used are an ion trap system (ThermoFinnigan, San Jose, CA) or a quadrupole time-of-flight mass spectrometer (Applied Biosystems, Foster City, CA). The method can further include the step of peptide mass fingerprinting, e.g. in a matrix-assisted laser desorption ionization with time-of-flight (MALDI-TOF) mass spectrometry method. The method can further include the step of sequencing one or more of the tryptic peptides. Results of this method can be used to identify proteins from primary sequence databases, e.g., maintained by the National Center for Biotechnology Information, Bethesda, MD, or the Swiss Institute for Bioinformatics, Geneva, Switzerland, and based on mass spectrometry tryptic peptide m/z base peaks.

Electronic Apparatus Readable Arrays

Electronic apparatus, including readable arrays comprising at least one predictive marker of the present invention is also contemplated for use in conjunction with the methods of the invention. As used herein, "electronic apparatus readable media" refers to any suitable medium for storing, holding or containing data or information that can be read and accessed directly by an electronic apparatus. As used herein, the term "electronic apparatus" is intended to include any suitable computing or processing apparatus or other device configured or adapted for storing data or information. Examples of electronic apparatus suitable for use with the present invention and monitoring of the recorded information include stand-alone computing apparatus; networks, including a local area network (LAN), a wide area network (WAN) Internet, Intranet, and Extranet; electronic appliances such as personal digital assistants (PDAs), cellular phone, pager and the like; and local and distributed processing systems. As used herein, "recorded" refers to a process for storing or encoding information on the electronic apparatus readable medium. Those skilled in the art can readily adopt any of the presently known methods for recording information on known media to generate manufactures comprising the markers of the present invention.

For example, microarray systems are well known and used in the art for assessment of samples, whether by assessment gene expression (*e.g.*, RNA detection, protein detection), or metabolite production, for example. Microarrays for use according to the invention include one or more probes of predictive marker(s) of the invention characteristic of response and/or non-response to a therapeutic regimen as described herein. In one embodiment, the microarray comprises one or more probes corresponding to one or more of markers selected from the group consisting of markers which demonstrate increased expression in short term survivors, and genes which demonstrate increased expression in long term survivors in patients. A number of different microarray configurations and methods for their production are known to those of skill in the art and are disclosed, for example, in U.S. Pat. Nos: 5,242,974; 5,384,261; 5,405,783; 5,412,087; 5,424,186; 5,429,807; 5,436,327; 5,445,93.4; 5,556,752; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,624,711; 5,700,637; 5,744,305; 5,770,456; 5,770,722; 5,837,832; 5,856,101; 5,874,219; 5,885,837; 5,919,523; 5981185; 6,022,963; 6,077,674; 6,156,501; 6261776; 6346413; 6440677; 6451536; 6576424; 6610482; 5,143,854; 5,288,644; 5,324,633; 5,432,049; 5,470,710; 5,492,806; 5,503,980; 5,510,270; 5,525,464; 5,547,839; 5,580,732; 5,661,028; 5,848,659; and 5,874,219; Shena, et al. (1998), Tibtech 16:301; Duggan et al. (1999) Nat. Genet. 21:10; Bowtell et al. (1999) Nat. Genet. 21:25; Lipshutz et al. (1999) Nature Genet. 21:20-24, 1999; Blanchard, et al. (1996) Biosensors and Bioelectronics, 11:687-90; Maskos, et al., (1993) Nucleic Acids Res. 21:4663-69; Hughes, et al. (2001) Nat. Biotechol. 19:342,2001. A tissue microarray can be used for protein identification (see Hans et al. (2004) Blood 103:275-282). A phage-epitope microarray can be used to identify one or more proteins in a sample based on whether the protein or proteins induce auto-antibodies in the patient (Bradford et al. (2006) Urol. Oncol. 24:237-242).

A microarray thus comprises one or more probes corresponding to one or more biomarkers identified herein, e.g., those indicative of ICS or germinal center activity. The microarray can comprise probes corresponding to, for example, at least 2, at least 5, at least 10, at least 15, at least 20, at least 25, at least 30, at least 35, at least 40, at least 45, at least 50, at least 75, or at least 100, biomarkers indicative of ICS or germinal center activity. The microarray can comprise probes corresponding to one or more biomarkers as set forth herein. Still further, the microarray may comprise complete marker sets as set forth herein and which may be selected and compiled according to the methods set forth herein. The microarray can be used to assay expression of one or more predictive markers or predictive marker sets in the array. In one example, the array can be used to assay more than one predictive marker or marker set expression in a sample to ascertain an expression profile of markers in the array. In this manner, up to about 44,000 markers can be simultaneously assayed for expression. This allows an expression profile to be developed showing a battery of markers specifically expressed in one or more samples. Still further, this allows an expression profile to be developed to assess capacity for adaptive immunity or degree of immunosuppression.

The array is also useful for ascertaining differential expression patterns of one or more markers in normal and affected (*e.g.*, blood, *e.g.*, B) cells. This provides a battery of predictive markers that could serve as a tool for ease of identification of subjects with affected adaptive immunity, *e.g.*, those with atrophic or hyperplastic germinal centers. Further, the array is useful for ascertaining expression of reference markers for reference expression levels. In another example, the array can be used to monitor the time course of expression of one or more bio markers in the array.

In addition to such qualitative determination, the invention allows the quantification of marker expression. Thus, predictive markers can be grouped on the basis of marker sets or short term or long term indications by the level of expression in the sample. This is useful, for example, in ascertaining the short term or long term indication of adaptive immunity in the sample by virtue of scoring the expression levels according to the methods provided herein.

The array is also useful for ascertaining the effect of the expression of a marker on the expression of other biomarkers in the same cell or in different cells. This provides, for example, a selection of alternate molecular targets for therapeutic intervention if a patient is predicted to be adversely affected, *e.g.*, immunosuppressed by the test agent.

Reagents and Kits

The invention also encompasses kits for detecting the presence of a polypeptide or nucleic acid corresponding to a marker of the invention in a biological sample (*e.g.* an immune system-associated body fluid such as a blood sample). Such kits can be used to assess capacity for adaptive immunity, *e.g.*, determine if a subject is at increased risk of developing immunosuppression or has increased adaptive immunity. For example, the kit can comprise a labeled compound or agent capable of detecting a polypeptide or a transcribed RNA corresponding to a marker of the invention in a biological sample and means for determining the amount of the polypeptide or RNA in the sample. Suitable reagents for binding with a marker protein include antibodies, antibody derivatives, antibody fragments, and the like. Suitable reagents for binding with a marker nucleic acid (*e.g.*, a genomic DNA, an mRNA, a spliced mRNA, a cDNA, or the like) include complementary nucleic acids. The kit can also contain a control or reference sample or a series of control or reference samples which can be assayed and compared to the test sample. For example, the kit may have a positive control sample, e.g., including one or more biomarkers described herein, or reference markers, e.g. housekeeping markers to standardize the assay among samples or timepoints. By way of example, the kit may comprise fluids (*e.g.*, buffer) suitable for annealing complementary nucleic acids or for binding an antibody with a protein with which it specifically binds and one or more sample compartments. The kit of the invention may optionally comprise additional components useful for performing the methods of the invention, *e.g.*, a sample collection vessel, *e.g.*, a tube, and optionally, means for optimizing the amount of biomarker detected, for example if there may be time or adverse storage and handling conditions between the time of sampling and the time of analysis. For example, the kit can contain means for increasing the number of B cells in the sample, as described above, a buffering agent, a preservative, a stabilizing agent or additional reagents for preparation of cellular material or probes for use in the methods provided; and detectable label, alone or conjugated to or incorporated within the provided probe(s). In one exemplary embodiment, a kit comprising a sample collection vessel can comprise *e.g.*, a tube comprising anti-coagulant and/or stabilizer, as described above, or known to those skilled in the art. The kit can further comprise components necessary for detecting the detectable label (*e.g.*, an enzyme or a substrate). For marker sets, the kit can comprise a marker set array or chip for use in detecting the biomarkers. Kits also can include instructions for interpreting the results obtained using the kit. The kit can contain reagents for detecting one or more biomarkers, *e.g.*, 2, 3, 4, 5, or more biomarkers described herein.

In one embodiment of the disclosure, the kit comprises a probe to detect at least one biomarker, e.g., a marker indicative of ICS (*e.g.*, a germline switch transcript, a circle transcript and a DNA recombination effector). In an exemplary embodiment of the disclosure, the kit comprises a probe to detect a biomarker selected from the group consisting of SEQ ID NOs: 1-21, 58, 59 and 60 (*i.e.*, to detect a biomarker selected from the group consisting of SEQ ID NOs:1, 2, 3, 4, 5, 6, 7, 8, 9, 10, 11, 12, 13, 14, 15, 16, 17, 18, 19, 20, 21, 58, 59 and 60). In preferred embodiments of the disclosure, the kit comprises a probe to detect a biomarker selected from the group consisting of GLT-$\mu$, AICDA (AID), CT-$\gamma$1&2, IGHG1 and/or IGHA2. In related embodiments of the disclosure, the kit comprises a nucleic acid probe comprising or derived from (*e.g.*, a fragment or variant (*e.g.*, homologous or complementary) thereof) a nucleic acid sequence selected from the group consisting of SEQ ID NOs:22-57. In preferred embodiments of the disclosure, the kit comprises a nucleic acid probe comprising or derived from (*e.g.*, a fragment or variant (*e.g.* homologous or complementary) thereof) a nucleic acid sequence selected from the group consisting of SEQ ID NOs: 53, 55, 34, 36, 35, 54, 37, 56, and 57.

For kits comprising nucleic acid probes, *e.g.*, oligonucleotide-based kits, the kit can comprise, for example: one or more nucleic acid reagents such as an oligonucleotide (labeled or non-labeled) which hybridizes to a nucleic acid sequence corresponding to a marker of the invention, optionally fixed to a substrate; labeled oligonucleotides not bound with a substrate, a pair of PCR primers, useful for amplifying a nucleic acid molecule corresponding to a marker of the invention, molecular beacon probes, a marker set comprising oligonucleotides which hybridize to at least two nucleic acid sequences corresponding to markers of the invention, and the like. The kit can contain an RNA-stabilizing agent.

For kits comprising protein probes, *e.g.,* antibody-based kits, the kit can comprise, for example: (1) a first antibody (*e.g.*, attached to a solid support) which binds to a polypeptide corresponding to a marker of the invention; and, optionally, (2) a second, different antibody which binds to either the polypeptide or the first antibody and is conjugated to a detectable label. The kit can contain a protein stabilizing agent. The kit can contain reagents to reduce the amount of non-specific binding of non-biomarker material from the sample to the probe. Examples of reagents include nonioinic detergents, non-specific protein containing solutions, such as those containing albumin or casein, or other substances known to those skilled in the art.

An isolated polypeptide corresponding to a predictive marker of the invention, or a fragment thereof, can be used as an immunogen to generate antibodies using standard techniques for polyclonal and monoclonal antibody preparation. For example, an immunogen typically is used to prepare antibodies by immunizing a suitable (*i.e.*, immunocompetent) subject such as a rabbit, goat, mouse, or other mammal or vertebrate. An appropriate immunogenic preparation can contain, for example, recombinantly-expressed or chemically-synthesized polypeptide. The preparation can further include an adjuvant, such as Freund's complete or incomplete adjuvant, or a similar immunostimulatory agent.

Antibodies include immunoglobulin molecules and immunologically active portions of immunoglobulin molecules, *i.e.*, molecules that contain an antigen binding site which specifically binds an antigen, such as a polypeptide of the invention, *e.g.*, an epitope of a polypeptide of the invention. A molecule which specifically binds to a given polypeptide of the invention is a molecule which binds the polypeptide, but does not substantially bind other molecules in a sample, *e.g.*, a biological sample, which naturally contains the polypeptide. Examples of immunologically active portions of immunoglobulin molecules include F(ab) and $F(ab')_2$ fragments which can be generated by treating the antibody with an enzyme such as pepsin. The disclosure provides polyclonal and monoclonal antibodies. Synthetic and genetically engineered variants (See U.S. Pat. No. 6,331,415) of any of the foregoing are also contemplated by the present disclosure. Polyclonal and monoclonal antibodies can be produced by a variety of techniques, including conventional murine monoclonal antibody methodology e.g., the standard somatic cell hybridization technique of Kohler and Milstein, Nature 256: 495 (1975) the human B cell hybridoma technique (see Kozbor et al., 1983, Immunol. Today 4:72), the EBV-hybridoma technique (see Cole et al., pp. 77-96 In Monoclonal Antibodies and Cancer Therapy, Alan R. Liss, Inc., 1985) or trioma techniques. See generally, Harlow, E. and Lane, D. (1988) Antibodies: A Laboratory Manual, Cold Spring Harbor Laboratory Press, Cold Spring Harbor, NY; and Current Protocols in Immunology, Coligan et al. ed., John Wiley & Sons, New York, 1994. Preferably, for diagnostic applications, the antibodies are monoclonal antibodies. Additionally, for use in in vivo applications the antibodies of the present invention are preferably human or humanized antibodies. Hybridoma cells producing a monoclonal antibody of the disclosure are detected by screening the hybridoma culture supernatants for antibodies that bind the polypeptide of interest, e.g., using a standard ELISA assay.

If desired, the antibody molecules can be harvested or isolated from the subject (*e.g.*, from the blood or serum of the subject) and further purified by well-known techniques, such as protein A chromatography to obtain the IgG fraction. Alternatively, antibodies specific for a protein or polypeptide of the invention can be selected or (*e.g.*, partially purified) or purified by, *e.g.*, affinity chromatography to obtain substantially purified and purified antibody. By a substantially purified antibody composition is meant, in this context, that the antibody sample contains at most only 30% (by dry weight) of contaminating antibodies directed against epitopes other than those of the desired protein or polypeptide of the invention, and preferably at most 20%, yet more preferably at most 10%, and most preferably at most 5% (by dry weight) of the sample is contaminating antibodies. A purified antibody composition means that at least 99% of the antibodies in the composition are directed against the desired protein or polypeptide of the invention.

An antibody directed against a polypeptide corresponding to a marker of the invention (*e.g.*, a monoclonal antibody) can be used to detect the marker (*e.g.*, in a cellular sample) in order to evaluate the level and pattern of expression of the marker. The antibodies can also be used diagnostically to monitor protein levels in tissues or body fluids (*e.g.* in a blood sample) as part of a clinical testing procedure, *e.g.*, to, for example, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of 125 131 35 3 suitable radioactive material include I, I, S or H.

Accordingly, in one aspect, the invention provides substantially purified antibodies or fragments thereof, and non-human antibodies or fragments thereof, which antibodies or fragments specifically bind to a polypeptide comprising an amino acid sequence encoded by a marker identified herein. The substantially purified antibodies of the invention, or fragments thereof, can be human, non-human, chimeric and/or humanized antibodies.

In another aspect, the invention provides non-human antibodies or fragments thereof, which antibodies or fragments specifically bind to a polypeptide comprising an amino acid sequence which is encoded by a nucleic acid molecule of a predictive marker of the invention. Such non-human antibodies can be goat, mouse, sheep, horse, chicken, rabbit, or rat antibodies. Alternatively, the non-human antibodies of the invention can be chimeric and/or humanized antibodies. In addition, the non-human antibodies of the invention can be polyclonal antibodies or monoclonal antibodies.

The substantially purified antibodies or fragments thereof may specifically bind to a signal peptide, a secreted sequence, an extracellular domain, a transmembrane or a cytoplasmic domain or cytoplasmic membrane of a polypeptide of the invention. The substantially purified antibodies or fragments thereof, the non-human antibodies or fragments thereof, and/or the monoclonal antibodies or fragments thereof, of the invention specifically bind to a secreted sequence or an extracellular domain of the amino acid sequences of the present invention.

The invention also provides a kit containing an antibody of the invention conjugated to a detectable substance, and instructions for use. Still another aspect of the invention is a diagnostic composition comprising a probe of the invention and a pharmaceutically acceptable carrier. In one embodiment, the diagnostic composition contains an antibody of the invention, a detectable moiety, and a pharmaceutically acceptable carrier.

Use of Information

The methods for processing approval of payment or processing of payment for a treatment regimen of a patient receiving an immunomodulator, *e.g.*, a regimen which increases or decreases ICS as described herein, include a step of reviewing the patient's biomarker expression levels, a further step of making a decision or advising on whether payment should be made for the treatment regimen based on the result of the evaluation, and a further step of transmitting or recording the decision or advice.

In one method, information, *e.g.*, about the patient's marker expression levels (*e.g.*, the result of evaluating a biomarker or biomarker set described herein), or about whether a patient is expected to have beneficial or detrimental effects on immunocompetence, is provided (*e.g.*, communicated, *e.g.*, electronically communicated) to a third party, *e.g.*, a hospital, clinic, a government entity, reimbursing party or insurance company (*e.g.*, a life insurance company). For example, choice of medical procedure, payment for a medical procedure, payment by a reimbursing party, or cost for a service or insurance can be function of the information. For example, the third party receives the information, makes a determination based at least in part on the information, and optionally communicates the information or makes a choice of procedure, payment, level of payment, coverage, etc. based on the information. In the method, the expression profile or trend in expression level of a biomarker or a biomarker set selected from or derived from the biomarkers described herein is determined.

In one embodiment, a premium for insurance (*e.g.*, life or medical) is evaluated as a function of information about one or more marker expression levels, *e.g.*, a biomarker or biomarker set, *e.g.*, a level of expression associated with have beneficial or detrimental effects on immunocompetence (*e.g.*, the trend in expression level or expression profile). For example, premiums can be increased (*e.g.*, by a certain percentage) if the biomarkers of a patient or a patient's biomarker set described herein are differentially expressed between an insured candidate (or a candidate seeking insurance coverage) and a reference value (*e.g.*, a non-afflicted person or nontreated person). Premiums can also be scaled depending on marker expression levels, *e.g.*, the result of evaluating a biomarker or biomarker set described herein. For example, premiums can be assessed to distribute risk, *e.g.*, as a function of marker expression levels, *e.g.*, the result of evaluating a biomarker or biomarker set described herein. In another example, premiums are assessed as a function of actuarial data that is obtained from patients that have beneficial or detrimental effects on immunocompetence.

Information about marker expression levels, *e.g.*, the result of evaluating a biomarker or biomarker set described herein (*e.g.*, the trend in expression level or expression profile), can be used, *e.g.*, in an underwriting process for life insurance. The information can be incorporated into a profile about a subject. Other information in the profile can include, for example, date of birth, gender, marital status, banking information, credit information, children, and so forth. An insurance policy can be recommended as a function of the information on marker expression levels, *e.g.*, the result of evaluating a predictive marker or predictive marker set described herein, along with one or more other items of information in the profile. An insurance premium or risk assessment can also be evaluated as function of the biomarker or biomarker set information. In one implementation, points are assigned on the basis of a result showing beneficial or detrimental effects on immunocompetence.

In one embodiment, information about marker expression levels, *e.g.*, the result of evaluating a biomarker or biomarker set described herein, is analyzed by a function that determines whether to authorize the transfer of funds to pay for a service or treatment provided to a subject (or make another decision referred to herein). For example, the results of analyzing a expression of a biomarker or biomarker set described herein may indicate that a subject has beneficial or detrimental effects on immunocompetence, suggesting that a treatment course is needed, thereby triggering an outcome that indicates or causes authorization to pay for a service or treatment provided to a subject. In one example, an expression profile or a trend in the expression level of a biomarker or a biomarker set selected from or derived from the biomarkers described herein is determined and payment is authorized if the expression profile or trend in expression level identifies a beneficial effect on immunocompetence. For example, an entity, *e.g.*, a hospital, care giver, government entity, or an insurance company or other entity which pays for, or reimburses medical expenses, can use the outcome of a method described herein to determine whether a party, *e.g.*, a party other than the subject patient, will pay for services (*e.g.*, a particular therapy) or treatment provided to the patient. For example, a first entity, *e.g.*, an insurance company, can use the outcome of a method described herein to determine whether to provide financial payment to, or on behalf of, a patient, *e.g.*, whether to reimburse a third party, *e.g.*, a vendor of goods or services, a hospital, physician, or other care-giver, for a service or treatment provided to a patient. For example, a first entity, *e.g.*, an insurance company, can use the outcome of a method described herein to determine whether to continue, discontinue, enroll an individual in an insurance plan or program, *e.g.*, a health insurance or life insurance plan or program.

In one aspect, the disclosure features a method of providing data. The method includes providing data described herein, *e.g.*, generated by a method described herein, to provide a record, *e.g.*, a record described herein, for determining if a payment will be provided. In some embodiments, the data is provided by computer, compact disc, telephone, facsimile, email, or letter. In some embodiments, the data is provided by a first party to a second party. In some embodiments, the first party is selected from the subject, a healthcare provider, a treating physician, a health maintenance organization (HMO), a hospital, a governmental entity, or an entity which sells or supplies the drug. In some embodiments, the second party is a third party payor, an insurance company, employer, employer sponsored health plan, HMO, or governmental entity. In some embodiments, the first party is selected from the subject, a healthcare provider, a treating physician, an HMO, a hospital, an insurance company, or an entity which sells or supplies the drug and the second party is a governmental entity. In some embodiments, the first party is selected from the subject, a healthcare provider, a treating physician, an HMO, a hospital, an insurance company, or an entity which sells or supplies the drug and the second party is an insurance company.

In another aspect, the disclosure features a record (*e.g.*, computer readable record) which includes a list and value of expression for the biomarker or biomarker set for a patient. In some embodiments, the record includes more than one value for each marker.

The present invention will now be illustrated by the following Examples, which are not intended to be limiting in any way

EXAMPLES

Example 1. Measurement of ICS in Mice

Biomarker transcripts were examined for their potential as surrogate measures of splenic germinal center atrophy upon treatment with a specific I Kappa B Kinase Beta (IKKβ) inhibitor, a beta-carboline. Splenic germinal center atrophy in mice can be due to inhibition of IKKβ in B lymphocytes, because targeted deletion of the IKKβ gene in B lymphocytes in mice induces a similar phenotype (Pasparakis et al. (2002) J. Exp. Med. 196:743-52, Li et al. (2003) J. Immunol. 170:4630-7, Ren et al. (2002) J. Immunol. 168:577-87). Expression of an endogenous reference transcript (18S), which is ubiquitously expressed and not regulated by the NF-κB pathway, also was measured, to allow normalization of the transcript data.

Female C57BL/6 mice (Charles River Laboratories, Bedford, MA) were used in these studies and treated with a beta-carboline, N-(6-chloro-7-methoxy-9H-beta-carbolin-8-yl)-2-methyl-nicotinamide (ML120B) following three different treatment regimens, A, B, and C. After the treatment, animals were euthanized and tissues were collected and frozen. Germinal centers were identified by immunohistochemisry (IHC) of cryosections (6 μm) using an antibody to mouse Bcl-6. T cells also were identified by IHC of serial cryosections, using an antibody to mouse CD3. The size and/or frequency of germinal centers and T cells were scored by a board-certified anatomic pathologist, using a 0 (low) to 4 (high) scale. Additionally, the samples were analyzed by qRT-PCR using the nucleic acid probes and primers listed in Table 1.

**Table 1 Mouse Quantitative Reverse-Transcriptase Polymerase Chain Reaction Reagent Sequences**

| Assay | Reference Sequence ID | Oligomer | Sequence |
|---|---|---|---|
| Mu-GLT | NC_000078 | Forward<br>Reverse | 5'-CTCTGGCCCTGCTTATTGTTG-3' SEQ ID NO:22<br>5'-GAAGACATTTGGGAAGGACTGACT-3' SEQ ID NO: 23 |
| G3-GLT | NC_000078 | Forward<br>Reverse | 5'-TGGGCAAGTGGATCTGAACA-3' SEQ ID NO:24<br>5'-CTCAGGGAAGTAGCCTTTGACA-3' SEQ ID NO:25 |
| G1-GLT | NC_000078 | Forward<br>Reverse | 5'-GGCCCTTCCAGATCTTTGAG-3' SEQ ID NO:26<br>5'-GGATCCAGAGTTCCAGGTCACT-3' SEQ ID NO:27 |
| GT-γ1&2 | NG_001019 | Forward<br>Reverse | 5'-GGGCTTCCAAGCCAACAGGGCAGGACA-3' SEQ ID NO:28<br>5'-GTTGCCGTTGGGGTGCTGGAC-3' SEQ ID NO:29 |

(continued)

| Assay | Reference Sequence ID | Oligomer | Sequence |
|---|---|---|---|
| AICDA-s | NM_009645.2 | Forward | 5'-GGCTGAGGTTAGGGTTCCATCTCAG-3' SEQ ID NO:30 |
| | | Reverse | 5'-GAGGGAGTCAAGAAAGTCACGCTGGA-3' SEQ ID NO:31 |
| 18S | X03205.1 | Forward | 5'-GTTAGCATGCCAGAGTCTCGTTCGTT-3' SEQ ID NO:32 |
| | | Reverse | 5'-CCGTTCTTAGTTGGTGG-3' SEQ ID NO:33 |
| Mu-GLT = Mu germline transcript; G1-GLT = Gamma 1 germline transcript; G3-GLT = Gamma 3 germline transcript; CT-$\gamma$1&2 = circle transcripts containing gamma 1 and 2 ; AICDA = activation-induced cytidine deaminase. | | | |

The following two paragraphs provide methods for qRT-PCR:

> Total ribonucleic acid (RNA) was isolated from 10 spleen cryosections, each 6 μM, using the RNEASY® Universal Tissue 8000 Kit (QIAGEN Inc., Valencia, CA) on a BIOROBOT® 8000 Workstation (QIAGEN INC., Valencia, CA), and deoxyribonucleic acidase (DNASE)-treated according to the manufacturer's protocol. The purity and yield of the RNA were assessed via spectrophotometry from 220 to 750 nm using the NANODROP® ND-1000 (NANODROP Technologies, Wilmington, DE). The integrity of the RNA was measured with the RNA 6000 NANO LABCHIP® Kit (AGILENT Technologies, Foster City, CA) on an AGILENT 2100 Bioanalyzer (AGILENT Technologies, Foster City, CA) and calculated using the RNA integrity number (RIN) algorithm (Schroeder et al. (2006) BMC Mol. Biol. 7:3). Deoxyribonucleic acidase-treated RNA was extracted from the samples and stored at -80°C. First strand complementary deoxyribonucleic acid (cDNA) synthesis was performed using a TAQMAN® Gold RT Master Mix (Applied Biosystems, Foster City, CA) according to the manufacturer's protocol, except that both oligothymidylic (oligo[dT]) and random hexamers were used for priming. The quality of synthesized cDNA was assessed by generating expression profiles of endogenous reference genes with a real-time polymerase chain reaction (PCR) system, the ABI PRISM® 7900HT Sequence Detection System (Applied Biosystems, Foster City, CA). Complementary deoxyribonucleic acid samples were stored at -20°C.

Sequences for all biomarkers (Table 1) were derived from mouse and human deoxyribonucleic acid (DNA) sequence (GENBANK® database [online] maintained by National Center for Biotechnology Information, Bethesda, MD). Quantitative reverse-transcriptase polymerase chain reaction assays were performed on an ABI PRISM® 7900HT Sequence Detection System (Applied Biosystems, Inc. (Foster City, CA) using a SYBR-green detection system (Qiagen Inc., Valencia, CA). All quantitative assays were run using universal thermal cycling parameters: hold at 95°C for 2 minutes to activate the DNA polymerase, then run 40 three-part cycles (95°C for 20 seconds, 58°C for 20 seconds, and 76°C for 20 seconds). Data were analyzed using sequence detection system (SDS) software, Version 1.7 (Applied Biosystems, Inc. (Foster City, CA). The first step was to generate an amplification plot for each sample, which showed the change in Rn ($\Delta$Rn) on the y-axis (where Rn is the fluorescence emission intensity of the reporter dye normalized to a passive reference) against the cycle number on the x axis. From each amplification plot, a threshold cycle (Ct) value was calculated. The Ct value is defined as the cycle at which a statistically significant increase in $\Delta$Rn is first detected and is displayed on the graph as the intercept point of the amplification plot and threshold. The Ct values were exported to an EXCEL® (MICROSOFT Corp., Redmond, WA) spreadsheet and relative expression (x) was calculated as shown in equation 2.

$$\text{EQUATION 2} \qquad X = \text{Power}(2,-Ct) \times 100{,}000{,}000$$

Mice were given a single oral dose of ML120B as indicated (n = 4/group) for 6 hours. The level of mouse mu heavy chain germline transcript was measured in RNA isolated from serial cryosections by qRT-PCR. The frequency of germinal centers and the level of mu germline transcripts decreased by 50% within the first 6 hours of exposure to ML120B, whereas the frequency of T cells remained unchanged (Figure 1).

Mice were given a single oral dose of ML120B as indicated (n = 4/group) for 18 hours. The level of mouse mu heavy chain germline transcript was measured in RNA isolated from serial cryosections by qRT-PCR. The frequency of germinal centers and the level of mu germline transcripts exhibited a dose-dependent decrease within the 18 hours of exposure to ML120B, with a 10-fold reduction in germinal centers and a 1000-fold reduction in mu germline transcripts occurring at 300 mg/kg (Figure 2). The frequency of T cells and level of 18S rRNA, in contrast, remained unchanged (Figure 2).

Mice were given twice daily oral dose of ML120B by gavage, as indicated (n = 4/group) for four days. The level of mouse mu, gamma 3 and gamma 1 heavy chain germline transcript, AICDA transcript, gamma 1 & 2 circle transcript and 18S rRNA were measured in RNA isolated from serial cryosections by qRT-PCR. The frequency of germinal centers and the level of mu, gamma 3 and gamma 1 heavy chain germline transcript, AICDA transcript, gamma 1 & 2 circle transcript exhibited a dose-dependent decrease to ML120B (Figure 3). Germinal centers expressing Bcl-6 decreased 10-fold at 300 mg/kg (Figure 3). Germline transcripts mu, gamma 3 and gamma 1 decreased from 10-1000-fold at 300 mg/kg (Figure 3). Transcripts encoding AICDA decreased 100-1000-fold at 300 mg/kg (Figure 3). Circle transcript gamma 1&2 decreased 10-fold at 100 mg/kg (Figure 3). The level of 18S rRNA, in contrast, remained unchanged (Figure 3).

Example 2 Measurement of ICS in Monkeys

A proprietary immunomodulator, Test Agent A, was used as a test agent in these studies. The effect of Test Agent A on lymphocyte transcripts (CD19, CD20, GLT-$\mu$, CT $\gamma$1&2, AID, TNF-$\alpha$, IL-1$\beta$, AND 18S) was measured using a well-established, robust transcript assay, the quantitative reverse-transcription polymerase chain reaction (qRT-PCR), which demonstrates excellent sensitivity and specificity.

**Materials and Methods**

Female cynomolgus monkeys (*Macaca fascicularis,* Charles River Laboratories (Sparks, NV; naive, nulliparous and non-pregnant, 2.0 to 4.0 kg) 4 animals per dosing group, 16 total). The monkeys were assigned to the study groups by weight-ordered distribution. Filtered tap water was available *ad libitum.*

The monkeys received Test Agent A, formulated in 0.1 M citrate buffer (pH 2.7 $\pm$ 0.05), in 10 ml/kg daily for 28 days. Citrate buffer in water (0.1 M; PH 2.7 $\pm$ 0.05) was the control used in this study. Both the Test Agent A and control articles were stored AT 5°C $\pm$ 3°C. The animals received Test Agent A or control formulation once daily for 28 days (dosing phase). Doses were based on the most recently recorded body weight. Animals were administered by oral gavage a single volume of 10 ml/kg at doses of 0 (vehicle control), 40, 60, or 100 mg/kg. Following each dose, any residual control/ Test Agent A remaining in the gavage tube was administered to the animal by administering 5 ml of tap water.

Once on Days -9, -2, 7, 14, 21, and 28, approximately 2.0 ml of peripheral blood from each animal was obtained via vein puncture and collected into PAXGENE® tubes (PREANALYTIX, Valencia, CA), following the manufacturer's instructions. Spleen specimens of all animals were flash frozen in liquid nitrogen at necropsy (Day 29). Blood samples and spleen specimens were stored at -70°C until shipped on dry ice to Millennium Pharmaceuticals, Inc. (Cambridge, MA) and stored at -80°C.

**Biomarker measurement**

Ribonucleic acid isolation Total ribonucleic acid (RNA) was isolated from 10 spleen cryosections, each 5 $\mu$M, using the RNEASY® Universal Tissue 8000 Kit (QIAGEN Inc., Valencia, CA) on a BIOROBOT® 8000 Workstation (QIAGEN INC., Valencia, CA), and deoxyribonucleic acidase (DNASE)-treated according to the manufacturer's protocol. Total RNA extraction from blood was conducted using the PAXGENE® 96 Blood RNA Kit (PREANALYTIX, Valencia, CA) according to the manufacturer's protocol. The purity and yield of the RNA were assessed via spectrophotometry from 220 to 750 nm using the NANODROP® ND-1000 (NANODROP Technologies, Wilmington, DE). The integrity of the RNA was measured with the RNA 6000 NANO LABCHIP® Kit (AGILENT Technologies, Foster City, CA) on an AGILENT 2100 Bioanalyzer (AGILENT Technologies, Foster City, CA) and calculated using the RNA integrity number (RIN) algorithm (Schroeder et al, 2006). Deoxyribonucleic acidase-treated RNA was extracted from the samples and stored at -80°C. First strand complementary deoxyribonucleic acid (cDNA) synthesis was performed using a TAQMAN® Gold RT Master Mix (Applied Biosystems, Foster City, CA) according to the manufacturer's protocol, except that both oligothymidylic (oligo[dT]) and random hexamers were used for priming. The quality of synthesized cDNA was assessed by generating expression profiles of endogenous reference genes with a real-time polymerase chain reaction (PCR) system, the ABI PRISM® 7900HT Sequence Detection System (Applied Biosystems, Foster City, CA). Complementary deoxyribonucleic acid samples were stored at -20°C.

Quantitative reverse-transcriptase polymerase chain reaction reagent design and validation Sequences for the reagents (Table 2) were derived from the human deoxyribonucleic acid (DNA) sequence (GENBANK® database [online] maintained by National Center for Biotechnology Information, Bethesda, MD).

TABLE 2 Custom quantitative reverse-transcriptase polymerase chain reaction reagent sequences

| Biomarker | Reference Sequence Identifier | Oligomer | Sequence | SEQ ID NO: |
|---|---|---|---|---|
| GLT-μ | NG_001019 SEQ ID NO: | Forward | CCTGAATTA*TTTCAGTTAAGCATGT | 34 |
| | | Reverse | CCTCGTCTCCTGTGAGAAT | 35 |
| | | Probe | CTCCATCA*CTTTCTCC | 36 |
| CT-γ1&2 | NG_001019 SEQ ID NO: | Forward | CAACAGGGCAGGACAC | 37 |
| | | Reverse | CCTCGTCTCCTGTGAGAAT | 35 |
| | | Probe | CTCCATCA*CTTTCTCC | 36 |
| IL-1β | NM_000576.2 SEQ ID NO: | Forward | GAGGATCTCCTGTCCATCAG | 38 |
| | | Reverse | CCAAATGTGGCCGTGGTTTCTGTCA | 39 |
| | | Probe | TCACCTCTCCTACTCACT | 40 |
| TNF-α | NM_000594 SEQ ID NO: | Forward | CTAGAAATTGACACAAGTGGACCTT | 41 |
| | | Reverse | CCCGGTCTCCCAAATAAATACATTC | 42 |
| | | Probe | GCCTTCCTCTCTCCA* | 43 |
| CD19 | NM_001770.4 SEQ ID NO: | Forward | CTGTGACTTTGGCTTATCTGATCT | 44 |
| | | Reverse | GCGTCACTTTGAAGAATCTCCTGGT | 45 |
| | | Probe | GCCTGTGTTCCCTTGTG | 46 |
| CD20 | NM_021950.3 SEQ ID NO: | Forward | CCAATGAAAGGCCCTATTGCTAT | 47 |
| | | Reverse | CAGTGAAGACATCCTCCTGAAGAGT | 48 |
| | | Probe | AATCTGGTCCA*AAAC | 49 |
| 18S | X03205.1 SEQ ID NO: | Forward | G(A*)CACGG(A*)CAGG(A*)TTGACAGATTGATAG | 50 |
| | | Reverse | GTT(A*)GCATGCCAGAGTCTCGTTCGTT | 51 |
| | | Probe | CCGTTCTT(A*)GTTGGTGG | 52 |
| AID | NM_020661.1 SEQ ID NO: | AOD[a] | ABI CATALOGUE NO. HS00757808_M1 | |
| IGL-κ | BC070336.1 SEQ ID NO: | AOD[a] | ABI CATALOGUE NO. HS00736177_M1 | |

GLT-μ = Germline transcript mu; CT-γ1&2 = circle transcripts containing gamma 1 and 2; IL-1β = Interleukin-1 beta; TNF-α = tumor necrosis factor alpha; IGL-κ = immunoglobulin light chain kappa; AOD = assay on demand.
* = SUPER™ Modified Base Technology from Nanogen (Bothell, WA).
[a]: assays on demand from Applied Biosystems, Inc. (Foster City, CA).

Probes were MGB ECLIPSE™ Probes (NANOGEN, Bothell, WA). Each primer and probe pair were synthesized by NANOGEN (Bothell, WA) and were validated by measuring PCR efficiency using synthetic templates and human cDNA standards. Validated assays demonstrated linear amplification and >99% efficiency over 7 orders of magnitude (data not shown). Validated primer and probe pairs were sent to Millennium Pharmaceuticals, Inc. (Cambridge, MA) and were revalidated on the human positive control cDNA standard prior to use in expression profiling studies (data not shown). The IGL-κ and AID assays used TAQMAN® reagents numbers Hs00736177_ml and Hs00757808_ml, respectively (Applied Biosystems, Inc. (Foster City, CA).

Quantitative reverse-transcriptase polymerase chain reaction assay

Quantitative reverse-transcriptase polymerase chain reaction assays were performed on an ABI PRISM® 7900HT Sequence Detection System (Applied Biosystems, Inc. (Foster City, CA). All quantitative assays designed using NANOGEN (Bothell, WA) guidelines were run using universal thermal cycling parameters: hold at 95°C for 2 minutes to activate the DNA polymerase, then run 40 three-part cycles (95°C for 20 seconds, 58°C for 20 seconds, and 76°C for 20 seconds).

**Data Analysis**

Raw Data Analysis Data were analyzed using sequence detection system (SDS) software, Version 1.7 (Applied Biosystems, Inc. (Foster City, CA). The first step was to generate an amplification plot for each sample, which showed the change in Rn (ΔRn) on the y-axis (where Rn is the fluorescence emission intensity of the reporter dye normalized to a passive reference) against the cycle number on the x axis. From each amplification plot, a threshold cycle (Ct) value was calculated. The Ct value is defined as the cycle at which a statistically significant increase in ΔRn is first detected and is displayed on the graph as the intercept point of the amplification plot and threshold. The Ct values were exported to an EXCEL® (MICROSOFT Corp., Redmond, WA) spreadsheet and relative expression (x) was calculated as shown in Equation 2 (see Example 1).

Statistical Analyses For each transcript examined in the blood, each animal's baseline was defined as the average of the Day -9 and Day -2 cycle numbers. The animal's baseline was subtracted from the animal's cycle number at each postdose time point. This difference was analyzed using a repeated measures linear regression model that included independent variables for the animal's baseline cycle number, dosage of the Test Agent A, time point (days postdose) at which the blood sample was taken, and the interaction between dose and time. In models where the dose-by-time interaction had a p-value greater than 0.05, the interaction term was removed from the model and the differences among doses were evaluated.

The model employed is shown in equation 3.

$$\text{Equation 3.} \quad y_{itd} - y_{it0} = y_{it0} + Treat_t + Day_d + (Treat*Day)_{td} + e_{itd}$$

where: $y_{itd}$ = Cycle number for animal i at treatment t and day d; $y_{it0}$ = Cycle number for animal i and treatment t at baseline (d = 0); $Treat_t$ = t level of treatment (vehicle, 40, 60 or 100 mg per kg of Test Agent); $Day_d$ = d level of day (7, 14, 21, or 28 days); and $(Treat*Day)_{td}$ = interaction between treatment and day (This tested the null hypothesis that there was no significant difference in the trend over time among the treatment groups); and $e_{itd}$ = unexplained error incorporating correlation from repeated readings over time on same animal.

For each transcript examined in the spleen at Day 29, a Kruskal-Wallis test was performed to assess if there were any significant differences among any of the Test Agent A dose groups with respect to the transcript cycle number. All hypotheses were tested at a type I error rate of 0.05 (2-sided). Since this was an exploratory analysis, no adjustments for multiple comparisons were made to account for the number of transcripts and tissue types examined.

Histology Hematoxylin and Eosin (H&E)-stained slides were prepared from spleen cryosections (5 μm) adjacent to those used for genomic analyses. Reactivity (hyperplasia) of spleen sections was assessed independently by 2 blinded reviewers, one of whom was a board-certified human anatomic pathologist. Reactivity was scored on a zero (minimal) to 4 (maximal) scale, based on measurements of the frequency and hyperplastic appearance of germinal centers in five 40 x fields. These analyses exhibited a Pearson correlation coefficient value ($r^2$) of 0.9, indicating that the analyses were consistent with each other.

Results and Discussion

General Immunology Assessment Histopathology of secondary lymphoid organs showed dose-dependent atrophy of GCs by the Test Agent A (see Figure 4). Test Agent A decreases follicular dendritic cells and inhibits expansion of B cells. There was no change in frequency of B or T cells in secondary lymphoid organ. There was more than 100-fold decrease in proliferating B cells and more than 100-fold decrease in follicular dendritic cells. Test Agent A increases the relative frequency of immature B cells in secondary lymphoid organ as seen by immunofluorescence. Test Agent A decreases the frequency of mature B cells in the secondary lymphoid organ. Image analysis of immunoflourescence-stained IgG+ germinal centers in Cyno spleens showed a dose-dependent decrease in the frequency of IgG+ germinal centers (Figure 5).

Test Agent A decreased the frequency of post-germinal center B cells in peripheral blood. As seen by flow cytometry of peripheral blood, there was no change in the frequency of total B cells. (Figure 6A). However, there was a decrease in post-germinal center (GC) B cells (Figure 6B) with a concomitant increase in pre-GC B cells (Figure 6C).

Histologic analysis of spleen cryosections Analysis of H&E sections from the spleen of each animal by 2 independent, blinded reviewers confirmed that germinal center atrophy had been achieved in this study. (See Figure 4) The vehicle control group (animal nos. 1101 through 1104) was completely homogenous, with each animal exhibiting highly reactive germinal centers and no indication of atrophy. The 40 mg/kg dose group (animal nos. 2101 through 2104) exhibited marked heterogeneity, with one animal exhibiting low germinal center reactivity, another exhibiting moderate reactivity, and 2 others exhibiting high reactivity. The 60 mg/kg dose group (animal nos. 3101 through 3104) was relatively homogeneous, with 3 animals exhibiting low reactivity and one exhibiting high reactivity. The 100 mg/kg dose group (animal nos. 4101 through 4104) was completely homogenous, with all 4 animals exhibiting low germinal center reactivity. A subset of animals in the 100 mg/kg group also exhibited symptoms of bacterial infection. The quantitative analyses of the 2 independent, blinded reviewers were consistent with one another ($r^2 = 0.9$), which indicates the heterogeneity observed between subjects was not due to variability in the analyses, but to inherent differences in the tissues. Analysis of the data by dose illustrated a statistically significant ($p < 0.05$) decrease in the reactivity of germinal centers in the 100 mg/kg dose group as compared to the vehicle control group.

Quantitative reverse-transcriptase polymerase chain reaction analysis of spleen cryosections Isolated RNA from cryosections bordering the H&E sections (i.e., serial sections) was analyzed with qRT-PCR transcript assays to determine if the expression profiles of these transcripts (18S, TNF-α, IL-1β, CD20, CD19, IGL-κ, GLT-μ, AID, and CT-γ1&2) reflected germinal center atrophy. There was a dose-response trend in the spleen transcripts, as transcript numbers for all genes examined decreased with higher doses of Test Agent A, with the largest differences observed for AID and TNF-α (Table 3) illustrates the values for the control (vehicle) animals and the 100 mg/kg-treated animals).

Table 3. Expression results after 28 days of treatment with Test Agent A

| Biomarker | Vehicle | | | 100 mg/kg | | |
|---|---|---|---|---|---|---|
| | Expression | St. Dev | T-test | Expression | St. Dev | T-test |
| CT gamma 1 and 2 | 20.99 | 11.39 | 1 | 3.84 | 2.08 | 0.025 |
| GLT-mu | 17.06 | 9.79 | 1 | 4.12 | 2.11 | 0.042 |
| AID | 0.0234 | 0.015 | 1 | 0.00048 | 0.00035 | 0.023 |
| TNF-alpha | 0.0986 | 0.072 | 1 | 0.0063 | 0.0037 | 0.044 |
| IL-1beta | 0.192 | 0.081 | 1 | 0.0398 | 0.030 | 0.013 |
| CD 19 | 0.045 | 0.0091 | 1 | 0.0179 | 0.0088 | 0.0050 |
| CD20 | 14.8 | 5.73 | 1 | 2.75 | 1.46 | 0.0064 |
| IgL-kappa | 46.6 | 49.0 | 1 | 3.44 | 2.56 | 0.13 |
| 18S RNA | 23252 | 4759 | 1 | 14343 | 3569 | 0.024 |

With the exception of CD 19, Kruskal-Wallis analyses of all transcripts showed significant ($p < 0.05$) differences among the treatment groups (Table 4).

TABLE 4 Kruskal-Wallis results for transcript levels in the spleen

| Protein | Kruskal-Wallis Test Overall p-Value[a] | Median Ct[b] | |
|---|---|---|---|
| | | 0 mg/kg | 100 mg/kg |
| 18S | 0.0060 | 12.03 | 12.73 |
| TNF-α | 0.0037 | 29.47 | 34.37 |
| IL-1β | 0.0086 | 28.86 | 31.71 |
| CD20 | 0.0151 | 22.55 | 25.09 |
| CD19 | 0.0905 | 31.18 | 32.30 |
| IGL-κ | 0.0415 | 20.79 | 24.93 |
| GLT-μ | 0.0086 | 22.56 | 24.29 |
| AID | 0.0143 | 32.18 | 38.78 |

(continued)

| Protein | Kruskal-Wallis Test Overall p-Value[a] | Median Ct[b] | |
|---|---|---|---|
| | | 0 mg/kg | 100 mg/kg |
| CT-γ1&2 | 0.0068 | 22.70 | 24.83 |

[a]: Overall p-value tested the hypothesis that there were no differences among treatment group medians.
[b]: Median data for 0 and 100 mg/kg dose groups included to illustrate significant differences

A large portion of the significance observed for each gene was attributed to the differences between the vehicle control group and the 100 mg/kg dose group (Table 4). These analyses also suggested a significant dose-dependent effect on 18S, the transcript used as an internal reference. This effect was small (<2-fold effect) and it is unknown whether it represented a biological effect or was a technical artifact. These data were nonetheless reanalyzed after normalizing the data for differences in 18S values, an endogenous reference transcript, and the conclusions remained unchanged (Table 5). The expression profiles of these transcripts correlated positively with histologic metrics of reactivity.

Table 5 Kruskal-Wallis results for transcript levels in spleen (data normalized to 18S)

| Protein | Kruskal-Wallis Test Overall p-Value[a] | Median delta Ct[b] | |
|---|---|---|---|
| | | 0 mg/kg | 100 mg/kg |
| 18S | --- | --- | --- |
| TNF-α | 0.0041 | 17.32 | 21.70 |
| IL-1β | 0.0257 | 16.98 | 18.98 |
| CD20 | 0.0496 | 10.69 | 12.46 |
| CD19 | 0.3314 | 19.03 | 19.67 |
| IGL-κ | 0.0506 | 8.77 | 12.30 |
| GLT-μ | 0.0127 | 10.49 | 11.56 |
| AID | 0.0136 | 20.24 | 25.81 |
| CT-γ1&2 | 0.0083 | 10.63 | 12.10 |

[a]: Overall p-value tested the hypothesis that there were no differences among treatment group medians.
[b]: Data were normalized to a conventional internal reference transcript, 18S (assumes that 18S is not regulated by Test Agent A). Median delta Ct = median Ct value of Protein X - Median Ct value of 18S for a given sample. Median data for 0 and 100 mg/kg dose groups included to illustrate significant differences.

The Test Agent A exhibited a slight dose-dependent effect on one immunohistologic marker of B cells, CD20, but not another, CD19, in the spleen. The effect on CD20 was small and, while statistically significant, was not reproduced in peripheral blood samples; the Test Agent A did not significantly affect CD20 levels in peripheral blood. The overall cellularity of the spleen (e.g., organ weights, histology) did not change in a dose-dependent manner. Additional immunohistochemistry analyses that focused on the content of B cells within cryosections from spleen samples indicated that the frequency of B cells expressing CD20 protein did not differ between dose groups (data not shown). These additional data suggest that the small differences observed in CD20 transcripts in the spleen are not systemic effects and are unlikely to be of biological significance.

Quantitative reverse-transcriptase polymerase chain reaction analysis of peripheral blood Changes in the levels of IGL-κ, GLT-μ, AID, and CT-γ1&2 transcripts in peripheral blood were also significantly dose-dependent, with transcript levels decreasing with higher doses of the Test Agent A (Table 6; treatment p-value).

Table 6 Kruskal-Wallis results for transcript levels in blood

| Protein | Time-by-Treatment Interaction p-Value[a] | Treatment p-Value[a] |
|---|---|---|
| 18S | NS | 0.3064 |
| TNF-α | NS | 0.2499 |

(continued)

| Protein | Time-by-Treatment Interaction p-Value[a] | Treatment p-Value[a] |
|---|---|---|
| IL-1β | NS | 0.5528 |
| CD20 | NS | 0.0954 |
| CD19 | NS | 0.2820 |
| IGL-κ | NS | 0.0007 |
| GLT-μ | NS | 0.0051 |
| AID | NS | 0.0335 |
| CT-γ1&2 | NS | 0.0212 |
| NS = not significant.<br>[a]: Overall p-value tested the hypothesis that there were no differences among treatment group medians. Dunn's Post Test compared each treated group to the vehicle. | | |

A large portion of the significance observed for each gene was attributed to the differences between the vehicle control group and the 100 mg/kg dose group. The other Test Agent A-treated groups did not differ greatly from the vehicle control group. There was considerable variability among animals within treatment groups (Figure 7A-D illustrate results for GLT-mu (ST1)). The repeated measures linear model indicated that there were no significant differences in the trends over time among the treatment groups for any of the transcripts (Table 6, time-by-treatment interaction p-value).

Comparison of histology and quantitative reverse-transcriptase polymerase chain reaction data Comparisons of histology and spleen qRT-PCR data demonstrated that levels of IGL-κ, GLT-μ, AID, and CT-γ1&2 transcripts are predictive of germinal center reactivity. Analyses of data from individual animals highlighted positive correlations between histologic germinal center reactivity data and levels of these transcripts. These positive correlations were not evident in analyses of the data by dose group, particularly in the 40 and 60 mg/kg dose groups, where interanimal variability was greatest. Histology indicated that animal nos. 1101, 1102, 1103, 1104, 2102, 2103, and 3101 exhibited high germinal center reactivity, while animal nos. 2101, 2104, 3102, 3103, 3104, 4101, 4102, 4103, and 4104 exhibited low germinal center reactivity. Analyses of data for all transcripts, from all spleens revealed that IGL-κ, GLT-μ, AID, and CT-γ1&2 exhibited a similar pattern. The No Observable Effect Level (NOEL) was determined to be 60 mg/kg, on the basis of the changes in these novel transcript biomarkers, which are considered nonadverse. This NOEL agrees with that determined by histologic assays. Infection was considered an adverse effect that defined the No Observable Adverse Effect Level (NOAEL) in previous investigations. Infections were not observed in this investigation.

More importantly, levels of IGL-κ, GLT-μ, AID, CT-γ1&2, CD19, and CD20 transcripts in peripheral blood exhibited the highest correlations with germinal center reactivity data from histology (Table 7).

Table 7 Comparison ($r^2$) of slopes of blood transcript levels to histology reactivity scores

| Blood Transcript | $R^2$ Value |
|---|---|
| IGL-κ | 0.65 |
| GLT-μ, | 0.42 |
| AID | 0.56 |
| CT-γ1&2 | 0.54 |
| CD19 | 0.63 |
| CD20 | 0.41 |
| TNF-α | 0.01 |
| IL-1β | 0.01 |
| 18S | 0.13 |

These correlations are most evident from analyses of individual animals and not by dose group. Transcript levels of IGL-κ, GLT-μ, AID, and CT-γ1&2 from the blood of individual animals were examined within dose groups. The linear slope of transcript level versus time was calculated, in order to control for differences in the magnitude of expression between animals and for variability in measurements at a specific time point. A drug effect could manifest itself as a decrease in transcript levels with time, yielding a negative slope value, the magnitude of which would be proportional to the magnitude of the decrease. If there were no drug effect, there would not be a change in transcript levels with time, yielding a relatively flat slope with a value approaching zero. The calculated slopes correlated with qRT-PCR and histology data from the spleens of individual animals. Animal no. 2101, for example, exhibited low germinal center reactivity values as determined by histology, splenic qRT-PCR, and blood qRT-PCR, and these values were comparable to those in the 100 mg/kg dose group (animal nos. 4101, 4102, 4103, and 4104). The magnitude of the decrease of blood switch transcripts over time (i.e. slope of days -2 to 28) predicts animals with normal versus atrophic germinal centers on day 29.

Animals were classified as normal or abnormal (i.e., potentially atrophic), with respect to the reactivity of their germinal centers, based on the slope values of the blood transcript levels. Peripheral blood biomarkers which are only expressed by B lymphocytes (IGL-κ, GLT-μ, AID, and CT-γ1&2), exhibited the strongest associations with the histology data (Table 8).

Table 8 Potential accuracy of blood transcript biomarkers for predicting splenic germinal center atrophy

| Blood Transcript | Classification | Potential Accuracy at Predicting Splenic Atrophy |
|---|---|---|
| IGL-κ | B cell-specific | 100% |
| GLT-μ, | B cell-specific | 88% |
| AID | B cell-specific | 88% |
| CT-γ1&2 | B cell-specific | 88% |
| CD19 | B cell-specific | 75% |
| CD20 | B cell-specific | 75% |
| TNF-α | ubiquitous | 62% |
| IL-1β | ubiquitous | 62% |
| 18S | ubiquitous | 50% |

The slope values of the blood IGL-κ transcripts exhibited the highest predictive potential, as they correctly designated all subjects as exhibiting normal or abnormal splenic germinal center reactivity (Table 9). The slope values of the blood GLT-μ, AID, and CT-γ1&2 transcripts were less correlative and somewhat less predictive, each classifying 88% of the subjects correctly (Table 10, Table 11, and Table 12 respectively).

Table 9 Use of the slope of blood IGL-κ levels over time at predicting reactivity (e.g., atrophy) of germinal centers in spleen

| Blood IGL-κ Slope | Animal No. | Spleen Histology Assessment |
|---|---|---|
| y = 0.0037x | 1104 | Normal |
| y = 0.0043x | 2103 | Normal |
| y = 0.007x | 2102 | Normal |
| y = -0.0156x | 1102 | Normal |
| y = -0.0342x | 1103 | Normal |
| y = 0.038x | 3101 | Normal |
| y = 0.061x | 1101 | Normal |
| y = -0.0666x | 3102 | Abnormal |
| y = -0.1003x | 2104 | Abnormal |
| y = -0.1102x | 4101 | Abnormal |
| y = -0.119x | 2101 | Abnormal |
| y = -0.1295x | 3104 | Abnormal |

(continued)

| Blood IGL-κ Slope | Animal No. | Spleen Histology Assessment |
|---|---|---|
| y = -0.2419x | 4104 | Abnormal |
| y = -0.2911x | 3103 | Abnormal |
| y = -0.2911x | 4102 | Abnormal |
| y = -0.3127x | 4103 | Abnormal |

Table 10 Use of the slope of blood GLT-μ levels over time at predicting reactivity (e.g., atrophy) of germinal centers in spleen

| Blood GLT-μ Slope | Animal No. | Spleen Histology Assessment |
|---|---|---|
| y = -0.0101x | 3101 | Normal |
| y = 0.0375x | 1101 | Normal |
| y = -0.0377x | 2102 | Normal |
| y = -0.0572x | 2103 | Normal |
| y = -0.0649x | 3104 | Abnormal |
| y = -0.0731x | 1104 | Normal |
| y = -0.0829x | 1103 | Normal |
| y = -0.099x | 2101 | Abnormal |
| y = -0.1007x | 4101 | Abnormal |
| y = -0.1093x | 4103 | Abnormal |
| y = -0.1214x | 1102 | Normal |
| y = -0.1558x | 3102 | Abnormal |
| y = -0.1692x | 2104 | Abnormal |
| y = -0.231x | 4104 | Abnormal |
| y = -0.2364x | 3103 | Abnormal |
| y = -0.2364x | 4102 | Abnormal |

Table 11 Use of the slope of blood AID levels over time at predicting reactivity (e.g., atrophy) of germinal centers in spleen

| Blood AID Slope | Animal No. | Spleen Histology Assessment |
|---|---|---|
| y = 0.0127x | 2102 | Normal |
| y = 0.0254x | 3101 | Normal |
| y = -0.0798x | 1104 | Normal |
| y = -0.07x | 2103 | Normal |
| y = -0.0908x | 1103 | Normal |
| y = 0.1081x | 1101 | Normal |
| y = -0.1267x | 3104 | Abnormal |
| y = -0.1304x | 1102 | Normal |
| y = -0.154x | 4101 | Abnormal |
| y = -0.1681x | 2101 | Abnormal |

(continued)

| Blood AID Slope | Animal No. | Spleen Histology Assessment |
|---|---|---|
| y = -0.1701x | 2104 | Abnormal |
| y = -0.1824x | 3102 | Abnormal |
| y = -0.2741x | 4103 | Abnormal |
| y = -0.2755x | 3103 | Abnormal |
| y = -0.2755x | 4102 | Abnormal |
| y = -0.3143x | 4104 | Abnormal |

Table 12 Use of the slope of blood CT-γ1&2 levels over time at predicting reactivity (e.g., atrophy) of germinal centers in spleen

| Blood CT-γ1&2 Slope | Animal No. | Spleen Histology Assessment |
|---|---|---|
| y = -0.0385x | 1101 | Normal |
| y = -0.0473x | 2103 | Normal |
| y = -0.0507x | 1104 | Normal |
| y = -0.0519x | 3101 | Normal |
| y = -0.0698x | 2102 | Normal |
| y = -0.0953x | 4101 | Abnormal |
| y = -0.0958x | 1103 | Normal |
| y = -0.1037x | 3104 | Abnormal |
| y = -0.1138x | 2101 | Abnormal |
| y = -0.1156x | 1102 | Normal |
| y = -0.1593x | 3102 | Abnormal |
| y = -0.1737x | 4103 | Abnormal |
| y = -0.1852x | 2104 | Abnormal |
| y = -0.2397x | 4104 | Abnormal |
| y = -0.2685x | 3103 | Abnormal |
| y = -0.2685x | 4102 | Abnormal |

The next most predictive subcategory of biomarkers included those which are only expressed by B lymphocytes (CD19 and CD20). CD19 and CD20 were less predictive than IGL-κ, GLT-μ, AID, and CT-γ1&2, each having correctly classified 75% of the animals (Table 8).

A third biomarker subcategory includes those whose expression is associated with NF-κB activity and which are ubiquitously expressed (TNF-α and IL-1β). These two transcripts were less predictive than CD19 and CD20, each having correctly classified 62% of the animals (Table 8).

The last biomarker studied, 18S, a non-mechanistic and ubiquitous biomarker, was not expected to be predictive. It correctly classified 50% of the animals as normal or abnormal, which is no better than random chance (Table 8).

Conclusion

Several biomarker transcripts (IGL-κ, GLT-μ, AID, and CT-γ1&2) were identified in peripheral blood to be predictive of splenic germinal center atrophy caused by exaggerated doses of Test Agent A within the context of this study. The NOEL was determined to be 60 mg/kg on the basis of changes in these novel transcript biomarkers, which are considered nonadverse. This NOEL agrees with that determined by histologic assays. Infection is considered an adverse effect that defined the noael in previous investigations. Infections were not observed in this investigation.

Example 3 Measurement of ICS in Peripheral Human Blood

Peripheral blood was collected from healthy human volunteers and utilized as a surrogate tissue to detect splenic germinal center atrophy. Samples were collected from 19 donors at 4 timepoints each (two weeks apart), the RNA was isolated as described for the monkey assays and the expression of ICS markers, *e.g.,* GLT-μ and circle transcripts CT-γ1&2 (using GLT1, GLT2, GLT3, CT1, CT2, and CT3), was determined. The detection reagents were designed to detect consensus sequences between human and monkey (see Table 13 for sequences). All the markers were detected in peripheral blood (Figures 8A and B). A relative expression level shows a general low level of transcripts. Since the volunteers generally were healthy, there was no expectation of differences between timepoints or between volunteers. Therefore, in order to show that differences could be detected, the samples were modified to increase the likelihood of detecting switch transcripts. Plasmablasts were enriched in the samples by negative selection of spinning through a polysaccharide cushion, then assayed or further selected by positive selection using magnetic beads conjugated to a CD138-detecting reagent (Miltenyi Biotec, Auburn, CA). The result is shown in Figure 9. The level of ICS transcript (circle transcript CT-γ1&2) is proportional to the frequency of human plasmablasts in whole blood sample.

Example 4 Measurement of ICS in Monkey

Examples 1-3 demonstrated that switch transcripts could be detected and changes in their levels could be measured in peripheral blood of mice, cynomolgous macaques and normal human subjects. Example 2 also demonstrated that levels of switch transcripts in peripheral blood reflected the degree of follicular germinal center atrophy in spleens of cynomolgous macaques. The goals of the current study were to 1) validate these previous observations in the context of a 13-week study. 2) assess if levels of switch transcripts in peripheral blood could also serve as antecedent markers of clinical symptoms of bacterial infection.

Both male and female cynomolgus monkeys (*Macaca fascicularis,* Charles River Laboratories (Sparks, NV; naive, nulliparous and non-pregnant, 2.0 TO 4.0 kg, 40 total). The monkeys were assigned to the study groups by weight-ordered distribution. Filtered tap water was available *ad libitum.*

The monkeys received Test Agent A, formulated, stored and administered as in Example 2, or control once daily for 13 weeks (91 days). Doses were 0, 20, 40, or 80 mg/kg.

Peripheral blood samples were collected using the same procedure as in Example 2. Samples were obtained prior to initiation of dosing (Weeks -2 and -1) and during Weeks 1, 2, 3, 4, 13, and 17 (end of recovery period). Blood samples were stored at - 70°C until shipped on dry ice to Millennium Pharmaceuticals, Inc. (Millennium, Cambridge, MA) and stored at -80°C.

Spleen biopsy specimens were collected from all euthanized animals. Thirty-two animals (4/sex/group) were euthanized one day after the last dose (Day 92) and 8 animals (four controls and four 80 mg/kg animals, 2/sex/group) were euthanized 4 weeks after the last dose (recovery group, Day 121 or 122). Necropsy was performed on all euthanized animals and spleen biopsy specimens were preserved in neutral-buffered 10% formalin.

RNA Isolation Total RNA extraction from the blood samples, RNA QC and cDNA generation was performed at Asuragen Inc. (Austin TX), using the general steps similar to those used in Example 2. cDNA samples were stored at -20°C.

Quantitative Reverse-Transcriptase Polymerase Chain Reaction Sequences for all biomarkers were derived from the human deoxyribonucleic acid (DNA) sequence (GenBank® nucleic acid database, National Center for Biotechnology Information, Bethesda, MD) Primers and MGB Eclipse™ probes for GLT-μ and CT1 (Nanogen, Bothell, WA) were designed from analysis. Each primer and probe pair was synthesized by Nanogen and was validated by measuring PCR efficiency using synthetic templates and human cDNA standards. Validated assays demonstrated linear amplification and >99% efficiency over 7 orders of magnitude (data not shown). Validated primer and probe pairs were sent to Millennium (Cambridge, MA) and were revalidated on the human positive control cDNA standard prior to use in expression profiling experiments. The Applied Biosystem Assay on Demand (AOD) assays found in Table 13 were configured into the Millennium DSD718 TaqMan® Low Density Array (Applied Biosystems; Foster City, CA).

Table 13 Biomarker reagents

| Biomarker | Reference Sequence Identifier | Oligomer | Sequence | SEQ ID NO: |
|---|---|---|---|---|
| GLT-μ ver1 (GLT1) | NG_001019 | Forward | ACAGTCTTAGGGAGAGTTTATGAC | 53 |
| | | Reverse | CACCACGTGTTCGTCTGTG | 54 |
| | | Probe | CTCCATCA*CTTTCTCC | 36 |

(continued)

| Biomarker | Reference Sequence Identifier | Oligomer | Sequence | SEQ ID NO: |
|---|---|---|---|---|
| GLT-μ ver2 (GLT2) | NG_001019 | Forward | GATATTCTGATAGAGTGGCCTTCAT | 55 |
| | | Reverse | CACCACGTGTTCGTCTGTG | 54 |
| | | Probe | CTCCATCA*CTTTCTCC | 36 |
| GLT-μ ver3 (GLT3) | NG-001019 | Forward | CCTGAATTA*TTTCAGTTAAGCATGT | 34 |
| | | Reverse | CACCACGTGTTCGTCTGTG | 54 |
| | | Probe | CTCCATCA*CTTTCTCC | 36 |
| CT-γ1&2 ver1 (CT1) | NG-001019 | Forward | CAACAGGGCAGGACAC | 37 |
| | | Reverse | CACCACGTGTTCGTCTGTG | 54 |
| | | Probe | CTCCATCA*CTTTCTCC | 36 |
| CT-γ1&2 ver2 (CT2) | NG_001019 | Forward | AGCAGAGCTGGCCGTA | 56 |
| | | Reverse | CACCACGTGTTCGTCTGTG | 54 |
| | Probe | Probe | CTCCATCA*CTTTCTCC | 36 |
| CT-γ1&2 ver3 (CT3) | NG_001019 | Forward | CCAGAAAGGCCCAGAGT | 57 |
| | | Reverse | CACCACGTGTTCGTCTGTG | 54 |
| | | Probe | CTCCATCA*CTTTCTCC | 36 |
| 18S IGHD | X03205.1 | AOD[a] | ABI Catalog No. Hs99999901_s1 | |
| | | AOD[a] | ABI Catalog No. Hs00378878_m1 | |
| IGKC | | AOD[a] | ABI Catalog No. Hs00736177_m1 | |
| IGLL | | AOD[a] | ABI Catalog No. Hs00760769_s1 | |
| AICDA | NM_020661 | AOD[a] | ABI catalog No. Hs002210688_m1 | |
| IGHA | | AOD[a] | ABI catalog No. Hs00740132_g1 | |
| CFB | | AOD[a] | ABI Catalog No. Hs00175252_m1 | |
| B2MG | | AOD[a] | ABI Catalog No. Hs99999907_m1 | |
| IGHG1 | | AOD[a] | ABI Catalog No. Hs00378340_m1 | |

Quantitative reverse-transcriptase polymerase chain reaction assays were performed on an ABI PRISM® 7900HT Sequence Detection System (Applied Biosystems, Foster City, CA). All quantitative assays designed using Nanogen guidelines were run using the following universal thermal cycling parameters: hold at 95°C for 2 minutes to activate the DNA polymerase, then run 40 three-part cycles (95°C for 20 seconds, 58°C for 20 seconds, and 76°C for 20 seconds). All quantitative assays for the TAQMAN® Low Density Array were run using the following cycling parameters: hold at 50°C for 2 minutes for AMPERASE® UNG (PCR Master Mix, Applied Biosystems, Foster City, CA) activation, then at 94.5°C for 10 minutes to activate DNA polymerase, then run 40 two-part cycles (97°C for 30 seconds and 59.7°C for 1 minute).

Raw Data Analysis Data were analyzed using Sequence Detection System (SDS) software, Version 2.2 (Applied Biosystems, Foster City, CA). The first step was to generate an amplification plot for each sample, which showed the change in Rn (ΔRn) on the y axis (where Rn is the fluorescence emission intensity of the reporter dye normalized to a passive reference) against the cycle number on the x axis. From each amplification plot, a threshold cycle (Ct) value was calculated. The Ct value is defined as the cycle number at which a statistically significant increase in ΔRn above background levels (i.e. noise) is detected. Ct values are displayed on a graph as the intercept point of the amplification plot and threshold. The Ct values were exported to an EXCEL® (Microsoft Corp., Redmond, WA) spreadsheet. Interpretation of raw Ct values can be confounded by variability in the amount of sample that was input into each assay. A conventional method of normalizing for such technical variability is to subtract the raw Ct value of an internal, invariant, endogenous reference transcript (18S or B2MG) from the raw Ct value of the transcripts of interest (in this case CT1, GLT3, IGLL, IGLK, AICDA, IGHG1, or IGHA1) to generate the delta cycle threshold (dCt) value. Transcripts for 18S rRNA (18S) or β-2 microglobulin (B2MG) are conventional invariant endogenous reference transcripts. Analysis of the data in this investigation illustrate that the 18S is the most invariant of the endogenous reference transcripts evaluated in this investigation. Cycle threshold and dCt are log base 2 ($log_2$) functions; an increase of one Ct represents a doubling of signal, whereas a decrease of one Ct represents a halving of signal. Levels of transcript are therefore represented as Normalized Level using Equation 3: [X = Power(2,-dCt) $\times$ 1000].

The majority of transcripts examined in this investigation (GLT3, CT1, AICDA, IGHG1 and IGHA1) are likely to be co-regulated because they are products of germinal center reactions. Trend analysis of changes in any single transcript in the context of other co-regulated transcripts allows the determination of whether a change in any single transcript represents a nonspecific trend in all the transcripts or is limited to a specific subset of transcripts. However, the normalized level of different transcripts may vary by 100,000-fold, which impedes direct comparative analyses. The data was normalized for variability in the magnitude of signal by calculation of fold changes from baseline, thus simplifying comparison across transcripts. Fold change from baseline was calculated by dividing the normalized value of a transcript at a given time-point by the mean of the two baseline normalized level values (Week -2 and Week -1).

Results

General Immune Function There were no treatment-related on clinical observations, except bacterial infection in a subset of high dose animals. Clinical pathology otherwise was normal. The anatomic pathology was normal, except there was atrophy of lymphoid germinal centers. An immunotoxicology assesment showed no inhibition of complement pathways; no inhibition of phagocytic function or respiratory burst; no inhibition of natural killer (NK) function; no alterations in frequency of major subsets of blood leukocytes; no change in levels of serum immunoglobulins; but inhibition of T cell-dependent antibody responses (TDAR) to keyhole limpet hemocyanin (KLH) (Figure 10) in high dose animals. The impaired TDAR is associated with clinical symptoms of infection (Fig 10); dose-dependent inhibition of the IgG response to neo- and recall antigen. Symptomatic animals exhibited the lowest anti-KLH titers, but overall Ig levels were unchanged.

Histologic Analysis Analysis of H&E sections from the spleen of each animal by 2 independent, blinded reviewers confirmed that atrophy of germinal centers occurred in this investigation in a dose-dependent manner. Minimal atrophy was observed in two animals of the 40 mg/kg group and one animal of the 80 mg/kg group. Mild atrophy was observed in one animal of the 40 mg/kg group and one animal of the 80 mg/kg group. Moderate atrophy was observed in two animals of the 80 mg/kg group. Significant differences were not observed between dose groups due to high inter-animal variability within the 40 and 80 mg/kg groups. Reactivity of each animal euthanized by Week 13 was also scored on a relative scale, essentially ranking each specimen from 1 (least reactive) to 30 (most reactive) based on assessment of the frequency and magnitude of hyperplasia of germinal centers. The relative scale facilitated comparisons of histologic changes to levels of transcripts in peripheral blood. The overall degree of germinal center atrophy achieved in this investigation is less than that achieved in the previous 28-day investigation (Example 2), which may have resulted from administering lower doses (20, 40 and 80 mg/kg compared to 40, 60 and 100 mg/kg) and/or attaining lower exposures in the 13 week study compared to the previous 28-day study.

Quantitative RT-PCR Significant differences were not observed for any transcript between dose groups. Levels of GLT3, CT1, AICDA, IGHA1, IGDG1, IGLK and IGLL transcripts exhibited coordinated variation over time within individual animals in the vehicle control group (see Figure 11A, for example). This variation most likely represents biological (not technical) variation in levels of these transcripts over time because the GLT3 and CT1 assays were conducted with a different technology (i.e. MGB Eclipse probes) and format (384-well plates) than the AICDA, IGHA1, IGDG1, IGLK and IGLL assays (ie, TaqMan probes and TLDA microfluidic cards.

An effect of Test Agent A on the levels of IGHA1, IGDG1, GLT3, AICDA, CT1, IGLK or IGLL transcripts was not observed for individual animals in the 20 mg/kg group.

An effect of Test Agent A on the levels of IGHA1, IGDG1, GLT3, AICDA, CT1, IGLK or IGLL transcripts was generally not observed for individual animals within the 40 mg/kg group, although some may have occurred in animals 3001, 3003 and 3501during Weeks 3-13.

Changes in the levels of IGHA1, IGDG1, GLT3, AICDA, CT1, IGLK and IGLL transcripts were observed in response to Test Agent A (80 mg/kg) for animals 4004, 4005 and 4006 (see Figure 11B, for example). Dosing of animals 4004 and 4006 was terminated during Week 4 due to the moribund condition of these animals. Animal 4005 was not moribund and dosing was continued, as this animal's condition consistently improved with administration of antibiotics with a return to normal clinical status by Week 17. The effects of Test Agent A on levels of IGHA1, IGDG1, GLT3, AICDA, CT1, IGLK and IGLL transcripts observed at Week 13 in animal 4005 were reversible, in that levels of these transcripts returned to their baseline levels by Week 17, four weeks after last dose of Test Agent A. There were no differences between the two control endogenous reference transcripts (18S and B2MG) between dose groups, nor changes in response to Test Agent A.

Comparison Of Atrophy Of Splenic Germinal Centers To Levels Of Transcripts In Peripheral Blood Comparisons of mean values for histologic germinal center atrophy with mean values for levels of switch transcripts in peripheral blood did not reveal significant correlations with dose (data not shown). Analyses of individual animals also were conducted by comparing the relative histologic reactivity of splenic germinal centers for an individual animal at necropsy (Week 13) to the change in levels of switch transcripts in peripheral blood from baseline (Week -2 & -1) to necropsy (Week 4 & 13). Animals that exhibited the most atrophy of germinal centers in spleen (e.g., animal 4006) also generally exhibited decreases in levels of several transcripts in peripheral blood. Quantitative analyses of these data revealed that decreases in blood levels of GLT3, CT1 and IGLL exhibited weak but positive correlations ($r^2$~0.2) with decreases in germinal center reactivity. Weaker correlations were observed for other transcripts. Animals that exhibited splenic germinal center atrophy generally had lower levels of GLT3 and CT1 in peripheral blood; however, animals 4503 and 4504 are exceptions in that they exhibited moderate atrophy yet high levels of GLT3 and CT1 in peripheral blood. It should be noted that these animals were also unique in that these sections contained lymphoid hyperplasia of the periarteriolar lymphoid sheath (PALS). Hyperplasia of lymphocytes is a hallmark of reactive germinal centers. It is possible that ICS did occur in the PALS of these two animals, as a compensatory response to atrophy of germinal centers and that this may be the source of disproportionately high levels of GLT3 and CT1 in peripheral blood. These morphologically exceptional spleens (4503 and 4504) heavily influence the outcomes of these comparisons. If animals 4503 and 4504 are considered outliers and are excluded from the analyses, the overall correlation between a decrease in germinal center reactivity (e.g. atrophy) and decreased levels of these transcripts in blood improved substantially for GLT3 and CT1 ($r^2$~0.6-0.7, Figure 12A & B). Excluding the same animals as outliers in reanalyses of the other transcripts also yielded higher correlations for AICDA, IGDG1 and IGLK (Figure 12C, E, G), but not for IGHA1, IGLL and 18S (Figure 12D, F& H).

Comparison Of Clinical Symptoms Of Infection To Levels Of Transcripts In Peripheral Blood Clinical symptoms of infection (i.e. Shigellosis) were observed on Day 13 for animal 4004, Day 20 for animal 4006 and Day 29 for animal 4005. The occurrence of clinical symptoms of infection was compared to changes in levels of transcripts in peripheral blood for time-points prior to and during the onset of symptoms (Weeks -2, -1, 1, 2, 3 & 4). Slope values for transcripts in individual animals represent the magnitude of change in that transcript from Week -2 through Week 4 and were quantified by calculating the linear regression of fold changes from baseline values over this time period (Table 14). Animal 4004, for example, exhibited the largest decreases in levels of GLT3 and CT1, whereas Animal 4501 exhibited the largest decrease in AICDA, 4005 for IGHG1, 4006 for IGHA, etc.. One can assess the relative predictive value of these potential safety biomarkers by ranking the slopes for each transcript and determining if the ranking value distinguishes clinically symptomatic animals (4004, 4005 & 4006) from asymptomatic animals. Ranking the slopes for GLT3 or CT1 segregated animals 4004, 4005 and 4006 into the top three positions; symptomatic animals exhibited the largest decreases in levels of GLT3 or CT1 (Table 14). Ranking slopes for AICDA, IGHG, IGHA or IGLK, segregated two of the three symptomatic animals (4005 and 4006) into the top three positions (Table 14) and the third animal (4004) segregated within the upper quintile of all animals (Table 14). Ranking slopes for IGLL, segregated two of the three symptomatic animals (4005 and 4006) within the upper quintile of all animals (Table 14). Ranking slopes for control transcripts 18S and B2MG did not segregate symptomatic from asymptomatic animals; slope values for animals 4004, 4405 and 4006 were distributed randomly among all animals examined (data not shown). These data collectively indicate that changes in levels of GLT3 or CT1 were the most accurate predictors of clinical symptoms of infection in this study.

**Table 14 Comparison of Symptomatic Infection With Changes in Levels of Transcripts in Peripheral Blood of Cynomolgus Monkeys From Week -2 to Week 4**

| | GLT3 | | CT1 | | AICDA | | IGHG1 | | IGHA | | IGLK | | IGLL | |
|---|---|---|---|---|---|---|---|---|---|---|---|---|---|---|
| **Rank** | **ID** | **Slope[a]** | **ID** | **Slope** | **ID** | **Slope** | **ID** | **Slope** | **ID** | **Slope** | **ID** | **Slope** | **ID** | **Slope** |
| 1 | **4004** | **y = -0.1708x + 1.4044** | **4004** | **y = -0.1708x + 1.4044** | 4501 | y = -0.1806x + 1.2691 | **4005** | **y = -0.1869x + 1.2106** | **4006** | **y =-0.218x + 1.2841** | **4005** | **y = -0.1869x + 1.2106** | 3504 | y = -0.1992x + 1.1967 |
| 2 | **4005** | **y = -0.1614x + 1.3337** | **4005** | **y = -0.1673x + 1.3039** | **4005** | **y = -0.1776x + 1.1608** | **4006** | **y = -0.185x + 1.2446** | **4005** | **y = -0.1997x + 1.1456** | **4006** | **y = -0.185x + 1.2446** | 3503 | y = -0.195x + 1.1923 |
| 3 | **4006** | **y = -0.1283x + 1.4198** | **4006** | **y = -0.1283x + 1.4198** | **4006** | **y = - 0.1638x + 1.1842** | 4501 | y = -0.1666x + 1.2456 | 1504 | y = -0.15x + 1.5727 | 4501 | y = -0.1666x + 1.2456 | 4001 | y = -0.1887x + 1.2514 |
| 4 | 2003 | y = -0.1063x + 1.2427 | 2503 | y = -0.1058x + 1.3538 | 3501 | y = -0.1479x + 1.2096 | 2004 | y = -0.1396x + 1.1076 | 4506 | y = -0.127x + 1.1347 | 2004 | y = -0.1363x + 1.1752 | 2004 | y = -0.1851x + 1.2243 |
| 5 | 2503 | y = -0.1016x + 1.3307 | 2003 | y = -0.0984x + 1.2191 | 3503 | y = -0.142x + 1.1498 | 3001 | y = -0.1372x + 1.1668 | 3501 | y = -0.1278x + 1.1921 | 3001 | y = -0.1275x + 1.0991 | **4006** | **y = -0.1755x + 1.2353** |
| 6 | 1504 | y = -0.0967x + 1.4225 | 2502 | y = -0.0942x + 1.3131 | 1001 | y = -0.1429x + 1.1696 | 4001 | y = -0.1235x + 1.1496 | 3001 | y = -0.1265x + 1.0822 | 1006 | y = -0.1269x + 1.3542 | **4005** | **y = -0.1625x + 1.1408** |
| 7 | 2502 | y = -0.0961x + 1.3434 | 1504 | y = -0.0821x + 1.3969 | 1506 | y = -0.1105x+ 1.0279 | **4004** | **y = -0.1169x + 1.2734** | 3004 | y = -0.1245x + 1.0928 | 4001 | y = -0.1235x + 1.1496 | 1504 | y = -0.1218x + 1.1602 |
| 8 | 2501 | y = -0.0844x + 1.2891 | 3501 | y = -0.075x + 1.4244 | 2003 | y = -0.1015x + 0.9907 | 2504 | y = -0.1153x + 1.0351 | 1002 | y = -0.1116x + 0.9902 | **4004** | **y = -0.1169x + 1.2734** | 3004 | y = -0.1013x + 1.0844 |
| 9 | 1506 | y = -0.0834x + 1.313 | 2004 | y = -0.072x + 1.2363 | 3502 | y = -0.0945x + 1.2817 | 1006 | y = -0.1013x + 1.3697 | 2003 | y = -0.1015x + 0.9907 | 3003 | y = -0.111x + 1.1087 | 3001 | y = -0.0967x + 1.0688 |
| 10 | 3002 | y = -0.0692x + 1.2162 | 2501 | y = -0.0725x + 1.2351 | 2503 | y = -0.0928x + 1.1107 | 3004 | y = -0.0999x + 1.0101 | 2504 | y = -0.0907x + 0.9453 | 3503 | y = -0.1096x + 1.1083 | 3502 | y = -0.094x + 1.7009 |

(continued)

| Rank | GLT3 | | CT1 | | AICDA | | IGHG1 | | IGHA | | IGLK | | IGLL | |
|---|---|---|---|---|---|---|---|---|---|---|---|---|---|---|
| | ID | Slope[a] | ID | Slope | ID | Slope | ID | Slope | ID | Slope | ID | Slope | ID | Slope |
| 11 | 3501 | y = -0.0685x + 1.4031 | 1506 | y = -0.0668x + 1.247 | 2004 | y = -0.0888x + 1.1156 | 1002 | y = -0.0928x + 0.9635 | 1006 | y = -0.08x + 1.1774 | 1504 | y = -0.0964x + 1.0503 | 3003 | y = -0.0939x + 1.1456 |
| 12 | 2004 | y = -0.0668x + 1.213 | 3002 | y = -0.0659x + 1.1969 | 1504 | y = -0.0841x + 0.9872 | 3503 | y = -0.0873x + 1.0192 | 2004 | y = -0.0888x + 1.1156 | 3501 | y = -0.0911x + 1.148 | 3501 | y = -0.0834x + 1.1457 |
| 13 | 2504 | y = -0.0607x + 1.163 | 2504 | y = -0.0518x + 1.1373 | **4004** | **y = -0.0748x + 1.2671** | 3003 | y = -0.0866x + 1.05 | 3003 | y = -0.0864x + 1.0732 | 3004 | y = -0.0911x + 1.1056 | 1501 | y = -0.0828x + 0.8933 |
| 14 | 4506 | y = -0.0477x + 1.2385 | 4506 | y = -0.0477x + 1.2385 | 1005 | y = -0.0703x + 1.0747 | 1506 | y = -0.0842x + 1.0152 | 4501 | y = -0.0858x + 1.1437 | 3504 | y = -0.0871x + 1.0592 | 1003 | y = 0.0817x + 1.094 |
| 15 | 1005 | y = -0.0467x + 1.187 | 4504 | y = -0.0462x + 1.0621 | 4504 | y = -0.0681x + 0.9775 | 1504 | y = -0.0808x + 1.0518 | 4001 | y = -0.0851x + 1.0022 | 3502 | y = -0.0848x + 1.2028 | 4501 | y = -0.075x + 1.0908 |
| 16 | 4504 | y = -0.0462x + 1.0621 | 4001 | y = -0.0413x + 1.1172 | 4502 | y = -0.065x + 1.1119 | 3501 | y = -0.0766x + 1.1437 | 4002 | y = -0.0677x + 0.9776 | 1506 | y = -0.0837x + 1.0692 | 4504 | y = -0.0655x + 1.0494 |
| 17 | 4001 | y = -0.0413x + 1.1172 | 3503 | y = -0.0395x + 1.1433 | 1006 | y = -0.058x + 1.3888 | 4002 | y = -0.0559x + 0.9637 | 1501 | y = -0.0667x + 0.9328 | 1002 | y = -0.07x + 0.9669 | 1505 | y = -0.0543x + 0.883 |
| 18 | 3503 | y = -0.033x + 1.1262 | 3001 | y = -0.028x + 1.1417 | 4002 | y = -0.0579x + 0.9865 | 4504 | y = -0.0559x + 0.9637 | 2501 | y = -0.0609x + 1.6551 | 2003 | y = -0.0737x + 1.0432 | 2503 | y = -0.0458x + 0.9905 |
| 19 | 3001 | y = -0.0337x + 1.1719 | 4502 | y = -0.0282x + 1.098 | 4001 | y = -0.0564x + 1.1328 | 2003 | y = -0.0509x + 0.9053 | 4502 | y = -0.0505x + 0.8973 | 3002 | y = -0.0734x + 1.2265 | 4503 | y = -0.0453x + 1.0088 |
| 20 | 4502 | y = -0.0282x + 1.098 | 1005 | y = -0.0226x + 1.092 | 1002 | y = -0.0546x + 0.9215 | 1001 | y = -0.0507x + 1.0576 | 1502 | y = -0.0434x + 1.0224 | 1005 | y = -0.072x + 1.0893 | 2003 | y = -0.0408x + 0.9189 |

(continued)

| Rank | GLT3 | | CT1 | | AICDA | | IGHG1 | | IGHA | | IGLK | | IGLL | |
|---|---|---|---|---|---|---|---|---|---|---|---|---|---|---|
| | ID | Slope[a] | ID | Slope | ID | Slope | ID | Slope | ID | Slope | ID | Slope | ID | Slope |
| 21 | 1004 | y = -0.0238x + 1.0586 | 1004 | y = -0.0178x + 1.0342 | 3504 | y = -0.0535x + 0.9562 | 4502 | y = -0.0413x + 1.003 | 4505 | y = -0.0408x + 0.9989 | 4002 | y = -0.0559x + 0.9637 | 1002 | y = -0.0398x + 0.8941 |
| 22 | 2002 | y = -0.0178x + 1.0078 | 1002 | y = -0.0151x+ 1.0865 | 3001 | y = -0.0451x + 1.0988 | 2503 | y = -0.037x + 0.9933 | 1506 | y = -0.017x + 1.1032 | 1501 | y = -0.0459x + 0.9372 | 1506 | y = -0.0356x + 0.971 |
| 23 | 1002 | y = -0.0148x + 1.1 | 2002 | y = -0.0109x + 0.9656 | 3003 | y = -0.0394x + 1.0028 | 1005 | y = -0.0375x + 0.9391 | 4503 | y = -0.0176x + 0.73 | 4504 | y = -0.044x + 1.0155 | 2501 | y = -0.0332x + 1.1874 |
| 24 | 1501 | y = -0.0055x + 1.067 | 1502 | y = -0.004x + 1.1115 | 4505 | y = -0.0359x + 0.9678 | 1004 | y = -0.0325x + 0.9241 | 3002 | y = -0.0164x + 1.272 | 4502 | y = -0.0413x + 1.003 | 4502 | y = -0.0332x + 0.9682 |
| 25 | 1505 | y = 0.0026x + 0.9714 | 1501 | y = -0.0042x + 1.0566 | 1004 | y = -0.031x + 0.9311 | 1505 | y = -0.0122x + 0.806 | **4004** | **y = -0.0164x + 0.8648** | 2101 | y = -0.0383x + 1.0457 | 1503 | y = -0.0066x + 0.8924 |
| 26 | 1502 | y = 0.0034x + 1.0817 | 1001 | y = 0.0101x + 1.0036 | 1503 | y = -0.021x + 0.8448 | 3504 | y = -0.006x + 0.8587 | 1005 | y = -0.0156x + 1.0548 | 2504 | y = -0.0318x + 0.8724 | 1005 | y = -0.0025x + 1.0638 |
| 27 | 1003 | y = 0.0039x + 0.9103 | 1505 | y = 0.0149x + 0.9614 | 1501 | y = -0.0077x + 0.9347 | 1501 | y = 0.0019x + 0.8438 | 1505 | y = -0.0006x + 0.7524 | 1505 | y = -0.027x + 0.8553 | 1001 | y = 0.0045x + 0.9491 |
| 28 | 4501 | y = 0.0189x + 0.993 | 1003 | y = 0.0165x + 0.8595 | 2002 | y = 0.0011x + 0.0893 | 4505 | y = 0.0021x + 0.9353 | 4504 | y = 0.0002x + 0.8913 | 1004 | Y =- 0.0258x + 0.9499 | 1006 | y = 0.0321x + 1.3645 |
| 29 | 3504 | y = 0.0233x + 0.8593 | 4501 | y = 0.0189x + 0.993 | 4503 | y = 0.0019x + 0.8963 | 4003 | y = 0.0021x + 1.0885 | 2002 | y = 0.0011x + 0.0893 | 1003 | y = -0.0144x + 0.9628 | 2002 | y = 0.0391x + 0.8381 |
| 30 | 1001 | y = 0.0255x + 0.9566 | 3504 | y = 0.0227x + 0.8557 | 1502 | y = 0.0083x + 0.9184 | 2501 | y = 0.0072x + 1.1496 | 2101 | y = 0.028x + 0.967 | 1502 | y = -0.0136x + 0.9466 | 4506 | y = 0.0453x + 1.0041 |

(continued)

| | GLT3 | | CT1 | | AICDA | | IGHG1 | | IGHA | | IGLK | | IGLL | |
|---|---|---|---|---|---|---|---|---|---|---|---|---|---|---|
| Rank | ID | Slope[a] | ID | Slope | ID | Slope | ID | Slope | ID | Slope | ID | Slope | ID | Slope |
| 31 | 4002 | y = 0.0264x + 0.9284 | 3004 | y = 0.0236x + 0.9067 | 2101 | y = 0.0084x + 0.9523 | 1502 | y = 0.0233x + 0.889 | 1001 | y = 0.0299x + 1.1096 | 1001 | y = 0.0011x + 1.0977 | 4002 | y = 0.0574x + 0.8364 |
| 32 | 4505 | y = 0.0266x + 0.9059 | 4002 | y = 0.0264x + 0.9284 | 2504 | y = 0.012x + 0.8623 | 3002 | y = 0.0308x + 1.1 | 3503 | y = 0.0417x + 0.7948 | 4505 | y = 0.0021x + 0.9353 | 4505 | y = 0.0859x + 0.8101 |
| 33 | 3004 | y = 0.029x + 0.8888 | 4505 | y = 0.0266x + 0.9059 | 1003 | y = 0.0254x + 1.0253 | 3502 | y=-0.0342x + 1.2773 | 2503 | y = 0.064x + 0.7354 | 4003 | y = 0.0021x + 1.0885 | **4004** | **y = 0.0905x + 0.6369** |
| 34 | 2101 | y = 0.0325x + 0.9206 | 4003 | y = 0.0382x + 0.8779 | 2501 | y = 0.0284x + 1.0457 | 1003 | y = 0.0375x + 0.883 | 3502 | y = 0.0654x + 0.9361 | 2002 | y = 0.0111x + 0.8426 | 1502 | y = 0.0979x + 0.8676 |
| 35 | 4003 | y = 0.0382x + 0.8779 | 1006 | y = 0.0391x + 0.8201 | 1505 | y = 0.0345x + 0.8829 | 2002 | y = 0.04x + 0.859 | 2502 | y = 0.0881x + 0.7674 | 2501 | y = 0.0201x + 1.3507 | 3002 | y = 0.1094x + 0.9183 |
| 36 | 1006 | y = 0.0494x + 0.7819 | 2101 | y = 0.0485x + 0.8757 | 3002 | y=0.03x+ 1.1414 | 2502 | y = 0.0593x + 0.8762 | 1003 | y = 0.1602x + 0.7026 | 1503 | y = 0.0328x + 0.8389 | 2101 | y = 0.1554x + 0.8416 |
| 37 | 3502 | y = 0.0561x + 0.7711 | 1503 | y = 0.0627x + 0.8778 | 3004 | y = 0.043x + 0.7108 | 4506 | y = 0.066x + 1.0079 | 3504 | y = 0.1823x + 0.6623 | 4506 | y = 0.066x + 1.0079 | 2502 | y = 0.1794x + 0.4851 |
| 38 | 1503 | y = 0.0585x + 0.8814 | 3502 | y = 0.0707x + 0.7175 | 4003 | y = 0.0681x + 1.0699 | 2101 | y = 0.0831x + 0.8645 | 1004 | y = 0.203x + 0.6491 | 2502 | y = 0.1217x + 0.7379 | 2504 | y = 0.2394x + 0.2681 |
| 39 | 3003 | y = 0.1291x + 0.5302 | 4505 | y = 0.0266x + 0.9059 | 4506 | y = 0.2193x + 0.9298 | 4503 | y = 0.1364x + 0.6808 | 4003 | y = 0.398x + 0.4401 | 4503 | y = 0.1364x + 0.6808 | 1004 | y = 0.3365x + 0.3899 |

(continued)

| | GLT3 | | CT1 | | AICDA | | IGHG1 | | IGHA | | IGLK | | IGLL | |
|---|---|---|---|---|---|---|---|---|---|---|---|---|---|---|
| Rank | ID | Slope[a] | ID | Slope | ID | Slope | ID | Slope | ID | Slope | ID | Slope | ID | Slope |
| 40 | 4503 | y = 0.1299x + 0.5066 | 4506 | y = -0.0477x + 1.2385 | 2502 | y = 0.3303x + 0.1932 | 1503 | y = 0.361x + 0.408 | 1503 | y = 0.402x + 0.2221 | | | 4003 | y = 0.3682x + 0.4393 |

ID = animal identification number.

Note: Bold font indicates animals that exhibited clinical symptoms of infection (observed on Day 13 for Animal 4004, Day 20 for Animal 4006, and Day 29 for Animal 4005).

a: Slope values for transcripts in individual animals represent the magnitude of potential changes in that transcript from Week -2 through Week 4 and were quantified by calculating the linear regression of fold change from baseline values over this time period. Slope values are listed in ascending order for each transcript and the corresponding animal identification number is included.

Animals with largest decreases in blood switch transcripts over time were those that developed clinical symptoms of infection. Of particular interest, decreases in levels of GLT3 and CT1 in peripheral blood preceded the onset of clinical symptoms of infection in animal 4005 by at least two weeks (Figure 8B). Similar pre-infection trends in the level of GLT3 and CT1 in peripheral blood appear to have occurred in animals 4004 and 4006. These data, combined with the knowledge that immunoglobulin ICS is required for resistance to infection, collectively indicate that decreases in GILT3 and CT1 are antecedent markers of infection. We therefore conclude that GLT3 and CT1 are useful predictive markers of clinical infection.

**Conclusions**

High exposures to Test Agent A caused atrophy of germinal centers, inhibition of antibody responses and bacterial infection. Test Agent A inhibits differentiation of B cells and antibody responses *de novo.* Change in levels of switch transcripts in Cyno tissue or peripheral blood correlate with germinal center atrophy, TDAR and with clinical symptoms of infection. Changes in levels of switch transcripts may be a noninvasive and antecedent marker of immunosuppression in clinical subjects.

## Claims

1. Use of a probe to detect a marker selected from the group consisting of germline transcript mu (GLT-μ), circle transcript containing gamma 1 (CT-γ1), and circle transcript containing gamma 2 (CT-γ2), in determining germinal center activity in a blood sample, wherein the marker comprises a nucleic acid sequence selected from the group consisting of SEQ ID NOs: 58, 59 and 60 or a complement of any thereof.

2. The use of claim 1 wherein the probe comprises a nucleic acid reagent at least 90% identical to a nucleic acid selected from the group consisting of SEQ ID NOs: 34, 35, 36, 37, 53, 54, 55, 56 and 57 or a complement of any thereof, or
wherein the probe consists of SEQ ID NO:59 or a nucleic acid fragment of SEQ ID NO:59 comprising at least 15 consecutive nucleotides of SEQ ID NO:59 or a complement of any thereof.

3. A kit comprising probes to detect markers selected from the group consisting of germline transcript mu (GLT-μ), circle transcript containing gamma 1 (CT-γ1), and circle transcript containing gamma 2 (CT-γ2), for use in determining germinal center activity in a blood sample, wherein the markers comprise a nucleic acid sequence selected from the group consisting of SEQ ID NOs: 58, 59 and 60 or a complement of any thereof,
wherein the kit comprises at least two probes to detect at least two markers.

4. The kit of claim 3 wherein one of the probes consists of SEQ ID NO:59 or a nucleic acid fragment of SEQ ID NO:59 comprising at least 15 consecutive nucleotides of SEQ ID NO:59 or a complement of any thereof.

5. The kit of claim 3, wherein at least one of the at least two probes comprise nucleic acid reagents, each at least 90% identical to nucleic acids selected from the group consisting of SEQ ID NOs:34, 35, 36, 37, 53, 54, 55, 56 and 57 or a complement of any thereof.

6. The kit of any one of claims 3 to 5, further comprising a stabilizer to add to the sample or a sample collection container comprising a stabilizer.

7. The kit of claim 6, wherein the stabilizer is an RNA stabilizer.

8. A method for determining whether a patient is susceptible to a disorder selected from the group consisting of opportunistic infection and lymphoma, comprising:

    a. Contacting a blood sample that has been obtained from a patient prior to treating with a therapeutic regimen with a probe to detect a marker selected from the group consisting of germline transcript mu (GLT-μ), circle transcript containing gamma 1 (CT-γ1), and circle transcript containing gamma 2 (CT-γ2), wherein the marker comprises a nucleic acid sequence selected from the group consisting of SEQ ID NOs: 58, 59 and 60 or a complement of any thereof;
    b. Measuring the amount of probe bound to the sample to determine the amount of the marker in the sample;
    c. Contacting an additional blood sample that has been obtained from the patient after treatment with the therapeutic regimen with a probe to detect a marker selected from the group consisting of germline transcript mu (GLT-μ), circle transcript containing gamma 1 (CT-γ1), and circle transcript containing gamma 2 (CT-γ2), wherein the marker comprises a nucleic acid sequence selected from the group consisting of SEQ ID NOs: 58, 59 and 60 or a complement of any thereof;
    d. Measuring the amount of probe bound to the additional sample to determine the amount of the marker in the additional sample; and
    e. Determining that the patient is susceptible to the disorder if the amount of marker in the sample after treatment is less than the level in the sample before treatment.

9. The method of claim 8, wherein the patient is being treated for cancer.

10. The method of claim 8 or 9, wherein the sample is a peripheral blood sample.

11. The method of any one of claims 8 to 10, further comprising enriching the sample for B cells.

12. The method of any one of claims 8 to 11, wherein the level of at least two markers is determined.

**13.** The method of any one of claims 8 to 12, wherein the amount of RNA of the biomarker in the sample is determined.

**14.** The method of claim 13, wherein the RNA in the sample is stabilized.

**15.** A method for determining germinal center activity, comprising:

a. Contacting a blood sample that has been obtained from a patient with a probe to detect a marker selected from the group consisting of germline transcript mu (GLT-$\mu$), circle transcript containing gamma 1 (CT-$\gamma$1), and circle transcript containing gamma 2 (CT-$\gamma$2), wherein the marker comprises a nucleic acid sequence selected from the group consisting of SEQ ID NOs: 58, 59 and 60 or a complement of any thereof; and
b. Measuring the amount of probe bound to the sample to determine the amount of marker in the sample and comparing the amount of marker in the sample to a baseline or a pre-determined standard, thereby determining germinal center activity.

**16.** The method of claim 15, wherein the patient is being treated for cancer.

**17.** The method of claim 15, wherein an amount of the marker which is more than baseline or a pre-determined standard indicates that adaptive immunity has recovered after removal of an immunomodulatory agent.

**18.** The method of claim 15, wherein an amount of the marker which is more than baseline or a pre-determined standard indicates that the patient has had an efficacious vaccination.

**19.** The method of claim 15, wherein an amount of the marker which is less than baseline or a pre-determined standard indicates that the patient is susceptible to a disorder selected from the group consisting of opportunistic infection and lymphoma.

**20.** The method of any one of claims 8 to 19, wherein the probe consists of SEQ ID NO:59 or a nucleic acid fragment of SEQ ID NO:59 comprising at least 15 consecutive nucleotides of SEQ ID NO:59 or a complement of any thereof.

**21.** The method of claim 15, wherein the probe comprises a nucleic acid reagent at least 90% identical to a nucleic acid selected from the group consisting of SEQ ID NOs: 34, 35, 36, 37, 53, 54, 55, 56 and 57 or a complement of any thereof.

**22.** A method for screening to identify an immunomodulator comprising:

a) contacting a blood sample comprising B cells with a test agent;
b) measuring the level of expression of a marker selected from the group consisting of germline transcript mu (GLT-$\mu$), circle transcript containing gamma 1 (CT-$\gamma$1), and circle transcript containing gamma 2 (CT-$\gamma$2), wherein the marker comprises a nucleic acid sequence selected from the group consisting of SEQ ID NOs: 58, 59 and 60 or a complement of any thereof; and
c) determining that the test agent is an immunomodulator if the level of expression of the marker is significantly different than the level in a sample that was not contacted with the test agent.

## Patentansprüche

**1.** Verwendung einer Sonde zum Erkennen eines Markers, welcher aus der Gruppe, bestehend aus einem mu-Keimbahntranskript (GLT-$\mu$), gamma 1 enthaltenden zirkulären Transkript (CT-$\gamma$1) und gamma 2 enthaltenden zirkulären Transkript (CT-$\gamma$2), ausgewählt ist, beim Feststellen von Aktivität im Keimzentrum in einer Blutprobe, wobei der Marker eine Nukleinsäuresequenz umfasst, welche aus der Gruppe bestehend aus SEQ ID Nr.: 58, 59 und 60 oder einem Komplement von einer davon ausgewählt ist.

**2.** Verwendung nach Anspruch 1, wobei die Sonde ein Nukleinsäure-Reagens aufweist, welches mindestens zu 90 % identisch mit einer Nukleinsäure ist, welche aus der Gruppe, bestehend aus SEQ ID Nr.: 34, 35, 36, 37, 53, 54, 55, 56 und 57, oder einem Komplement von einer davon ausgewählt ist, oder
wobei die Sonde aus SEQ ID Nr.:59 oder einem Nukleinsäurefragment von SEQ ID Nr.:59, welches mindestens 15 aufeinanderfolgende Nukleotide von SEQ ID Nr.:59 umfasst, oder einem Komplement von einem davon besteht.

**3.** Kit, welcher Sonden zum Erkennen von Markern, welche aus der Gruppe bestehend aus einem mu-Keimbahntran-

skript (GLT-μ), gamma 1 enthaltenden zirkulären Transkript (CT-γ1) und gamma 2 enthaltenden zirkulären Transkript (CT-γ2) ausgewählt sind, zum Verwenden beim Feststellen der Aktivität im Keimzentrum in einer Blutprobe, wobei die Marker eine Nukleinsäuresequenz aufweisen, welche aus der Gruppe, bestehend aus SEQ ID Nr.: 58, 59 und 60 oder einem Komplement von einer davon ausgewählt ist,
wobei der Kit mindestens zwei Sonden zum Erkennen von mindestens zwei Markern umfasst.

**4.** Kit nach Anspruch 3, wobei eine der Sonden aus SEQ ID Nr.:59 oder einem Nukleinsäurefragment von SEQ ID Nr.:59, welches mindestens 15 aufeinanderfolgende Nukleotide von SEQ ID Nr.:59 umfasst, oder einem Komplement von einer davon besteht.

**5.** Kit nach Anspruch 3, wobei mindestens eine der mindestens zwei Sonden Nukleinsäure-Reagenzien umfasst, welche jeweils mindestens zu 90 % identisch mit Nukleinsäuren sind, welche aus der Gruppe, bestehend aus SEQ ID Nr.:34, 35, 36, 37, 53, 54, 55, 56 und 57 oder einem Komplement davon, ausgewählt sind.

**6.** Kit nach einem der Ansprüche 3 bis 5, ferner umfassend einen Stabilisator zum Zugeben zu der Probe oder einen Probensammelbehälter, welcher einen Stabilisator umfasst.

**7.** Kit nach Anspruch 6, wobei der Stabilisator ein RNA-Stabilisator ist.

**8.** Verfahren zum Feststellen, ob ein Patient für eine Erkrankung, welche aus der Gruppe, bestehend aus opportunistischer Infektion und Lymphom, anfällig ist, umfassend:

a. In-Kontakt-Bringen einer Blutprobe, welche einem Patienten vor der Behandlung mit einem therapeutischen Regime abgenommen wurde, mit einer Sonde zum Erkennen eines Markers, welcher aus der Gruppe, bestehend aus einem mu-Keimbahntranskript (GLT-μ), gamma 1 enthaltenden zirkulären Transkript (CT-γ1) und gamma 2 enthaltenden zirkulären Transkript (CT-γ2), ausgewählt ist, wobei der Marker eine Nukleinsäuresequenz umfasst, welche aus der Gruppe, bestehend aus SEQ ID Nr.: 58, 59 und 60, oder einem Komplement von einer davon ausgewählt ist;
b.Messen der an die Probe gebundenen Sondenmenge, um die Menge des Markers in der Probe festzustellen;
c. In-Kontakt-Bringen einer zusätzlichen Blutprobe, welche dem Patienten nach der Behandlung mit dem therapeutischen Regime abgenommen wurde, mit einer Sonde zum Erkennen eines Markers, welcher aus der Gruppe, bestehend aus einem mu-Keimbahntranskript (GLT-μ), gamma 1 enthaltenden zirkulären Transkript (CT-γ1) und gamma 2 enthaltenden zirkulären Transkript (CT-γ2), ausgewählt ist, wobei der Marker eine Nukleinsäuresequenz umfasst, welche aus der Gruppe, bestehend aus SEQ ID Nr.: 58, 59 und 60, oder einem Komplement von einer davon, ausgewählt ist;
d. Messen der an die zusätzliche Probe gebundenen Sondenmenge, um die Menge des Markers in der zusätzlichen Probe festzustellen; und
e. Feststellen, dass der Patient für die Erkrankung anfällig ist, wenn nach der Behandlung die Menge des Markers in der Probe geringer als das Niveau in der Probe vor der Behandlung ist.

**9.** Verfahren nach Anspruch 8, wobei der Patient wegen Krebses behandelt wird.

**10.** Verfahren nach Anspruch 8 oder 9, wobei die Probe eine periphere Blutprobe ist.

**11.** Verfahren nach einem der Ansprüche 8 bis 10, ferner das Anreichern der Probe mit B-Zellen umfassend.

**12.** Verfahren nach einem der Ansprüche 8 bis 11, wobei das Niveau von mindestens zwei Markern festgestellt wird.

**13.** Verfahren nach einem der Ansprüche 8 bis 12, wobei die RNA-Menge des Biomarkers in der Probe festgestellt wird.

**14.** Verfahren nach Anspruch 13, wobei die RNA in der Probe stabilisiert wird.

**15.** Verfahren zum Feststellen von Aktivität im Keimzentrum, umfassend:

a.In-Kontakt-Bringen einer Blutprobe, welche einem Patienten abgenommen wurde, mit einer Sonde zum Erkennen eines Markers, welcher aus der Gruppe, bestehend aus einem mu-Keimbahntranskript (GLT-μ), gamma 1 enthaltenden zirkulären Transkript (CT-γ1) und gamma 2 enthaltenden zirkulären Transkript (CT-γ2), ausgewählt ist, wobei der Marker eine Nukleinsäuresequenz umfasst, welche aus der Gruppe, bestehend aus SEQ ID Nr.: 58, 59 und 60, oder einem Komplement von einer davon ausgewählt ist; und
b.Messen der Sondenmenge, welche an die Probe gebunden ist, um die Menge des Markers in der Probe festzustellen und die Menge des Markers in der Probe mit einem Ausgangswert oder einem vorgegebenen Standard zu vergleichen, wodurch die Aktivität im Keimzentrum festgestellt wird.

**16.** Verfahren nach Anspruch 15, wobei der Patient wegen Krebses behandelt wird.

**17.** Verfahren nach Anspruch 15, wobei eine Menge des Markers, welche höher als ein Ausgangswert oder als ein vorgegebener Standard ist, anzeigt, dass die erworbene Immunität nach dem Entfernen eines immunmodulatorischen Agens zurückgewonnen wurde.

**18.** Verfahren nach Anspruch 15, wobei eine Menge des Markers, welche höher als ein Ausgangswert oder als ein vorgegebener Standard ist, anzeigt, dass der Patient wirksam geimpft worden ist.

**19.** Verfahren nach Anspruch 15, wobei eine Menge des Markers, welche geringer als ein Ausgangswert oder als ein vorgegebener Standard ist, anzeigt, dass der Patient für eine Erkrankung, welche aus der Gruppe, bestehend aus opportunistischer Infektion und Lymphom, ausgewählt ist, anfällig ist.

**20.** Verfahren nach einem der Ansprüche 8 bis 19, wobei die Sonde aus SEQ ID Nr.:59 oder einem Nukleinsäurefragment von SEQ ID Nr.:59, welches mindestens 15 aufeinanderfolgende Nukleotide von SEQ ID Nr.:59 umfasst, oder einem Komplement von einer davon besteht.

**21.** Verfahren nach Anspruch 15, wobei die Sonde ein Nukleinsäure-Reagens umfasst, welches mindestens zu 90 % identisch mit einer Nukleinsäure ist, welche aus der Gruppe, bestehend aus SEQ ID Nr.: 34, 35, 36, 37, 53, 54, 55, 56 und 57, oder einem Komplement von einer davon, ausgewählt ist.

**22.** Verfahren zum Screening, um einen Immunmodulator zu identifizieren, umfassend:

a) In-Kontakt-Bringen einer Blutprobe, welche B-Zellen umfasst, mit einem Testagens;
b) Messen des Expressionsniveaus eines Markers, welcher aus der Gruppe, bestehend aus einem mu-Keimbahntranskript (GLT-$\mu$), gamma 1 enthaltenden zirkulären Transkript (CT-$\gamma$1) und gamma 2 enthaltenden zirkulären Transkript (CT-$\gamma$2), ausgewählt ist, wobei der Marker eine Nukleinsäuresequenz umfasst, welche aus der Gruppe, bestehend aus SEQ ID Nr.: 58, 59 und 60, oder einem Komplement von einer davon, ausgewählt ist; und
c) Feststellen, dass das Testagens ein Immunmodulator ist, wenn das Expressionsniveau des Markers wesentlich anders als das Niveau in einer Probe ist, mit welcher das Testagens nicht in Kontakt gebracht wurde.

## Revendications

**1.** Utilisation d'une sonde pour détecter un marqueur choisi dans le groupe constitué du produit de la transcription de la lignée germinale mu (GLT-$\mu$), du produit de la transcription en cercle contenant gamma 1 (CT-$\gamma$1), et du produit de la transcription en cercle contenant gamma 2 (CT-$\gamma$2), dans la détermination de l'activité du centre germinatif dans un échantillon de sang, dans laquelle le marqueur comprend une séquence d'acide nucléique choisie dans le groupe constitué des SEQ ID N° 58, 59 et 60 ou d'un complément de l'une quelconque de celles-ci.

**2.** Utilisation selon la revendication 1 dans laquelle la sonde comprend un réactif à acide nucléique au moins 90 % identique à un acide nucléique choisi dans le groupe constitué des SEQ ID N° 34, 35, 36, 37, 53, 54, 55, 56 et 57 ou d'un complément de l'une quelconque de celles-ci, ou
dans laquelle la sonde est constituée de la SEQ ID N° 59 ou d'un fragment d'acide nucléique de SEQ ID N° 59 comprenant au moins 15 nucléotides consécutifs de SEQ ID N° 59 ou d'un complément de l'un quelconque de ceux-ci.

**3.** Trousse comprenant des sondes pour détecter des marqueurs choisis dans le groupe constitué du produit de la transcription de la lignée germinale mu (GLT-$\mu$), du produit de la transcription en cercle contenant gamma 1 (CT-$\gamma$1) et du produit de la transcription en cercle contenant gamma 2 (CT-$\gamma$2), pour utilisation dans la détermination de l'activité du centre germinatif dans un échantillon de sang, dans laquelle les marqueurs comprennent une séquence d'acide nucléique choisie dans le groupe constitué des SEQ ID N° 58, 59 et 60 ou d'un complément de l'une quelconque de celles-ci,

laquelle trousse comprend au moins deux sondes pour détecter au moins deux marqueurs.

4. Trousse selon la revendication 3 dans laquelle l'une des sondes est constituée de la SEQ ID N° 59 ou d'un fragment d'acide nucléique de SEQ ID N° 59 comprenant au moins 15 nucléotides consécutifs de SEQ ID N° 59 ou d'un complément de l'un quelconque de ceux-ci.

5. Trousse selon la revendication 3, dans laquelle au moins une des au moins deux sondes comprend des réactifs à acides nucléiques, chacun au moins 90 % identique aux acides nucléiques choisis dans le groupe constitué des SEQ ID N° 34, 35, 36, 37, 53, 54, 55, 56 et 57 ou d'un complément de l'une quelconque de celles-ci.

6. Trousse selon l'une quelconque des revendications 3 à 5, comprenant en outre un stabilisant à ajouter à l'échantillon ou à un récipient de collecte d'échantillons comprenant un stabilisant.

7. Trousse selon la revendication 6, dans laquelle le stabilisant est un stabilisant d'ARN.

8. Méthode destinée à déterminer si un patient est susceptible de développer un trouble choisi dans le groupe constitué d'une infection opportuniste et d'un lymphome, comprenant :

a. La mise en contact d'un échantillon de sang qui a été obtenu auprès d'un patient avant le traitement par un régime thérapeutique avec une sonde pour détecter un marqueur choisi dans le groupe constitué du produit de la transcription de la lignée germinale mu (GLT-μ), du produit de la transcription en cercle contenant gamma 1 (CT-γ1) et du produit de la transcription en cercle contenant gamma 2 (CT-y2), dans laquelle le marqueur comprend une séquence d'acide nucléique choisie dans le groupe constitué des SEQ ID N° 58, 59 et 60 ou d'un complément de l'une quelconque de celles-ci ;
b. La mesure de la quantité de sonde liée à l'échantillon pour déterminer la quantité du marqueur dans l'échantillon ;
c. La mise en contact d'un échantillon de sang additionnel qui a été obtenu auprès du patient après le traitement par le régime thérapeutique avec une sonde pour détecter un marqueur choisi dans le groupe constitué du produit de la transcription de la lignée germinale mu (GLT-μ), du produit de la transcription en cercle contenant gamma 1 (CT-γ1) et du produit de la transcription en cercle contenant gamma 2 (CT-γ2), dans laquelle le marqueur comprend une séquence d'acide nucléique choisie dans le groupe constitué des SEQ ID N° 58, 59 et 60 ou d'un complément de l'une quelconque de celles-ci ;
d. La mesure de la quantité de sonde liée à l'échantillon additionnel pour déterminer la quantité du marqueur dans l'échantillon additionnel ; et
e. La détermination que le patient est susceptible de développer le trouble si la quantité de marqueur dans l'échantillon après le traitement est inférieure à la teneur dans l'échantillon avant le traitement.

9. Méthode selon la revendication 8, dans laquelle le patient est traité pour un cancer.

10. Méthode selon la revendication 8 ou 9, dans laquelle l'échantillon est un échantillon de sang périphérique.

11. Méthode selon l'une quelconque des revendications 8 à 10, comprenant en outre l'enrichissement de l'échantillon en lymphocytes B.

12. Méthode selon l'une quelconque des revendications 8 à 11, dans laquelle la teneur d'au moins deux marqueurs est déterminée.

13. Méthode selon l'une quelconque des revendications 8 à 12, dans laquelle la quantité d'ARN du biomarqueur dans l'échantillon est déterminée.

14. Méthode selon la revendication 13, dans laquelle l'ARN dans l'échantillon est stabilisé.

15. Méthode de détermination de l'activité du centre germinatif, comprenant :

a. La mise en contact d'un échantillon de sang qui a été obtenu auprès d'un patient avec une sonde pour détecter un marqueur choisi dans le groupe constitué du produit de la transcription de la lignée germinale mu (GLT-μ), du produit de la transcription en cercle contenant gamma 1 (CT-γ1) et du produit de la transcription en cercle contenant gamma 2 (CT-γ2), dans laquelle le marqueur comprend une séquence d'acide nucléique choisie dans le groupe constitué des SEQ ID N° 58, 59 et 60 ou d'un complément de l'une quelconque de celles-ci ; et

b. La mesure de la quantité de sonde liée à l'échantillon pour déterminer la quantité de marqueur dans l'échantillon et comparer la quantité de marqueur dans l'échantillon à une valeur de référence ou à un étalon prédéterminé, en déterminant ainsi l'activité du centre germinatif.

**16.** Méthode selon la revendication 15, dans laquelle le patient est traité pour un cancer.

**17.** Méthode selon la revendication 15, dans laquelle une quantité du marqueur qui est supérieure à la valeur de référence ou à un étalon prédéterminé indique que l'immunité adaptive a récupéré après élimination d'un agent immunomodulateur.

**18.** Méthode selon la revendication 15, dans laquelle une quantité du marqueur qui est supérieure à la valeur de référence ou à un étalon prédéterminé indique que le patient a reçu une vaccination efficace.

**19.** Méthode selon la revendication 15, dans laquelle une quantité du marqueur qui est inférieure à la valeur de référence ou à un étalon prédéterminé indique que le patient est susceptible de développer un trouble choisi dans le groupe constitué d'une infection opportuniste et d'un lymphome.

**20.** Méthode selon l'une quelconque des revendications 8 à 19, dans laquelle la sonde est constituée de la SEQ ID N° 59 ou d'un fragment d'acide nucléique de SEQ ID N° 59 comprenant au moins 15 nucléotides consécutifs de SEQ ID N° 59 ou d'un complément de l'un quelconque de ceux-ci.

**21.** Méthode selon la revendication 15, dans laquelle la sonde comprend un réactif à acide nucléique au moins 90 % identique à un acide nucléique choisi dans le groupe constitué des SEQ ID N° 34, 35, 36, 37, 53, 54, 55, 56 et 57 ou d'un complément de l'une quelconque de celles-ci.

**22.** Méthode de criblage destinée à identifier un immunomodulateur comprenant :

a) la mise en contact d'un échantillon de sang comprenant des lymphocytes B avec un agent d'essai ;

b) la mesure du niveau d'expression d'un marqueur choisi dans le groupe constitué du produit de la transcription de la lignée germinale mu (GLT-$\mu$), du produit de la transcription en cercle contenant gamma 1 (CT-$\gamma$1) et du produit de la transcription en cercle contenant gamma 2 (CT-$\gamma$2), dans laquelle le marqueur comprend une séquence d'acide nucléique choisie dans le groupe constitué des SEQ ID N° 58, 59 et 60 ou d'un complément de l'une quelconque de celles-ci, et

c) la détermination que l'agent d'essai est un immunomodulateur si le niveau d'expression du marqueur est sensiblement différent du niveau dans un échantillon qui n'est pas entré en contact avec l'agent d'essai.

Figure 1

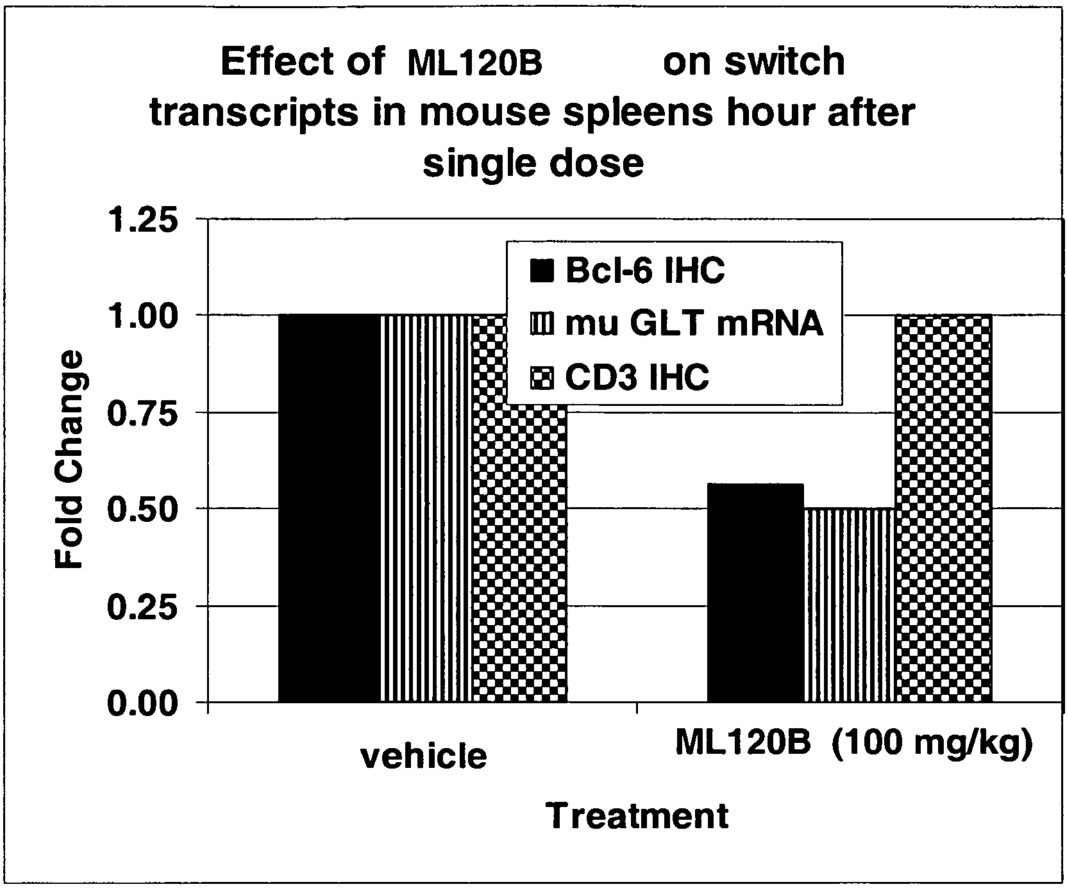
Effect of ML120B on switch transcripts in mouse spleens hour after single dose
1.25
1.00
0.75
0.50
0.25
0.00
Fold Change
Bcl-6 IHC
mu GLT mRNA
CD3 IHC
vehicle
ML120B (100 mg/kg)
Treatment

Figure 2

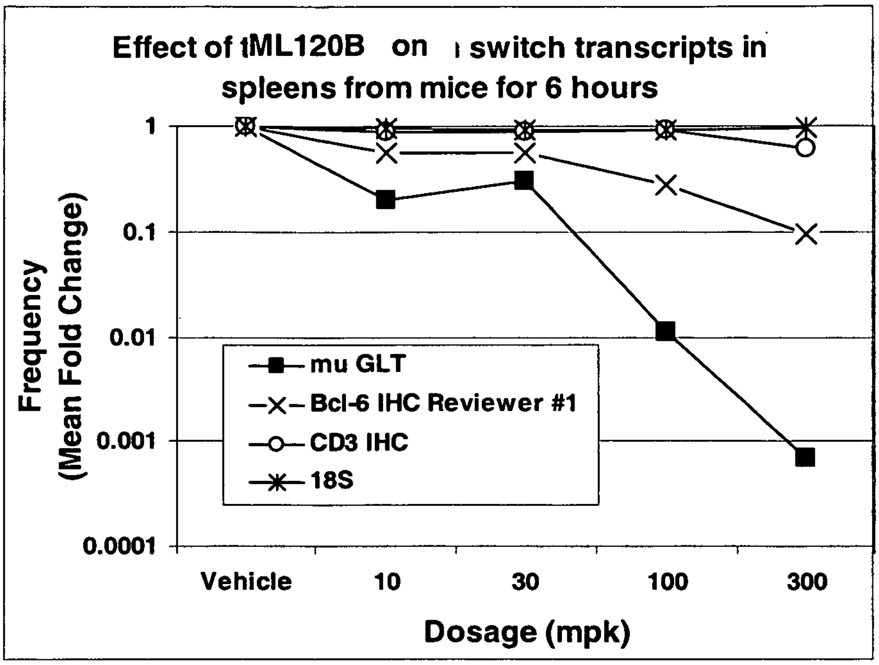


Figure 3

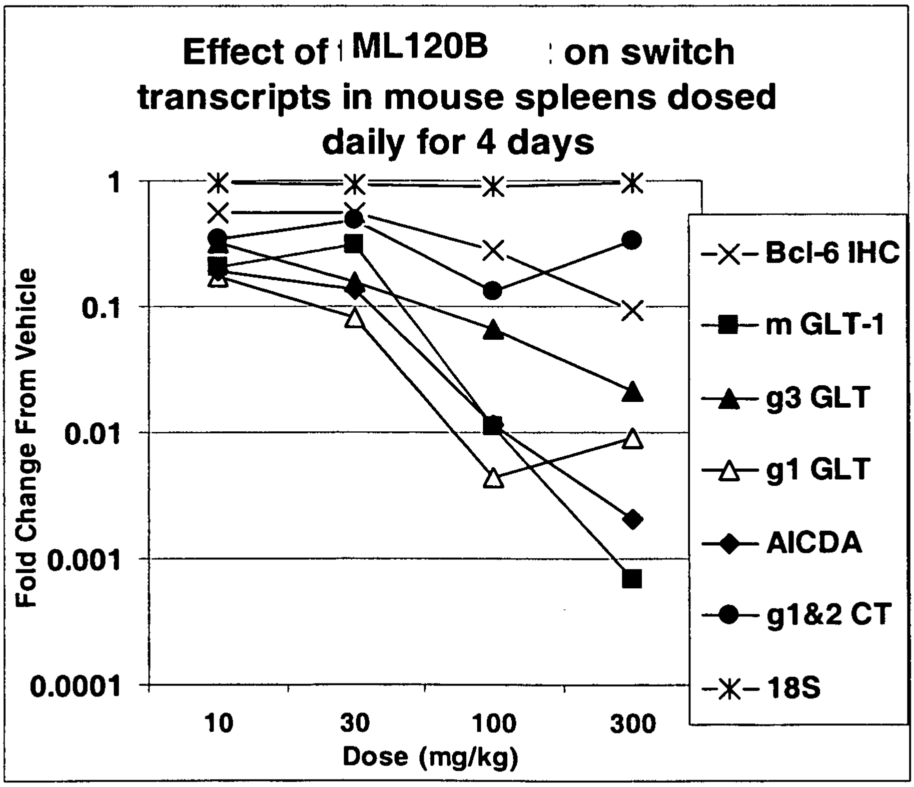

Figure 4

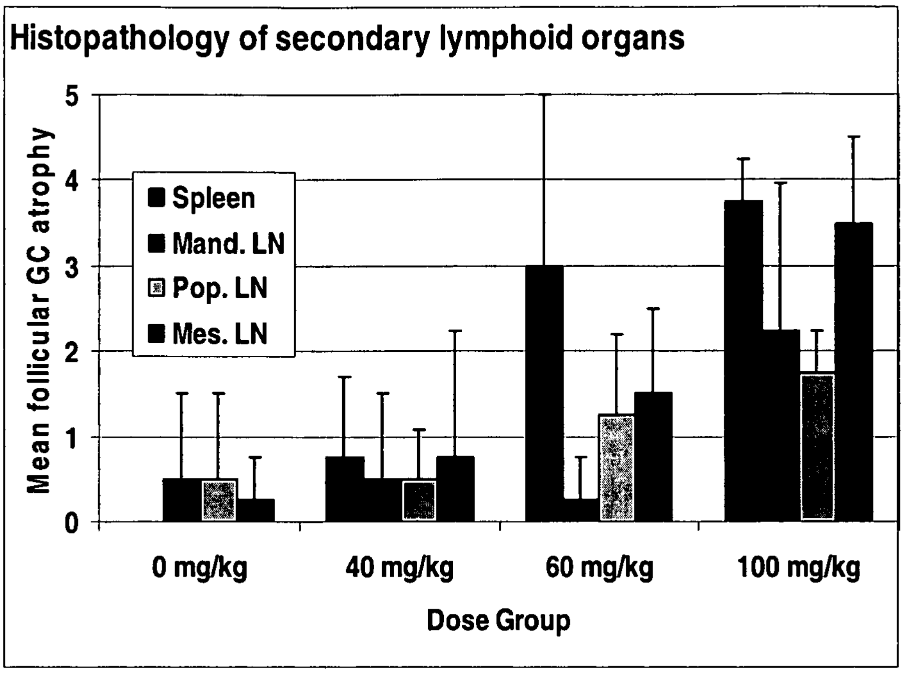


Figure 5

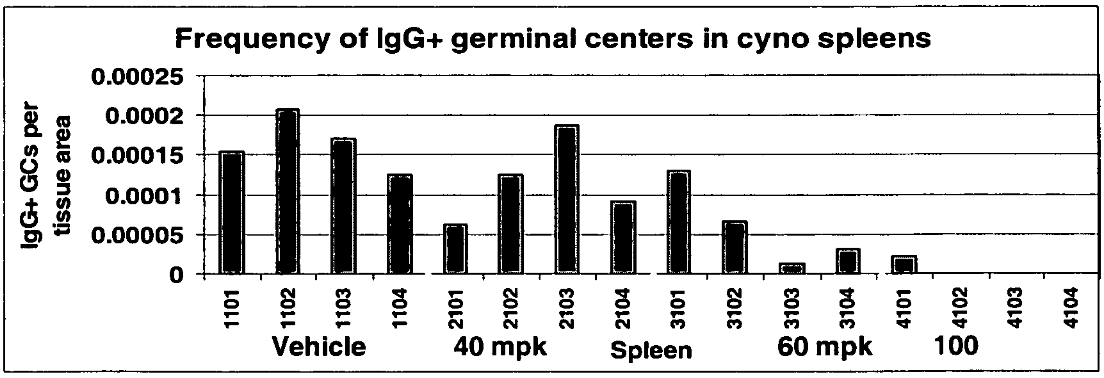

Figure 6A

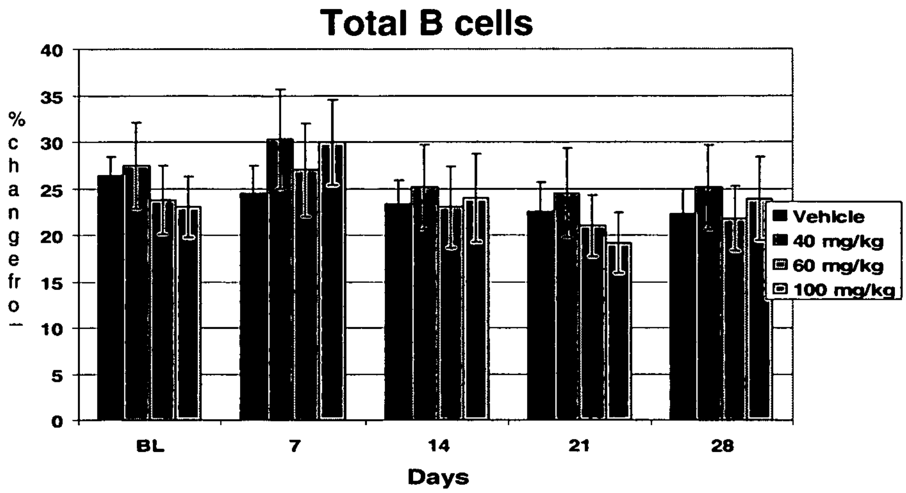


Figure 6B

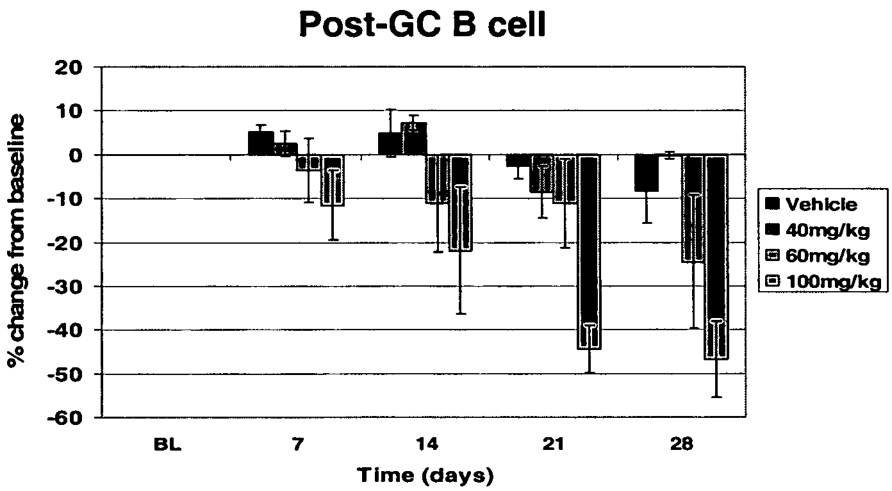


Figure 6C

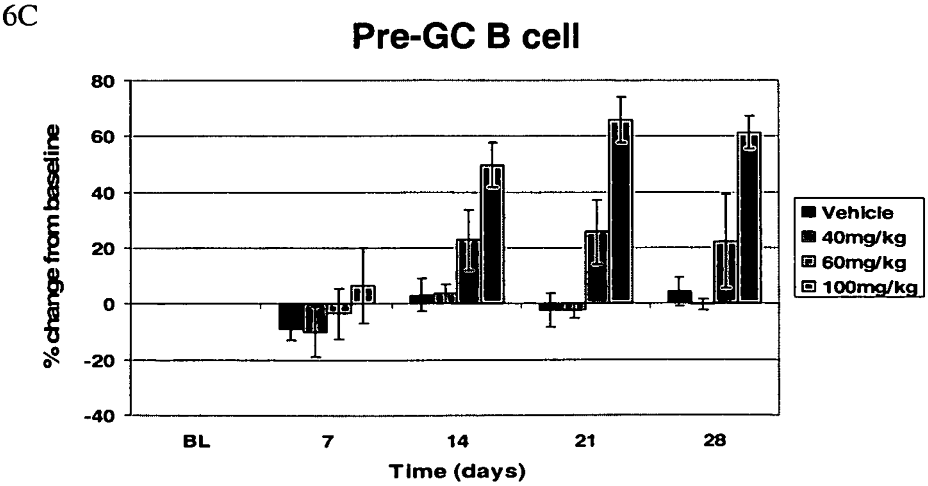

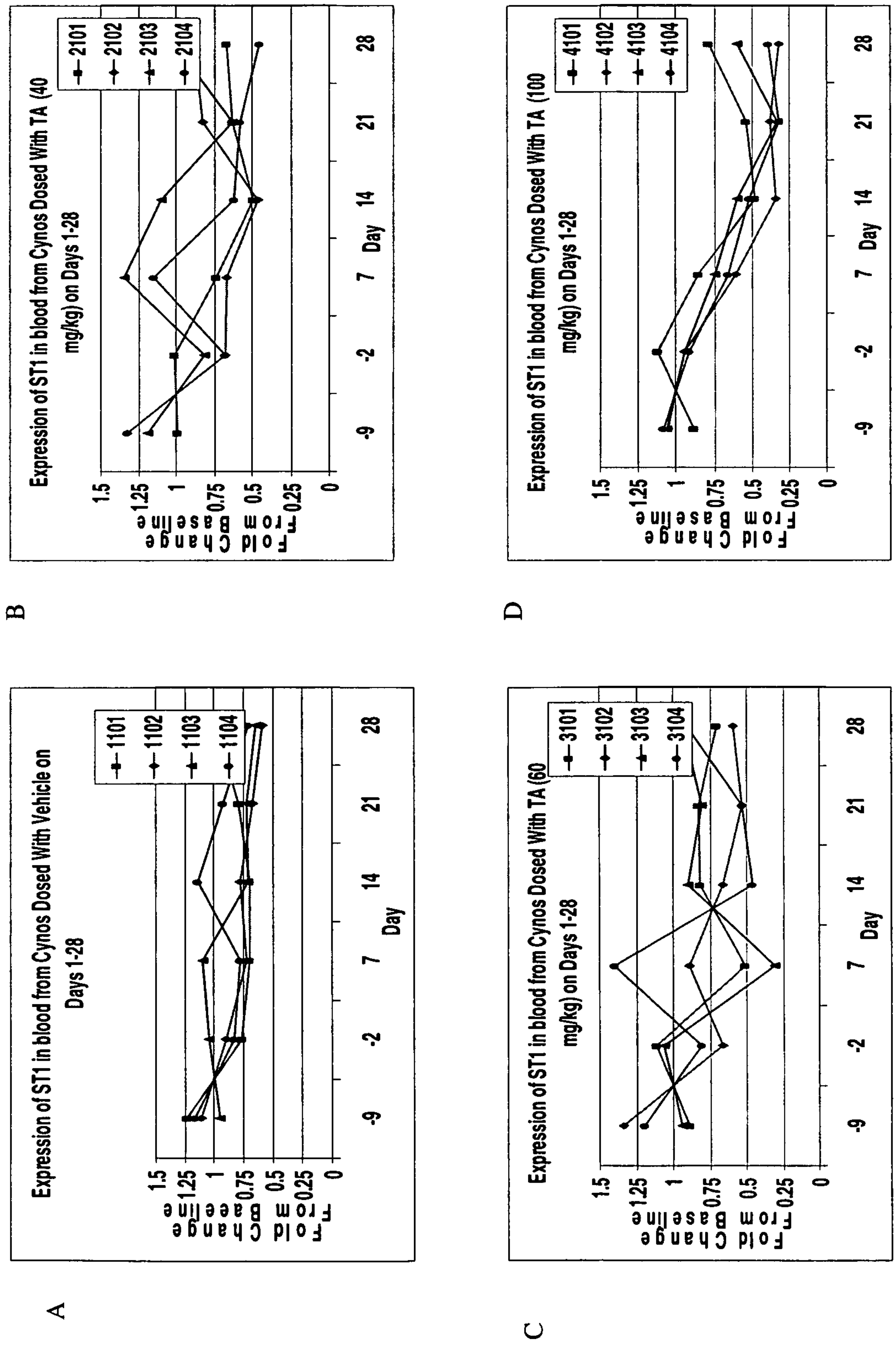
A
Expression of ST1 in blood from Cynos Dosed With Vehicle on Days 1-28
Fold Change From Baseline
Day
1101
1102
1103
1104
B
Expression of ST1 in blood from Cynos Dosed With TA (40 mg/kg) on Days 1-28
Fold Change From Baseline
Day
2101
2102
2103
2104
C
Expression of ST1 in blood from Cynos Dosed With TA (60 mg/kg) on Days 1-28
Fold Change From Baseline
Day
3101
3102
3103
3104
D
Expression of ST1 in blood from Cynos Dosed With TA (100 mg/kg) on Days 1-28
Fold Change From Baseline
Day
4101
4102
4103
4104

Figures 7 A-D

Figure 8A

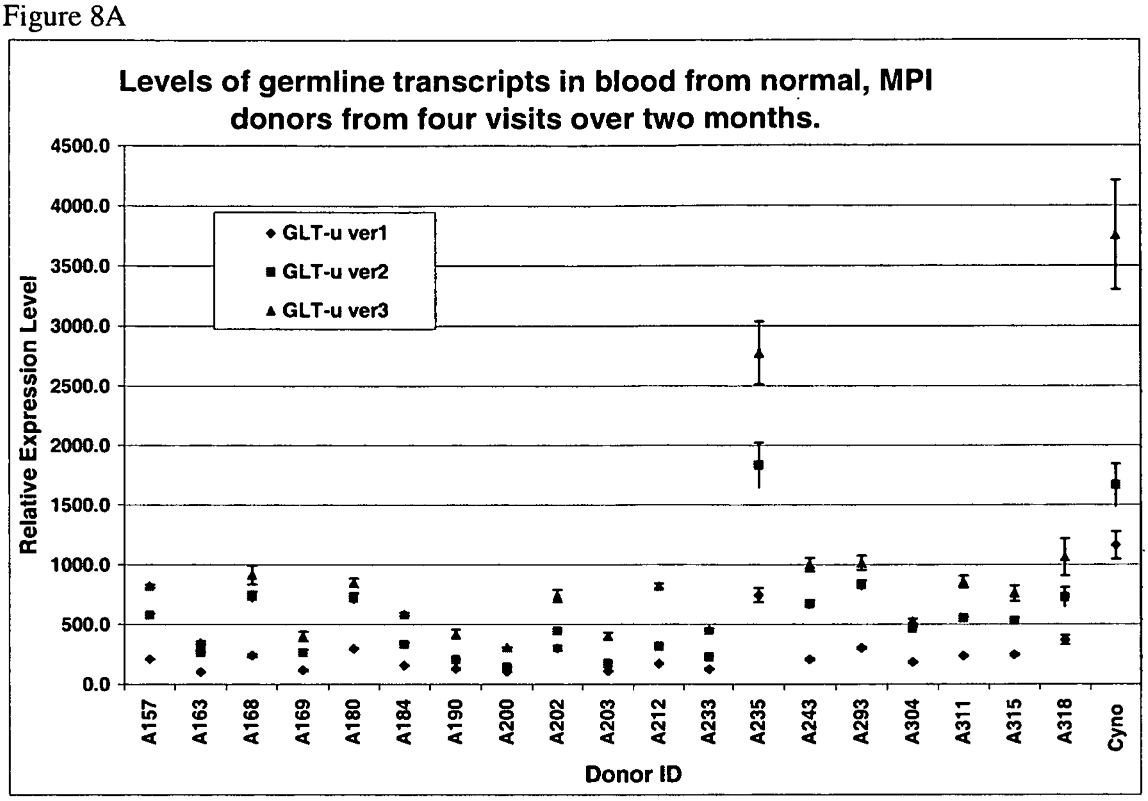


Figure 8B

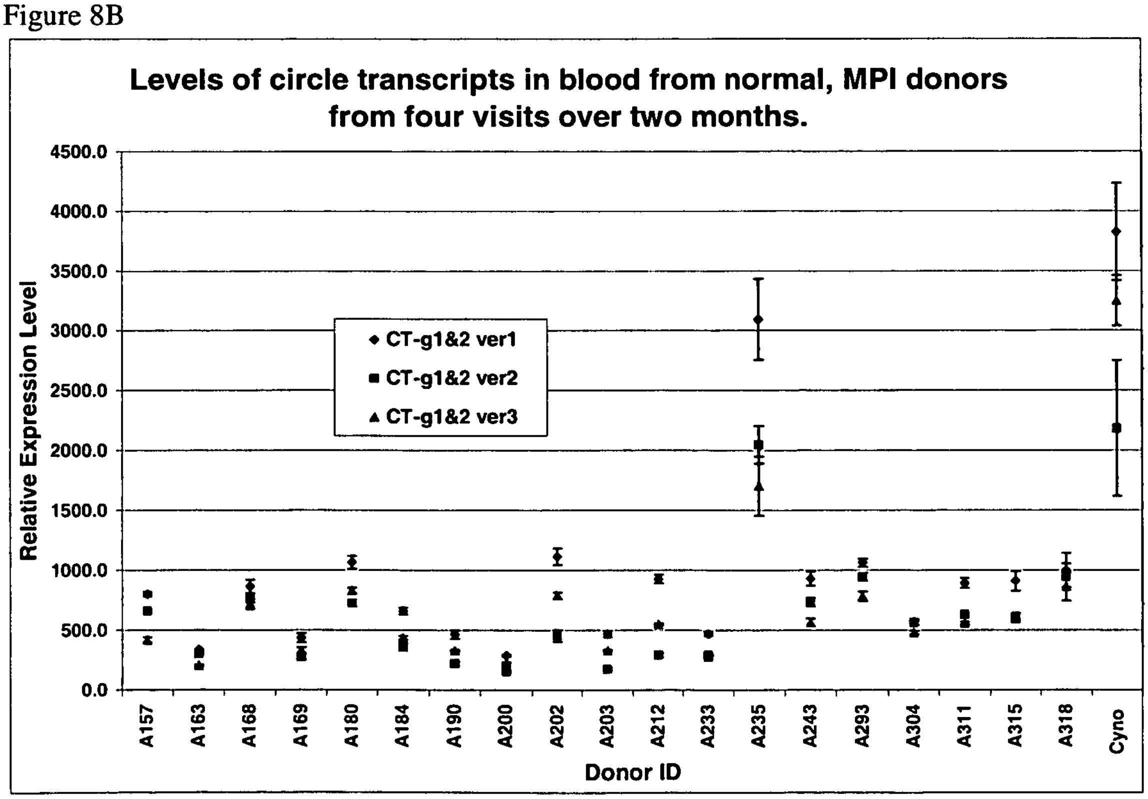

Figure 9

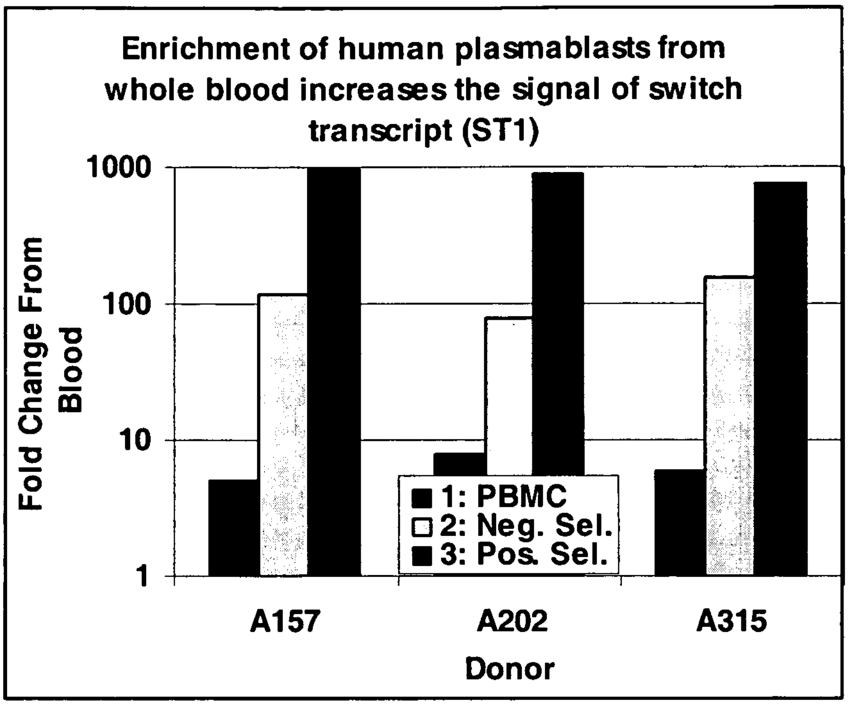


Figure 10

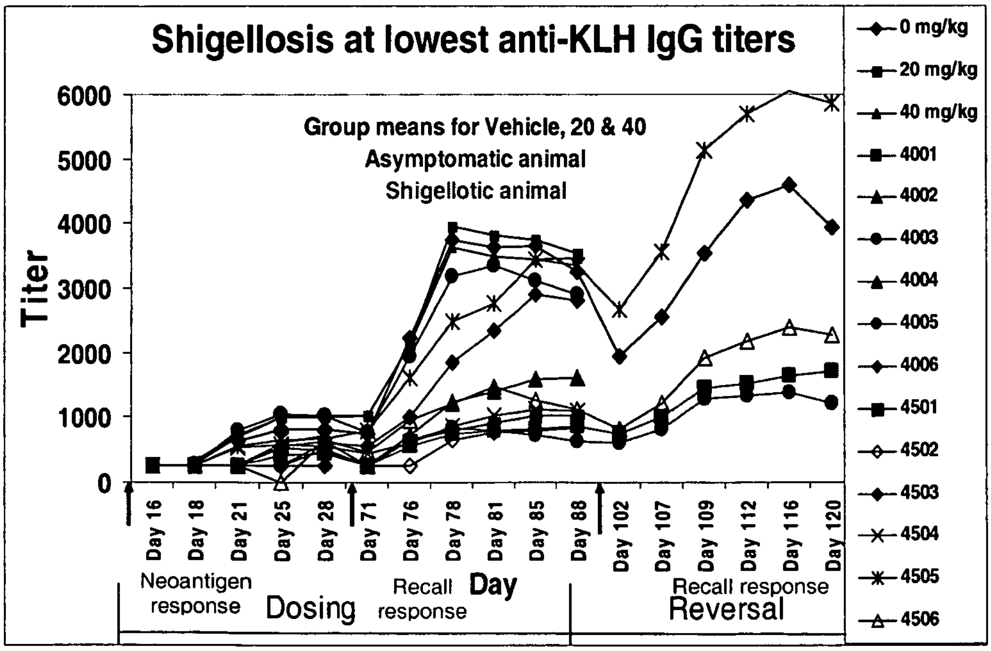

Figure 11A

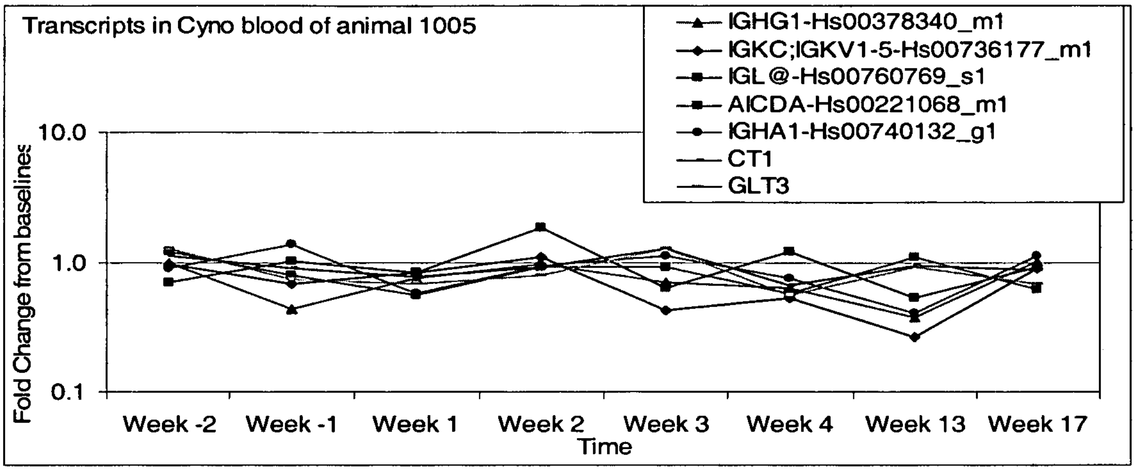


Figure 11B

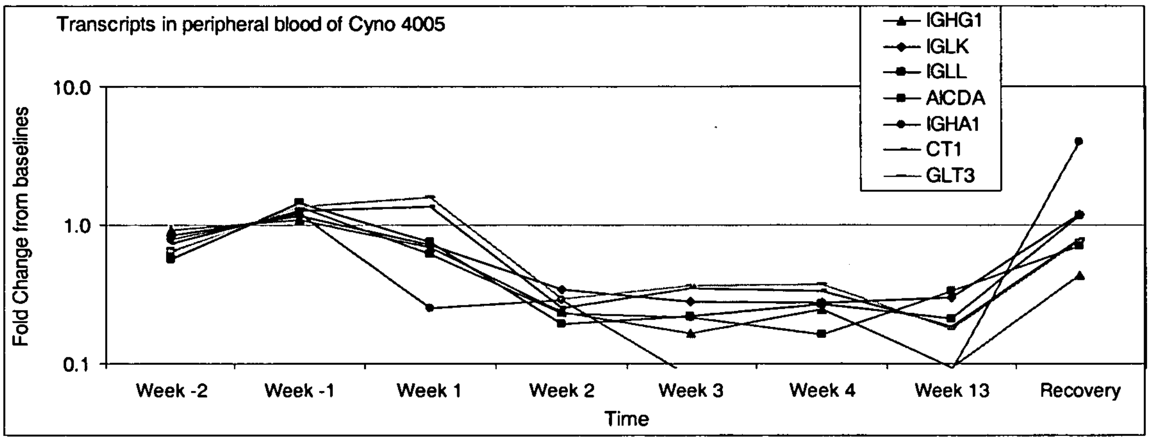

Fig 12 A-H

12A

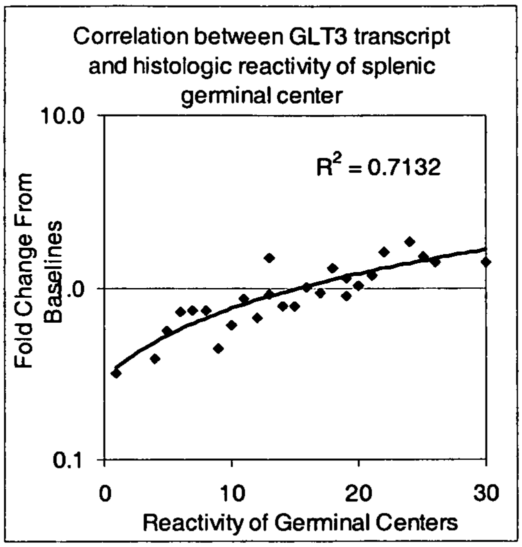


12B

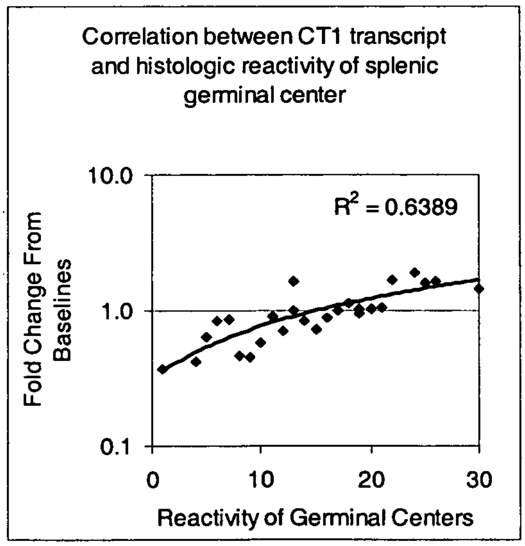

12C

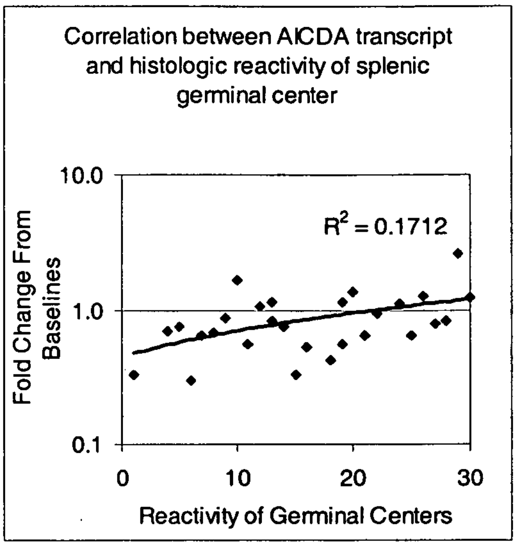
Correlation between AICDA transcript and histologic reactivity of splenic germinal center
Fold Change From Baselines
10.0
1.0
0.1
$R^2 = 0.1712$
0
10
20
30
Reactivity of Germinal Centers

12D

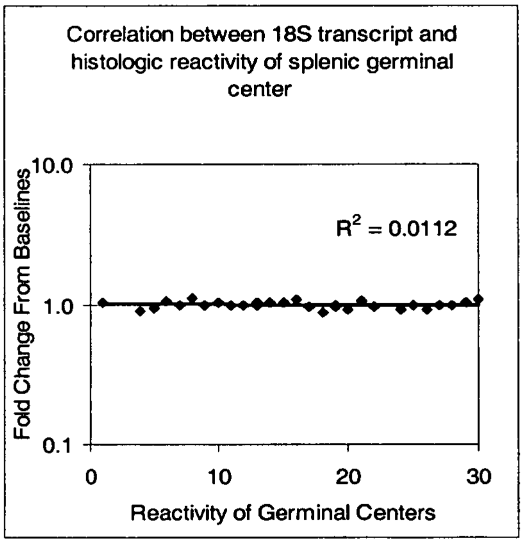
Correlation between 18S transcript and histologic reactivity of splenic germinal center
Fold Change From Baselines
10.0
1.0
0.1
$R^2 = 0.0112$
0
10
20
30
Reactivity of Germinal Centers

12E

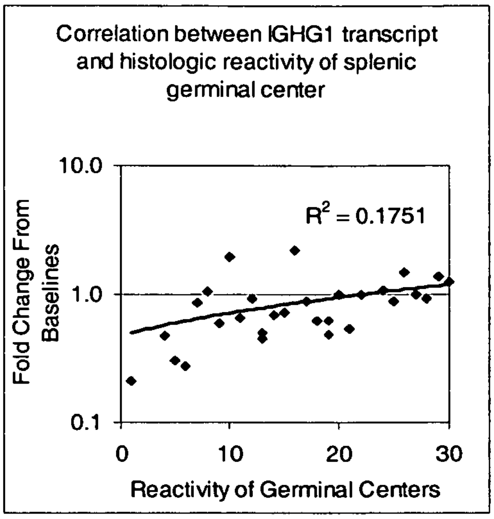
Correlation between IGHG1 transcript and histologic reactivity of splenic germinal center
Fold Change From Baselines
10.0
1.0
0.1
R2 = 0.1751
0
10
20
30
Reactivity of Germinal Centers

12F

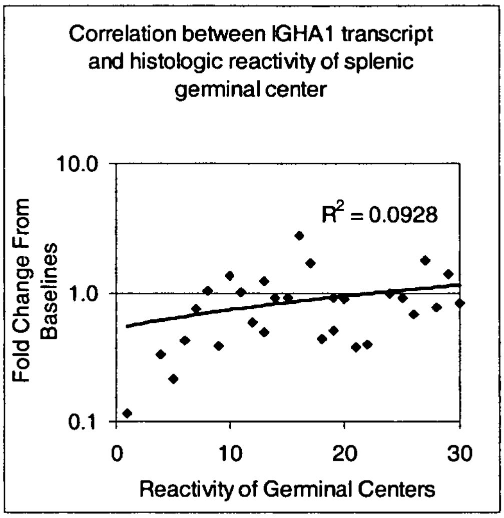
Correlation between IGHA1 transcript and histologic reactivity of splenic germinal center
Fold Change From Baselines
10.0
1.0
0.1
R2 = 0.0928
0
10
20
30
Reactivity of Germinal Centers

12G

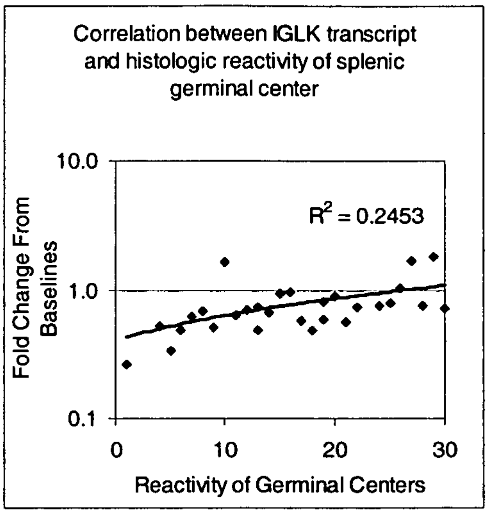
Correlation between IGLK transcript
and histologic reactivity of splenic
germinal center
10.0
1.0
0.1
Fold Change From
Baselines
$R^2$ = 0.2453
0
10
20
30
Reactivity of Germinal Centers

12H

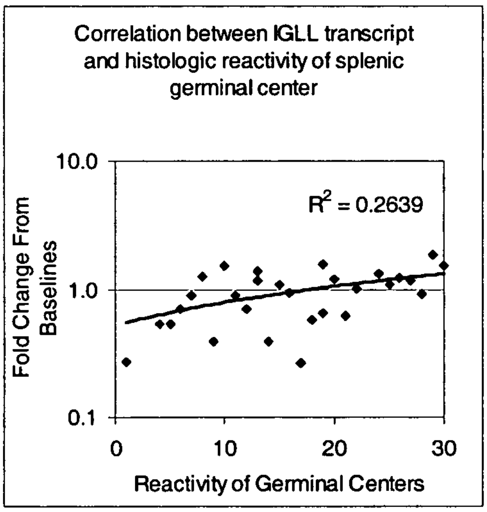
Correlation between IGLL transcript
and histologic reactivity of splenic
germinal center
10.0
1.0
0.1
Fold Change From
Baselines
$R^2$ = 0.2639
0
10
20
30
Reactivity of Germinal Centers

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4843155 A, Chomczynski
- US 5346994 A
- US 5539083 A
- WO 0105935 A, Friend
- US 6033862 A, Matsuda
- US 6627637 B
- US 7026331 B
- US 20040235839 A
- US 5693617 A
- US 6831099 B
- US 6310057 B
- US 5756764 A
- WO 05002572 A
- US 7276530 B
- US 5631169 A, Lakowicz
- US 4868103 A, Stavrianopoulos
- US 7045610 B
- US 4683202 A, Mullis
- US 5854033 A, Lizardi
- US 5242974 A
- US 5384261 A
- US 5405783 A
- US 5412087 A
- US 5424186 A
- US 5429807 A
- US 5436327 A
- US 544593 A
- US 5556752 A
- US 5472672 A
- US 5527681 A
- US 5529756 A
- US 5545531 A
- US 5554501 A
- US 5561071 A
- US 5571639 A
- US 5593839 A
- US 5624711 A
- US 5700637 A
- US 5744305 A
- US 5770456 A
- US 5770722 A
- US 5837832 A
- US 5856101 A
- US 5874219 A
- US 5885837 A
- US 5919523 A
- US 5981185 A
- US 6022963 A
- US 6077674 A
- US 6156501 A
- US 6261776 B
- US 6346413 B
- US 6440677 B
- US 6451536 B
- US 6576424 B
- US 6610482 B
- US 5143854 A
- US 5288644 A
- US 5324633 A
- US 5432049 A
- US 5470710 A
- US 5492806 A
- US 5503980 A
- US 5510270 A
- US 5525464 A
- US 5547839 A
- US 5580732 A
- US 5661028 A
- US 5848659 A
- US 6331415 B
- US 61127522 B


### Non-patent literature cited in the description


- **SNAPPER et al.** *Immunity,* 1997, vol. 6, 217-23
- **CHAUDHURI ; ALT.** *Nat. Rev. Immunol.,* 2004, vol. 4, 541-52, 655
- **CHAUDHURI ; ALT.** *Nat. Rev. Immunol.,* 2004, vol. 4, 541-52
- **REVY et al.** *Cell,* 2000, vol. 102, 565-75
- **IMAI et al.** *Nature Immunol.,* 2003, vol. 4, 1023-28
- **ROLINK et al.** *Immunity,* 1996, vol. 5, 319-30
- **MANIS et al.** *J. Exp. Med.,* 1998, vol. 187, 2081-9
- **CASELLAS et al.** *EMBO J.,* 1998, vol. 17, 2404-11
- **ZELAZOWSKI et al.** *J. Immunol.,* 1997, vol. 159, 2559-62

- **GIRSCHICK et al.** *J. Immunol.,* 2001, vol. 166, 377-86
- **RAMESH et al.** Primary Immunodeficiency Diseases: A Molecular and Genetic Approach. Oxford University Press, 1999, 233-49
- **CASTIGLI et al.** *Proc. Natl. Acad. Sci. USA,* 1994, vol. 91, 12135-9
- **FERRARI et al.** *Proc. Natl. Acad. Sci. USA,* 2001, vol. 98, 12614-9
- **KUNKEL ; BUTCHER.** *Nat. Rev. Immunol.,* 2003, vol. 3, 882-9
- **FRASCA et al.** *J. Immunology,* 2008, vol. 180, 2741-2746
- **AUSUBEL et al.** Current Protocols in Molecular Biology. John Wiley & Sons, 1987
- **CHOMCZYNSKI ; SACCHI.** *Anal. Biochem.,* 1987, vol. 162, 156-159
- **CHIRGWIN et al.** *Biochemistry,* 1979, vol. 18, 5294-99
- **SAMBROOK et al.** Molecular Cloning--A Laboratory Manual. Cold Spring Harbor Laboratory, 1989
- **AUSUBEL et al.** Current Protocols In Molecular Biology. Current Protocols Publishing, 1994, vol. 2
- **EGHOLM et al.** *Nature,* 1993, vol. 363, 566-568
- **HUGHES et al.** *Nat. Biotech.,* 2001, vol. 19, 342-7
- **LITTELL ; MILIKEN ; STROUP ; WOLFINGER ; SCHABENBERGER.** SAS for Mixed Models. SAS Institute, Inc, 2006
- **KOOMEN et al.** *J. Mass. Spectrom.,* 2000, vol. 35, 258-264
- **JAMES.** *AIDS Treatment News Archive,* 1994, vol. 209
- **HATTIS et al.** *Env. Health Perspect.,* 1991, vol. 90, 229-238
- **SCHENTAG.** *Am. J. Health-Syst. Pharm.,* 1999, vol. 56 (3), S21-S24
- **NICOLAU.** *Am, J. Health-Syst. Pharm.,* 1999, vol. 56 (3), S16-S20
- **ANOLIK et al.** *J. Immunol.,* 2008, vol. 180, 688-692
- **NAGASHIMA et al.** *Blood,* 2006, vol. 107, 4266-73
- **DEVOS et al.** *Eur. J. Immunol.,* 2004, vol. 34, 3446-55
- **NYMAN et al.** *Blood,* 2007, vol. 109, 4930-5
- **ANOLIK et al.** *Arthritis Rheum.,* 2007, vol. 56, 3044-56
- **SACKSTEIN ; BORENSTEIN.** *J. Invest. Med.,* 1995, vol. 43, 68-77
- **SCHEINMAN et al.** *Science,* 1995, vol. 270, 283-6
- **KUPER et al.** *Toxicol. Pathol.,* 2007, vol. 35, 226-32
- **GORE et al.** *Toxicology,* 2008, vol. 197, 23-35
- **WOODLAND et al.** *Blood,* 2008, vol. 111, 750-60
- **SJOLANDER, S. ; URBANICZKY, C.** *Anal. Chem.,* 1991, vol. 63, 2338-2345
- **SZABO et al.** *Curr. Opin. Struct. Biol.,* 1995, vol. 5, 699-705
- **RIVAS, G. ; MINTON, A.P.** *Trends Biochem Sci.,* 1993, vol. 18, 284-7
- **HEEGAARD, N.H.** *J. Mol. Recognit.,* 1998, vol. 11, 141-8
- **HAGE, D.S. ; TWEED, S.A.** *J. Chromatogr. B. Biomed. Sci. Appl.,* 1997, vol. 699, 499-525
- Current Protocols in Molecular Biology. John Wiley & Sons, 1987
- Current Protocols in Molecular Biology. John Wiley & Sons, Inc, 1995
- **SAMBROOK et al.** Molecular Cloning: A Laboratory Manual. Cold Spring Harbor Press, 1989
- **CHURCH ; GILBERT.** *Proc. Natl. Acad. Sci. USA,* 1984, vol. 81, 1991-1995
- **BARANY.** *Proc. Natl. Acad. Sci. USA,* 1991, vol. 88, 189-193
- **GUATELLI et al.** *Proc. Natl. Acad. Sci. USA,* 1990, vol. 87, 1874-1878
- **KWOH et al.** *Proc. Natl. Acad. Sci. USA,* 1989, vol. 86, 1173-1177
- **LIZARDI et al.** *Bio/Technology,* 1988, vol. 6, 1197
- **LIAO et al.** *Arthritis Rheum.,* 2004, vol. 50, 3792-3803
- **SHENA et al.** *Tibtech,* 1998, vol. 16, 301
- **DUGGAN et al.** *Nat. Genet.,* 1999, vol. 21, 10
- **BOWTELL et al.** *Nat. Genet.,* 1999, vol. 21, 25
- **LIPSHUTZ et al.** *Nature Genet.,* 1999, vol. 21, 20-24
- **BLANCHARD et al.** *Biosensors and Bioelectronics,* 1996, vol. 11, 687-90
- **MASKOS et al.** *Nucleic Acids Res.,* 1993, vol. 21, 4663-69
- **HUGHES et al.** *Nat. Biotechol.,* 2001, vol. 19, 342
- **HANS et al.** *Blood,* 2004, vol. 103, 275-282
- **BRADFORD et al.** *Urol. Oncol.,* 2006, vol. 24, 237-242
- **KOHLER ; MILSTEIN.** 256. *Nature,* 1975, 495
- **KOZBOR et al.** *Immunol. Today,* 1983, vol. 4, 72
- **COLE et al.** In Monoclonal Antibodies and Cancer Therapy. Alan R. Liss, Inc, 1985, 77-96
- **HARLOW, E. ; LANE, D.** Antibodies: A Laboratory Manual. Cold Spring Harbor Laboratory Press, 1988
- Current Protocols in Immunology. John Wiley & Sons, 1994
- **PASPARAKIS et al.** *J. Exp. Med.,* 2002, vol. 196, 743-52
- **LI et al.** *J. Immunol.,* 2003, vol. 170, 4630-7

- **REN et al.** *J. Immunol.,* 2002, vol. 168, 577-87
- **SCHROEDER et al.** *BMC Mol. Biol.,* 2006, vol. 7, 3

SEQUENCE LISTING

<110> Millennium Pharmaceuticals, Inc. Fedyk, Eric R.

<120> Methods and Kits for Monitoring the
Effects of Immunomodulators on Adaptive Immunity

<130> MPI08-003P1WOM

<150> US 61/127522
<151> 2008-05-14

<160> 60

<170> FastSEQ for Windows Version 4.0

<210> 1
<211> 6121
<212> DNA
<213> Artificial Sequence

<220>
<223> Nucleotides 961381 to 967501 of GenBank Accession No. NG_001019 (Jan 10, 2006 version).

<400> 1

```
gtaagatgtt taagaaatta aacagtctta gggagagttt atgactgtat tcaaaaagtt 60
ttttaaatta gcttgttatc ccttcatgtg ataactaatc tcaaatactt tttcgatacc 120
tcagagcatt attttcataa tgactgtgtt cacaatcttt ttaggttaac tcgttttctc 180
tttgtgatta aggagaaaca ctttgatatt ctgatagagt ggccttcatt ttagtatttt 240
tcaagaccac ttttcaacta ctcactttag gataagtttt aggtaaaatg tgcatcatta 300
tcctgaatta tttcagttaa gcatgttagt tggtggcata agagaaaact caatcagata 360
gtgctgaaga caggactgtg gagacacctt agaaggacag attctgttcc gaatcaccga 420
tgcggcgtca gcaggactgg cctagcggag gctctgggag ggtggctgcc aggcccggcc 480
tgggctttgg gtctccccgg actacccaga gctgggatgc gtggcttctg ctgccgggcc 540
gactggctgc tcaggcccca gcccttgtta atggacttgg aggaatgatt ccatgccaaa 600
gctttgcaag gctcgcagtg accaggcgcc cgacatggta agagacaggc agccgccgct 660
gctgcatttg cttctcttaa aactttgtat ttgacgtctt atttccacta gaaggggaac 720
tggtcttaat tgcttgatga agagcaggag actcatttat gtgagtcttt tgagtgacca 780
ttgtctgggt cactcccatt taactttccc taaagcccat ttgaaggaga ggtcgcacga 840
gctgctccac aacctctgaa tggggatggc atgggtaatg atgcttgaga acataccaag 900
ccccactggc atcgcccttg tctaagtcat tgactgtagg tcatcatcgc acccttgaaa 960
gtagcccatg ccttccaaag cgatttatgg taaatggcag aattttaagt ggcaaattca 1020
gataaaatgc atttcttggt tgtttccaat gatgactgtt atctagaggg aatttaaagg 1080
caggggttta ctgcagactc agaagggagg ggatgctccg ggaaggtgga ggctctgagc 1140
atctcaatac cctcctcttg gtgcagaaga tatgctgcca cttctagagc aaggggacct 1200
gctcattttt atcacagcac aggctcctaa attcttggtc tcattctcaa gatgttttaa 1260
tgactttaaa gcagcaaaga aatattccac ccaggtagtg gagggtggta atgattggta 1320
atgctttgga accaaaaccc aggtggcgct ggggcaggac tgcagggaac tggggtatca 1380
agtagaggga gacaaaagat ggaagccagc ctggctgtgc aggaacctgg caatgagatg 1440
gctttagctg agacaagcag gtctggtggg ctgaccattt ctggccatga caactccatc 1500
cagctttcag aaatggactc agatgggcaa aactgaccta agctgaccta gactaaacaa 1560
ggctgaactg ggctgagctg agctgaactg ggctgagttg aactgggttg agctgagctg 1620
agctgagctg ggctaagttg caccaggtga gctgagctga gctgggcttg gctgcactaa 1680
gctgggctga gctgggcagg gctgggctga gctgagctgg gctgggctga gctgggctgg 1740
gctgggctgg gctgagcggg tctgagcggg gctgagctga gctaggctgg gctgggctga 1800
gctgggctga gctgggctga gctgggctga gcaaggctag gctgagctgg gctgagctga 1860
gctgggccga gcaaggctag gctgagctga gctgagctgg gctgcgctga gctgggctgg 1920
gctgcgctga gctgggctgg gctgagctgg gctaggctgg gctgagctgg gctgagctag 1980
gctgggctgg gctgggctga gcggggctga gcggggctga gctgagctag gctgggctga 2040
gcggggctga gctgagctag gctgggctgg gctgggctga gccaagctga accgggttga 2100
gcgtgctgtg ctgggctgag ccaagctagg ctgagctgag ccaagttgag cttagctggg 2160
ctgagctaac ctgggcaagg ctgagctggg ctgagctaac ctggactggg gctgagctaa 2220
cctgggcaga gctgagctgg gctgagctaa cctggactgg ggctgagcta acctgggcag 2280
```

```
agctgagctg ggctgagcta acctgggctg gggctgagct aacctgggct gggctcagct 2340
gagctgagct acgctgggct gggctgggct gagccgagct gaactgggct gagcaggctg 2400
tgtcgggctg agccaagctg ggccgagctc agcagagctg agccgagctg agcttagctg 2460
ggctgagcta accagggctg ggctgagctg ggctgagctg agctgagctg aactgggctg 2520
aacgggctga ggcgagcagg gccgagctga gcagagctaa gccgaggctg ggctgggcta 2580
acctgggctg ggccgagctg agcagagcta agccgaggct gggctgggct aacctgggct 2640
gggctgagct gagctgggtt gggcagggct gggctgggct gagctaagct gaactagggt 2700
gagctgggcc gagccaggct ggtttgggct gagttgagct gacctggact ggggctgagc 2760
taacctgggc tgggctcagc tgagctgagc tacgctgggc tgggctgggc tgagccgagc 2820
tgaactgggc tgagcaggtt gtatcaggct aagccaagct gggccaagct cagcagagcc 2880
aagccgagct gagcttagct gggctgagct aaccagggct gggctgagct gggctgagct 2940
gagctgagct gaactgggct gaacgggctg aggcgagcag ggccgagctg agcagagcta 3000
agccgaggct gggctgggct aacctgggct gggctgagct gagctgggtt gggcaaggct 3060
gggctgggtt gagttgggct gagctaagct gagctagggt gagctgggct gagccaggct 3120
ggattgggct gagttgagct gacctcaact gagctgacct aggctgagct gagctgagct 3180
gaagtaggct gtgctgggct gagctgggat gacctgggct gggctgagct gatttgggct 3240
gggttgagca gacctgggct gagccgggtt gagctgagct gaaccaggat gagctgggct 3300
gagctgagct gggctgggtg gtccaggctg ggctgacctg gaccaggctg ggccaggttg 3360
agctgggcta agccgagctg agctgagctg agctgggatg atctgggctg ggctgggctg 3420
ggctgagctg acctgggctg ggctgggctg agctgagctg agctgagtta agctgagctg 3480
agctgaactg ggctgtgctg agctaggctg ggctgggctg agctgggctg ggctgggctg 3540
agccagattg tgcctggctg aactgagctg ggctaagctg agctgggctg agctgggctg 3600
agctgagctg ggctgagtgg ggcggggctg agctgagccg gactgggctg ggctgggctc 3660
agctgagctg agctgaactg ggctgggctg aactgggctg ggctgagctg agctgaactg 3720
ggctgggctg aactgggctg ggctgagctg agcttggatg agctgggctg aactgggctg 3780
ggttgagctg ggctgggctg agttgagcca ggctgatctg ggctgagccg agctgggtta 3840
agccgagctg ggttgggctg ggctgggttg ggctgggctg agccggactg ggttgggctg 3900
agctgagctg gactgggctg agctgagctg gtctgggctg gctgagctga gctgtgttga 3960
gccaagctag gctgggctgg gctgagctgg gctgaactgg gctgagcgga gctgagctga 4020
gctgggctgg gttgagcaga gctgggggct gagctgggct gggctgggct gagttaagct 4080
gggctgacct gggctgagtt aagctgggct gacctgggct gagctaagct gggttgagcc 4140
gatccgggct gggctgggct gggctgagcc aggctaggtt gagctgggct gggctgggct 4200
ctgctgtgct gtgctgaaca gggctgagct gaactgagct gagctgggct gagctgggct 4260
ctgctgtgct gtgctgagca gggctgagct gaactgagct gagctgggtt gagctaagct 4320
aggctgggct gggctgagct gggatgagct gggctgggct gggctgggct ctgctgtgct 4380
gtgctgaaca gggctgagct gaactgagct gagctgggct gagctgggct ctgctgtgct 4440
gtgctgagca gggctgagct gaactgggct gagctgggct gagctgggct gagttgagca 4500
gagctgggtt gagcagagct gggctgggct gggctgagtt gagccaggct gacctgggct 4560
gagccaagct gggttgagcc aaactgggct gggctgggct gagccaggct ggattgagct 4620
gggctgggct gggctaagct gagccgagca aggttgagcg agctgggctg ggttgggctg 4680
agctgagctg ggctgggctg agctgggctg tgctgcactg agctgggctg agctgggttg 4740
aactgtgctg agctgagctg ggctgtgctg aactatgctt agctgggctg ggctatactg 4800
ggcttagctg ggctgggcta aactgggctt agctgggctg ggctatactg ggcttagctg 4860
ggctgggcta aactgggctt agctgggctg ggctatactg ggcttagctg ggctgggctg 4920
agctgagatg gtcttaggtg gtctgagctc agctaggctg ggctgagctg gtctgagctc 4980
atctgagttg ggctgagctg agcttggctt tgctgagctg gggtggggtg ggctgggctg 5040
gattgagctg gcctgggctg ggatgaactg gattgagctg gcctgggctg ggatgaactg 5100
gaggacatgg cactgggcca atcttcatga tcttgttgga catagatgga tagcctcagc 5160
tgagtctaca ctgcgttccc catcacaccc accctcccta tactcactcc caggcctggg 5220
ttgtctgcct ggggagactt cagggtagct ggagtgtgac tgagctgggg gcagcagaag 5280
ctgggctgga gggactctat tggctgcctg cggggtgtgt ggctccaggc ttcacattca 5340
ggtatgcaac ctgggccctc cagctgcatg tgctgggagc tgagtgtgtg cagcacctac 5400
gtgctgatgc ctcgggggaa agcaggcctg gtccacccaa acctgagccc tcagccattc 5460
tgagcaggga gccaggggca gtcaggcctc agagtgcagc agggcagcca gctgaatggt 5520
ggcagggatg gctcagcctg ctccaggaga ccccaggtct gtccaggtgt tcagtgctgg 5580
gccctgcagc aggatgggcc gaggcctgca gccccagcag ccttggacaa agacctgagg 5640
cctcaccacg gccccgccac ccctgatagc catgacagtc tgggctttgg aggcctgcag 5700
gtgggctcgg ccttggtggg gcagccacag cgggacgcaa gtagtgaggg cactcagaac 5760
gccactcagc cccgacaggc agggcacgag gaggcagctc ctcaccctcc ctttctcttt 5820
tgtcctgcgg gtcctcaggg agtgcatccg ccccaaccct tttccccctc gtctcctgtg 5880
agaattcccc gtcggatacg agcagcgtgg ccgttggctg cctcgcacag gacttccttc 5940
ccgactccat cactttctcc tggaaataca agaacaactc tgacatcagc agcacccggg 6000
gcttcccatc agtcctgaga gggggcaagt acgcagccac ctcacaggtg ctgctgcctt 6060
ccaaggacgt catgcagggc acagacgaac acgtggtgtg caaagtccag caccccaacg 6120
g                                                                 6121
```

<210> 2

<211> 81061
<212> DNA
<213> Artificial Sequence

<220>
<223> Nucleotides 967201 to 1048261 of GenBank Accession No. NG_001019 (Jan 10, 2006 version).

<400> 2

```
tgtcctgcgg gtcctcaggg agtgcatccg ccccaaccct tttccccctc gtctcctgtg 60
agaattcccc gtcggatacg agcagcgtgg ccgttggctg cctcgcacag gacttccttc 120
ccgactccat cactttctcc tggaaataca agaacaactc tgacatcagc agcacccggg 180
gcttcccatc agtcctgaga gggggcaagt acgcagccac ctcacaggtg ctgctgcctt 240
ccaaggacgt catgcagggc acagacgaac acgtggtgtg caaagtccag caccccaacg 300
gcaacaaaga aaagaacgtg cctcttccag gtgagggccg ggcccagcca ccgggacaga 360
gagggagccg aagggggcgg gagtggcggg caccgggctg acacgtgtcc ctcactgcag 420
tgattgccga gctgcctccc aaagtgagcg tcttcgtccc accccgcgac ggcttcttcg 480
gcaacccccg caagtccaag ctcatctgcc aggccacggg tttcagtccc cggcagattc 540
aggtgtcctg gctgcgcgag gggaagcagg tggggtctgg cgtcaccacg gaccaggtgc 600
aggctgaggc caaagagtct gggcccacga cctacaaggt gaccagcaca ctgaccatca 660
aagagagcga ctggctcagc cagagcatgt tcacctgccg cgtggatcac aggggcctga 720
ccttccagca gaatgcgtcc tccatgtgtg gccccggtga gtgacctgtc cccaggggca 780
gcacccaccg acacacaggg gtccactcgg gtctggcatt cgccaccccg gatgcagcca 840
tctactccct gagccttggc ttcccagagc ggccaagggc aggggctcgg gcggcaggac 900
ccctgggctc ggcagaggca gttgctactc tttgggtggg aaccatgcct ccgcccacat 960
ccacacctgc cccacctctg actcccttct cttgactcca gatcaagaca cagccatccg 1020
ggtcttcgcc atccccccat cctttgccag catcttcctc accaagtcca ccaagttgac 1080
ctgcctggtc acagacctga ccacctatga cagcgtgacc atctcctgga cccgccagaa 1140
tggcgaagct gtgaaaaccc acaccaacat ctccgagagc caccccaatg ccactttcag 1200
cgccgtgggt gaggccagca tctgcgagga tgactggaat tccggggaga ggttcacgtg 1260
caccgtgacc cacacagacc tgccctcgcc actgaagcag accatctccc ggcccaaggg 1320
taggccccac tcttgcccct cttcctgcac tccctgggac ctcccttggc ctctggggca 1380
tggtggaaag cacccctcac tcccccgttg tctgggcaac tggggaaaag gggactcaac 1440
cccagcccac aggctggtcc ccccactgcc ccgccctcac caccatctct gttcacaggg 1500
gtggccctgc acaggcccga tgtctacttg ctgccaccag cccgggagca gctgaacctg 1560
cgggagtcgg ccaccatcac gtgcctggtg acgggcttct ctcccgcgga cgtcttcgtg 1620
cagtggatgc agagggggca gcccttgtcc ccggagaagt atgtgaccag cgccccaatg 1680
cctgagcccc aggccccagg ccggtacttc gcccacagca tcctgaccgt gtccgaagag 1740
gaatggaaca cgggggagac ctacacctgc gtggtggccc atgaggccct gcccaacagg 1800
gtcaccgaga ggaccgtgga caagtccacc ggtaaaccca ccctgtacaa cgtgtccctg 1860
gtcatgtccg acacagctgg cacctgctac tgaccctgct ggcctgccca caggctcggg 1920
gcggctggcc gctctgtgtg tgcatgcaaa ctaaccgtgt caacggggtg agatgttgca 1980
tcttataaaa ttagaaataa aaagatccat tcaaaagata ctggtcctga gtgcacgatg 2040
ctctggccta ctggggtggc ggctgtgctg cacccaccct gcgcctcccc tgcagaacac 2100
cttcctccac agcccccacc cctgcctcac ccacctgcgt gcctcagtgg cttctagaaa 2160
cccctgaatt ccctgcagct gctcacagca ggctgacctc agacttgcca ttcctcctac 2220
tgcttccaga aagaaagctg aaagcaaggc cacacgtata caggcagcac acaggcatgt 2280
gtggatacac atggacagac acggacacac acaaacacat ggacacacag agacgtgcta 2340
acccatgggc acacacatac acagacatgg acccacacac aaacatatgt ggacacacat 2400
gtacaaacat gcacaggcac acaaagagaa cactgactac aggcacacac acacacgggc 2460
acactaccca caggcacaca acagatggac acgcgtacac agacatgcac acacccacag 2520
gcacaacacg tgcgcatgcc ggccggcccc cgcccacatt ctcccagggc cctgccggat 2580
actctgtccc tgcagcagtt tgctccctgc gctgtgctgg ccccggggct ttgggcccag 2640
gctctgcttg tccttctgtc tctgcttgga ggtgctgcca tggcacccag cttgggctct 2700
gcctggggag cggaggcccc agggatagca tgtgacccct gctgaggcca ggctcctgat 2760
gaaggcagca gatagccccc acacccaccg gtgagcagaa ccagagcctg tgccatgtgc 2820
tgagagcagg cagtgactaa gcatatgggc ccagagggca gagtggctgc cctgggcagc 2880
tgctcctctt agcaggaggc ctcaggagat gagctagagc aagtctgccc ctgcaaatac 2940
cacctgctcc ccaacccaca gcagggagca ggcgaggtca gacagcagca gcccgggaag 3000
gaccgagccc cagcagggaa ggcagggccc gagtgaggtc tccacaccca acgcacagtg 3060
ctgtctctaa ctggggccac ctccgagtcc ccgccacact cttggccctt tggagtcctg 3120
ggctccaggt gtctcccaag ggcccatctg tgcaggggat gcaacccccc gaatgtcctc 3180
atcccactgt ggagctcagg tctctgtctg ctccctgggt cctggcaggg taggacaagt 3240
ccgccaggat gtccccatgc agactctgct ccaagaggga gctggagagt cagggccttg 3300
gtgagggagt caggatcggg ttccccccag ctcagtcctc ccacctgcca gcccccacag 3360
cacagggcag ggccacaccc cctgcttccc cctccaggag agtcaggaca tgctggccgc 3420
tgctccgctg ggccccgcc ctccagcccc caccttggtc tgtgtgctgc atcccccacg 3480
```

```
ctctctctgc caccccagga ctctgaggaa aagacctcag agtcccagcc ctgcccagcc 3540
tcggcctgtg cccccgctgc atcaggcttt caggggccca gcccatgccc tgggcagtgc 3600
ccgagccccc ctgcacttgc tctccccacc cctgggtgca gcacagccta ggggccaagg 3660
gtgggcctag aggatgggcc ccgggggggc tttgctgggt gccaccccag cctgacccta 3720
ttcccccgtg ctgtgtctcc tgcagagggg gaggtgagcg ccgacgagga gggctttgag 3780
aacctgtggg ccaccgcctc caccttcatc gtcctcttcc tcctgagcct cttctacagt 3840
accaccgtca ccttgttcaa ggtagcacgg ctgtggcaca gggaggaggg cgcagggcga 3900
atgtggggcc cagggagcag cctgggctgg acgtctagcc cggaggcccc cacaccaccc 3960
cactgggtca tctctgcccc ggctcccttc ccgaccacag ggaaagcatt tcacactgtc 4020
tctgttgcct gtaggtgaaa tgatcccaac agaagaacat cggagaccag agagaggaac 4080
tcaaaggggc gctgcctccg ggtctggggt cctggcctgc gtggcctgtt ggcacgtgtt 4140
tctcttcccc gcccggcctc cagttgtgtg ctctcacaca ggcttccttc tcgaccggca 4200
ggggctggct ggcttgcagg ccacgaggtg ggctctaccc cacactgctt tgctgtgtat 4260
acgcttgttg ccctgaaata aatatgcaca ttttatccat gaaactgctt tctggtgagg 4320
gtttttgttt ctttttcaaa actttcatgc tacatgggca tctcaagggg gaccccagtt 4380
ccaaagggag ctgtggaaaa gagcgctggg tcagccagcg caggggggttc gaggaaagcc 4440
acgtgcccag gaaaggggcc gcagaagcag gtgggccaga ctcagactcg ggcatgccc 4500
agcctgatgg aaggaagggg actgagcagg agagggttcc aggcctggtc ctccaagcac 4560
agcctgaatt gggagactgg ggctcaggcc tcgggggcct ctgtgtgtgc tccacatgcc 4620
tacaactgcc cgggtcacct tgccacccctt cccagcaagc ccagacagtt cttggccttg 4680
ccccaaacct tcatgatgtg tggtgcacgc caccgggatc caggaggtgc aggctgagcc 4740
ctcgagagca tgtgggcctc accgggatcc aggaggtgca ggctgagccc tcgagagcat 4800
gtgggcctgt ctgcacagtg tgggggcctt gcactccaca ggagcacagg ggtggggtag 4860
cagtcgcgcc cgtggcaggg gagtggaagt tggagcaaaa gtttcaggtg aacgagtgtc 4920
ttagtctaat tgggcagcta tcacaaaaca ctatcggctg caaagcttga gcaacagaca 4980
tttgcctccc acagcgctgg aggctgaaag tctaagatca aggcgccggc aacttcaacg 5040
tctggcgagg gccaattccc agctcagaga cgcgcctcct cactgcatcc tcgcgcagcc 5100
gaagatggtg aaggagctct cagggtctct gtcatacagg cactaagccc gtcacgaggg 5160
tctctgtcat acaggcacta agcccgtcac gagggtctct ctcatacagg cactaagccc 5220
gtcacgaggg tctctctcat acaggcacta agcccgtcac gagggtctct ctcatacagg 5280
cactaagccc gtcacgaggg tctctctcat acaggcacta agcccgtcac gagggtctct 5340
ctcatacagg cactaagccc gtcacgaggg tctctctcat acaggcacta agcccgtcac 5400
gagggtctct ctcatacagg cactaagccc gtcacgaggg tctctctcat acaggcacta 5460
agcccatcac gaggtcctca tcattgccca gatgctaggg ctcccagtgc catccccttg 5520
tggatgagga tggggggcgt gaccatgtcg tccttagaag ccgttaggga atgcatagtg 5580
gggaggacaa gtctctgcca tcaccctgaa gatgggcagg tggtggccag gcttggggca 5640
gtgccgaaaa atgactcccc aggtaggtgg gagcaggatc aaagagcagg ggattttttc 5700
agagatggca gccaagccaa agttgggggc tcatgccagg aggctcactt ggccatggtg 5760
cagtcaccca ggggtgaccc acagtccgtg gcaaagtcaa ggacagcagt ggctgtgaga 5820
ccagggtgga ggcaggtggc tgggggggaga ggaccaggct ggaaggactg ggcagacccc 5880
aaggccatag aagtgtccta gaggaagcat ccaggatggc gtcttgggag ggctcataag 5940
ctgggggccc agaaggaggc catttcaggt tctgaggagc agcctggcac ttgctctgag 6000
ccagctgtca tgggtatctg gggctgctg ggtacaagtt gtgtccctcc cactaaatgt 6060
gctctccaca tggacccggc ccgcccttct gtccctgctg gatccctgag ctggcaccag 6120
ccctgccctc agagacaatg tccaggagac aggtggaggt gcacgtgtgg gtccctgggg 6180
aaatccatcc tccaaccgtg ggctccgagt cgcctcccag cctctcgctc cagcttcacc 6240
ccatgtagct catcacaggc tcaacctccc agcctggggg aggatggagt gaggagcccc 6300
acactgccca gggcacaccc agggggctgg ggagtctgca ctgggctggg gcagggaggc 6360
ctcgtgcagc ctgtggggct ggcagctcag gacaacactc gtatccgtta actgtggccc 6420
tggcaacact gcaccccaga ctgcgtggct taaacaacag acgtttattc cgtcctggtt 6480
ctggaggccg ggcatctggg atggaggcct cggcggggct ggctcctctg tgtcacggga 6540
gactctgttc caggctctct ccctgctgct gggctttgcc ggccgtctct ggtgctcttg 6600
gcttatggaa gcagcaccat cttcacaggg cgttctcccc acgtgctgtc tgtgcccaga 6660
ttcccccttt tcatgaggac agcagtcata ttggatcaga ggcttgccct actccaggt 6720
gacctcatct gaacttgatt gcagctgcaa agactgtttc cagacaaggt cacattctgt 6780
ggtcctgggg gttaggactt cgacacatta atttacaggg gacacatttt aacccatgac 6840
agtttgccct ccgttccccc cataatcatg tccttctcac atgcaaaatc cctgcatccc 6900
atagcaacat ctccagaacg gctgacccct tccagcacca actcttagtc cacaatctca 6960
gcgaaatatc acctgcatca agtgtgggag aaacccaggg tgagattcat tctgggtgaa 7020
attcctctcc atctgtggac ctttgtaact tgacaagaag ttatctgctt ccaaagtaca 7080
atgatggggc aggcatagga tggacattcc tattctaaaa gggatacata gaaagggaga 7140
gaggagccat gggtcccaag caagcccaaa cccagcaggg aaagctgcat tagatttaag 7200
gctcaaggct catcctcctg ggtccgtgct ccatcttcca ggcccactgg ggtgacccca 7260
tgctcttcgc ccatggtggc cagagccccc acagcctcct catctttggc tctgatctca 7320
aagtcaccct tccttcattt tttctggtct tctttccctt ccatccaatt ggcattgttt 7380
cttctgtttt acaattcaca aaatccttgt cagcttccca tgaaattcat gggggtttga 7440
gtcatcagac aagagagccc tgcacagtta tatcctcaat aaccttatct ctactcctgg 7500
```

```
tttctgctga gatggttgat tggatccata agtcatgatt ccaatctctt taacaaatgg 7560
ttgcccagac acatccttgg ccttgtttcc agagcatgct attggacagg ctgagaattt 7620
tccagctgtt caagttgtgg gtcctgttta ctgaacactt tctctcactt tttactataa 7680
gcagtcagga gaaatcaggt tgtgccttca acacttttct tagaaatatc ctcagctaaa 7740
atccaggttc atcacttgta agtcctactt accataaata gaatgcaatt cagccaagtg 7800
ctttgccact ttctaacaaa ggtcaccttt cctccaattt tcaataacat cttcctcatt 7860
tctgtctggg gcctcaccag aggcatcttt aacattcaca tttctagcaa cattctgttt 7920
gtgacaattt atgtattctc taagatgaca ggagttttct ctatagctct cctctttctt 7980
tctgagcccc caccaggatc accttcaatg tccaaatttc taccatagtc ttcaaggaaa 8040
tctaggcttt ttctaacatg cacctgaaaa ctcttctcac ctctacacat taccctatcc 8100
caaattcatt tccacagttt taggtatcaa ttaaatcaga taaataaatc attaccctga 8160
attatttcag ttaagcatgt tagttggtgg cataagagaa aactcagtca gatagtgctg 8220
aagacaggac tgtggagaca ccttagaagg acagattctg ttcagaatca ctgatgcggc 8280
gtcagcagga ctggcctagt ggaggctctg ggagggtggc tgccaggccc ggcctgggct 8340
ttgggtctcc ccggactacc tggagctggg atgcgtggct tctgccgccg ggccgactgg 8400
ctgctcaggc cccagccctt gttaatggac ttggaggaat gattccatgc caaagctttt 8460
gcaaggttcg cagtgaccag gcacccgaca tggtaagaga caggcagccg ccgctgctgc 8520
attcacttct cttaaaactt tgtatttgat gtcttatttc cactagaagg cgaattggtc 8580
ttaattgctt ctcttaagcc accacttcta aagccaaaat ctgttttagt ttcttatgag 8640
ctattgtaac aaagtaccac aactgcatgg ctgaaacaac agaagtgtat tctgtctcat 8700
ttctggaggc caggtgtctg agatcaagct gtgggcaggg ttggctcccc tgggttggca 8760
gggagaatct gttccaggtg tctgtcctgg cttttgttgg tttgctggaa agctttggtg 8820
ttccttggct tttggaaaca gctcctccgc ctccatcttt accttcacat ggcattctgc 8880
ctgcgtgcat gtctgtgtct aaatttccac tttgcataag gacaccagcc atattgggtc 8940
agaggctcac ctgcctccag cgtgacctca tcttaacttg attgcatctg caaaactctg 9000
cttccaaaga agggcacatt ctgaggccct acgggtcaga gcttcaacac gtccatttat 9060
ttggagacac aactcaaccc ataacaagac ccatccctag tgtctcacca gtggtgctgt 9120
agagggaggc ctggcagggc tgacatccca gaacctcagg gaaaaatgaa aacagcccag 9180
aggggtccaa gaggtggctc gaggaaggga attaggaaca cactgttcag gtggaagatt 9240
ctgagatggg ccgtgaatac tgcctaggat gtggagggtt gacaatgggt tgacaatcct 9300
gctcagaaaa tcccaggata aaggatgtaa atggggagaa gaggagggtc atccagaatt 9360
tgggaaagca gggcgacagt ttctgcccca agggagaagg gaaggaggat ggggccaccg 9420
ccacaccaga tgaccttgcg taccaggcca aagaacggga acacctggcc ccacctgagc 9480
agcaatagtc agtgtggtgg tgggcagaca tgggtggagg caggggggtga gtagaaggtt 9540
agactaagac ggagcacctg gggcctccag ggacccaggc aagaaccctg cacttgctca 9600
gctgccctgg gtacccaggt ctccaggaaa gtgaggctga gagccaagcc cagcaggcag 9660
ccacacattc tgggacctgc caccctacag cctgctgtcc atgagtaaca ccccttacaa 9720
ggggccaggt cagctcatgg gtttatccca ggcagcagag cccctggggc caggaatcag 9780
ggagaggagc atccaatccc accagctcct ctggagccac tcagagggcc agacgcatgg 9840
ccctccacag ggaccccatg gcccctgcag ggcagctgag gacccgtggc tgggagcctg 9900
ggcaggaggg tcatacagcc ctaggcccgt tgctcccagg cttgagtgcc cctcctcccc 9960
tcagggggccc aagggaagtg ggttccagag aggttgggggg cagcagggaa ggtggaggtc 10020
ccaggaatgc ccagaggggc accaaagcct ctggagggaa gacccctccc ttccaggagc 10080
tctcggcaac aagagcccag ggtccacaaa gccacaggtc ccactcggtt attctgactc 10140
acaacacagg agcggcagca ggggcattcg tgttcacggg ccacttggtc agccccgctc 10200
accctgggca ctcctcctgg gcccctttttc cctgccttcc ctgtcaccct gctgccaggg 10260
tcctctgccc tgccctgccc cttgtcctca gagcctccag cctcagactc ccactgtgtc 10320
tgtcttccag cacccaccaa ggctccggat gtgttcccca tcatatcagg gtgcagacac 10380
ccaaaggata acagccctgt ggtcctggca tgcttgataa ctgggtacca cccaacgtcc 10440
gtgactgtca cctggtacat ggggacacag agccagcccc agagaacctt ccctgagata 10500
caaagacggg acagctacta catgacaagc agccagctct ccaccccccct ccagcagtgg 10560
cgccaaggcg agtacaaatg cgtggtccag cacaccgcca gcaagagtaa gaaggagatc 10620
ttccgctggc caggtaggtc gcaccggaga tcacccagaa gggcccccca ggacccccag 10680
caccttccac tcagggcctg accacaaaga cagaagcaag ggctgggctg tgaggcaacc 10740
cccacctccc cctcagagca cgttcctccc ccttcaccct gtatccaccc ctccggaccc 10800
tccccatctc agtccctccg ctccctctct ctgaggccca tctcccaata cccagatcac 10860
tttccttcca gacccttccc tcagtgtgca cggaggcagc ttgcccagca aaggtgactg 10920
tctagtgggc ttcccacagc caagctccca ccccatgctg cggcccttcc cttcttcctg 10980
cttggctgcc tgtgcccccc acctgcctgt ccacaaccca gcctctggta catccatgcc 11040
ctctgccctc agcctcacct gcacttttcc ttggatttca gagtctccaa aggcacaggc 11100
ctcctcagtg cccactgcac aaccccaagc agagggcagc ctcgccaagg caaccacagc 11160
cccagccacc acccgtaaca caggtgagaa gccccttccc tgcacactcc accccccaccc 11220
acctgctcat tcctcagccg cctcctccag gcagcccttc ataactcctt gtctgagtct 11280
ccaagtcaca ctttggtaag gagagggaca ctgaacggac ctctaacaaa cacctactgc 11340
cagccagccc cagtctgggg gccagcagat gccaaacagc cagcagactc ccagagcaga 11400
cctgggccgg ctccctggcc catggaccca gctctgcctc gctgagctga ggcatgggct 11460
ctcagcgcag cctcacatag agccaccctg ccgaggcagt ccggcttgca gactcacagg 11520
```

```
tcacttgggc cgcagcagcc cctccccgtg accctcgcct cccgcccgcc ccagcctggc 11580
tctctccaag tgttggatct tggtggccag cctgcttctc accctcaccc tgcctgccac 11640
ctcagaatgg caggggaaag agggccctca ccaagaactt tatctgagga gtctgaggct 11700
tgtgactctg acctgcctga gatgtccatg tggccggggg gacgggttca gtgttcggga 11760
gaactcgggt acgtgcctga ctttctctga gtagggcagg aagctgttag gagaagcagc 11820
agtgaggtgg gctggaccaa caggcagaat gactgtccct cagccaccct ctgggatgtg 11880
ggtcaagctc tgacaaaggc atggcacagc catggtggcc cctgcttgga tgagtggcca 11940
cggtgccctc accctgggcc agaatctgcc tccactctgc aggtgcagaa acacgacatt 12000
cccgtctcta aacacaccta gctcctaggc ttggggtggg cctatcaaat gcagggagat 12060
ggacacagca caagggccag agcttcccat gagaaaggtg agggcagctg ctccctgacc 12120
cgggcatctg cacttgtccc tctccaccct cctcatgggc agtggagact cagcaacaaa 12180
acaagttgag tgcattagca gccagctctg gagccaagtc actcacccca cggccttggc 12240
tgctggtgga ggggccttcc cctgggcagc ctccaagaag acggccaagt gctcttactc 12300
agaccacggc gctgcttcct ggcacctcga tttcccacaa caacatgggg tgcagacagg 12360
ctagggcccc ctgccctggg gcctggacgg catccagtta aagatgaccc ttcacgggcg 12420
gtgcctgagg tgtgctgacc tcagcagcta agccctcagg tctggtctgc actgccccac 12480
ctggaggacc caactgaccc agacacagcc agggttatgg catgaccccg tggacggtga 12540
cccacaggcc agatgcagcc aggggctgtt ttgtgtggcc tagaaatgtc tttacagttg 12600
tagtgggatg gaggaggaag aggaagagag gaggggagag aaaagcaggg aaggggaaaa 12660
agaggagttc aatgcaaccc caaaagccag aacagttttg agctgaaaga acaaggcagg 12720
aaacatccca gtacctgact tcaaaacata ctataaagca gttgtaatca aaacaggatc 12780
ataaaaacag acacacagac ccatggaaca gaaaagcgag cccagaaata aatctacatg 12840
cttgcagtcc attgattttc aacaaaggca ccaggaaaac acaatgggga gaggacagtt 12900
tcctcaataa atagtgctgg ggaaactgga tatccatgtg cagactaatg aaactacaca 12960
aaaatcaatt gaaaacagtc taggccaggc gcggtggctc atgccggtaa tcccagcact 13020
ttgggaggcc gagacaggcg gatcacctga ggtcaggagt tcgagaccag cttggccaac 13080
atggcgaaac ccggtctcca ctaaaaatac aaaaattagc acatggtggc ctacgtctgt 13140
tatcccagct tttcaggagg ctgaggcagg agaatcgctt gaatccggga ggtgaaggtt 13200
gcagggagcc aagattgcgc cactgcattc cagcctgggc aatggagcga gactgtctca 13260
aaaaaaaaaa aaaaaagaaa agaaaacagt ctaaaggttt aactgaacag ataaagctac 13320
tagaagaaaa catagggggga aaactccatg acattagtct gagcaacgat ttttggatat 13380
gatcccaaaa gctcaggcag cactagtcac aaaagccaag atacagaacc aacctaagca 13440
cccctcagca gatgcacagg taaagaaaat gtggtacgta tggggcacaa tggaatacga 13500
ttcagccttt aaaaacagtg aaattctgtc attggcaaca atgtagatga acctgaagga 13560
cacttatgct aagtgaaata agccaggcac agaaggagca atactgcatg attgcactta 13620
catctggcag gttaaaaagg caaactctta gaggcagaca gtagagaggt ggtgccaggg 13680
agcgggcact ggtggctggg gagatgttgg tcaaagggca caaaactgca gttgggagga 13740
attagttcag gacatccctt gtacatgggg acagtggtta gtaacaacgg attgtatcct 13800
tgaaaaccgc taagaaaata gtttttaagt gttcttgaca caaaaagtga cacgtatgtg 13860
agatactgca tggtcattag ctggatttag ccattccaca atgtacacat atttcaaaca 13920
ttgtgttgta tatgataaac atgtataatt tttgtcaatt aaaaattttt aggaagagga 13980
ggagaagaga agaagaagga gaaggagaaa gaggaacaag aagagagaga gacaaagaca 14040
ccaggttttt tctgacccct gggctatcaa aacacctatt gcccaataac tagttggccg 14100
ttggtgccct aaactattga agcgattgct gttatgtgga tgggccccgg acacttagaa 14160
actcgtgacc cctgaggacc cccacgagga cagtcagggt ccccccgaac tcagggagca 14220
ctgaggaagg agctcttaga ggcgtggggc ccctcaggcc cctcagaggg ctctgccaca 14280
tgggtcaggg gcaggctgag gggggagtccc aggctccatg cccagcctct gtgcctctga 14340
ccagggtgtc ccccacaccg cctcctcccc agtgccctcc actggccaca cctggccaga 14400
agctggggag aggagagcac agtggttaag tcagtccctg cagggagacg gcaccagaaa 14460
aacctggcct gtggatgagt cccggcctgg cagccacaga gcagagagct ctagaagcaa 14520
cgaaggcccg agtctgctca gggaagagcg ggcagcagcc ccagggccgg acagtgacca 14580
agagtggcac cgcccatggc tcaacgggtc tttgcccaca gatcccccag cccctggaga 14640
cagggtctgt gtgcctggcc gtgcaggcag gcaccacact caggggggagg ccactgtgga 14700
gctctgtgca gagccccggg cgggagccta ctgctcccga aggtccggcc acagctgctc 14760
tcgtttgctc tcccctgcag agtgtccgag ccacacccag cctcttggcg tctacctgct 14820
aacccctgca gtgcaggacc tgtggctccg ggacaaagcc accttcacct gcttcgtggt 14880
gggcagtgac ctgaaggatg ctcacctgac ctgggaggtg gccgggaagg tccccacagg 14940
gggcgtggag gaagggctgc tggagcggca cagcaacggc tcccagagcc agcacagccg 15000
tctgaccctg cccaggtcct tgtggaacgc ggggacctcc gtcacctgca cactgaacca 15060
tcccagcctc ccaccccaga ggttgatggc gctgagagaa cccggtgagc ctggctccca 15120
ggtggggaga cgagggtgcc cacagcctgc tgacccctac gcctgcccca gggccatgac 15180
cccagctggg ccccagcagc accggtcatc ctccacagga aaggagaagg gaggcaccag 15240
caccctggcc ggccccactt ctctcccagt gcccccgtgg ccagaggctg acagcctccc 15300
ccacctcccc gcagctgcgc aggcacccgt caagctttcc ctgaacctgc tggcctcgtc 15360
tgaccctccc gaggcggcct cgtggctcct gtgtgaggtg tctggcttct cgccccccaa 15420
catcctcctg atgtggctgg aggaccagcg tgaggtgaac acttctgggt ttgcccccgc 15480
acgcccccct ccacagcccg ggagcaccac gttctgggcc tggagtgtgc tgcgtgtccc 15540
```

agccccgccc agccctcagc cagccaccta cacgtgtgtg gtcagccacg aggactcccg 15600
gactctgctc aacgccagcc ggagcctaga agtcagctgt gagtcacccc caggcccagg 15660
gttgggacgg ggactctgag gggggccata aggagctgga atccatacta ggcaggggtg 15720
ggcactgggc aggggcgggg ctaggctgtc ctgggcacac aggccccttc tcggtgtccg 15780
gcaggagcac agacttccca gtactcctgg gccatggatg tcccagcgtc catccttgct 15840
gtccacacca cgtgctggcc caggctggct ggcacagtgt aagaggtgga tacaacccct 15900
cgccgtgccc tgaggagtgg cggtttcctc ccaagacatt ccccacggct gggtgctggg 15960
cacaggcctt ccctggtgtg accgtgaatg tggtcaccct gaacagctgc cctctctggg 16020
gacatctgac tgtccaagac cacagtcagc acctctggga gccagagggg tctccagaga 16080
cccccagatg tcaggcttgg gctcagtgcc cagcgaaagg tcagccccac acatgcccat 16140
aatgggcgcc cacccagagt gacagccccc agcctcctgc caggcccacc cttttccgcc 16200
cccttgaggc atggcacaca gaccagtgcg cccactgccc gagcatggcc ccagtgggat 16260
gtggtggcca cgaggggctg tacacacagc aggaggctgt ccgccctgct cagggcctgc 16320
tgcctatgcc ccagctgtcc aaccaaggga ggcatggaag ggcccctggt gtaagctgga 16380
gccaggcacc caggcccccg gccaccctgc agagccaagg aaaggaagac acccaagtca 16440
acaaggggca gggctgaggg ctgtcccagg ctcttttggc ccgaggggct gccagcagcc 16500
ctgacccggc atgggccttc cccaaaagcg accctgtgag gtggcctcac agagaacccc 16560
ctctgaggac agtgtctgac cctgcctgcc tcacacagat gggccccaca gcagtgggca 16620
acctgggggg cagcagccca acctgaccct gcagggactg ccccctgcag cagcagctgc 16680
ttctcagtcc cccaacctcc ctgtccccgc cagagggtct tccccgaagc tgcagcccca 16740
acccatggct gcccacctgg aaccgggact ccctgtccac tgcccctcc ccttcggggc 16800
cccatctgtg ctggggccca ggttcggcct acagattccc atcattgcca tggcctcctg 16860
accttgccta tccaccccca accaccggct ccatgctgac cctcccccag gctcccacgc 16920
ccagctggcc ggccatcccc aggcacagac agtctgggat ctcacaggtt agcctggacc 16980
atccacctgg ccagacctgg gagaggctgg aagctgccct gccaccatgc tccagggccc 17040
caggttgcag tactatgggg tgagggtgtg tgtgcacacc tgtgtgtacc taggatatcc 17100
gagtgtaccc ttgtgccccc aagcacaagt ctccctccca ggcagtgagg cccagatggt 17160
gcagtggtta gagctgaggc ttatcccaca gagaaccctg gcgccttggt caaggaagcc 17220
cctatgcctt tcttgcctcg atttcccctc ttgtctgctg agccagcagg ggccacgtcc 17280
tgggctgctg tgaggaggaa gcaagttggt gctaggaggg gctcctgtgt gtgcatgggc 17340
gggaggggtg caggtatctg agcaccccgg tctccacttg agagagcagg gcaggagctc 17400
cctgacccac ccagactaca cacgctgtgt ccacgtgtct cccattatct gtggcagagg 17460
atccggcttc tttctcaatt tccagttctt cacaaagcaa tgcctttgta aaatgcaata 17520
agaaatacta gaaaaatgat atgaacagaa agacacgccg attttttgtt attagatgta 17580
acagaccatg gccccatgaa atgatcccgg accagatccg tccacacccg ccactcagca 17640
gctctggccg agctcacagt acaaccacaa taaactcttg ttgaatgaac tctaggaagt 17700
ctgtgacgtg gctggttctt gtcaatgctt cctgcctgcc cacaggctct tcctcgtgga 17760
tggggctgtg cttgccacgg aagcgcgttt ttcccggcct aggcttgcct tgggccccac 17820
tgccgtctcc agctggagat gaccttctat acacacattt gctcatgaca gacccttgct 17880
tagcccccctt ccatggctcc ctcctgctgc tgggataaaa tcaccttgcc tggatatccc 17940
ctcctgggcc cctttccacc ctccttagtc agcaccccca gttcagggca cctgctttcc 18000
ccgctgcgga gaagccactc tctccttgct gcccggctgt gtcttgcctt ccacaccttg 18060
tcacagtggc cacttcctaa ggaaggcctc cctgtgtgca ggtgtgcaga agtgccccag 18120
cctcccgtca cctttgtcac gggagcccaa tccatgagag tctatggttc tgtctgtctg 18180
ccccactcag ggcagcgaca agtccaggcg gggaggacac agtaggcaga gatttgtcga 18240
ggggacatat gagcaagagg gtgaggctgg gagctccctg gagataacca cgcctcctgg 18300
gaagactcgc cgtcatttca gctccacgct gtgcgggggt gggtggaggg gtagcctggc 18360
cctcatgacc agggagcttc tcactcagcc cctgttcctc cccagacctg gccatgaccc 18420
ccctgatccc tcagagcaag gatgagaaca gcgatgacta cacgaccttt gatgatgtgg 18480
gcagcctgtg gaccgccctg tccacgtttg tggccctctt catcctcacc ctcctctaca 18540
gcggcattgt cactttcatc aaggtcaggg gagcggccag gctctcagtg accctcgggg 18600
tgggtgtggg gcaaggtgcc cttccagggg acatgccaga gctggtccag ggatcctgga 18660
ccaggcagag gcagggctga gggagcctgg aggacatgca ggccctctgt ggcctgtgga 18720
cactgtcgaa ggccctcttg accctgtgga taaaggacaa cacccccctcc cctgctcctc 18780
tgtctcccct gcccctccac ccctcaggct tctagccccc tgtctgaccc caggggctgt 18840
ctttcaggtg aagtagcccc agaagagcag gacgccctgt acctgcagag aagggaagca 18900
gcctctgtac ctcatctgtg gctaccagag agcagaaagg acccaccctg gactcttctg 18960
tgtgcaggaa gatgcgccag cccctgcccc cggctcccct ctgtccgcca cagaatccag 19020
tcttctagac caggggggacg ggcacccatc actccgcagg cgaatcagag ccccccctgcc 19080
ccggccctaa cccctgtgcc tccttcccgt gcttccccca gagccagcta cacccctgcc 19140
ccggccctaa cccccatgcc tccttcctgt gcttccccca gagccagcta gtcccacctg 19200
cagcccgctg gcctccccat aaacacgctt tggttcattt cacctgcctc ctgttctttg 19260
tcccagtggt ctggattcac ctcaagttaa aggacacagg gagctaacgg caacggggag 19320
ggagtttggg gtgtcagcag caacagggaa cgcaatagcc aaagctgtca cagcaagaac 19380
tgagcggaga ccttgctctg aagttcatgg gttaagaaac gcccctcccc acaccaaaca 19440
caaacatttc acgctcactc gtacatgcac acacatgcac acaagcatct atgcacacat 19500
gcacatgtgc acagctcacg aataaacata tatgcacaca catgcagtac actcacatgc 19560

aaacaggcag atgcacaggc atgggcacac atgcacaagg catgcatgtg caggtctgca 19620
gacacacaca tctgcatgca catacacgga cgtgcacctg cacacacaca agtgcacata 19680
ggcatcaaag acacacatgc tcgtgcacgc gcatacacac agacatgcac acttatgcat 19740
gcacacgcca tgacaggatg gcacgggaac aaccttgcac atggagcctg ttctgtgtgt 19800
atcgagagcc tgcacctctt actgtctgca tgactgtggg caagagcctg aactccgtac 19860
ctccatttcc tcatcagaat ggggtgaaaa tacacctgtc aagtagagtg aggtgagaag 19920
tgagtaaaat cctatctgaa aggggctcag caaggaccca ggtattggtc ggacctcctg 19980
ttatcatctg ctgtgggctg aacatttgtg tcctcctgaa attcatacat tcgtatattc 20040
ataccttttcc ccctgaaatc ctaacctcca atgtaatggt attagaagat ggggcttttg 20100
ggggtgctga ggtcataaaa ggggggccct catgaagagg attagcaccc ttatgagagg 20160
ggccccagag agctccctca cccttctcc cctgtgagga gatgccgtct atgagctggg 20220
atgcaggcct caccagacac ccatctgctg gagccttggt ctgggacatt cagcctccag 20280
aatggtggga aataagtttc tgttgtttct cagccaccac gtctgtagta tgtggaagtc 20340
atcagaatca aaattgagtc acctgtggtt tttttttttt ctaaatccct gacaaataga 20400
gcctaggaag gccaagaaga gaagagggtt ctcatccata aacacttgat aacaaaaact 20460
atcaccaagg actctacaaa aactgcaact ggcacaaaga ccatcacaac cttacacaga 20520
aagtacttct gtgaggacat cttcccagca acgggctgtc caacctcaga ctggcattgc 20580
ctttgttatt ggtccttgta gagagggtaa ttatctcaaa gcaatcatgt aatcctcctc 20640
atttttcctt tgaaagcctt ggtctccctt tgcctccctg aatacgcaca tagctgatca 20700
tggcaggtgt atcccactgc agtgctctac ctccaaatag atatcttttt cttttagacg 20760
gcatttctct tgttatttag attgagatac atggagtcag aagtgggatg tggagaagga 20820
tcactgctgg aaggagtcag taattcttgg gccggtgtgc agtattcact tgagcccttt 20880
gagctctcag ctttttctga gctactccgt cttctctcag gccaaccctc cctctttttg 20940
gaagatgctt ttttaatatt atgtggggtt tgtttattag actgccttaa taaaggacct 21000
tatgtccctc ctgggatgat aaaaggcttt tcgtgctttc tggcaagtcc tgtttagcat 21060
aaagacattc cagcctgagt actctggttt ccacagagtt tgcattctgt ctctgaggga 21120
tgtcttctct ggtgaggtgc ctcctttaat actttgttgg atatgcatgc ctgggtgaac 21180
atatttgtga gcacccttat cttgccttct tttgctgtgg ttatgtgtat ctgtaaatga 21240
cctggctctt ttcctctgct tgcttctaaa aatctttgga gagcaaaata aacattctaa 21300
atggtgggca caggctgtct cattagaagc tgctacagca gtcaccaccg tctaaagcac 21360
cagtccaaac tgccggcgtg gcctcatagt atctgtagaa tgttctttac tgtaaaaaga 21420
ttaataagaa gggggatggt ttttaaatat taaggcacag taagttttct ggaactcccg 21480
ctggctacgt ctttgtgcac atttttaaac taatgggcaa attacgtcaa ggaaaattta 21540
gagttccaat ggttattttt cgaactctaa caaaaaccct gcaacagtag tcatcatgca 21600
gtgcctccta agtcctctat ctctctattt tgtttttctg cctactttaa atctgctgac 21660
tttttttactc atgttgcaat aaaactcact ggttacagca ttccagccaa gatatttttg 21720
gtttttttgtt tgtttgtttt ttgtttttttg tttttttaga tggagtctcg ctctgtcacc 21780
caggctggaa tgcagtggcg ctatcttggg tcgctgcaac ctctgcctct cgggttcaag 21840
tgattctcct gcctcagcct cctgagtagc tgattacagg tgcccgccac catgcccagc 21900
taatttttgt atttagtag agacaggctt tcactgtgtt ggccaagctc gtcttgaact 21960
cctgacctca agtgatccac ctgccttggc ctcccaaagt gctaaaacta taggcgtgtg 22020
ccaccgtgcc tggcccaaga tttttttaaag tttcctaaag gttttaaatt agtggcttta 22080
caaattacaa cagctctatg gtaaccaatg acctagatgt aagagtcatt tcttttgaaa 22140
atgtagatta gctttgcttg gctaacaact gcttaggctg atggaatagc taactgaagg 22200
actgatggtc tcaaagaaca gaactaggta aatatttaca gaaattgggc tttcagatcg 22260
aacaggccaa aatcttgagc ttagagcagt aatataaggt atctctgtct gccataaaaa 22320
ttttgctttg ccacaggtgc cagaaaaagg aaaaacactg ctaaatgctt ccctgcatgc 22380
attgtctagt ccagaaaatc agaccagcaa ccaaaaaata gatttgttac tagtatagac 22440
gagttgaaga ttttgttttt catatacaat tcagccagtc ctagctaaaa cgcaatcact 22500
ggaaatttaa ccttaaattc atttaaaact gaaaaaggtc ttttgaaatc aaactaatgc 22560
agaaactgct ttacccaaaa ttctgatcca tggccctcat tagacgaccc atcaggacaa 22620
ataaagttta gcttgtgaac aagtcccagt tttgtcaaaa atataatttg gattgaagtg 22680
tcttttagaa actgacacat ttgtgttatt atctcatgac caaaattcta aaatgaaagc 22740
tacagtgtct ttatttgtgt gcgtatgtgg ttaagtgtgt ttatgcatat gtatgtgtat 22800
tatgttgtat gttgttatct acatggtaaa atctggcatc atcaacaaaa aatctattaa 22860
gggattctat ttagattggc ttagataaat aagcattcat aaaaaatatg tactaattaa 22920
cccaaatgcc ttttagttca tgtgacttag gtatatcttt aataaacaaa tcagttttaa 22980
aattgttggt aaaataaaaa tacaaatgtt ctcagaattg tcagcatata tttttccctg 23040
agtttactcg tcagacagct ttatatttgt ctctgataga tgttttaaga tgtcagggtt 23100
tgacacaaag atgataaaac tataaaccca tcctaaaaca gaataatctt tgtatgatct 23160
ttgataaata agactaattt ggctgagtgc agtggttcat gcctgtaatt ccagcacttt 23220
gggaggccaa ggtgggcaga tgactagagg tcaggacttt gagaccagcc tggccaacac 23280
agcaaaaccc catccctact aaaagtacaa aattagccag gtgtggtggt gggcataatc 23340
ccagctactt gggagcctga ggcaggaaag tcactggaac ccgggaggca gaggttgcaa 23400
tgagccaaga gagcgccact gcactccagc ctaggcaaca gcgagactcc atctcaaaaa 23460
aaaaaaaaaa ggctaattta atattgcagg cttaataaaa acagctgtat cttatgagct 23520
atcgacaaaa tacccataca cttcactaag tttcttacta agtgaacatc taataatcac 23580

```
aggctataaa aatggttaaa agggaaataa ctttaagtaa tggctagctt tgtctaatat 23640
ctcaattttc ataagtaatc taggtaaatt attaaaaata aattaattaa gcctggtgtc 23700
atggcacacg cctatagtcc tagctactca ggaggctgag gtgggaggat tgcttgagcc 23760
cggaagttca gaccaccctg agcgatgcaa caagaccccc atcactaaaa taaataaata 23820
aataagataa atgtcaagaa aataaatgtt tataaataag cttcttatgt aatttaagat 23880
cttaaaatta tgttatcttc agttaaataa tagatactca ttaaatgtct gggtcatttc 23940
caaataagat ttttaaaact aacacaaatt actgaacata aatgtttgtt cttggcttct 24000
taaattttat agaaattttg tagaaagact aaatttattt gggtctatta atatatgtaa 24060
aaattatgtc atagaaatat gttttcaaaa attataaaat ttttctcatc tataaaatag 24120
tgatatgtga caaatggtta aaatttgctt gctagaaaat aaggttacta agagttaaaa 24180
ttctaattaa tatatataat tctgtctaca aagtatacca caaaaaataa gatgtgtttt 24240
tcatttttaa aaactataag acaggcattt ataatttatt ttactgcaaa aaaaaattta 24300
tctaatttgg agtttgttta aaggttgttt caaagcacag atttaggaaa aaagtaaaaa 24360
caagatggaa aagaaccact ctcatctgcc cccacgtaag acgtacctgc tccccttcca 24420
ccacgactgt gtttcccaag gcctctccag ccatgtggaa ctgaatttaa tctacaaata 24480
gagttgatca agtaacttta aggtatatgg acaataatat gctttgcagc ataaagacct 24540
gaaaacaaac taaatgccca tcagtgggaa attgtttcaa taaaccacag aacattccgt 24600
ggaaaactcc acagccattc aaaagaatga agggggagag atcatcatgg tggacaggag 24660
tcaggactag attgcagtcc gactgggatg gacagagcac atgtggaggc tggcaccgtg 24720
aattttagct ccagaacaac tgcaggaata aatcaggaat cccaagagga tccacagacc 24780
atgtgaagga agcagcctgc tcctgcagga cccagagaca ccccaaatac tgtgagtgcc 24840
caaactgtgg aagtgggaat gggagatcgt ccatcctgca cacacaccct cactagagaa 24900
accaaaggtc tagttggtgg gaaaaggttc caaccttacc tggagctgag tcaatttaga 24960
aagctgagcg aaatacaggg gtacagggag cagcgggaaa ggccctggga gcttcctggg 25020
tccccaggca ggccattcct gcatggcacc atatgaatac tttgggaggg tggccagagg 25080
cacagggaaa atgtcacagg gagaaggaag tctccagctg aacgcagggg agggcacgaa 25140
tcctgcagac cccactggta gaggaacaac caaagccctt cttcatagct gggaagtagg 25200
tagcctgggg caagttctct gcttgcccaa tgcctggaaa cagactcagt gctgttggcg 25260
aggggcatgg tgtgagtgag acccaccttc agtttgcatg ggagctgggt gacacctgtg 25320
actgccggct ttccccactt ccctgacaac ctgcatgact cagcagaggc agtcataatc 25380
ctcctaggtt cacaactcca ttgacctggg aacctcacag ccatccccc cagcagctgc 25440
agcaagaact gtccaaggag actctgtgag ctcagacaca cctagccctg cccgcatctg 25500
atggtccttc cctacccccc cttgtagttg aagacagagg gcatatactc ttgggagttc 25560
tagggcccta cccactgctg gttcctctcc atactaccac cactgatgct ctctggaaaa 25620
cgccatctcc tggcaggagg ccaaccagca caaaaacaga gcattaaacc accaaagcta 25680
agaaccttca tggagtccat ttcaccgccc caccacctcc actgggacag ttgctggtat 25740
gcacggcgga gaaacccaca gacagttcac atcacaggac tctgtgcaga caaccccccag 25800
tacaagcctg gagcctggta gacttgctgg gtggctagat ccagaagaga aataacaatc 25860
accacagctc ggctctcagg aagccacatc cacaggaaaa gggggagagt gctacatcaa 25920
gggaacaccc cgtgggacaa aggaatctga gcaacagcct tcagccccag accttccctc 25980
agacagagcc tactcaaatg agaaggaacc agaaaaccag ctctggtaat atgacaaaac 26040
aaagctcttt aacaccccca aaaaatcgca ctagctcacc agcaatggat ccaaaccaag 26100
aagaaatccc tgatttacct gaaaaagaat tcaggaggtt ggttattaag ctaatcatgg 26160
aggcaccaga gaaaggcaaa gcccgatgca aggaaatcca aaaaaagata caagaagtga 26220
agggagaaat gttcaaggaa atagatagca taaagaaaaa caataaaaac ttcaggaaac 26280
attggacaca cttatagaaa tgcaaaatgc tctggaaagt ctcagcaaca gaactgaata 26340
agtagaagaa agaaattcag agctcgaaga caaggtcttt gaattaaccc aatccaacaa 26400
agacaaagaa aaaagaataa gaaaatatga gcaaagcctc caagaagtct aggattatgt 26460
taaatgaaca aacctaagat aattggtgtc cctgaggacg aagagaaatc taaaagtttg 26520
gaaaacatgt ttgggggaac aattgaggga agcttcccca gccttgccag agacatagac 26580
atctaaatac aagaggcaca aagaacacct gggaaattca tcacaaaaag atctttaaaa 26640
ctaaaactaa aacaagcaat ctacaaaacc taggcacatt gtcatctggt tatctaaagt 26700
taagatgaag gaaagaatct taagagctgt gagacaaaag caccacatgc ttagtttcac 26760
tggatacaaa attcttggct gataattgtt ctgttggagg aggatgaaga tagggctcca 26820
atctcttcta gcttgtagtg tttctgctga gaaatctgct gttaatctga taggttttcg 26880
tttataggtt acctggtgct ttcggttcaa aaattttttt aatttccatc ttgatttcgt 26940
ttttgaccca ataatcattc aagagcagtt tatttaattt ccatgtattt gcatggtttt 27000
gaaggttcct tttggagttg atttccagtt ttattccact gtggtctgag agagtgcttg 27060
atataatttc aattttctta aatttattga ggcttgcttt gtggcctatc atacggtctg 27120
tcttggagaa tgttccatac gccgttgaat agaatgtgac catatgatag gaaataaact 27180
ccaaaagaag ccgaacaaca acagtgacac aacctgttga aacctctggg atacagcaaa 27240
ggcggtgcca agaggaaagt tcacacccct aggcgcctac gtcaaaaaga ctgaaaaagc 27300
acaaattgac attctaaggt cacccctcag ggaaccagag aaacaagaac aaaccaaatc 27360
caaacccagc agaagaaagg aattaatcaa gatcagagca aaactaaagg aaattgagac 27420
aaaaaaatac aaaagataaa tgaaacaaaa agctggttct ctgaaaagat aaataaaatt 27480
gatagaccat tagcaagatt aaccaagaaa agaagaaagt cccaataacc tcaataggaa 27540
acgaaatggg aaatattaca actgacacca cagaaataca aaagatcatt caaggctact 27600
```

```
atgaacgcct ttacacacat taactagaaa acctagaaga gatggataaa ttctcaggaa 27660
aatacaaccc tcctagctta aatcaggaag aattatatac cctgaagaga ccaataacaa 27720
gcagcgagat tgaaatggta attttaaaat taccaagaac aaaaaaaagt ccaggaccag 27780
attcacagta gaattctacc agacattcaa agaagaattg gtcctattgg tactattcca 27840
caagacagag aaagcgggaa gcctccctaa ttcattctat gaagccagca tcaccctaat 27900
accaaaaccg ggaaaggacg taaccaaaaa agaaaactac agaccgatat ccctgatgaa 27960
gatagatgcc agaaatcctt aacaaaatgc tagctaacca agtccaacaa catatcaaaa 28020
agataatcca ccccaatcaa gtgggtttca taccagagat gcagggatgg tttaacatac 28080
gcaagtcagt taatgtgata caccacataa acagaattaa aaacaaaaat cacatgatca 28140
tctcaataga tgcagaaaca gcatttgaca aaatccagca tcgctttatg attaaaactc 28200
tcagcaaatc agcataccaa gggacatacc tcagtgtaga aaaagccatc catgacaaca 28260
taattctgaa tggggaaaag ttgaaagcat tccctctgag aattgaaaca agacaaggat 28320
gcccactgtc accactcctc ttcaacacag tattggaagt cctagccaga gcaatcagac 28380
aagggaaaga aataaagggc atccatatcg ataaagagaa agtcaaactg tcactactga 28440
tgatatgatt gtttacctgg aaaaccctaa agactcctcc agaaagctcc taggactgaa 28500
aaaaaaattc agcaaagttt ccagatagaa gattaatgta cacaaatcag tagctactct 28560
atacaccaac agtaattaag cagagaatca aatcaagaac tcaacccctt ttacaatagc 28620
tgcaaaaaat aaaataaaat acttagaaat ataccttacc aaggaaggga aagacctcta 28680
caaggaaaac tacaaaacgc tgctgaaaga aatcatagat gacacaaaca aatggaaaca 28740
cataccacgc tcatggatgg gtagaatcaa tattgtgaaa attaccacac tgccaaaagc 28800
aatctacaaa ttcaatgcaa tccccatcaa aataccatca tcattcttca cagaattaga 28860
aaaagcaatt ctaaaattca tatggaggcc aggcatggtg gctcatgcct gtaatcctag 28920
cactttggga ggccaaggca gtaggatcac ttgaggtcag gagttcaaga ccagcctggc 28980
caacatggtg aaaccccatc tctactaaaa atacaaaaac tagccgggtg tggtggcatg 29040
tgcctgtaat tccagctact actcaggagg ctgaggcagg agaatcactt gaacctggaa 29100
ggtttgcagt gagcccagat tgtgccattg tactccagcc tgggcaaaag aatgagaatc 29160
tgtcacaaaa aaaaattcat atgaaaccaa aaaagagcct acatagccaa ggcaagacta 29220
agcaaaaaga acaaatctgg aggcaacaca cggcctgatt ccaaactcta ctataaggcc 29280
atagtcacca aaacagcatg gtactggtat aaaaataggc acatagacca atgtaacaga 29340
atagagaacc cagaaataaa cccaagtact tacagccaac tgatctttga caaagcaaac 29400
aaaaacgtaa agtggggaaa ggacacccta ttcaacaaat ggtgctggga taactggcaa 29460
gccacgtgta ggagaatgaa actggatctt catctctcat cttatacaaa aatcaactca 29520
agatggatca aagacttaaa tctaagacct gaaactgtaa aaattctaga aactataaaa 29580
atttccaata acattggaaa aacccttcca ggcattggct taggcgagga cttcatgacc 29640
agaacccaaa agcaaatgca ataaaaacaa agataaatag ctgggacata attaagctaa 29700
agagcttctg cacagcaaaa ggaacagtca gcagagtaaa cagacaaccc acagagtgag 29760
agaaaatctt cacaatctgt acatctgaca aaggactaat atccagaatc tacaacaaac 29820
tcaaacaaat cagcaagaaa aaaacagaca atcccatcaa aaagtggggt aaggacatga 29880
atagacaatt ctcaaaagaa gatatgcaaa tggccaacaa atatatgaaa aaatgctcaa 29940
catcactaat gattagggaa atgcaaatca aaaccacaat gcgataccac cttacccctg 30000
caagaatggc cataatcaaa aaatcaaaaa acattagatg ttggtgtgga tgcagtgatc 30060
aggggacact tctacactgc tggtgggaat gtaaactagt acagccgcta tggaaaacag 30120
tgcagagatt ccttaaagga ctaaaagtag aactactatt tgatcctgca atcccactac 30180
tgggtatcta cccagaggaa aagacgtcat tctacgaaaa agatacttgc acactcatgt 30240
ttgtagcagt acaattttca actgcaaact tgtggaacca acccaaatgc ccatcaatca 30300
acaagtggat aaaggaactg tagtatgtat atgtgatgga atactactca gccacaaaaa 30360
ggaatgaatt aatagcattc acagcgacct ggatgaggct ggaggctatt attctaagtg 30420
aagtaactca ggaatggaaa accaaacatc gtatgttctc actgacatgt gagagctaag 30480
atatgaggat gcaaaggcat aagaatgata caatagactt tgggaacttg gggtgaggg 30540
tgagagagga aagagggaga aaagactaca aatatggtgc agcatatgct gctcgggtga 30600
tgggtgcacc aaaatctcac aaatcaccac taaagaactt attcatgtaa ccaaacacca 30660
cctgtattcc aataacctat ggaaaaataa acattaaaaa aattaagaag actgaaagaa 30720
ttaaaaaatt taaaaaacag taagtagaag agaggtattt gaaggaagtt atgggtatga 30780
agatgtattt ttggtacgga aggttaaaaa gaaaagagaa ttttttttaa aaaggaagaa 30840
tcttgcgtga taaatttttg tcataaagta aaatgactgc ttactttaaa aaaagaggta 30900
tcacacacac cagggcctgt tgtggggtgg ggagaggggg gagggatagg attaggagat 30960
atacgtaatg taaatgacga gttaatgggt gcagcacacc aacatggcac atgtatacat 31020
atgtaacaaa cctgcacgtt gtgcacatgt accctagaac ttagagtata ataaatatat 31080
atatatataa agaaaattcc acacctgaag ccatgtgaca ggctacagac aaaacacagt 31140
taaaactttg tttcatgtac aaagttattt aaaatattgt acaaaatcac cttcaggctg 31200
tctgtataag gtgtacatga aatacaaatg aattttgtgt ttagacttgg gtctatccaa 31260
gatatctcat tatatatatg caaatatccc aaaatccaaa aaatataaaa tctgaaacac 31320
ttctggtccc aagcatttca gacaggggat cgttagcctg taatgagagg gagtatgggt 31380
caattatgac gttgaagatt caaaaaaaat tttttttaa ttttcagtca agctctacca 31440
actacactaa ctagaaaaca ctcaggcaga ttgcttttg ctaggttaat atggcatgat 31500
tacaactgtg catatgaata acatgtatca gattcctttt gtttgggaat tatttcatta 31560
caacactaaa cactgcttat gtacgctggg tctcttttc aaagactaca aataatgctt 31620
```

ggttctttgc tttcaaaata taccaaacat gaggtacaag aatgtaccca tccatatttg 31680
accaggggta gaattaaaag gtttgggttg aagaagatga cataacatcc caagagggtt 31740
gcatccctag gggtcatggg aacaaatgtg gccaagtccc tttttaactg gaaaggattt 31800
cagatccctg aacaagacaa ttcatcagtc ttaagcgtag taagtcatca gcaaataaga 31860
gaatgataaa cactagaaaa ataatcaaat cattgtctaa attcattata aaactatatg 31920
agaaaactat aaaactatat gagagagata aacagagaca agaagacaag gataaaataa 31980
aaaaagaggt atcaaacaaa gcagaacgcc tcaacatgtc ataaaacatc tgagtaagtc 32040
ataataaggt ttgcaaataa tgaatttacg aaaggaattt tgcgtgtgat caagatggct 32100
ataattagaa gggaattatt tataagtctt tctaaagact gaggtttgct attaaaaata 32160
tgctaatata aaactaaaga tttagtttcc tgtgttagaa caacaaagtt atcttgaagt 32220
attgatctgt tcttaaaact acaagaagtt tttattttta attctaaaat ctgtttcttt 32280
aacagaccat ttctattgct tcctgggatc catttacttt ccctagtttc aggttggaag 32340
tcctcttcat gtaaaacgag aatttcattt cttgacatag tcttttcccc ctaaagcttc 32400
tcagttttag atttcagaac ttcaacttct gttgtatttc acagcacatg atttatagat 32460
catgtatata aatatggatt tatagatcat gtatataaat acagacttat ccatcagtgc 32520
cttcagctct tcctccccat gagatggcct ggggtgatag ctctctcttt caactttttt 32580
tttatcaact cctataacat tttttctccc attacaactc tgttgttatg gctcgatgct 32640
gaaatgttta tcccgaaagt ctagaaaaca aatgttctct ccagtataat tccagtataa 32700
ttgaatttc ccttgtaacc aggaagtttc tcatgctgtt gctttttcta tgtgttcccc 32760
tgctcaggta ctagttatct tgtttacatt tctctactaa tggtttacac ttatagcctt 32820
ggacatactc tttctctgcc taattaaact cagtgtcttt ttcatcagat ttgacttcca 32880
gtttatctac atgggcttcc caagaggaga cacaatcaca ctgcaggagg catttcttta 32940
acttttgggt aagtagccta aaaaaaacaa agattttgta ttttattagg ataatttttt 33000
gtgttgtctt tatgaggttt ttggttactt aggtaacttg agctttgaag aagttaggtc 33060
cttttaatcc atgtaacttt ccgtattact cttcaagtct tttgatgatc actggttgaa 33120
tacatggcta tatttaatag tgacctgaga ttctgctttg attagccatg ttgaaccttt 33180
gacatctttg gcaggcttcc tcaggatcaa aattctaaaa taagtctttt tttatctaga 33240
attgacttag ggattttaca gttagacccc tggaaagcct caaagaattt atctctcatc 33300
ctatagagaa aataaatgat taggcttatt tggtaaatta tatgggaaac attgtcaaat 33360
aataagtgat attagatctt ctttcagtta catttgtggg tatgctattg atatgatgtt 33420
tcaaagatta tataaattca tatcagtcta taacgttatc agccataatt ttggttatgc 33480
tacatcttct ttaaagctat atttgtatgg agacgttact gatgtgagta tattctaaag 33540
attatgtgaa atttataaaa ccctgatggt tctgatgtga tgctatcagt cacagtcatg 33600
attctgtttg ctaccttaaa acactatagt aatttttaaa aagtcaattt ccttatcaat 33660
tgctgattat aatgaatttt tatcagatgt ttaaccatgg ccattttgtt tttgtgaccc 33720
agagttattg ttttgatttt tctccaaaag catttgtaat cagctattgt ccaaaattgc 33780
ttttcatgga agagactcaa acaggaactc ttaaatacgg tttcccgata aaagatcaat 33840
ggactcaata aaaaattttc agaactctaa taaagaaact gagaaattca aaaacctcca 33900
atcaagctca agcagaaaag ctgacttcat gatattgaag aagtgatgag gggaatattt 33960
ttatgaattt tatttgaagc atcgttcgtt cttaaatgtt ttgtttgcca gatttaagga 34020
aattttctct cgtaagtcat ctatagttta cagtaattta atacagtata ctttttgtga 34080
acaaagatga aagcaattat tttccccccct acgtgactcc tccaaaattt agaaactatt 34140
cacaagtgtt cttatgacga tgtggccatt tgtataagtc cagataaaaa ctagttgtct 34200
cctcactgca ggatgtaatt ggaaacatca ggtatattac taaggctttg gctgaaatac 34260
catatttgaa aaatatacat agaatgccta gtttcagccg ggcggggtgg ctcacccctg 34320
taatcgcagc actttggaag gccaagccag gcggatcacc cgaggtcagg agttcgagac 34380
cagcctggcc aacatggcaa aaccccatct caatcccgtc tctactaaaa atacaaaaat 34440
tagccaggtg tggtggtggg cacctataat cccagctgct cgggagactc aggcaggaga 34500
ctcttttgaa catggaaggc agaggttgca gtgtgccaag atcacgccac tgcactccag 34560
cctcggcaac aaagtgagac tccgtctcaa aaaaagaaaa aagaatgcct ggtttccagg 34620
gttccttata atgagtaaaa atcatcactt cctggcaagc ccagaaacct taaaactgta 34680
agtaaaagct aaagcctgtc ttggtttggc ttaccagcat aaagaggtat tgaagtacga 34740
gattcctatg tgatcaatgt acagaggaaa acttatgctt ccaaagaaaa gctgtaacac 34800
acctgctttt agattgtagc tctgtgcatt attttcaagt tcttgttttc tacctataga 34860
ctagatcctg aattcttcta gattattcca atccaacttt cttccatgga gaaaaatgag 34920
aactgctctg ttcctgaagc cctataagct gaagctagat aaatgctaag aaacaagtct 34980
catgcttgat gtccgagcca gacagaaagt tcaccagact gcccaatgcc acgactagag 35040
acattcaaac tgcaaagcaa gatgaggaat ttcacatttt cactctgtag acagcttctc 35100
ccaagtcatg ggaacaagac tccatatcat aatgggactc ttacctgtcc tagggcctac 35160
agtttttact tagcttccta ttttatttat tcaattaatt gccttgaatc ctggattcat 35220
tatttattca attaattgcc tttaatccta gacattgcat aatcctatta tgcaaactag 35280
gattgtcatg ttattactaa ttttactttt tattttccct ttttaaactt tgtatctgtt 35340
acttgctgaa ttttttcaga agtacaactt ctaacagaat aatgctagcc cagcactttg 35400
agataatagc aaaagaccac accacagaca aaattgaact taatcattta ctccaggtag 35460
acttagcctg aaagccactg tcttcaaacc tcctttgtta ctcaaatgtg gctaaaagga 35520
ttttgacact gactcctaga catcaatcac tccttcaaac atgtgaccag accagacacc 35580
tgggacaggc ccatccaaat actgagggac atcagaacct accacagcat ggtccatcag 35640

tgaggcttcc agagaaagac cttgaccaaa gggagaaaat atgatgaaag tcgtcagaat 35700
caaaatggag tcccttgtgt taaaaaacaa acaaacaaac gaaactctga catataaagc 35760
cagagaaggc tgagaagcat tctcatgcat aaacacctac tagcaaaaac tatcacaaaa 35820
gactataaaa aacacagcct tgcacaaagg ccattgcaac cttacacaaa aaaatacttc 35880
tgtgaggaca gctgcccagc aactgcctgt gcgcctcata ctggcatcac ccttgttatt 35940
gatccttgta gccaaagatt atttcaaaac gatcatgtaa tcgtcctcat ttttccttta 36000
aaaacctttg tcgtctttta cctccctgaa tatacacata gtttatgatg gtacgtgtgc 36060
tcccactgca gtgcttgggg accaagctcc ctccacacct cactgttccc aaataaatat 36120
tttctgttag agcctctctc tgttcattat ttagctcgac aggtatttgg tacagcagcc 36180
aaaacaggcc aagacattgc ccggaatctt ccagcagtag actgggggcc cggatttgac 36240
cctggtcagt tggacacggg cttctctcac tccaccagat gaggaacaaa tagtgaggtg 36300
tggcgggagc ttggtcccca aacagctcct ccggccattt gccctcccca cccacggacc 36360
agagacagga cgagggtgag ccccggtgtc agccatgtgc cccaaccccg gtgcctcatt 36420
tgctcctagg atgcgtcctg taggtcatct tcttgcagct gtcccgctca gtctcccctc 36480
aacccctgct catgctcttg gtgagcttat caatattatg gtttccaata gcttctgttc 36540
atagtttcaa ctcatatctg tccctaagtg tggactttta ttccacgcgt tggcactggc 36600
agtcagctat gtgcctccta ctcaaggcat ctgaaactgc aggtgggtag ttggctaagt 36660
caggccccaa aagtgtcttc atccagattc ccagaactgt gactctgaga tcttacatgg 36720
caaaagggac tttgcagatg agattaagcc aatgatcttg agatgaggag aggggcctgg 36780
attacttggg tggctccgaa tgtgggcaca ggtgtcacag gagggaggca gagggagacg 36840
tgagaaggag aaggtgccat gactccgaag ccagaggctc ctgctgatcc tccggcccca 36900
cccccacccc acggaccatg tggtcttttt aaaacatgaa tctatcagga cggcccattc 36960
aaacccacag agaggttccc actgcaccaa gaacaaagtc agaactctca ggcctgaccc 37020
actgccctcg gccaccctgg agccccagtg cccccagacc cggtcctggc ctcagagtgt 37080
aacaggctcg ctcccaccac ccatctgcat ggcaggcgcc ttgccatcgc ctcccctctg 37140
agagcacaga cagccacagc tggcctccgg gtgaaaggat ggatgaacag tcaggccggg 37200
cgagtgctct cagagcctgc tggccaacgg aggcagccgc tcaggcctct gcctgagcca 37260
tgtgcgatca cagctgctgc ccagctgtcc caaggaggca gctcttggct tttcccctgg 37320
gagtcgaatt cgcagggagt cccatccaca ggcccctggg aggcgggaag acatctcggt 37380
ctccaaagcc agtaacctgt gattcccctg aggatggtgg gaacctggtg tggggcagat 37440
gatctgtatt tgagctttgt ttctctctag caattatacc taggaaatac cctaaaatgc 37500
acgacgcaat gcacattgac cccagcaaac tccccaccct gcgcctgttt ctagtgccgg 37560
ggctgctcca gcacagcctg tgcacttgac cgaatgtcta cagccctggc cctcagccac 37620
actgtgttgt tttcatgcac cttcacccaa cagaattgct ccaacagagt gattcagggg 37680
gctgccctcc agcaccactt cccctttcca tgttacataa atgtccattg agtcctgact 37740
gactacagaa atctgtcttc tgggggcatg tgatgcccca ggtggcccag ggccccgcac 37800
aggaatgtgt ttacagcttt cccggcactg aagccacagt cgtaactcag cctcctgaca 37860
tgggccaggg ccctcgcctc cgcacgctct gcagctcacc cgtgtcgcca cagcctgctg 37920
ggcctggacg ccaccctctg aggtggcact cggggcagaa acatctgttt ccagctggta 37980
acaaacagga catgtctgcc tcccacacct aaatcaatgc ttgacaagcc tggcctgatc 38040
tcggggcgct caccaccagc cacctgcctg ggcccagccg acctcctctc ccttggtatt 38100
ttgagggaag ggtctgagcc ctttctgccc ccctagactt gtggacagag gccccccaag 38160
acctggacag aaatcaggcc atagtctcca gccccaaaga gacttccaga ggcctgcacc 38220
tctcctctcc agccgaggcc ctgatgtctg tggctgtggc cacagccggc tctatacctg 38280
ccaagtttat tctagaaaac attggtttgg acaagacctc aaaacggaat tgggaaaatg 38340
aaagagaaaa gagaaaggat ttctgctctc attgtaagga caaaaagcaa attgaagacc 38400
agcactgata cagatcaata atcacaccag aaaaaaatat ggcaaaatat ctatattctc 38460
ttaaggtgaa taagtgttct ctcagaatga ggctaaggcc aggaacagtg aaaagtaaga 38520
ctgataaatt tgcccacaag gaaaaaaata aagtctctat aaggacaaag ccaccaaaaa 38580
caaagtaagc accccaggaa agaaagtgac caaagtatct aacatgaaag agagcctgta 38640
ttcgcatgag ctcccgagaa acagaaccca tagatgtgga tgtggatatc gacagaaata 38700
ggtatcaaca tagacacagg ctatggccac aggtatggat agagggagag atgcgagaga 38760
gagattttaa ggaattggct cacacagttg tggagacgaa tgtccaaaat ctgcagggca 38820
aaccagcagg ctggtgaccc agagaagagc tgaggctgct gctcccgtcc aaagttcagc 38880
ctgctggcag aattctgtcc tccttgggga ctgccgtctg ttttctctta aggcctccaa 38940
ctgcctggat gaggcccacc cacattatgg atgatgatct gccttaccca aagtccacag 39000
gttgaaatgt taataacatc taaaaatcac ctgcactatc tagactggtg tttggccaaa 39060
tatctgggta gtatggcctg cccaagttaa cacctaaaat taactatcag agtccaagta 39120
agtgaatgta aaaaacaaac aaacaaaaaa aaaaaaacat gatgaatgag acaagctcga 39180
ccttgaagaa gttttcagtg gaacgtgggg aagaggagtt ctctttagcc agctgcagac 39240
acaaatagcc aatagaacac atggagagtg ttaaaagtca caaatcgtca atggaaagca 39300
acttacaatg tgatgttcat tgaacatcta ctgtgtgcca ggccaagggc taaggtggac 39360
acgtgcagtg tgtctgcact gcggcactaa ggtcctggtg gggaatggat aatacaatta 39420
aaaacaaatc acaaagaaac cagaagagga aaaaaagcaa ttaaaaacaa ataaatgctc 39480
cataaaggac aacagcgcca gctgagggtg tggccatcaa tgccaggcat ccagaatatg 39540
tccttgggga catggaccac tgggaggtgc atcccaggca aaaggagcag gaggggaagg 39600
gtccaggcca gaaacatgct tgccaagttc aagagcggtg ggaaggccag tgggcttggg 39660

```
aaggagtggg ctggaaggac aggccagagg ccagactgca tggggccaga ctggccctga 39720
ttccagcctt ggacttcact ctgtggtgat aggagaccct cagagggact tggggagggg 39780
ttgtgtgttt tcctgggagc cacagaaaag tgagtgacac tgggctgagg aaggaagtca 39840
caggacccac cagtgtctgc gcaggagtcc cagtggaaga gcagaaaaaa tccaggagaa 39900
gatacaaaag tattcaaaag taattcaaaa tagcaaaaat ccaaaatatt caataattta 39960
aatggtatta cttaaaatag tattataaat aataaaaata tattattaaa aataatagat 40020
tactggcttt aataccaatg tacagaaatc cgttttattt ctatagacca gcaacaaacg 40080
gaaaatgtga ttcttataaa gatgccagat gtctgcttct attaagggca tacagggaac 40140
tcggaccagt tagcaaatct gaaagaagta ttttaaaaat ctgtttgaag gtaatggaga 40200
gataccagag tggtgaataa tagccaccaa agtagctgcc atttgcagtc acttcccccct 40260
gagcccctcc ccaaaagcat ttgctgattc tgggatagtc agtgagaagc tgagcagaaa 40320
tcctgacaat cttagagcta gggagaaaaa ctcagaggcc aggactcacc aggagaaagg 40380
cgcttgggaa gtacatgcac tttggattag aaacccatgg actttcctta taggagcaaa 40440
ggtgaaggaa atcatccctc acaggaactg caaccagatg cactttattc aggtccttag 40500
aaaagctcaa cctctaccac tgggttaagt tgatctcaga ttttgaattc caccaggaac 40560
cagccagaag catatgaaaa ccgtctctgg aaaattataa cgtcatttta ggttcagttg 40620
ctcaacccaa tacttctgaa tacagtatcc agcacacaat cacaaagaac cacacataca 40680
agtagacgaa gcaacatgag taagaacagc aaaaacggaa gatcaacagg gactccagac 40740
ctcagctatg ggaattagca catgaagact ttcttaatat tttttatttc aatagctttt 40800
gggatacaag tagtttttgg ttacacggat gaattctata gtggttaatt ctgagatttt 40860
agggcaccca tcatccaagt agtgtacact gtacccaata tgtagttttt tatccctcac 40920
ctcccccacc cacttgtaat cccagtgctt tgagatgcca ggaggtttgg gaccagcctg 40980
ggcaacatgg caagacccta tctctacaaa aaatgtttaa taattacctg ggcatagtgg 41040
tacttgtctg tagcccccac tatctgagag gttaggtggg ataatcactt gagccagaag 41100
tttggggcta cagggagctg tgattgaatc actgcactcc agcttgggtg acagagtgag 41160
gccccatctc tgaaaaaaaa taaaaataag taaaatttta aaactgataa aataacagtg 41220
ctactaggtt tgaagagttt aaacacaatc tgaaaatttc atcataaatt gaaaactata 41280
aaacacacat agcagatctt ttttaattca gaattttaga acagaagtat acaatgaaga 41340
gctcaaatga tgggtttaag agcagactta acacagctga agaaagaatt agtgaatcag 41400
aagttggatc agaagaaaat atctagaatg aaggatagac aaaaacacag catggaactg 41460
gagggttaaa agaagagagt atccaccgat aaggtctaac atataggtag ttagagtccc 41520
agaaggagaa gtgagaagga agagaagaaa catttttaaa gaggcaattg cagaaatctt 41580
cccaaactta aggaaatatc ctaccccatc attcatatgg aataaaatac gtagaaaatc 41640
tcatctaggc caggcgtggt ggctcatgcc tgcaatctca gcaactttgg gaggctgagg 41700
caggcagatc acttgaggcc aggagcttca gaccaacctg gccaacatgg tgaaaccctg 41760
tctctagtaa aaatacaaaa attagctggg tgtggtagta ctcatctgta attccagctg 41820
cttgggaagc tgaggcagga gaatctcttg aacccgggag gtggaggttg cagtgagcca 41880
agatcgcacc actgcactcc agcctgggtg acagagcaag actctgaaga aaataaaata 41940
aaataaaacc acatctatcc ataacattat aaaactcctg aagactaaaa aaaaaaaaga 42000
ggctatctta agagtagcca aggtcgggga agctcacaat acctttaaag gaggaccagt 42060
gaggctggca gctaatgtcg taacagaaac aagaacagaa tgagatgctg tcttaggctg 42120
gcagctaatg tcgtaacaga aacaagaaca gaatgagatg ctgtctttac agtgctaaaa 42180
gaaatacctg ctaagccagg gaaactatct ttggaaaata aagatgactt ttctttttct 42240
cttcttttca attttcattc cctagggcta tcctaacaag gaccgtaaag aacagaagtg 42300
tattcttgca cagctctgga agctggaagt ctaaactcaa ggtgtcagga gggctgggct 42360
ctctctaaag cctctagggt aggatgcttc ttgccccttc tagcttcttt tttttttttt 42420
ttttatttct aaaacgactt tattgctaag aaccatgatt attacttaaa atagattttt 42480
cagttactga tataaaatat gttccttttg agaacacatg gacacataga ggggaatgac 42540
acacactggg gcctattgga gggtggaggc cgggaggagg gagagggtca ggaaaaataa 42600
ctaaggggca ctaggcttaa tacctgggtg atgaaatcat ctgtacgaca gatccccatg 42660
gcacacgttt acctgcgtaa caaacctgca cgtgtacccc tgaactcaaa ataaaaggtt 42720
ttttaaaaaa tgttctttgt gcttctaaaa atgtctaaat aatcctatat gcatttttct 42780
tatttgcctc ttatgcgtag cttacagatt taaaagtaca atgttgcttg ttttgtattc 42840
atcttggtgg atctaataat ttacaaatag gacataggca tgctgataca gtcagcagtt 42900
aattcctcct tctgacagtg gggtttggac atacaaccag caatgcgtgc aggtggagag 42960
acatagagga tctttttttt tttttttttt tttgagatgg aattttgctc ttgttgccta 43020
ggctggagtg caatggttcg atctcggttc actgcaacct ccgtctcctg ggttcaagcg 43080
attctcctgc ctcagcctcc tgagtagctg ggattacagg catgcgccac cacgcccagc 43140
taattttgta tttttagtag agatggggtt tcaccatgat ggtcaggctg gtctccaact 43200
cccaatctca ggtgatccac ccgcctcggc ctcccaaagt gctgggatta caggcgtgag 43260
ccaccacgcc ctgtacgaag ttttaggtca tctgtgattc acaaacgacc ggcttcgaga 43320
agctctggaa cgtgccaaac aaggcactgt ggagtagttt ggctccatgt gacctaagag 43380
aggaaaatct gttgtctttg ggaacattac acaattcagg taagatatgt gcatattctc 43440
aagggaaatt taaggtgctc atactcaata aataaacaca agagcagcgt tacagataat 43500
attcaaagag acagaggctt cttcatacct gatgtggtgg gaagaccact ggatgactgg 43560
acactgagat acgtggttga cagtctcgtc tctgaaacac atgatttgag ttttgagctt 43620
agacaaactt gtttttctcc agcatcagtt ttttcatcag taaacaataa taataatact 43680
```

gtgtttatgc caacatcatt aggaagatca ctttaaatgg tgtatataaa aggatccatc 43740
aactttaaag ccagtaccca atatacactc atagatatca gcgtattaca gaaaaatagt 43800
atttattttg tggtcaagca tatctggatt ctaatcccaa ttctactaat tgctccctta 43860
tcttttgtgt cccgtgatca tcaatgtttt tatcattaga atcttgataa tatctagctc 43920
accatgtttt tctggaaatt atatgagata acatatgcaa aggacttagt gcagcaccag 43980
atgtgactct tccagctcct gtggttgccg actgccctgg gtgttcccgg gcctgcagac 44040
gcatcgctcc agcctctgcc tctgtcttca tgcagtgttc tccctgtatc tgtgtctaaa 44100
tgtttctctt cttataatgg cactggccat agtggatcta ggacccactc tactgtagag 44160
tggcctcatg ttatcgtgat tatatctgcc aagtccctct ttccaaataa ggccacattc 44220
tcaagttctt ggtggacata aatttggggg tgactctatt caacccagta tactttctct 44280
tcctgagaag agcagtttgg actcaaagct gttcggtggg cagatcaagg gttggggggt 44340
gtctgcatgg tgggggtagg aggagagatg caggggccca gagcgggaag ccagtgtgga 44400
gtccaggctc ggagcaccca catgagcaga ggtgggcagc ctgatataga aagtcagatc 44460
ttaagtgggg tgagaagggt tccagatggg gaggagtgca cactagaatg gagggggagc 44520
cagaatggca gaggggaagg gagtactggc agagagggca ggttggtcac acaaggggat 44580
tggtcaagta agtaaataca ccgaaaagaa tagaaaagag agaaggcagt tccaaatacc 44640
aaaagggagt gatatggttt ggatctgtgt ccccacccaa atctcatgtc gaattgggtt 44700
tcaatctgtg tccccaccca aatctcatgt cgaattgggt ttggatctgt gtccccaccc 44760
aaatctcatg tcgaattgta atccccggtg ttggaggtgg ggcctggtgg gaggtggttg 44820
gatcacgggg gtgggttctc atgaatggtt tagcaccatc cccctagtgc tgttttcctt 44880
atagagtcct cccgagatct ggctgtttaa aagcacgtgg cacctccctg gtctcctttt 44940
ctcctgctcc gggaatgtaa gacatgcctg cttcccttca ccttcacctt ccaccatgat 45000
tttaagttcc tgaggcctcc ccagaagcca agcagaagcc actatgcttc ccggacagcc 45060
tgcagaaccg tgagtcaatt aaacctcttt tccttataaa ttacccagcc tcaggtattt 45120
ctttatagca gttcaagaac agactacagg gagaaactgg aatgaatcct atggtactgg 45180
attggaattt gatccgtaag tttgaattca tggtttctaa cataaatagc cattatatag 45240
gagttgtcct gatttgtaga aaacacacta aaatattcat gaaggaggag gtgtcaggtc 45300
aaaaaaatac tctcttgaat gattcagata aaaattgttt gtacttttct tgcaacttcc 45360
aatttttggc tgaagctaaa gaaatatttt cagacaaaac agaatatttg ttgccagaag 45420
acacacacta aaggaactgc ttaaagatag gtttaagaaa gaaggaaaat gatcccagag 45480
gaagcctcag agatgaaaaa aagaatttaa gagtgtaaag tacacaggtg agtacatttt 45540
aagagttatt aactaataac aatgatgatg tgatggatag tgaggataga aaattaaaac 45600
aattcagatg catagcaata acatataagt aatgaagggc taataacact gcatcatcat 45660
atctgaaaag aagatcaaag aattagtgac aaatttcttg ataaatttaa ctaatagaaa 45720
aaaatgcaac tgtaagaaag tccatccaaa gggaagcaag gaaaaaggga taaaagaaga 45780
ccaacaagga cagcattatc tcacttattt gtggaatctt aaaagagttg agctcataaa 45840
agtagatatt agaatggtgg ttaccagagg ctagaggcag agagggagag aatggagcgt 45900
ggttggtcaa agggtacaaa gtctcaggta gacaggagga ataagttctg agatctattg 45960
catgacagag tgactatatt cagtaataat gtatattttg agataactaa gagagtaaat 46020
tgaaagggtc tctccacaaa aaaataagta aatgaggtaa cagatatatt aattagcttg 46080
attcaatcat tccacgttgt ttacatgtat gaaaatattg tggctgggca cagtggctca 46140
cacctgtaat cccagcactt tgggaggcca aggcgggtgg atcacttgat gtcaggaatt 46200
tgagaccagc ctggccaaca tggtgaaacc tagtctccac taaaaataca aaaatcagct 46260
gggcgtggtg gcacatgcct gtaatcctag ctacttggga ggctgaggca tgagaatcac 46320
ttgaacccag gagacagagg ttgtagtgag ctgagatcat gccactgcac tccagcttgg 46380
gtgacagagc cagactctgt ctcgaagaaa caaacaaaca aacaaaaaac attgcattga 46440
actccataaa tgtatctaat tatgatttgt caattagaaa ttatattaat tttaaaacca 46500
cattgctaga taggagattt aggctcaaat atatcagtat ttacattaaa tgtaaatggg 46560
ctgggagtaa tgtcactgaa aatggcagct ccaaaaatgt gtcccttcac gaaagcaaca 46620
attaagctgg caaaaaactg acagaattca ttttttcata actctagaat ataaccaaaa 46680
acttaaaaca aagggtgctt aatgaagaaa gaaactgcta aatttgggta agagagaatt 46740
gtggaatttt cttacctgcc tataaagccc tcattttcca gctcagaagt agccagggca 46800
acagcagccc atcttcccgg tgtggtttgc tggtgccaga gggagtaaaa aagccgttgt 46860
catcaaagaa ttgtgcttgc gaatctcaac ctatctgaca gctctctgag ggatcagctc 46920
aggatcttgc ctttgttttg ctcaccctgc tcctgccctt cctcccctgt gtcctacccc 46980
caacaaacac acacagaatt ctctacagga tggaacagcc tcctgggcag catacatgga 47040
aagtatttaa aggtatatac ttgtcacaac catctaggtc aacagataat agaaagggaa 47100
aacagtagac aggctaaaac gcctaggaag aaaaaggcta aggaaggaaa cgtggggaaa 47160
tgagaacttt aaaaaactcc cacatatact aaggaattca aacagccaca tgtatttcta 47220
gatgtatgct cagataagac ctgagaagac cctaagcttt tacctctggc tggtcattag 47280
gcttcacatg agcaggaagt aaaggctaag gcagagttgt aaatgacctg gttaagtgtt 47340
gcagcagtgc tctaacccag agccagcctg caaagaacag gagggtcttc ttcctctttc 47400
tttctttctt tcttcttttg gcttaagaaa tttaagaaga tctgtgaaaa cactagctga 47460
ccactaaact aacataacaa agatttcagt ggccacatat gacaaaagtc ttcacaaaag 47520
tagtttagac aagtcactaa acaactataa cacaaaacaa gcagcaataa caaaccctag 47580
ggagaagaaa gaatctagtt tcccatatta taatatttat tcaaatttc cagtttttaa 47640
caaagaagta tgagacatgc agaaaaacaa gaaatatgac cctttcacag gaaaaggtaa 47700

```
attaacggaa acttcccctg aggaagccca ggctgtgaac ttactagaaa aaggccttaa 47760
atcaactgtc ttaaatattc tcaaagagct aaaggaaacc aggagaacaa tgcctcagaa 47820
ctagtgatta tcagtaaaga gatagaaatt ataaaatagc gccaaataga attcttgaaa 47880
tgcaaagtac tgtaactaaa gtgaaaaact cactacaggt tcaagaggaa acttgagcag 47940
gtagaagaat cagtgaactt gaaggtaggc caattaaaat tctcccaact aaagagcaga 48000
aaaaagataa ggaaaatgag cttaatttga acaatatgat caataaattt gatctactag 48060
gcatatacag accttagaaa gagcagctac agaatgcacc ttcattttga gccaggtgga 48120
atattcacac attataccat ttatgaccac aaaggaagat ttaacacatt ttgaaaggct 48180
aaaagtattt agttatgatg tgatcacagt gctaagtcag aaataaataa caaagaggta 48240
agttaaaata cacatatgct tggaaaataa gataaaaact gctaaataac cctgaagcta 48300
aagaagaaat cataatggaa acattaaaat tttaaaaagt ttaaaatatt tttaactgaa 48360
taataaggga aatactacat atcaaaacat ggtgagatgc agcctaaaca gtgcttagag 48420
gaaaacttat agccttagaa gcatgtatta tgaaagataa aatcctgaaa gttgatgaac 48480
taaaaattca cccgaagaag ttaaaaaaga atgtaaaacc aactgaaaat agaaggaaag 48540
agatcattaa aataatataa gaaaatgatt gaactgagga taataaagca aagaaatgat 48600
caaccaagtg aattaacaaa attgataaat ctctgataac acttgttggg gaagaaaaga 48660
gaaaatgcaa ataactaatg tcagaaataa aaagggacga cacacacatc ctgcagatgt 48720
ttaaaatgta aaaaagaata tttgggaaag ctttaagcta ctaagtgtga acaattggat 48780
gaaatctaac tggataaatg taagtattta aaaagtttaa tctacaattt ataaccttac 48840
cccagagaaa actgtgaacc agacatgatt tcataggga tttctgatca atatttaggg 48900
aaatatcaac actcttagac aaactcttcc agggatctga agaagagtcc atgccctgta 48960
agctactgta tgaagccagt gtaatctgag ttccgttggg actgcaaccg ataaagtaat 49020
tcatcacaga aatgacctca ggaaactggc cttcagaacg ggcttctggg aagttgtctc 49080
acatatttga ggaccacagg cataattcat tgcccgcagg aaatggggca gccatacatg 49140
acatgtggtg gcaccatgat gtctcagact gtcactgaca ggagtgcatc caaagtacaa 49200
ggagagaaaa aggcatgggc aggtcaagat gcagtagcac tttgttgtca tggcaacaag 49260
agggctgcta aagattatgg agtcaccaac tttttctcat gaccatagag agtgtacaca 49320
ggggaaaaga aacagtgaaa gctatgaaca tagcaaaaag aacatataca gagagccatg 49380
gacgcttgaa ggagtccctt acctcctggt catgggtcag atagagctga tacccaacca 49440
attcactaca gatgaaagtc ttagaaattg tggggctaca acatgcaaag gtttatcatc 49500
atcccacata acactaaaca ggaggctcta actgcattct tgttggacat gtatccaatt 49560
tcagaatagt gttccaagaa tctgcctcct aggaccactt ttgttggtgt cagggatccc 49620
cagaagctga tcttgagaga atattttaag tggacagttt gtttgagagg tgatgccagg 49680
aaacactggt ggaagagttg gggatgaaag gaagccaata aaaagtgcac taataagcat 49740
gttaccatgg aaataactgt aactgaaatt caccagggcc ctcaggaagc cagtgtcaaa 49800
caaacacctt ctcagtacgt ccccccaaca cacaccagca gcaagggagt ttggggtatt 49860
tatatgctaa cttattagtc attggttgat ggcagtttgt gcagagcaaa atcctgtgca 49920
acttcaggcc tgttgtgcac agaaaccctg actaccagag aaaaccctca ggcaagaaga 49980
tgcagatgct agcagtttga ggttacacca gtatacacta aagtgtaaag gcccaaggca 50040
cctggagtgc accaaaaact attgccacag gtgtacaaat gttcaaatta acaagaaaat 50100
gccatgtcat cctccaaagt catggcacca attcacattc ccatcagcaa atctgagacc 50160
ttatccaata ccaatatcct gacctgaaac tagatcatca tttatttgca tctgtgtttc 50220
cttcttttat gtatcctttt gtctcttgat cagagttagc cacactcttt attttgtatg 50280
catcattccc tccctttcca taaaaaaaat ttatcaaata tggccgggcg cagtggctcc 50340
catctgtaat cccagcacgt tgggaggccg aggcaggcag atcaccagag gtcaggagtt 50400
caagaccagc ctggccaaca tggagaaacc ccgtctctac taaaaataca aaaattagct 50460
gggtgtcatg gcacacacct gtagtcccag ctactcagga ggctgatgca ggacaatctc 50520
ttgaaccttg gatgtggagg ttgcagtaag ccaagatggc accactgcac tccagcctgg 50580
atgacagagg cagactgtct caaaaaaaaa aaattatcaa atatgtatgt atatctgaac 50640
ttgtctgatt ctgtttgccc ttgagctatg taaaattata tcatactata tgcagttgtc 50700
tgcattggca tttttaccca attttttac atcttgtttc caagattcat ctatgttgtt 50760
gtggggagtt gtaatttatt cacttttact gctgtatagg tttccagaat ttattttcct 50820
tggataattg tttcaaaatt tttctattat tagctgcctt actattgcat tcttgttcct 50880
gtctgttgag atcctgttat aggctgaata tgtcctcacc tctccccaaa ttcatatatg 50940
gaagccctaa tcctcaatgt gatggatttg gagatggggc ctttgggaag tgattagttt 51000
ttgatgaagt caggagggtg ggcgcccacg gtggtggtgg tcatgagggt ggtcctcata 51060
ggaagaggaa gaaacggaag agcctcactc tctctgccat gtgaatgcac catgagaagg 51120
tggccatctt caagccagga agacagccct catcagaacc cagccacgct ggcacccaga 51180
ttctggactt ccaacctcca gaacaaagaa aactaaattt ttgttgttta agccacccag 51240
tctatggtat ttttgtcagg acagcccaca ctaagatgga tactagtggg agagtttctc 51300
tatgaaatat gcacaggagt ggaagtactg aggtatagaa tattatccct atttagtttt 51360
ctggaaagtg ttttatcaag catgttacca tgacaattaa ctagagtcta acacctctgg 51420
ggaattttga ttatcgttgt agaatatatg ccttagaatt atcaccaggg acaagtgacc 51480
tcaggcattt acacacacaa gctcccatca gccatgggtt aagagctgct ccaatgagta 51540
ctaattcctg gcacttctgt tctgtattca tttaggctct ggaagccaca ggaagcaatc 51600
agacaaaaag tttcaggtgt cgtggctggg catagtggct tatgcttgta ataccagcac 51660
tttgggaggc tgaagcagga ggatcacttc agcccaggag ttcagaacca gcctgggcaa 51720
```

```
tatagtgaga ccacatttct acaaaaaatt taaaaattag ctgagtgtgg tgatgcatgc 51780
ctatagtccc tgttactcag taggctaagg cagaaggatc gcctgagccc aggaagttga 51840
ggttgcagtg agccgtggtc tcaccactgt gtgcattcca gcctaggtga cagagcaaga 51900
ctctctctct ctctctctct ctctctctct ctctctatat atatatatat atatatatat 51960
atatatatat atatatatat atatatggct gggtgcagtg gctcatgcct aataatccca 52020
gcactttggg aggccaaggc gggcagatcc ctgaggtcag gagttcaaga ccagcctggc 52080
caacgtggtg aaaccccacc tctactaaaa atacaaaaat tagccaagca tggtggcgca 52140
tgcctgtagt cccagctatt tgggaggctg gagtagggga atcattgaac ccagaaggcg 52200
gaggttgcag tgagccaagg tcatgccacc acactccagc ttgggcaaca gagcgagact 52260
ctgtaaaaaa aaaaaaaaaa aagaaaagaa aagaaaagaa aaaagaaaaa aaaaggaaca 52320
cagaaggcac aaacagcatc cactacagtc acccctcgca tgtctaaggt gcacttgcac 52380
cccacagtga tgtcaccact tcctattatt tcctctgaaa atgaacccat tttgagatgg 52440
gcttgggcca gaattaccct tacccactga tccctgtaaa cactcactct aacaggtgag 52500
gagtgaagag aatagggagg cttctatggt attctggtcc ctcaattcat tatctgggtt 52560
cgtggaacaa ctacagattc tgctgttgca actgatcccg aggccttcat tggcctttgt 52620
catctccctt ttggagatag gtttctaagg gtttcccttg accatggcaa gaacttctgc 52680
tgggctatgc tctatgcctg gtgggatgat ccaaacctcc atttctgca gaattacaga 52740
ccccaacaaa aatgtcctta ttccaggtct tgaggtccca ctccttcatt attggggtgc 52800
tttccttagt ggaagagaac tttgagctcc tggattcagc cttctttgta atcccactac 52860
tcttacaatc aggttctggg cccaatttc agtagtttct tctccagctg caggagatgg 52920
agtttttctt aatgctgtga tgaaggtttt ctgaaatttg caccatttcc tgagttgaaa 52980
ggggttcaac tgagcctgtt gttatatttc ttcagtgatt caatttcact aaacaacaac 53040
caaactattc caccattctt atgatcctgt ctttctcaaa ggccagagaa attacataag 53100
gagcacatcc cccaacactg taacactttc tatctatgtc ccattatcat aatgccagga 53160
gaagttattt acaaagggtt atttacaaaa gtgcaagtga gatgtaagag aaccactagg 53220
gatagtgcag taagccaggt taggtaacag gagggctgtt gctgcccaca aaaccaaatc 53280
tcaaggtgtt ttagacaaga cacaaactgt aaaagactta ttggtaaatt taactataca 53340
aaaattaaaa agtcatgtcc atcaaaagat aaagaaagtg aaagacaagt cacaacccgg 53400
gaaaatacat atgcaacacc taaacttaac aaaggaatat tatcaaaaat gtgtaaactc 53460
ctacaaataa agaagaaaag gataaatatc ccaatagaaa ataaaggcca acagcttagg 53520
caacatggca aaaccccatc tctacaaaaa atacaaaaat tagctgggca tggtggcaca 53580
cacctatagt cctagctact tgggaagctg aggtgggagg atcgcttgac cccaggaggt 53640
cgaggctgca gtcagccatg attgtgccac tgcactccag cctgggagac agagtcagac 53700
cctgtctcaa tcaatcaacc aatcaatcaa gtaaaataaa gaccaaaaga cacgaacagg 53760
tatttcagag aataaaaaaa atacataagg ccaataaaca gtttagaagg tgtcagcttc 53820
actagcaatc agaaaaaaag tcaaatcaag agaagaatat tcaagaaatc atgttagcaa 53880
aatgatgttg gcaaaaatta agaagttgaa ccataatcat taagacatag attcacagcc 53940
tccaagtacg aatgatcctc cgacctcagc cccccaagta actgggacta caggtgcatg 54000
ccaacatgcc cagctaattt tttttctttt ttgttttttt catttatttg tagagactgg 54060
gttttgccat gttgcccagg ctagtctcga actcctgtgc tcaagcaatc cgccccatct 54120
tggcctccca aagtgctggg attacaggcg taagccaccc caccgggccc agaatgcttt 54180
cttaatctaa aaactcccct aagtggccag gcgcagtggc tcacgcctgt aatcccagca 54240
ctttgggagg ccaaggcggg tggatcacga ggtcaagaga tggagaccat cctggcaaac 54300
atggcgaaac cccgtctcta ctaaaaatac aaaaattagc tgggcatggt ggcgcgtgcc 54360
tgtattccca gctactcagg agactgaggc aggagaactg cttgaaccca ggaagcagag 54420
gttgcagtga gccgagatca caccactgca ctccagcctg ggcgacagag cgagactccg 54480
tctcaaaaca aaaacaaaaa ccaaactccc ctaagtaggg ctattttcct gtccctgatg 54540
ccaccttgac aacataagtc aaccactgtt ccacatttgt cttttaggaa cgccccagga 54600
ctgttggctg ctgtccatgg tgctgccttg gtcagacacg tggtgtccaa gctgctgtcc 54660
gtggttttgc tttcattttt ctgtgtagca taatgatttt tttgtttatt tctttgttta 54720
attaattgat tttctctttt tagaacagcg ctaggtttgc aaacaaattg aacaaaaagt 54780
ccatagactt tttacatacc cagcgcctct ctatacatat acagtgtcac ctactgtcat 54840
tgtgcatctg tatggcacat ttgttacaaa tgatgagtcg atattgatac attatcatta 54900
actgaaatcc gtagtttacc ttagggttca ctacttgtgt tgtatagttc atgagttttg 54960
acaaatgtgc aatgtcacgt atccaccatg gcagtgttat gcagaatagt atcccctata 55020
tttcactatt cattcctgac ccctcacccc aaaccctggc aactgctgat ctttctactc 55080
tctctgtagt tttgcctttc caaatatata gtttggaaac gtacattatt tagccttctc 55140
caactggctg ctttcactta gcaatatgta tttaagattc ctccatgcgt tctgtggttt 55200
aatagttttt tgtttttgtt ttttgttttt gatagggtct tcctctgtca cccaggctgg 55260
agtgcagtgg tgcaatcata tttcactata gcttcagcct cccaggctca agcgatcctc 55320
ttgccttagc ctccaacaca gctgggatta caggcaccac catcatgccc atttttaat 55380
ttttcataga gatggtatct ggctatattg cccaggctga tctagaactc ttgggctcaa 55440
gtgatcctcc tgtctcaacc tcccaaagtg ctgggatcac agtgtgagcc accatgcctg 55500
cccttgatag ctcacttttt attgctgaat aatattccac tgtccggatg taccacggtt 55560
aactgaacca ttcacctgtg aatggtatct tagttacttc caagtttggc aattgcgagg 55620
aaagctgcta taaaagctca cgtgcagatt tttgtgtgga cataagtttt caactcattt 55680
gaataaatac ctaggagtgt gattgtatgg taagataaga tttagctttt aaaactgtca 55740
```

aactgtcttc caaagtggct gcaccatttg tattcccacc agcaatgaaa gagagttctt 55800
tatgcctcac atcctccaca gcatttggcg ttgagagctt tttgtttcat ttgttttctt 55860
tgggattttc tccattctag tagctggtcg tggtagctca ttgttgtttt catttgccat 55920
cccaatggca caccacgtgg agcatatttt catgtgctta tttgccatcc gcaagccttc 55980
tttgctgagg tgtctgttca gatcttttgc ccatgtttta attgggttgc ttgttttctt 56040
attgtttaaa tttaataatg gttctttaag tgttttagat acaagtcttt tatcagatat 56100
atattttctc ctcccagtct gtggcctgcc tttttcattc tcttgacaat gtctttcaca 56160
tgtagacatt ttttcatttt aataaacctc agttaactaa tttttctttt tatacaaatt 56220
gtgatttttg tgttatatct aaaaactcat tgccaagccc aagatcatct ggatttctc 56280
ctgttatctt ctaaaatttc atagttttag gctttacatt tgggcctatg atccactttg 56340
agttgatttt tgtgaaaagc ataaggcctg cgtctagatt cttttttttt tttttttttt 56400
ttttttgct ttggtgccat gtagtgaaaa gatgacgctt tctccattga actgcctttg 56460
ctcctttgtt gaagatcagt tgactatgtt tatgggcgtc tattatcaga aaattttgtt 56520
ctatggattt atttgtctat cctttcacca attccacact cctgattatt gtggcttcac 56580
agtaagtctt gcataaaagt tgggaagagt cagtcctcta actttgttct tcttcaatat 56640
tgtgttgtct cttctgagta ctttcccttc ccatataaac tgtagaatca gtttgccaac 56700
atccataaaa taatgtggtg ggaatttgat taggattgtg ttaaatctat atataaagtt 56760
gggaagaact gacatcttaa gaatattgtt tttcttttca tgaacgtgga atatctctcg 56820
actgatttag atcttctttc atttctttta tcacagtttt atagttttcc tagtatatat 56880
cttgtatatg ttttattaga tttataccca agtatttcag tttctggtgc taatttaaat 56940
ggtattcggt tttccacttt aaatcccaat tgttcattgc tggtatatag ggaaaaaatg 57000
gcttttgccc attaaactta acgtccttca aatttgcttt taggccattc ttgcgttgct 57060
ataaagaaat acttgaggtt gggtaatttt tttttttttt aaaagaggtt taattggctc 57120
acggttctac aggctgtaca gaaagcatgg cagcctctgc ttctggggag gcctcaggaa 57180
gcttccaatc atggcaaaca gcaaagggag agaagccata tcacatggcc gaagcagaag 57240
caagaaagag actctgtgga gggggaggtg gcacacactt ctaacgacta gatctcgtct 57300
gaactctgag cgagagctca ctcatcacca aagggctggc acaaaccatt catgaggatc 57360
tgcccccatg atttaaacac ctcccagcag gccccacctc cagcattggg gattacattt 57420
caacatgaga tttgggtagg aacaaatacc caaactatat cattgctata atcacttatt 57480
agttccagga ggtttttgt cgattatttg ggatttctg cagagatatc atgtcatctg 57540
atgacaaacg cagcttcatt tcttctcctc caatctgtat atcttatact tgcttttcat 57600
gttttaatgc attagctaag acttccagca tggtgttaat tgggactggt aaggggacct 57660
actcacctgt tgcttctcac catgaagtac aatgttagca gtaggttttt tgtagatgtc 57720
ctttataaat ttgagagtat tccccttat tcaaagtttg ctgagagttt ttatgataaa 57780
ttgagttgta ttttgtcaga cactttttg ttatctattg atatgaccac atgagttttc 57840
ttaatctttt gaagagatgg attatattaa ttgactttta ggtggaaagc ttatattaat 57900
tgattttgaa tgtccaatca gccttgtttc tattactgag agttctccag attaacagaa 57960
ccaatagaat agggtgtgtg tgtgtgtgtg tgtgtgtgtg tgtgagagag agagagagag 58020
agagagacag agagagagag agaattttaa ggaattggct cacatgggtg tggaggcctg 58080
caggtccaaa atcatcaaat cggtagtgtg gaacctgacc tagggaagag atgatgttcc 58140
agcttgagtc caaagatctt ctggaggcaa aattccatct tcctcagggg acctcaatct 58200
gtcttttta atagtcttca actgattgga tgaggcccac ccacatttg gaggatgatc 58260
tgctttactt aaagtctaac aacttaaatg ttaatcctgt ctaaaaaaat accttcacaa 58320
catccagact gatgtttgac caaatattgg gttccaagac ctagccaagt tgacaagtaa 58380
aatcagccat catatttgca tacctgggat aaatctcaat gggtatatta tttttatac 58440
atggttggat ttcatttgct agtatttcat taacattaga ttttacactg tcgatattga 58500
aggtaagcaa tctttgaaaa agactactag aactcaccca tagctgtttt tctccttcct 58560
ttgactcctg ctagactgtg tttcccagtg ccatcacatg tggatggaga cctgttacca 58620
gttctgttca atgagtaatg tgaataagtc agacagtact ttgaagagtt gtgatgctgt 58680
aacaaaaaat aatattatgt ggctcatgcc tgtaatccca gcacttcggg aagctgaggc 58740
aagaggatga tttgaactga gtaattcaag atcagcctgg gcaacatagc aatactccat 58800
ctctagaaaa aaaattaaaa attagccaag catggaggca catgcctgta gtcccagcca 58860
ccagggaggc tgaggtggaa ggatcacctg agcctgcaga agcagagggt gcagtgagct 58920
gagatcatac cactgcaccc cagcataggt gacaaagtga gactctatct caaaaaaata 58980
aaataaaaaa cttgccttag aatatgagaa gcagttagca aggaagctgg tgtcaggctg 59040
gaggactgca gatccttgtc atgccttaat aaatcagttg ttaacatttg gaaggcagag 59100
tccctgccta ttcagcctgc agctctaggg taagagattg caaaatcaag tattaagagt 59160
ttgttggcta ctattagctc ccttcattaa ttaactagag aaaagaggtt aggtcagaga 59220
attggtggct ttgtaagcaa aaattttaaa agaatagaga aaatccaaaa cttacaagga 59280
gggagtggaa aagctggctt cttctagaac ccaaacaaga caggacacca agaaaggttt 59340
tagatgacga ggatggccaa ggtgtgattt gcagtgtggc cttcccatcc atggcctctt 59400
tcacagataa tctaattttt aatttattat ttttgtggat acataataat tatatattaa 59460
tgtatttatg gggtacacat gatattttga tacatgccta gaatgtgtaa tgatcaaatc 59520
ggggtagtta aaatatctat cacctcaaaa tttatcattt ctttgtgttg gaaacatttc 59580
aaatcttctc ttctagctat tttgaaatat acaataaatt attgttaact atagtcacca 59640
cactgtgctg ttaaacacta gaacttattc cttctaacca actgttttgt gtgtgtgtgc 59700
gtgtgtgttt tttttgaga cagagtcttg ctctatcatc caggctgaag tgcaatggtg 59760

caatctcgac tcactgcagc ctccgtctcc cgggttcaag cgattctcct gcctcagcct 59820
cccaagtagg tgggattaca ggcacatgcc atatgccctg ctatttttg tacttttact 59880
agaaacaggg tttcaccatg ttgtccagat tggtctcgaa ctcctgacct caggtgatcc 59940
acccacttca gcctcaaagt gctgggatta caggcgtgag ccacaacacc caacctaccc 60000
aattgtattt ttgtatccat tataccaacc tctcttatct cctccccaca gcagtgatcc 60060
ttcccagcct ctggtaacca ccattctcct ctctacctct atgagatcca catttttagc 60120
tcccacatat gaaagaggac acatgatatt tgactttctg tgcctggctt atttctcttt 60180
aacataatga cctccctttc catccatgtt gctggaaatt ataggatttc attctttttt 60240
atggctgaat agtattccat tatgtatata taccacattt tctttatcca tttatccatt 60300
gatggacact taggttgata ccctatctta gctattgtga acagtgctgc aataaatatg 60360
ggagtgtaga tatatctcca aaatattgat ttcctttctt ttggatatat atgcatcagt 60420
gaaattgctg aattatatgg tagttctatt tttaattttt tgaagaactt ctacactgtt 60480
ttcaataatg gctgtactaa tttacattcc aactagcagt gaatgagcat tctcctttct 60540
ccacatcctc atcagcatct gttccttttt gtctttggta ataactattc taaatggggt 60600
aagataatat ctcattgtga tttcgatttg catttcctag atcattagta atgttgagca 60660
ttttttcatg tacatgttgg tcacacttgc atgtcttctt ttgagatatg catattcatg 60720
tcttttgtcc atttaatgg gatttttctt gttgtggagt tgaattcctt gtatattctg 60780
aatattagtc ccttgttgga tgaattttat cccatcaaaa agttgactct tcactctgtt 60840
gactgtttcc ttgctgtgca gaagcttttt agtttaatat agtcccattt gtctatttct 60900
gtttctgttg ccagtcattt tgaggtgtta gccataaaat atttgcttat gacaatgtcc 60960
tgtagtgttt tccctgtttt cttctagtag ttttataatt tttagtctta tgtttaagtc 61020
tttaatacat tttagttgtt tttttaattc agtgagagat tgggacctag ttttattctt 61080
ctgcatatga atatccagtt ttcctagcat catttattga agaggctgtc ctttcccagc 61140
actttcattg aaaatcagta ggctataaat atgtggttgt tttgtttcta ggttctcaat 61200
tctgttccat tggtctgtgt ctatttttat accaatgtca tgctgttttg tttcctatat 61260
cctcatgata tattttgaag tcaggtagta tgatgcttcc agctttattc tttttcctaa 61320
gggttgcttt ggctatacaa gctctttttt ggtttcataa taattttggg attgttttc 61380
catttctttg aaaaaatgac attgatattt taatagcgat tacattgaat ctgtagattg 61440
ctttggatag tatggtcatt ttaacaatat taattctccc aattcatgag catggaaggt 61500
ctttccattt gtttgtgtcc tcttcaattt ctttcatcag tgccttgtag ttttccttgc 61560
agaagtcttt cacctccttg gtgaaattta tttatatgga tttttgtagc tattgtacat 61620
gggattgcct tcttggtttc tttttcagct agttcattat cagggtatag aaatactact 61680
gactttcata tattgatttg tatcttgcaa ctttactaaa tttatttatc agacctaaga 61740
gtttttggtg gagtctttgg gttttttcct ttcagccagt atatatcttt taagtggaaa 61800
attctatctg tttacattca aggttattat tgatacgtgg ggacttattt ctgccatttt 61860
gttggttgtt ttcttgttgt tttgtctatc ctgtgttcct ttctttctct cttattgttt 61920
atcattgtgg cttggtggtt cctgtagtgg taacatttgt gtcttttctc ttcctctttt 61980
tttgtatttg ccgtgcggct gtaagtttta tacatttatg tgttttcacg atggtagaga 62040
tcatcctttt gcttccaagt atagaactcc cttaagcatt tcttgtaagg ccaatatagt 62100
ggtgatgaat ttccccaggc tccagtggct cacataggtg ctggctgtgg tgggcaggat 62160
gggatgatcc tttgccccac ccagtaacag tagcagtgtg acgggagagc ctgtcctcag 62220
ggcatgtgaa gtgcacagtg gccctgctat ggggtcactg cccgtggcat gtacttcagc 62280
cctggaagca gcagcagctt catcatagct tatttaattc agcggaccaa gagcataaac 62340
acctgagctt cctcttttta tctgctcatt ctgcttccca agttctcaag gacttatgtg 62400
gacagtcttc ccgcacttgc caaaaactca gttcattctg ttataaatct cagtgcctct 62460
acctgctaca gacacggagc tcaccccgtc agtggcaccc cttctgaccc agaagtgtcc 62520
ctaggtgtct tcgtggcctc tgccatgggt gctggtatgc cattgtggga ggtgggggc 62580
agtgggagtc aggaggttgc tcctctgggt ggagctgcct gccaatcaag atcctgactc 62640
tacctccacc ccatgtgacc aggctgcagg ggcaggcctt agtgggcacc ggtggtgccc 62700
tggtcacaag acaccctgga ggtgaagggg gccttccaat tctaaagctg tagctgcaga 62760
cttgggcagt gcaaatcctg tcaggaccaa acaccaaatt ctatcaccca ctcaagcttg 62820
aagttaagtg ggagggagag agtccctgca ggacccttgc tatagttgga tgtttgtccc 62880
ccaagacttc atgttgaaat tcgaccccca gtgttggagg tggagcctgg tagaaggtgt 62940
ttggattttg ggtgcagatc ccaaaatgct ctcctttgag ggtgagttct cactcttatt 63000
cctgctagag ctggctggtg aaaggagcct ggcatctccc ctctttcttg cttcctctct 63060
ccccatgtga tctctgcaca taccatggtt cttgtacagc ctgcagaacc atgagccaaa 63120
taaacctctt ttctttataa attacccagc ttcaggaatt cccttatagc aatttaatgg 63180
acaaagacaa cacctgacag ccactctccc acatgcctcc agcaatgctc tgtgggtttg 63240
cacctccaac taagtcattt aggcaggatt taatggaagg acaccttgct gacacagatc 63300
tgggctcagt gcaagagccc ctgggacact gaggactcca gagctggctc cggcagagtg 63360
aagagtagca gaatatgcca ctttcaaaca agaataattt tgagccgaag acaattaaaa 63420
agaagcatac ccaagaaaaa aatccctgcc cactcccttt ctaccaggaa aagcagagga 63480
ttcttagtcc ttggagacaa ctctagatgc ttatcagccc agagaaggca ctagaggaat 63540
ctacacaaca aactagcaag ctatcttctg ttagcttccc ctatatattt acttcccaga 63600
atttgctgcc ctacaggttg aaagcccctt tcctttgtct tgccaattct gtaaaaatga 63660
ttgcccattt gctaagatgc tccgtaagct cacgttccaa ccgcccccttt gaattcctca 63720
tctctgacgg ctcccacata gaggcataat gcacatgtta gtaaacttct gtttgttttt 63780

ctcatgttag tatcttgtta atctaattga caagacccca gctaatgaac ctaagatggg 63840
tagaaggggg aaaaatgttt cttcccctac aagagcaagc acctccccac acactggagg 63900
aggagagaga gccgctccga gatctgcagg aacagaggct ccttgggctg tctgttgagg 63960
aaggcaataa ggtccaggca accctgcaag gagagagcca gggtcaccac ttagcatcag 64020
catccccttc ctctgatctc ctgcaggcag ccagcccacc caaagagctg acccatccag 64080
cacccaagga gtgaggcagg ccagagtcca ctccaagggc agaggctgtg gggcaggtgg 64140
ggagccagcc tggaggatgt tagcagagcc aggtgggtca tggcacccac agcctcgggg 64200
atccagcttg agagagaccc tgagggccag gcttgtcaga gctgataaca gatgagttgt 64260
gatgacaggg ctcattgtca gacactgtag gctgtcaggc tgtcaacctg agcagactac 64320
caagtcttcc catccgcaaa acagctcacc ccagctatca ggggtgggca cccagaggag 64380
agaccattct catctccacc tgaactcttt tggtgctccc agaacattct gatgctgaaa 64440
aggagtgtag aatcagatag aaacaattcc tcttgtagct tccatgatgg ttggatggta 64500
aagtacaact ggctttgtta gttcattttg cattgctgta gaggaatacc tgaggctcag 64560
taacttataa agaaaagagg tttatttggc tcagggtttt gcaggccata aaagaaccct 64620
ggtgccagca tctgcttctg gtgagggcat caaggagctt acaaccatgg cagaagttga 64680
agggggagag agcatgtaac atggtgagag agagagcaag agagggaaag aggaggtgcc 64740
aagctctttt aaacaaccag ctcttgtgtg aaccagtaga gcaaaactca ctcatcacca 64800
agaggatggc agcaaggcca ttcatagga tccatggcat gacccaaaca tttccactag 64860
gccccacctc caacattggg gatcacattt caacaggaga tttggagggg acaaatatcc 64920
aaaccatgtc actccacact gtccccccaa atctcatgtc cctctcacat tgcaaaatat 64980
aatcatccct tcccaatagt gcccaaaaaa tctcaacttg ttctaggatc tactcaatca 65040
aaactccgaa gtttcatctg agactcaagg catgcccctt ccacctatga ggctgtaaga 65100
tccaaaacaa gttgctactt ccaagataca gtgatggtac aggcattggg taaacatttc 65160
ccttccttcc aaaaggaata aattggccaa aagaaggagg gggaagaagc aacaggcccc 65220
acacaattct gagagaaaga cattaaactt taaagctcca aaataatcct tgactccatg 65280
tcctgcatcc agggcacact ggtgtgagga gcgggttccc aaggccttgg gctggtctgc 65340
ccccatggct ttgcagagtg cagcccacat ggctgctctc atgggttgta gttgagtgcc 65400
tgaatctttt ccaggctctg ggcgcaagct gctggtggct ctaccattct tgggtatgga 65460
gggcagcagc cccattccca gagctccact aggcagtacc ccactgggga ctctgtgaag 65520
gggctccaac cctacatttt ccctccacac tgccctactg gaggctctgt gggagaactc 65580
cacccctgtg gcaggtttct gtctgggctc ccaggctttc ttttttgtct ctctcttttt 65640
ttatttttat ttcaatagtt ttaggttttt ggttacatag ataagttctt tcatggtaat 65700
ttctgagatc ttggcacact tgtcacccaa gcagtgtacg ctgtacccaa tatgtaatct 65760
tttatccctc acctccatcc cccacttgcc ctcaagtatc caaagtccat tatatcattc 65820
ttatgccttt gcatccttat agtttagctc ccacttataa gtgagaacat atgatgtttg 65880
tttttccatt cctgagttat ttcacttaga ataatggcct ccaactccat ccagtttgct 65940
gcaaatgcca ctattttatt cctttttatg gctgagtagt atcccatggt gtatatatac 66000
cacatttttt atccactcat tggtcaatgg gcatttaggc tggctccata tttttgcaat 66060
tacaaggagt tgcaattgca ctgctctaaa tatgtgtgtg caagtgtctt tttcatataa 66120
tgacttattt ttttctggat atcgaatagt gggactgctg gatcaaatgg tgattctact 66180
tttagttatt taaggaatct ccatactgtt ttccaaagtg gttgtactag tttacattcc 66240
caaccaggag tgtaaaactg ttcccttttc accatatcca tgacaacatc tgttagtttt 66300
taatttttaa ttatggtcat tcttacagaa gtgaggtggt atctcattgc agttttaatt 66360
tgcatttccc caaaaattag tgatattgag catttcttta tatgtttgtt ggccatttgt 66420
atatcttctt ttgagaattg tctattcatg tcgttcgccc actttttgat ggtatttttt 66480
ttattgatga tttgtttgaa ttccttgtag attctggata ttagtccttt atcagatgca 66540
tagtttgcaa atattttctc ttattctgtg ggctgtctgt ttactctctg attatttctt 66600
ttgctgtgca gaagtttttt ttgtctaatt aggtcacatt tatttattta tttatttatt 66660
tttggttttg ttgcacttgc ttttgggttc ttgatcatga actctttgcc taagccaatg 66720
tctagaagag ttttaccaat gttatcttct agaattttca tggtttcagg tcttagattt 66780
aagcctttca tccatcttga gttgattttt atataaagtg agagatgagg atccagcttc 66840
attcttctac atgtggcttg ccaattatcc cagcaccatt tgttgaatag gacatctttt 66900
ccccacttta catttttgtt tgctttgtca aagatcagtt ggatataagt atttgacttc 66960
atttctggat tctctgttct gttccattgg tctaatgcct atttttatac aagtaccgtg 67020
ctattttggt aactatagcc ttgtagtata gtatgaattt gggtaatgta atgcctccag 67080
atttgttcac cttgcttagt cttgctttgg ctatgtgggg tctttttttgg ttccaaatga 67140
attttagaat tgttttttat atttctgtga agaatgatgg tggcactttg atgggaattg 67200
cattaaatct gtagattgtt tttggcagta tggtcatttt cacaatattg attctaccca 67260
tccaagagca tgggatgtgt ctccatttgt ttgcgtcatt gatgattgct ttcagcagtg 67320
ttttgtagtt tttcttgtac agatctttta cctccttggt taggtatgtt cctaagtatt 67380
ttattttttg caggtgttgt aaaagggatt gagttcttga tttgtttctc agcttggtca 67440
ttgttggtgt atagcagtgc tgctaattta tgtacattga ttttgtatcc tgaaatttta 67500
ctgaattaat ttaacagatc tagaagcttt ttggatgagc tttagggttt tctaggtatg 67560
tgatcatatc atcagtgaac agcaacagtc tgacttcctt tttactgatt tggatgggca 67620
cccagacttt cctatacacc ctctgaaatg taggtaggag ctgccatgcc tccttcactc 67680
ttgtattctg cccacctgtg ggcttagcac catgtggaag ctaccaaggc ttccagcttg 67740
caccttctgg agtggcagcc tgagctgcac ctgggactct ttgagccttg gctgaacctg 67800

gagcagcaac ctcccaagat accacaggga agcaaggccc cagccctgac ccccataaca 67860
atttcatcct cctaggcctc tgggcctgtg atgggagaga ctgtcccaaa gacttctgaa 67920
atgcctttaa gaccttttc tcattgtctt tgctattagc atttagttgc cttttagtta 67980
tgctaatctc tctagcaagt ggttgctcca taaggcattt gggttccttg cctgaaaagg 68040
ctcttttctt ctcaacctca tggccagcct gcaaatttc caaatgttta tgttctgctt 68100
accttttaat tataaattct aattttaagt catttttttt gctcccatat ttgatttagg 68160
ccattcaaag cagccaggtt acttcttgga tgctctgctg ctttgaaatt tcttccacca 68220
gataccttag gtcatcactg tcaatctcag cctttcagaa agccctggaa catggacaca 68280
atacagccaa gctccttgct gggggcataa caagcatcac ctttactcca gttcctaata 68340
aattcctcat ttctacctga gacctcatcg gcctggcctt tactgtccat atttctatca 68400
gcactttgat cacaactatg taaccagtct ctaaaaagtt ccaaatttcc cctcatcttc 68460
ctgtcttctt cctgggccct ccaaactctt ccaacctctg cccattaccc agttccaaag 68520
ccacttccac attttcaggt atctttatag caacaccgca ctccttgcta ccaattttct 68580
gttttcatcc attttgcatt gctataaaag aatacctgag gctggataat ttgtaaagaa 68640
aaggaagttt atttggctca ctacaagctg tacaagaagc ataatgccag tatctgcttc 68700
tggtgaggcc tctggaagtt tacaatcaag gcagaaggca aaaaaggagc cagcatagga 68760
caggcatggt ggctcatgcc tgtaatccca gcactttggg aggccaaggc aggtggatca 68820
cttgaggtca ggagttcgag accagcctga ccaacatggt gaaaccccat ctctactaaa 68880
aatacaaaat tatccgggta tggtggtgca cacctgtagt cccagctact agggaggctg 68940
aggcaggaga attgcttgaa cccaggagac agaggttgca gtgagtgagc cgagatcaca 69000
ccattgcact ccagcctggg caacaagagt gaaactccat ctcaaaaaaa aaaaaaaaaa 69060
aggagccagc atgtcacatg gtgagaatgg tgagagggag aaagagagat aagaaggaag 69120
tgtcaggttt ttttaaacaa ccagctcttg tgtgaactaa tagagtgaga aatcacccat 69180
caccaagggg atggcaccaa accattcatg agagatctgg ccttatgacc ccaatacctc 69240
ccaccaggcc tcacctccaa cactggggat catatttcaa tgtgaaattt ggaggggaca 69300
aacatctaaa tcatatcagg aactccatgg tactgctaac ccccaaatct ctaggggtga 69360
tattgagtga catgcatggt actgaacgat acttggtcag gttctcccag tgaagcccaa 69420
gcaccctcag ggtacagggc tgggaggccc aatcacacca gctcagccca ggacttggct 69480
gagtctgccc tggtattaga aggtagcaga cctagaactg ggttgaacaa gatcaggcca 69540
ctttgtgcat gttgccaaag gagaaactga aggatcagaa gtgggcagta agttttcagt 69600
gagcccaggg gtcataagta gcaaaatgtg gaattcagta aggggtagag gctggaagag 69660
ttttgaggtg tgtgttagaa aaagcctagg tttccttgaa gactatgtta gaaatttgga 69720
cattaaaggt gatcctggtg ggggctcaga aagaaagagg agagctatag agaaaactcc 69780
tgtcatcttg gagaatacat aaattgtcac gaacagaatg ttgctagaaa cgtgaatgtt 69840
aaagatgcct ctgctgaggc tccagacaga aataaggaag gtgttattga aaacgaaggt 69900
gacccttgtc atacagtggc aaagcacttg gctgaactgt gttccgtttt gtggaaagta 69960
gaaattatac atgatgaact tggatgttta gctgacgaag ttcctaagaa aattgttgaa 70020
ggcattcttg atttctcctt gctgcttaca gtaaaatatg agaagagaga gatcagctga 70080
agagggaagt gctgaggtgg ctgggtcagc tgacccaagg gcccttcaag ctgatttagg 70140
ctgctcacct tggtctcaga atcaccacgg ccatggccgg tcagtcacag gtgatccagc 70200
agctgctgca ggccgagaag caggatgcca agaaggtgtc tggggcccac aagcaaaaga 70260
accagaggct gaagcagccc aaaggagcag cttaggctga aattgaacag cactgcctgc 70320
agaaggagaa agagttcaag gccaaggaag ctgcagcact aggatctcgc agacccagga 70380
taaccatcct ccagacctac ttccagcaga acagggaaga agtcttagat aaccttttgg 70440
cctttgtctg cagcatccag ccagaaatcc ctgaaaacta tcgcataagt agatattaaa 70500
agagagaagc acctgttaaa tggactggca ctttagatgc cctcatggaa taagaagctc 70560
tagttatatt cttataagaa gacattaact tatctctgta tattatagag taggcccatt 70620
cacttttcgg agagaagcaa atccagtttc tttgtacaga cttagaattt atctaggctg 70680
ggcatggtgg ttcacgcctg taatcccagc attttcgggg gccaaggcgg gcagatggct 70740
tgagctcagg agtttgagac ctgcctgaac aacacagtga gactgtgtct ctacaaggaa 70800
atataaaaat tagccagata tggtggtaca tgcttatagt cctacctact tggaagagtg 70860
aggcaggagg atcacttggg cccggaaggc agaggttata gtgagccaag attgtgccac 70920
tgcactccag cctgggtgct agagtgagac cctgtctcaa aaaaaaaaaa cttaaagatt 70980
tcatcttttt acctcatgtt tcttaggaat ataatgggta aatattgtct atttttattat 71040
gccttttggc tcaagcaata tatatacata tatatatatc actgttgatg ttttctttct 71100
tgtatcaagt ttaaaagaaa aagcaaaaca atcctttgaa aaaaggaagg aattaaatca 71160
ttttttcctt aatgcttttt tgaaggtcag gggctttatc tatgaaaaag tagtagtttc 71220
tgtaacctgt gtgaagcatc agccagcctt aaagtagtcc attgctgcta ataattagaa 71280
cagtgaatat tactagtata attgtttcag cttcttaatc aaaataacta gatgatagaa 71340
ttcaagaact tgttacatgt tattactcgg tgtactacta atcatttaaa agtaaagcct 71400
atcatgcaaa taaataaata aataaataaa taaatacatg agaccagata aaatgaagga 71460
agaatgactt tgagatcaga gccacaggtg aggaggctgt tggggctgct gttgccacct 71520
tgggcccaga gtgtggggcc accccagtgg gcctggaaga tggagcatta accgaggagg 71580
atgaatcttg agacttaaat ctaataaagg gccaactaga agcccctagt gctcatcatc 71640
ctcataaaga aagccagaaa agtcaataga caactatatt ttaacgaaaa taactgaggg 71700
agagcactga gtgcatcaga ggagtaacag aaaccctgat aagcacagaa atgtgggctg 71760
gccacataga aaacagaagg aaacacacgg cctccaccac tccatccccc aaccaggatt 71820

atctgggaat caggaggaat ttctgacatg aggaggtaag caagaggatc ccagtggcac 71880
ccactaacac ctcggacacc tgcagacctc gccactgggg tcccctgcag tttgcactgg 71940
agggagttgc ctggaggcca catagctgtg ctctccaaaa aaggtgctga caccgtaccc 72000
cactcccaca tggctagtgc ttcaccacac tggaatagga actatggctg gagtgtgtct 72060
tacccaagga gtgagtagtc acagctcccc ttcatctctg aggctaagct gccactgaac 72120
aacccagact agtggtccca cgtccccaag tcaagctatg ggcatctatt aaacccttcc 72180
ccacagagcc aagtggatca gctctaccca ctcctccccc tgaccctttg agtccaagct 72240
aaagcagttt cttgcctcct agcaaaacag gactttggcc atgcctttct agtgtctacg 72300
ttgaagcagc accctgcatt ccaggaacta atgccttggc cacccagaag gtcacacact 72360
gcagtgctta ggctgaagca gcaacctgta tcctacagaa atggtgcctg ggccactcag 72420
aacaatcaca ccctctgata cataaatgca ggactttcct tacagcagag aaggcctgag 72480
ctgaagcaac acatcacctt ctagggaatc agtgccctgg cttaagctga gcagcagcac 72540
atcccaaggc tgagctgata tagtacccta tgccccagga aaacagagca gtggctaagc 72600
tgagacatct cagcctacag gccaaacaac tgtagtgccc tgcttccctg gagcttgcct 72660
aggcccctag ggtctgagct gctgaggcac cctctccctg ggaagtagaa tcagcactgt 72720
gctgttccct gcctactccc caccaaggcc caaattacag ctatgctctg ccatcttggg 72780
atacttgctg acactgtacc tggtctcaca tagtctggga tcctgctgag ccctaccatc 72840
ctagggtcta gagtcaatac catacagtgc ctcatcccct gggaccaaag tttccattgt 72900
gccctattgg ctcagcttcc caaattgcag ccataccata ctccccaggt ccacacctcc 72960
agaacacccc ttcttcccca gagttggaca aatgctgcac cccccaggaa tagactcaca 73020
gctacaacct ggccccttga gcaaaacctg ttaaagggta cctcagagtc acagatccta 73080
gcactgtgag ccacctaatc caaacctgcc acagaaactg aacctacacc ccaagaccca 73140
gatgccacag tagattcttg aaaccaggag actaggactc cagttgcaca gctactctaa 73200
ttttctgcac ctggaatcca gcaccattgc agctgcacgt tggccacatt agacctgcta 73260
ccaagagaga gccccttgac taaatctcct cattgtgggg aaacaagaag aggaggacta 73320
caaaagccct tgacactgag tacattaacc tgtgccacca ctacatccac aaacttctac 73380
agcctagtcc actgaggtgc ccacagttat tgctgaggtt gaatgcagtt gaagaagacg 73440
catggaagct atgccactgc acctattcag aaacagagtc atcacacttt cccaaccaaa 73500
acactaaacc caactgcagg tgaaattgtt tctctaaaaa acccactctt gaaagtttgg 73560
aagaggtgac tatttcgcca gatgcacaga catcaatgca ggaacacaat aaacatgaga 73620
aagcaaggaa atatgacacc accaaaggaa cataactctc attaatagac ctcaatggaa 73680
aaagaaatca atgtattgct ggaaaaggaa ttcaaaataa tgatcttaag ataactcaat 73740
aagatacaag aaaacagata aacaattcaa tgaattcagg aaagtaattt acagtatgaa 73800
tgaaaatttc aacagagata gaaataaaaa agaagcaaac taaaattctg caggtgaaga 73860
attcaatgaa taaaattaaa aaatacagta atgagcttca acagcagata tgatgaagca 73920
gaagaaagga agagatagat cccaatacaa taatagttga taacttcaac accactctca 73980
gcactggaca gataatccag acagaaagtc aacaaagaag cattcaattt aaaaagcact 74040
ttagaccaaa tgcatctaac agatatttac acaacatttc atccaacagc tgtgaaatac 74100
acattctttt catcagcaca tggaatattc tccaggataa accatacagt aagtcacaaa 74160
acaagtctca acaaatttga aagaattaaa attatatcaa atatcattta tgaccacaat 74220
aaaactcgaa atcaataaga ggaactttta agactgtaca aagacatggg aaattaaata 74280
atgtactcct gagtgaccaa taggaattaa gaagggaatt ttagaatgtc aacaaagaaa 74340
atgaaaatag aaatgcaacg tactagaatt tataagatat agcaaaaagc agcattaaga 74400
tggaagttta tggcaataaa tacctacatc aaaaaagcag agagatttca aataaatctc 74460
atccttgaaa atgcatgtca aggaactagg aaatcaagaa taaaccaaac ccaaaattag 74520
tagaaagaaa gaaataataa agatcagagc agaaataaat gaaattcaga ctaaaaaaaa 74580
aaaagatcaa caaaatgaaa aattgaattt ttcaaaagat aaacaaaaat gacaaataat 74640
tagctagact aaacaaaaaa gacccaaaag aatgaaatca gaaatgaaaa cagaaacatt 74700
acaactcata tcacagaaac acaaaggata attagagacc atacaccaac agattagaaa 74760
acctagcaga aacaggtata tttctggaca atacaaccta ccaagactga acccagaaga 74820
aatagaaagc ctgcacaaac cagtcatgag cattgagatt gaatctgtaa caaaaagtct 74880
cccatcaaag gaaagcccaa agaaattcct gttgaaatac caatgacatt cctatcaaaa 74940
tgccaattct acagtaatcc aatggcttca gtggggaatt ctaccaaaca ctaaaaaaac 75000
taatacctat tcttctgaaa atcttcccga aaattgaaga ggagggaatt cttctaaatt 75060
ccttgtatga ggccactatt atggtaatta tgattatgat tatgattatg tgattattat 75120
tatgattcca aaaccagaca aggacacaac aaaaaaagca aactataggc taaatactct 75180
gatgaagata atagatgaaa accttcaacc aaatactagc aaattgaatc cagcagcata 75240
ttaaaaacat tatttatcat gatcaagtga gatttatccc aggaatgcaa ggatggttca 75300
acatatgcaa atcacatcaa cagaatgaag gacaaaaacc gtattatcat cataacagac 75360
acagaaaaag tatttgataa aatccaacat cccttcataa aaattctcag caagttaggt 75420
atgaaaggaa tatacctcaa cacaataaga gctatatgtg acaaacccac agccaacatc 75480
atactgaaca tgcaagagtt gaaagctttt ctctaaaatc tggaacaagc caatgatatc 75540
aggccagatg tagtggctca tgcctctaat tctagcactt tgggaggcca aggcaggagg 75600
atcacttgag cccaacaaag gaaacaatca acagagtgaa aagacaatct acagaatgtt 75660
agaaaatatt tgcaaactct ccatccaaca agggactaat atttagaata cattggaaac 75720
tcaaacaact aaacagaaaa gaaaacaaat ttttttatta aaaataggca aatgaaaaaa 75780
tatttaaaag tttttaaaac caaaaaagga actgaataga cgtctctaaa aggaagatat 75840

acgaatggcc aacagacata tgagaaaatg ctcaacatta ctaatcataa ggaaaatgca 75900
aattgaaacc acaataagat ataatctcac cccagttaaa atggctatta ttaaaaaagg 75960
caaaaaacaa ttgctgtcaa agattcagag aaaggggagc tttaatgcat tattggttga 76020
aatcttgtct ttggaattag cacagccatt gaaaaacaat aaagagcttc ctcaaaaaat 76080
ggaaatgagt actgccccat gatcccacca tcctctaccg ggtatttatc caaaggaaat 76140
ctatgtcaaa gagacatcta cactacatgt tcattgcagc actattcaca atgccagtgt 76200
tggaatcaac ctacgtgtcc atcaacagat gaatgcataa agaaaatgta gtgtttatac 76260
acaaacacac acagagagag agagagagag gaattctatt ctgccatgaa aaagaatgaa 76320
atcttgtcat ctgaggtaac atggatgagc ttggaggaca tgttaagtga aataaagcta 76380
gacacagaca gccaaatatc acatgttctc actcatatat ggaagccaaa taagttgatc 76440
tcatagaaat agggagtaga atagtggtaa ccagaggctg ggaatgggag gggataggat 76500
agctagaagt cgattaataa ataaaaaatt acagtcaggt aggaggaata ggccccggtg 76560
ttctctaaca ccatagggtg actataacta acaacaattt attgtatatt ttcatacggc 76620
tagaagagca aattttgatt gttcccagca caaagaagtg ataaatgttt gaggtgatgg 76680
atatgctaat tgttgcagga agtcagggac cccgaatgga gggaccagct ggagccatgg 76740
cagaggaaca taaattgtga agatttcatg gacatttatc agttcccaaa taatactttt 76800
ataatttctt atgcctgtct ttactttaat ctcataatcc tgttatcttc ataagctgag 76860
gatgtacgtc acctcaggac cactgggata attgtgttaa ctgtacaaat tgattgtaaa 76920
acatatgtgt ttgaacaata tgaaatcagt gcaccttgaa aaagacagaa taacagcgat 76980
ttttagggaa taagggaaga caaccataag gtctgactgc ctgaggggtt gggcaaaaag 77040
agccatattt ttcttcttgc agaaagccta taaatgaacg tgcaagtagg gaagatatcg 77100
ctaaattatt ttcctagcaa ggaatattaa tactaatacc ctgggaaagg aatgcatccc 77160
tgggggaggt ctataaacgg ccgctctggg aatgtctgtc ttatgtggtt gagataagga 77220
ctgagataag gactgagata cgccctggtc tcctgcagta ccctcagctt attagggggg 77280
tgaaaaactc caccctggta aatttgtggt cacactggtt ctctgctctc aaactctgtt 77340
ttctgttgtt taagatgttt atcaagataa tatatgcact gctgaacata gacccttatc 77400
agtagttctg tttttgccct ttgccttgtg atctttgttg gacccttatc agtggttctg 77460
cttttgccct ttgtcctgtt ccctcagaag catgtgatct ttgttagacc cttattagta 77520
gttctgcttt ttgcctttga agcatgtgat ctttgtaccc actccctgtg cttacacccc 77580
ctccctttt gaaaccctta acaaaaaacc tgctggtttg aggctcaggc ggtcatcaca 77640
gtcctactga tatgtgatgt cacccccggc tgcccagctg taaaatcctc tctttatact 77700
gtctctcttt atttctcagc tggccaacaa ttatggaaaa cagaaagaac ctacattgaa 77760
atattggggg caggttcccc caatactaat tatcctgatt tgatcatcac ccattgtata 77820
tatgtatcca aatatcacaa tgtaccccaa aatatataca attattatgt gtcaattaaa 77880
aacaatcata aaacttttaa acagctaaaa taaaagtata ttgttttctt caaaaaaaat 77940
ctaatgcagt tttccctact aggttttggg cttgcttagg acccatggct cctctctccc 78000
ttccaatgta tcccttttgg aataggaatg tccatcctat gcctgcccca tcattgtact 78060
ttggaagcag ataacttctt gtcaagttac aaaggtccac agatggagag gaatttcacc 78120
ccagaatgaa tcaccctgca tttctcccac acttgatgca ggtgatatgt cgctgagatt 78180
gtggactaag agttggtgct ggaaggggtt agccatcgtg gagatgttgc tatgggatgc 78240
agggattttg cctgtgagaa ggacatgatt atggggggag cggagggcaa actgtcatgg 78300
gttaaaatgt gtcccctata aattcatgtg ttgaagtcct aacccccagg accacagaat 78360
gtgaccttgt ttggaaacag tctttgcagc tgcaatcaag ttcggatgag gtcaccctgg 78420
agtagggcaa gcctctgatc caatatgact gctgtcctca tgaaaagggg gaatctgggc 78480
acagacgcac gtgtggagaa cgccctgtga agatggtgct gcttccacaa gccaagagca 78540
gcagagacgg ccggcaaagc ccagcagcaa ggagagagcc tggaacagag tctccatgac 78600
acagaggagc cagccccacc gagacctcca tcccagatga ccggcctcca gaaccaggac 78660
ggaataaacg tctgttgttt aagccacgca gtctggggtg cagtgttgcc agggccgcac 78720
ttaacggata cgagtgttgt cctgagctgc cagccccaca gactgcacaa ggcctccctg 78780
ccccagccaa gtgcagtctc cccagccccc tgggtgtgcc atgggcagtg tggggcccat 78840
cactccgtcc tcccccaggc tgggaggttg agcccattat gagctccatg gggtgaagct 78900
ggagcgagag gctgggagcc gactgggagc ccgcggctgg aggatggatt tccccaggga 78960
cccacacgtg cacctccacc tgtctcctgg acgttctctc tgagggcagg gctggtgcca 79020
gctcagggat ccagcaggga cagaagggcg ggccgggtcc ttgtggagag cacatttagt 79080
gggagggaca tgatttcccc tcacaagtgt ccattcttcc tgttccttgc tggacgcttc 79140
ctcttccatt ctggacactt cctgtgcgac acctcctcgg gctttcccga ggccctctgg 79200
cctcattccg ttccctgcta cctcccactt ccacgtacgt ccttgcccag ctcttccctc 79260
tatccagagc ttctgcctgg caaggtccct gctgagatca gtccaggctc ccccagcaca 79320
ggtaggagcc ttgcacatgc ccttggacct ccccaccctg catgatgcca gcatccccag 79380
gccccaggga ggccccattt ctctctctgc tggtagtcca gtggccctgg agtcccactg 79440
caggtggggt gtgcccctga actctgagga agctaagtac cctgccctca gacaggctat 79500
cccccctgct cagccccagg gccctgcccc ctaccccttc ccctcacctg caccacaggc 79560
tctggccaac tctgcccagg ccctgaatgg gcccctctgg ctcccctctg ctgctacact 79620
gccctgcacc acctccactc agcctcagtg tgttcatccg cctgtcccac gtcccctcgg 79680
cccccaggag cacagctggt ggccctggct cctcgcagcc catcttgttc cttctggagc 79740
accagcctca gaggccttcc tgtgcagggt ccactcggcc agccctggga ccctcctggt 79800
ctcaagcaca cacattctcc ctgcagccag acctgcccct gcctgtgagc tcagacctga 79860

```
gccttggaac gccttccctt ctccatccca gctcgccttt gccagctgct cagcaggatg 79920
aactcacact cccctccctg caccatgagt gagagtcagc tggagagatg cccaggcaaa 79980
agcagccacc agggcccagt gggggccaga agcttcagat gagaggccca ggtattgaga 80040
ggctgagacc acgggcagaa tggtcataat cgttgccagt ctcagtccag ccccagggac 80100
tcagagacag agaaaagagc agcacacaag gtccgggctc cccaccttct cccgtgagta 80160
cgggggagta tgggggcagc caccaccccc atccccacac acccatgagg cagcctcagc 80220
tgtgtctgga ctccccctcg ccctctgaca cagaaaccac cagaagaaaa gggaacttca 80280
ggaagtaagt ggtgccgccg gtttcaatcc tgttcttagt gtttgcagcg tggagttcac 80340
accccctgggg acctgggggc cgagctgtga tttcctagga agacaagtgg cagctgacag 80400
cgtgggcaag gctgcccaca tgtacctcgc cacaacagga agggctgaga cccccacctc 80460
ggtgagtggg gtcagcacag ggcaggagca caggctcagg agaaggacag agcctgggcg 80520
cagccatcgg cgcttctgga cctgagctgc tgaacaggct gcaagaggct ggggagaggc 80580
tggggcgagg ccagccccac atggaagcct aagcggagcc agcacagggg aggtgggcag 80640
ccttcaggca ccgatgccca cccagtgcga gacgacaggg accgtgggca ggggcttcca 80700
agccaacagg gcaggacaca ccagaggctg actgaggcct ccaggacgac cgggctggga 80760
gtgtgaggaa catgacggga tggggcagag ccagccatgg ggtgatgcca ggatgggcat 80820
gaccgacctg agctcaggag gcagcagaga gagggaggag gagaggcccc aggtgaaccg 80880
aggggcttgt ccaggccggc agcatcaccg gagcccaggg cagggtcagc agagctggcc 80940
gtagggccct cctctcagcc aggaccaagg acagcaggtg agctgggagc agagcaggga 81000
gggtgagtgt ggcagcagga caggagggtg gaagccaagg agcccagagg cagaggcagg 81060
g                                                                 81061
```

<210> 3
<211> 2791
<212> DNA
<213> Homo sapiens

<220>
<221> CDS
<222> (77)...(673)

<400> 3

```
gaaccatcat taattgaagt gagatttttc tggcctgaga cttgcaggga ggcaagaaga 60
cactctggac accact atg gac agc ctc ttg atg aac cgg agg aag ttt ctt 112
                  Met Asp Ser Leu Leu Met Asn Arg Arg Lys Phe Leu
                  1               5                   10

tac caa ttc aaa aat gtc cgc tgg gct aag ggt cgg cgt gag acc tac   160
Tyr Gln Phe Lys Asn Val Arg Trp Ala Lys Gly Arg Arg Glu Thr Tyr
        15                  20                  25

ctg tgc tac gta gtg aag agg cgt gac agt gct aca tcc ttt tca ctg   208
Leu Cys Tyr Val Val Lys Arg Arg Asp Ser Ala Thr Ser Phe Ser Leu
    30                  35                  40

gac ttt ggt tat ctt cgc aat aag aac ggc tgc cac gtg gaa ttg ctc   256
Asp Phe Gly Tyr Leu Arg Asn Lys Asn Gly Cys His Val Glu Leu Leu
45                  50                  55                  60

ttc ctc cgc tac atc tcg gac tgg gac cta gac cct ggc cgc tgc tac   304
Phe Leu Arg Tyr Ile Ser Asp Trp Asp Leu Asp Pro Gly Arg Cys Tyr
                65                  70                  75

cgc gtc acc tgg ttc acc tcc tgg agc ccc tgc tac gac tgt gcc cga   352
Arg Val Thr Trp Phe Thr Ser Trp Ser Pro Cys Tyr Asp Cys Ala Arg
            80                  85                  90

cat gtg gcc gac ttt ctg cga ggg aac ccc aac ctc agt ctg agg atc   400
His Val Ala Asp Phe Leu Arg Gly Asn Pro Asn Leu Ser Leu Arg Ile
        95                  100                 105

ttc acc gcg cgc ctc tac ttc tgt gag gac cgc aag gct gag ccc gag   448
Phe Thr Ala Arg Leu Tyr Phe Cys Glu Asp Arg Lys Ala Glu Pro Glu
    110                 115                 120

ggg ctg cgg cgg ctg cac cgc gcc ggg gtg caa ata gcc atc atg acc   496
Gly Leu Arg Arg Leu His Arg Ala Gly Val Gln Ile Ala Ile Met Thr
```

```
125                 130                 135                 140

ttc aaa gat tat ttt tac tgc tgg aat act ttt gta gaa aac cat gaa    544
Phe Lys Asp Tyr Phe Tyr Cys Trp Asn Thr Phe Val Glu Asn His Glu
                145                 150                 155

aga act ttc aaa gcc tgg gaa ggg ctg cat gaa aat tca gtt cgt ctc    592
Arg Thr Phe Lys Ala Trp Glu Gly Leu His Glu Asn Ser Val Arg Leu
            160                 165                 170

tcc aga cag ctt cgg cgc atc ctt ttg ccc ctg tat gag gtt gat gac    640
Ser Arg Gln Leu Arg Arg Ile Leu Leu Pro Leu Tyr Glu Val Asp Asp
        175                 180                 185

tta cga gac gca ttt cgt act ttg gga ctt tga tagcaacttc caggaatgtc 693
Leu Arg Asp Ala Phe Arg Thr Leu Gly Leu  *
    190                 195

acacacgatg aaatatctct gctgaagaca gtggataaaa aacagtcctt caagtcttct 753
ctgtttttat tcttcaactc tcactttctt agagtttaca gaaaaaatat ttatatacga 813
ctctttaaaa agatctatgt cttgaaaata gagaaggaac acaggtctgg ccagggacgt 873
gctgcaattg gtgcagtttt gaatgcaaca ttgtccccta ctgggaataa cagaactgca 933
ggacctggga gcatcctaaa gtgtcaacgt ttttctatga cttttaggta ggatgagagc 993
agaaggtaga tcctaaaaag catggtgaga ggatcaaatg tttttatatc aacatccttt 1053
attatttgat tcatttgagt taacagtggt gttagtgata gatttttcta ttcttttccc 1113
ttgacgttta ctttcaagta acacaaactc ttccatcagg ccatgatcta taggacctcc 1173
taatgagagt atctgggtga ttgtgacccc aaaccatctc tccaaagcat taatatccaa 1233
tcatgcgctg tatgttttaa tcagcagaag catgttttta tgtttgtaca aaagaagatt 1293
gttatgggtg gggatggagg tatagaccat gcatggtcac cttcaagcta ctttaataaa 1353
ggatcttaaa atgggcagga ggactgtgaa caagacaccc taataatggg ttgatgtctg 1413
aagtagcaaa tcttctggaa acgcaaactc ttttaaggaa gtccctaatt tagaaacacc 1473
cacaaacttc acatatcata attagcaaac aattggaagg aagttgcttg aatgttgggg 1533
agaggaaaat ctattggctc tcgtgggtct cttcatctca gaaatgccaa tcaggtcaag 1593
gtttgctaca ttttgtatgt gtgtgatgct tctcccaaag gtatattaac tatataagag 1653
agttgtgaca aaacagaatg ataaagctgc gaaccgtggc acacgctcat agttctagct 1713
gcttgggagg ttgaggaggg aggatggctt gaacacaggt gttcaaggcc agcctgggca 1773
acataacaag atcctgtctc tcaaaaaaaa aaaaaaaaaa aagaaagaga gagggccggg 1833
cgtggtggct cacgcctgta atcccagcac tttgggaggc cgagccgggc ggatcacctg 1893
tggtcaggag tttgagacca gcctggccaa catggcaaaa ccccgtctgt actcaaaatg 1953
caaaaattag ccaggcgtgg tagcaggcac ctgtaatccc agctacttgg gaggctgagg 2013
caggagaatc gcttgaaccc aggaggtgga ggttgcagta agctgagatc gtgccgttgc 2073
actccagcct gggcgacaag agcaagactc tgtctcagaa aaaaaaaaaa aaaagagaga 2133
gagagagaaa gagaacaata tttgggagag aaggatgggg aagcattgca aggaaattgt 2193
gctttatcca acaaaatgta aggagccaat aagggatccc tatttgtctc ttttggtgtc 2253
tatttgtccc taacaactgt ctttgacagt gagaaaaata ttcagaataa ccatatccct 2313
gtgccgttat tacctagcaa cccttgcaat gaagatgagc agatccacag gaaaacttga 2373
atgcacaact gtcttatttt aatcttattg tacataagtt tgtaaaagag ttaaaaattg 2433
ttacttcatg tattcattta tattttatat tattttgcgt ctaatgattt tttattaaca 2493
tgatttcctt ttctgatata ttgaaatgga gtctcaaagc ttcataaatt tataacttta 2553
gaaatgattc taataacaac gtatgtaatt gtaacattgc agtaatggtg ctacgaagcc 2613
atttctcttg atttttagta aacttttatg acagcaaatt tgcttctggc tcactttcaa 2673
tcagttaaat aaatgataaa taattttgga agctgtgaag ataaaatacc aaataaaata 2733
atataaaagt gatttatatg aagttaaaat aaaaaatcag tatgatggaa taaacttg   2791
```

<210> 4

<211> 198

<212> PRT

<213> Homo sapiens

<400> 4

```
Met Asp Ser Leu Leu Met Asn Arg Arg Lys Phe Leu Tyr Gln Phe Lys
 1               5                  10                  15
Asn Val Arg Trp Ala Lys Gly Arg Arg Glu Thr Tyr Leu Cys Tyr Val
            20                  25                  30
Val Lys Arg Arg Asp Ser Ala Thr Ser Phe Ser Leu Asp Phe Gly Tyr
        35                  40                  45
Leu Arg Asn Lys Asn Gly Cys His Val Glu Leu Leu Phe Leu Arg Tyr


    50                  55                  60
Ile Ser Asp Trp Asp Leu Asp Pro Gly Arg Cys Tyr Arg Val Thr Trp
65                  70                  75                  80
Phe Thr Ser Trp Ser Pro Cys Tyr Asp Cys Ala Arg His Val Ala Asp
                85                  90                  95
Phe Leu Arg Gly Asn Pro Asn Leu Ser Leu Arg Ile Phe Thr Ala Arg
            100                 105                 110
Leu Tyr Phe Cys Glu Asp Arg Lys Ala Glu Pro Glu Gly Leu Arg Arg
        115                 120                 125
Leu His Arg Ala Gly Val Gln Ile Ala Ile Met Thr Phe Lys Asp Tyr
    130                 135                 140
Phe Tyr Cys Trp Asn Thr Phe Val Glu Asn His Glu Arg Thr Phe Lys
145                 150                 155                 160
Ala Trp Glu Gly Leu His Glu Asn Ser Val Arg Leu Ser Arg Gln Leu
                165                 170                 175
Arg Arg Ile Leu Leu Pro Leu Tyr Glu Val Asp Asp Leu Arg Asp Ala
            180                 185                 190
Phe Arg Thr Leu Gly Leu
        195
```

<210> 5
<211> 2053
<212> DNA
<213> Homo sapiens

<220>
<221> CDS
<222> (71)...(1012)

<400> 5

```
cacagccaca gccagggcta gcctcgccgg ttcccgggtg gcgcgcgttc gctgcctcct 60
cagctccagg atg atc ggc cag aag acg ctc tac tcc ttt ttc tcc ccc    109
           Met Ile Gly Gln Lys Thr Leu Tyr Ser Phe Phe Ser Pro
           1               5                   10

agc ccc gcc agg aag cga cac gcc ccc agc ccc gag ccg gcc gtc cag   157
Ser Pro Ala Arg Lys Arg His Ala Pro Ser Pro Glu Pro Ala Val Gln
    15                  20                  25

ggg acc ggc gtg gct ggg gtg cct gag gaa agc gga gat gcg gcg gcc   205
Gly Thr Gly Val Ala Gly Val Pro Glu Glu Ser Gly Asp Ala Ala Ala
30                  35                  40                  45

atc cca gcc aag aag gcc ccg gct ggg cag gag gag cct ggg acg ccg   253
Ile Pro Ala Lys Lys Ala Pro Ala Gly Gln Glu Glu Pro Gly Thr Pro
                50                  55                  60

ccc tcc tcg ccg ctg agt gcc gag cag ttg gac cgg atc cag agg aac   301
Pro Ser Ser Pro Leu Ser Ala Glu Gln Leu Asp Arg Ile Gln Arg Asn
            65                  70                  75

aag gcc gcg gcc ctg ctc aga ctc gcg gcc cgc aac gtg ccc gtg ggc   349
Lys Ala Ala Ala Leu Leu Arg Leu Ala Ala Arg Asn Val Pro Val Gly
        80                  85                  90

ttt gga gag agc tgg aag aag cac ctc agc ggg gag ttc ggg aaa ccg   397
Phe Gly Glu Ser Trp Lys Lys His Leu Ser Gly Glu Phe Gly Lys Pro
    95                  100                 105

tat ttt atc aag cta atg gga ttt gtt gca gaa gaa aga aag cat tac   445
Tyr Phe Ile Lys Leu Met Gly Phe Val Ala Glu Glu Arg Lys His Tyr
110                 115                 120                 125

act gtt tat cca ccc cca cac caa gtc ttc acc tgg acc cag atg tgt   493
Thr Val Tyr Pro Pro Pro His Gln Val Phe Thr Trp Thr Gln Met Cys
                130                 135                 140
```

```
gac ata aaa gat gtg aag gtt gtc atc ctg gga cag gat cca tat cat    541
Asp Ile Lys Asp Val Lys Val Val Ile Leu Gly Gln Asp Pro Tyr His
            145                 150                 155

gga cct aat caa gct cac ggg ctc tgc ttt agt gtt caa agg cct gtt    589
Gly Pro Asn Gln Ala His Gly Leu Cys Phe Ser Val Gln Arg Pro Val
        160                 165                 170

ccg cct ccg ccc agt ttg gag aac att tat aaa gag ttg tct aca gac    637
Pro Pro Pro Pro Ser Leu Glu Asn Ile Tyr Lys Glu Leu Ser Thr Asp
    175                 180                 185

ata gag gat ttt gtt cat cct ggc cat gga gat tta tct ggg tgg gcc    685
Ile Glu Asp Phe Val His Pro Gly His Gly Asp Leu Ser Gly Trp Ala
190                 195                 200                 205

aag caa ggt gtt ctc ctt ctc aac gct gtc ctc acg gtt cgt gcc cat    733
Lys Gln Gly Val Leu Leu Leu Asn Ala Val Leu Thr Val Arg Ala His
                210                 215                 220

caa gcc aac tct cat aag gag cga ggc tgg gag cag ttc act gat gca    781
Gln Ala Asn Ser His Lys Glu Arg Gly Trp Glu Gln Phe Thr Asp Ala
            225                 230                 235

gtt gtg tcc tgg cta aat cag aac tcg aat ggc ctt gtt ttc ttg ctc    829
Val Val Ser Trp Leu Asn Gln Asn Ser Asn Gly Leu Val Phe Leu Leu
        240                 245                 250

tgg ggc tct tat gct cag aag aag ggc agt gcc att gat agg aag cgg    877
Trp Gly Ser Tyr Ala Gln Lys Lys Gly Ser Ala Ile Asp Arg Lys Arg
    255                 260                 265

cac cat gta cta cag acg gct cat ccc tcc cct ttg tca gtg tat aga    925
His His Val Leu Gln Thr Ala His Pro Ser Pro Leu Ser Val Tyr Arg
270                 275                 280                 285

ggg ttc ttt gga tgt aga cac ttt tca aag acc aat gag ctg ctg cag    973
Gly Phe Phe Gly Cys Arg His Phe Ser Lys Thr Asn Glu Leu Leu Gln
                290                 295                 300

aag tct ggc aag aag ccc att gac tgg aag gag ctg tga tcatcagctg    1022
Lys Ser Gly Lys Lys Pro Ile Asp Trp Lys Glu Leu  *
            305                 310

aggggtggcc tttgagaagc tgctgttaac gtatttgcca gttacgaagt tccactgaaa 1082
attttcctat taattcttaa gtactctgca taagggggaa aagcttccag aaagcagcca 1142
tgaaccaggc tgtccaggaa tggcagctgt atccaaccac aaacaacaaa ggctaccctt 1202
tgaccaaatg tctttctctg caacatggct tcggcctaaa atatgcagaa gacagatgag 1262
gtcaaatact cagttggctc tctttatctc ccttgccttt atggtgaaac aggggagatg 1322
tgcacctttc aggcacagcc ctagtttggc gcctgctgct ccttggtttt gcctggttag 1382
actttcagtg acagatgttg gggtgttttt gcttagaaag gtccccttgt ctcagccttg 1442
cagggcaggc atgccagtct ctgccagttc cactgccccc ttgatctttg aaggagtcct 1502
caggcccctc gcagcataag gatgttttgc aactttccag aatctggccc agaaattagg 1562
gctcaatttc ctgattgtag tagaggttaa gattgctgtg agctttatca gataagagac 1622
cgagagaagt aagctgggtc ttgttattcc ttgggtgttg gtggaataag cagtggaatt 1682
tgaacaagga agaggagaaa agggaatttt gtctttatgg ggtggggtga ttttctccta 1742
gggttatgtc cagttggggt ttttaaggca gcacagactg ccaagtactg ttttttttaa 1802
ccgactgaaa tcactttggg atattttttc ctgcaacact ggaaagtttt agtttttttaa 1862
gaagtactca tgcagatata tatatatata tttttcccag tcctttttttt aagagacggt 1922
ctttattggg tctgcacctc catccttgat cttgttagca atgctgtttt tgctgttagt 1982
cgggttagag ttggctctac gcgaggtttg ttaataaaag tttgttaaaa gtttaaaaaa 2042
aaaaaaaaaa a                                                      2053
```

<210> 6
<211> 313
<212> PRT
<213> Homo sapiens

<400> 6

```
Met Ile Gly Gln Lys Thr Leu Tyr Ser Phe Phe Ser Pro Ser Pro Ala
 1               5                  10                  15
Arg Lys Arg His Ala Pro Ser Pro Glu Pro Ala Val Gln Gly Thr Gly
            20                  25                  30
Val Ala Gly Val Pro Glu Glu Ser Gly Asp Ala Ala Ala Ile Pro Ala
        35                  40                  45
Lys Lys Ala Pro Ala Gly Gln Glu Glu Pro Gly Thr Pro Pro Ser Ser
    50                  55                  60
Pro Leu Ser Ala Glu Gln Leu Asp Arg Ile Gln Arg Asn Lys Ala Ala
65                  70                  75                  80
Ala Leu Leu Arg Leu Ala Ala Arg Asn Val Pro Val Gly Phe Gly Glu
                85                  90                  95
Ser Trp Lys Lys His Leu Ser Gly Glu Phe Gly Lys Pro Tyr Phe Ile
            100                 105                 110
Lys Leu Met Gly Phe Val Ala Glu Glu Arg Lys His Tyr Thr Val Tyr
        115                 120                 125
Pro Pro Pro His Gln Val Phe Thr Trp Thr Gln Met Cys Asp Ile Lys
    130                 135                 140
Asp Val Lys Val Val Ile Leu Gly Gln Asp Pro Tyr His Gly Pro Asn
145                 150                 155                 160
Gln Ala His Gly Leu Cys Phe Ser Val Gln Arg Pro Val Pro Pro Pro
                165                 170                 175
Pro Ser Leu Glu Asn Ile Tyr Lys Glu Leu Ser Thr Asp Ile Glu Asp
            180                 185                 190
Phe Val His Pro Gly His Gly Asp Leu Ser Gly Trp Ala Lys Gln Gly
        195                 200                 205
Val Leu Leu Leu Asn Ala Val Leu Thr Val Arg Ala His Gln Ala Asn
    210                 215                 220
Ser His Lys Glu Arg Gly Trp Glu Gln Phe Thr Asp Ala Val Val Ser
225                 230                 235                 240
Trp Leu Asn Gln Asn Ser Asn Gly Leu Val Phe Leu Leu Trp Gly Ser
                245                 250                 255
Tyr Ala Gln Lys Lys Gly Ser Ala Ile Asp Arg Lys Arg His His Val
            260                 265                 270
Leu Gln Thr Ala His Pro Ser Pro Leu Ser Val Tyr Arg Gly Phe Phe
        275                 280                 285
Gly Cys Arg His Phe Ser Lys Thr Asn Glu Leu Leu Gln Lys Ser Gly
    290                 295                 300
Lys Lys Pro Ile Asp Trp Lys Glu Leu
305                 310
```

<210> 7
<211> 2156
<212> DNA
<213> Homo sapiens

<220>
<221> CDS
<222> (71)...(1900)

<400> 7

```
gcgcatgcgt ggattgtcgt cttctgtcca agttggtcgc ttccctgcgc caaagtgagc 60
agtagccaac atg tca ggg tgg gag tca tat tac aaa acc gag ggc gat    109
           Met Ser Gly Trp Glu Ser Tyr Tyr Lys Thr Glu Gly Asp
            1               5                   10

gaa gaa gca gag gaa gaa caa gaa gag aac ctt gaa gca agt gga gac   157
Glu Glu Ala Glu Glu Glu Gln Glu Glu Asn Leu Glu Ala Ser Gly Asp
    15                  20                  25

tat aaa tat tca gga aga gat agt ttg att ttt ttg gtt gat gcc tcc   205
Tyr Lys Tyr Ser Gly Arg Asp Ser Leu Ile Phe Leu Val Asp Ala Ser
 30                  35                  40                  45
```

```
aag gct atg ttt gaa tct cag agt gaa gat gag ttg aca cct ttt gac    253
Lys Ala Met Phe Glu Ser Gln Ser Glu Asp Glu Leu Thr Pro Phe Asp
                50                  55                  60

atg agc atc cag tgt atc caa agt gtg tac atc agt aag atc ata agc    301
Met Ser Ile Gln Cys Ile Gln Ser Val Tyr Ile Ser Lys Ile Ile Ser
            65                  70                  75

agt gat cga gat ctc ttg gct gtg gtg ttc tat ggt acc gag aaa gac    349
Ser Asp Arg Asp Leu Leu Ala Val Val Phe Tyr Gly Thr Glu Lys Asp
        80                  85                  90

aaa aat tca gtg aat ttt aaa aat att tac gtc tta cag gag ctg gat    397
Lys Asn Ser Val Asn Phe Lys Asn Ile Tyr Val Leu Gln Glu Leu Asp
    95                  100                 105

aat cca ggt gca aaa cga att cta gag ctt gac cag ttt aag ggg cag    445
Asn Pro Gly Ala Lys Arg Ile Leu Glu Leu Asp Gln Phe Lys Gly Gln
110                 115                 120                 125

cag gga caa aaa cgt ttc caa gac atg atg ggc cac gga tct gac tac    493
Gln Gly Gln Lys Arg Phe Gln Asp Met Met Gly His Gly Ser Asp Tyr
                130                 135                 140

tca ctc agt gaa gtg ctg tgg gtc tgt gcc aac ctc ttt agt gat gtc    541
Ser Leu Ser Glu Val Leu Trp Val Cys Ala Asn Leu Phe Ser Asp Val
            145                 150                 155

caa ttc aag atg agt cat aag agg atc atg ctg ttc acc aat gaa gac    589
Gln Phe Lys Met Ser His Lys Arg Ile Met Leu Phe Thr Asn Glu Asp
        160                 165                 170

aac ccc cat ggc aat gac agt gcc aaa gcc agc cgg gcc agg acc aaa    637
Asn Pro His Gly Asn Asp Ser Ala Lys Ala Ser Arg Ala Arg Thr Lys
    175                 180                 185

gcc ggt gat ctc cga gat aca ggc atc ttc ctt gac ttg atg cac ctg    685
Ala Gly Asp Leu Arg Asp Thr Gly Ile Phe Leu Asp Leu Met His Leu
190                 195                 200                 205

aag aaa cct ggg ggc ttt gac ata tcc ttg ttc tac aga gat atc atc    733
Lys Lys Pro Gly Gly Phe Asp Ile Ser Leu Phe Tyr Arg Asp Ile Ile
                210                 215                 220

agc ata gca gag gat gag gac ctc agg gtt cac ttt gag gaa tcc agc    781
Ser Ile Ala Glu Asp Glu Asp Leu Arg Val His Phe Glu Glu Ser Ser
            225                 230                 235

aag cta gaa gac ctg ttg cgg aag gtt cgc gcc aag gag acc agg aag    829
Lys Leu Glu Asp Leu Leu Arg Lys Val Arg Ala Lys Glu Thr Arg Lys
        240                 245                 250

cga gca ctc agc agg tta aag ctg aag ctc aac aaa gat ata gtg atc    877
Arg Ala Leu Ser Arg Leu Lys Leu Lys Leu Asn Lys Asp Ile Val Ile
    255                 260                 265

tct gtg ggc att tat aat ctg gtc cag aag gct ctc aag cct cct cca    925
Ser Val Gly Ile Tyr Asn Leu Val Gln Lys Ala Leu Lys Pro Pro Pro
270                 275                 280                 285

ata aag ctc tat cgg gaa aca aat gaa cca gtg aaa acc aag acc cgg    973
Ile Lys Leu Tyr Arg Glu Thr Asn Glu Pro Val Lys Thr Lys Thr Arg
                290                 295                 300

acc ttt aat aca agt aca ggc ggt ttg ctt ctg cct agc gat acc aag    1021
Thr Phe Asn Thr Ser Thr Gly Gly Leu Leu Leu Pro Ser Asp Thr Lys
```

```
                305                 310                 315

agg tct cag atc tat ggg agt cgt cag att ata ctg gag aaa gag gaa     1069
Arg Ser Gln Ile Tyr Gly Ser Arg Gln Ile Ile Leu Glu Lys Glu Glu
        320                 325                 330

aca gaa gag cta aaa cgg ttt gat gat cca ggt ttg atg ctc atg ggt     1117
Thr Glu Glu Leu Lys Arg Phe Asp Asp Pro Gly Leu Met Leu Met Gly
    335                 340                 345

ttc aag ccg ttg gta ctg ctg aag aaa cac cat tac ctg agg ccc tcc     1165
Phe Lys Pro Leu Val Leu Leu Lys Lys His His Tyr Leu Arg Pro Ser
350                 355                 360                 365

ctg ttc gtg tac cca gag gag tcg ctg gtg att ggg agc tca acc ctg     1213
Leu Phe Val Tyr Pro Glu Glu Ser Leu Val Ile Gly Ser Ser Thr Leu
                370                 375                 380

ttc agt gct ctg ctc atc aag tgt ctg gag aag gag gtt gca gca ttg     1261
Phe Ser Ala Leu Leu Ile Lys Cys Leu Glu Lys Glu Val Ala Ala Leu
            385                 390                 395

tgc aga tac aca ccc cgc agg aac atc cct cct tat ttt gtg gct ttg     1309
Cys Arg Tyr Thr Pro Arg Arg Asn Ile Pro Pro Tyr Phe Val Ala Leu
        400                 405                 410

gtg cca cag gaa gaa gag ttg gat gac cag aaa att cag gtg act cct     1357
Val Pro Gln Glu Glu Glu Leu Asp Asp Gln Lys Ile Gln Val Thr Pro
    415                 420                 425

cca ggc ttc cag ctg gtc ttt tta ccc ttt gct gat gat aaa agg aag     1405
Pro Gly Phe Gln Leu Val Phe Leu Pro Phe Ala Asp Asp Lys Arg Lys
430                 435                 440                 445

atg ccc ttt act gaa aaa atc atg gca act cca gag cag gtg ggc aag     1453
Met Pro Phe Thr Glu Lys Ile Met Ala Thr Pro Glu Gln Val Gly Lys
                450                 455                 460

atg aag gct atc gtt gag aag ctt cgc ttc aca tac aga agt gac agc     1501
Met Lys Ala Ile Val Glu Lys Leu Arg Phe Thr Tyr Arg Ser Asp Ser
            465                 470                 475

ttt gag aac ccc gtg ctg cag cag cac ttc agg aac ctg gag gcc ttg     1549
Phe Glu Asn Pro Val Leu Gln Gln His Phe Arg Asn Leu Glu Ala Leu
        480                 485                 490

gcc ttg gat ttg atg gag ccg gaa caa gca gtg gac ctg aca ttg ccc     1597
Ala Leu Asp Leu Met Glu Pro Glu Gln Ala Val Asp Leu Thr Leu Pro
    495                 500                 505

aag gtt gaa gca atg aat aaa aga ctg ggc tcc ttg gtg gat gag ttt     1645
Lys Val Glu Ala Met Asn Lys Arg Leu Gly Ser Leu Val Asp Glu Phe
510                 515                 520                 525

aag gag ctt gtt tac cca cca gat tac aat cct gaa ggg aaa gtt acc     1693
Lys Glu Leu Val Tyr Pro Pro Asp Tyr Asn Pro Glu Gly Lys Val Thr
                530                 535                 540

aag aga aaa cac gat aat gaa ggt tct gga agc aaa agg ccc aag gtg     1741
Lys Arg Lys His Asp Asn Glu Gly Ser Gly Ser Lys Arg Pro Lys Val
            545                 550                 555

gag tat tca gaa gag gag ctg aag acc cac atc agc aag ggt acg ctg     1789
Glu Tyr Ser Glu Glu Glu Leu Lys Thr His Ile Ser Lys Gly Thr Leu
        560                 565                 570

ggc aag ttc act gtg ccc atg ctg aaa gag gcc tgc cgg gct tac ggg     1837
```

```
Gly Lys Phe Thr Val Pro Met Leu Lys Glu Ala Cys Arg Ala Tyr Gly
    575                 580                 585

ctg aag agt ggg ctg aag aag cag gag ctg ctg gaa gcc ctc acc aag     1885
Leu Lys Ser Gly Leu Lys Lys Gln Glu Leu Leu Glu Ala Leu Thr Lys
590                 595                 600                 605

cac ttc cag gac tga ccagaggccg cgcgtccagc tgcccttccg cagtgtggcc     1940
His Phe Gln Asp  *

aggctgcctg gccttgtcct cagccagtta aaatgtgttt ctcctgagct aggaagagtc 2000
tacccgacat aagtcgaggg actttatgtt tttgaggctt tctgttgcca tggtgatggt 2060
gtagccctcc cactttgctg ttccttactt tactgcctga ataaagagcc ctaagtttgt 2120
actatatact gttaaaaaaa aaaaaaaaaa aaaaaa                           2156
```

<210> 8
<211> 609
<212> PRT
<213> Homo sapiens

<400> 8

```
Met Ser Gly Trp Glu Ser Tyr Tyr Lys Thr Glu Gly Asp Glu Glu Ala
 1               5                  10                  15
Glu Glu Glu Gln Glu Glu Asn Leu Glu Ala Ser Gly Asp Tyr Lys Tyr
            20                  25                  30
Ser Gly Arg Asp Ser Leu Ile Phe Leu Val Asp Ala Ser Lys Ala Met
        35                  40                  45
Phe Glu Ser Gln Ser Glu Asp Glu Leu Thr Pro Phe Asp Met Ser Ile
    50                  55                  60
Gln Cys Ile Gln Ser Val Tyr Ile Ser Lys Ile Ile Ser Ser Asp Arg
65                  70                  75                  80
Asp Leu Leu Ala Val Val Phe Tyr Gly Thr Glu Lys Asp Lys Asn Ser
                85                  90                  95
Val Asn Phe Lys Asn Ile Tyr Val Leu Gln Glu Leu Asp Asn Pro Gly
            100                 105                 110
Ala Lys Arg Ile Leu Glu Leu Asp Gln Phe Lys Gly Gln Gln Gly Gln
        115                 120                 125
Lys Arg Phe Gln Asp Met Met Gly His Gly Ser Asp Tyr Ser Leu Ser
    130                 135                 140
Glu Val Leu Trp Val Cys Ala Asn Leu Phe Ser Asp Val Gln Phe Lys
145                 150                 155                 160
Met Ser His Lys Arg Ile Met Leu Phe Thr Asn Glu Asp Asn Pro His
                165                 170                 175
Gly Asn Asp Ser Ala Lys Ala Ser Arg Ala Arg Thr Lys Ala Gly Asp
            180                 185                 190
Leu Arg Asp Thr Gly Ile Phe Leu Asp Leu Met His Leu Lys Lys Pro
        195                 200                 205
Gly Gly Phe Asp Ile Ser Leu Phe Tyr Arg Asp Ile Ile Ser Ile Ala
    210                 215                 220
Glu Asp Glu Asp Leu Arg Val His Phe Glu Glu Ser Ser Lys Leu Glu
225                 230                 235                 240
Asp Leu Leu Arg Lys Val Arg Ala Lys Glu Thr Arg Lys Arg Ala Leu
                245                 250                 255
Ser Arg Leu Lys Leu Lys Leu Asn Lys Asp Ile Val Ile Ser Val Gly
            260                 265                 270
Ile Tyr Asn Leu Val Gln Lys Ala Leu Lys Pro Pro Pro Ile Lys Leu
        275                 280                 285
Tyr Arg Glu Thr Asn Glu Pro Val Lys Thr Lys Thr Arg Thr Phe Asn
    290                 295                 300
Thr Ser Thr Gly Gly Leu Leu Leu Pro Ser Asp Thr Lys Arg Ser Gln
305                 310                 315                 320
Ile Tyr Gly Ser Arg Gln Ile Ile Leu Glu Lys Glu Glu Thr Glu Glu
                325                 330                 335
Leu Lys Arg Phe Asp Asp Pro Gly Leu Met Leu Met Gly Phe Lys Pro
            340                 345                 350
Leu Val Leu Leu Lys Lys His His Tyr Leu Arg Pro Ser Leu Phe Val
```

```
                355                     360                     365
Tyr Pro Glu Glu Ser Leu Val Ile Gly Ser Ser Thr Leu Phe Ser Ala
    370                     375                     380
Leu Leu Ile Lys Cys Leu Glu Lys Glu Val Ala Ala Leu Cys Arg Tyr
385                     390                     395                     400
Thr Pro Arg Arg Asn Ile Pro Pro Tyr Phe Val Ala Leu Val Pro Gln
                    405                     410                     415
Glu Glu Glu Leu Asp Asp Gln Lys Ile Gln Val Thr Pro Pro Gly Phe
            420                     425                     430
Gln Leu Val Phe Leu Pro Phe Ala Asp Asp Lys Arg Lys Met Pro Phe
        435                     440                     445
Thr Glu Lys Ile Met Ala Thr Pro Glu Gln Val Gly Lys Met Lys Ala
    450                     455                     460
Ile Val Glu Lys Leu Arg Phe Thr Tyr Arg Ser Asp Ser Phe Glu Asn
465                     470                     475                     480
Pro Val Leu Gln Gln His Phe Arg Asn Leu Glu Ala Leu Ala Leu Asp
                    485                     490                     495
Leu Met Glu Pro Glu Gln Ala Val Asp Leu Thr Leu Pro Lys Val Glu
            500                     505                     510
Ala Met Asn Lys Arg Leu Gly Ser Leu Val Asp Glu Phe Lys Glu Leu
        515                     520                     525
Val Tyr Pro Pro Asp Tyr Asn Pro Glu Gly Lys Val Thr Lys Arg Lys
    530                     535                     540
His Asp Asn Glu Gly Ser Gly Ser Lys Arg Pro Lys Val Glu Tyr Ser
545                     550                     555                     560
Glu Glu Glu Leu Lys Thr His Ile Ser Lys Gly Thr Leu Gly Lys Phe
                    565                     570                     575
Thr Val Pro Met Leu Lys Glu Ala Cys Arg Ala Tyr Gly Leu Lys Ser
            580                     585                     590
Gly Leu Lys Lys Gln Glu Leu Leu Glu Ala Leu Thr Lys His Phe Gln
        595                     600                     605
Asp
```

<210> 9
<211> 3310
<212> DNA
<213> Homo sapiens

<220>
<221> CDS
<222> (34)...(2232)

<400> 9

```
ggcgggcgac caaagcgcct gaggaccggc aac atg gtg cgg tcg ggg aat aag  54
                                    Met Val Arg Ser Gly Asn Lys
                                    1               5

gca gct gtt gtg ctg tgt atg gac gtg ggc ttt acc atg agt aac tcc  102
Ala Ala Val Val Leu Cys Met Asp Val Gly Phe Thr Met Ser Asn Ser
        10                  15                  20

att cct ggt ata gaa tcc cca ttt gaa caa gca aag aag gtg ata acc  150
Ile Pro Gly Ile Glu Ser Pro Phe Glu Gln Ala Lys Lys Val Ile Thr
    25                  30                  35

atg ttt gta cag cga cag gtg ttt gct gag aac aag gat gag att gct  198
Met Phe Val Gln Arg Gln Val Phe Ala Glu Asn Lys Asp Glu Ile Ala
40                  45                  50                  55

tta gtc ctg ttt ggt aca gat ggc act gac aat ccc ctt tct ggt ggg  246
Leu Val Leu Phe Gly Thr Asp Gly Thr Asp Asn Pro Leu Ser Gly Gly
                60                  65                  70

gat cag tat cag aac atc aca gtg cac aga cat ctg atg cta cca gat  294
Asp Gln Tyr Gln Asn Ile Thr Val His Arg His Leu Met Leu Pro Asp
```

```
            75                  80                  85

ttt gat ttg ctg gag gac att gaa agc aaa atc caa cca ggt tct caa     342
Phe Asp Leu Leu Glu Asp Ile Glu Ser Lys Ile Gln Pro Gly Ser Gln
        90                  95                  100

cag gct gac ttc ctg gat gca cta atc gtg agc atg gat gtg att caa     390
Gln Ala Asp Phe Leu Asp Ala Leu Ile Val Ser Met Asp Val Ile Gln
    105                 110                 115

cat gaa aca ata gga aag aag ttt gag aag agg cat att gaa ata ttc     438
His Glu Thr Ile Gly Lys Lys Phe Glu Lys Arg His Ile Glu Ile Phe
120                 125                 130                 135

act gac ctc agc agc cga ttc agc aaa agt cag ctg gat att ata att     486
Thr Asp Leu Ser Ser Arg Phe Ser Lys Ser Gln Leu Asp Ile Ile Ile
                140                 145                 150

cat agc ttg aag aaa tgt gac atc tcc ctg caa ttc ttc ttg cct ttc     534
His Ser Leu Lys Lys Cys Asp Ile Ser Leu Gln Phe Phe Leu Pro Phe
            155                 160                 165

tca ctt ggc aag gaa gat gga agt ggg gac aga gga gat ggc ccc ttt     582
Ser Leu Gly Lys Glu Asp Gly Ser Gly Asp Arg Gly Asp Gly Pro Phe
        170                 175                 180

cgc tta ggt ggc cat ggg cct tcc ttt cca cta aaa gga att acc gaa     630
Arg Leu Gly Gly His Gly Pro Ser Phe Pro Leu Lys Gly Ile Thr Glu
    185                 190                 195

cag caa aaa gaa ggt ctt gag ata gtg aaa atg gtg atg ata tct tta     678
Gln Gln Lys Glu Gly Leu Glu Ile Val Lys Met Val Met Ile Ser Leu
200                 205                 210                 215

gaa ggt gaa gat ggg ttg gat gaa att tat tca ttc agt gag agt ctg     726
Glu Gly Glu Asp Gly Leu Asp Glu Ile Tyr Ser Phe Ser Glu Ser Leu
                220                 225                 230

aga aaa ctg tgc gtc ttc aag aaa att gag agg cat tcc att cac tgg     774
Arg Lys Leu Cys Val Phe Lys Lys Ile Glu Arg His Ser Ile His Trp
            235                 240                 245

ccc tgc cga ctg acc att ggc tcc aat ttg tct ata agg att gca gcc     822
Pro Cys Arg Leu Thr Ile Gly Ser Asn Leu Ser Ile Arg Ile Ala Ala
        250                 255                 260

tat aaa tcg att cta cag gag aga gtt aaa aag act tgg aca gtt gtg     870
Tyr Lys Ser Ile Leu Gln Glu Arg Val Lys Lys Thr Trp Thr Val Val
    265                 270                 275

gat gca aaa acc cta aaa aaa gaa gat ata caa aaa gaa aca gtt tat     918
Asp Ala Lys Thr Leu Lys Lys Glu Asp Ile Gln Lys Glu Thr Val Tyr
280                 285                 290                 295

tgc tta aat gat gat gat gaa act gaa gtt tta aaa gag gat att att     966
Cys Leu Asn Asp Asp Asp Glu Thr Glu Val Leu Lys Glu Asp Ile Ile
                300                 305                 310

caa ggg ttc cgc tat gga agt gat ata gtt cct ttc tct aaa gtg gat     1014
Gln Gly Phe Arg Tyr Gly Ser Asp Ile Val Pro Phe Ser Lys Val Asp
            315                 320                 325

gag gaa caa atg aaa tat aaa tcg gag ggg aag tgc ttc tct gtt ttg     1062
Glu Glu Gln Met Lys Tyr Lys Ser Glu Gly Lys Cys Phe Ser Val Leu
        330                 335                 340

gga ttt tgt aaa tct tct cag gtt cag aga aga ttc ttc atg gga aat     1110
```

```
Gly Phe Cys Lys Ser Ser Gln Val Gln Arg Arg Phe Phe Met Gly Asn
    345                 350                 355

caa gtt cta aag gtc ttt gca gca aga gat gat gag gca gct gca gtt     1158
Gln Val Leu Lys Val Phe Ala Ala Arg Asp Asp Glu Ala Ala Ala Val
360                 365                 370                 375

gca ctt tcc tcc ctg att cat gct ttg gat gac tta gac atg gtg gcc     1206
Ala Leu Ser Ser Leu Ile His Ala Leu Asp Asp Leu Asp Met Val Ala
                380                 385                 390

ata gtt cga tat gct tat gac aaa aga gct aat cct caa gtc ggc gtg     1254
Ile Val Arg Tyr Ala Tyr Asp Lys Arg Ala Asn Pro Gln Val Gly Val
            395                 400                 405

gct ttt cct cat atc aag cat aac tat gag tgt tta gtg tat gtg cag     1302
Ala Phe Pro His Ile Lys His Asn Tyr Glu Cys Leu Val Tyr Val Gln
        410                 415                 420

ctg cct ttc atg gaa gac ttg cgg caa tac atg ttt tca tcc ttg aaa     1350
Leu Pro Phe Met Glu Asp Leu Arg Gln Tyr Met Phe Ser Ser Leu Lys
    425                 430                 435

aac agt aag aaa tat gct ccc acc gag gca cag ttg aat gct gtt gat     1398
Asn Ser Lys Lys Tyr Ala Pro Thr Glu Ala Gln Leu Asn Ala Val Asp
440                 445                 450                 455

gct ttg att gac tcc atg agc ttg gca aag aaa gat gag aag aca gac     1446
Ala Leu Ile Asp Ser Met Ser Leu Ala Lys Lys Asp Glu Lys Thr Asp
                460                 465                 470

acc ctt gaa gac ttg ttt cca acc acc aaa atc cca aat cct cga ttt     1494
Thr Leu Glu Asp Leu Phe Pro Thr Thr Lys Ile Pro Asn Pro Arg Phe
            475                 480                 485

cag aga tta ttt cag tgt ctg ctg cac aga gct tta cat ccc cgg gag     1542
Gln Arg Leu Phe Gln Cys Leu Leu His Arg Ala Leu His Pro Arg Glu
        490                 495                 500

cct cta ccc cca att cag cag cat att tgg aat atg ctg aat cct ccc     1590
Pro Leu Pro Pro Ile Gln Gln His Ile Trp Asn Met Leu Asn Pro Pro
    505                 510                 515

gct gag gtg aca aca aaa agt cag att cct ctc tct aaa ata aag acc     1638
Ala Glu Val Thr Thr Lys Ser Gln Ile Pro Leu Ser Lys Ile Lys Thr
520                 525                 530                 535

ctt ttt cct ctg att gaa gcc aag aaa aag gat caa gtg act gct cag     1686
Leu Phe Pro Leu Ile Glu Ala Lys Lys Lys Asp Gln Val Thr Ala Gln
                540                 545                 550

gaa att ttc caa gac aac cat gaa gat gga cct aca gct aaa aaa tta     1734
Glu Ile Phe Gln Asp Asn His Glu Asp Gly Pro Thr Ala Lys Lys Leu
            555                 560                 565

aag act gag caa ggg gga gcc cac ttc agc gtc tcc agt ctg gct gaa     1782
Lys Thr Glu Gln Gly Gly Ala His Phe Ser Val Ser Ser Leu Ala Glu
        570                 575                 580

ggc agt gtc acc tct gtt gga agt gtg aat cct gct gaa aac ttc cgt     1830
Gly Ser Val Thr Ser Val Gly Ser Val Asn Pro Ala Glu Asn Phe Arg
    585                 590                 595

gtt cta gtg aaa cag aag aag gcc agc ttt gag gaa gcg agt aac cag     1878
Val Leu Val Lys Gln Lys Lys Ala Ser Phe Glu Glu Ala Ser Asn Gln
600                 605                 610                 615
```

```
ctc ata aat cac atc gaa cag ttt ttg gat act aat gaa aca ccg tat    1926
Leu Ile Asn His Ile Glu Gln Phe Leu Asp Thr Asn Glu Thr Pro Tyr
                620                 625                 630

ttt atg aag agc ata gac tgc atc cga gcc ttc cgg gaa gaa gcc att    1974
Phe Met Lys Ser Ile Asp Cys Ile Arg Ala Phe Arg Glu Glu Ala Ile
            635                 640                 645

aag ttt tca gaa gag cag cgc ttt aac aac ttc ctg aaa gcc ctt caa    2022
Lys Phe Ser Glu Glu Gln Arg Phe Asn Asn Phe Leu Lys Ala Leu Gln
        650                 655                 660

gag aaa gtg gaa att aaa caa tta aat cat ttc tgg gaa att gtt gtc    2070
Glu Lys Val Glu Ile Lys Gln Leu Asn His Phe Trp Glu Ile Val Val
    665                 670                 675

cag gat gga att act ctg atc acc aaa gag gaa gcc tct gga agt tct    2118
Gln Asp Gly Ile Thr Leu Ile Thr Lys Glu Glu Ala Ser Gly Ser Ser
680                 685                 690                 695

gtc aca gct gag gaa gcc aaa aag ttt ctg gcc ccc aaa gac aaa cca    2166
Val Thr Ala Glu Glu Ala Lys Lys Phe Leu Ala Pro Lys Asp Lys Pro
                700                 705                 710

agt gga gac aca gca gct gta ttt gaa gaa ggt ggt gat gtg gac gat    2214
Ser Gly Asp Thr Ala Ala Val Phe Glu Glu Gly Gly Asp Val Asp Asp
            715                 720                 725

tta ttg gac atg ata tag gtcgtggatg tatggggaat ctaagagagc           2262
Leu Leu Asp Met Ile  *
        730

tgccatcgct gtgatgctgg gagttctaac aaaacaagtt ggatgcggcc attcaagggg 2322
agccaaaatc tcaagaaatt cccagcaggt tacctggagg cggatcatct aattctctgt 2382
ggaatgaata cacacatata tattacaagg gataatttag accccataca agtttataaa 2442
gagtcattgt tattttctgg ttggtgtatt atttttctg tggtcttact gatctttgta 2502
tattacatac atgctttgaa gtttctggaa agtagatctt ttcttgacct agtatatcag 2562
tgacagttgc agcccttgtg atgtgattag tgtctcatgt ggaaccatgg catggttatt 2622
gatgagtttc ttaacccttt ccagagtcct cctttgcctg atcctccaac agctgtcaca 2682
acttgtgttg agcaagcagt agcatttgct tcctcccaac aagcagctgg gttaggaaaa 2742
ccatgggtaa ggacggactc acttctcttt ttagttgagg ccttctagtt accacattac 2802
tctgcctctg tatataggtg gttttcttta agtggggtgg gaaggggagc acaatttccc 2862
ttcatactcc ttttaagcag tgagttatgg tggtggtctc atgaagaaaa gacctttttgg 2922
cccaatctct gccatatcag tgaaccttta gaaactcaaa aactgagaaa tttacttcag 2982
tagttagaat tatatcactt cactgttctc tacttgcaag cctcaaagag agaaagtttc 3042
gttatattaa aacacttagg taacttttcg gtctttccca tttctaccta agtcagcttt 3102
catctttgtg gatggtgtct cctttactaa ataagaaaat aacaaagccc ttattctctt 3162
tttttcttgt cctcattctt gccttgagtt ccagttcctc tttggtgtac agacttcttg 3222
gtacccagtc acctctgtct tcagcaccct cataagtcgt cactaataca cagttttgta 3282
catgtaacat taaaggcata aatgactc                                    3310
```

<210> 10

<211> 732

<212> PRT

<213> Homo sapiens

<400> 10

```
Met Val Arg Ser Gly Asn Lys Ala Ala Val Val Leu Cys Met Asp Val
 1               5                  10                  15
Gly Phe Thr Met Ser Asn Ser Ile Pro Gly Ile Glu Ser Pro Phe Glu
            20                  25                  30
Gln Ala Lys Lys Val Ile Thr Met Phe Val Gln Arg Gln Val Phe Ala
        35                  40                  45
Glu Asn Lys Asp Glu Ile Ala Leu Val Leu Phe Gly Thr Asp Gly Thr
    50                  55                  60
Asp Asn Pro Leu Ser Gly Gly Asp Gln Tyr Gln Asn Ile Thr Val His
65                  70                  75                  80
```

Arg His Leu Met Leu Pro Asp Phe Asp Leu Leu Glu Asp Ile Glu Ser
85 90 95
Lys Ile Gln Pro Gly Ser Gln Gln Ala Asp Phe Leu Asp Ala Leu Ile
100 105 110
Val Ser Met Asp Val Ile Gln His Glu Thr Ile Gly Lys Lys Phe Glu
115 120 125
Lys Arg His Ile Glu Ile Phe Thr Asp Leu Ser Ser Arg Phe Ser Lys
130 135 140
Ser Gln Leu Asp Ile Ile Ile His Ser Leu Lys Lys Cys Asp Ile Ser
145 150 155 160
Leu Gln Phe Phe Leu Pro Phe Ser Leu Gly Lys Glu Asp Gly Ser Gly
165 170 175
Asp Arg Gly Asp Gly Pro Phe Arg Leu Gly Gly His Gly Pro Ser Phe
180 185 190
Pro Leu Lys Gly Ile Thr Glu Gln Gln Lys Glu Gly Leu Glu Ile Val
195 200 205
Lys Met Val Met Ile Ser Leu Glu Gly Glu Asp Gly Leu Asp Glu Ile
210 215 220
Tyr Ser Phe Ser Glu Ser Leu Arg Lys Leu Cys Val Phe Lys Lys Ile
225 230 235 240
Glu Arg His Ser Ile His Trp Pro Cys Arg Leu Thr Ile Gly Ser Asn
245 250 255
Leu Ser Ile Arg Ile Ala Ala Tyr Lys Ser Ile Leu Gln Glu Arg Val
260 265 270
Lys Lys Thr Trp Thr Val Val Asp Ala Lys Thr Leu Lys Lys Glu Asp
275 280 285
Ile Gln Lys Glu Thr Val Tyr Cys Leu Asn Asp Asp Asp Glu Thr Glu
290 295 300
Val Leu Lys Glu Asp Ile Ile Gln Gly Phe Arg Tyr Gly Ser Asp Ile
305 310 315 320
Val Pro Phe Ser Lys Val Asp Glu Glu Gln Met Lys Tyr Lys Ser Glu
325 330 335
Gly Lys Cys Phe Ser Val Leu Gly Phe Cys Lys Ser Ser Gln Val Gln
340 345 350
Arg Arg Phe Phe Met Gly Asn Gln Val Leu Lys Val Phe Ala Ala Arg
355 360 365
Asp Asp Glu Ala Ala Ala Val Ala Leu Ser Ser Leu Ile His Ala Leu
370 375 380
Asp Asp Leu Asp Met Val Ala Ile Val Arg Tyr Ala Tyr Asp Lys Arg
385 390 395 400
Ala Asn Pro Gln Val Gly Val Ala Phe Pro His Ile Lys His Asn Tyr
405 410 415
Glu Cys Leu Val Tyr Val Gln Leu Pro Phe Met Glu Asp Leu Arg Gln
420 425 430
Tyr Met Phe Ser Ser Leu Lys Asn Ser Lys Lys Tyr Ala Pro Thr Glu
435 440 445
Ala Gln Leu Asn Ala Val Asp Ala Leu Ile Asp Ser Met Ser Leu Ala
450 455 460
Lys Lys Asp Glu Lys Thr Asp Thr Leu Glu Asp Leu Phe Pro Thr Thr
465 470 475 480
Lys Ile Pro Asn Pro Arg Phe Gln Arg Leu Phe Gln Cys Leu Leu His
485 490 495
Arg Ala Leu His Pro Arg Glu Pro Leu Pro Pro Ile Gln Gln His Ile
500 505 510
Trp Asn Met Leu Asn Pro Pro Ala Glu Val Thr Thr Lys Ser Gln Ile
515 520 525
Pro Leu Ser Lys Ile Lys Thr Leu Phe Pro Leu Ile Glu Ala Lys Lys
530 535 540
Lys Asp Gln Val Thr Ala Gln Glu Ile Phe Gln Asp Asn His Glu Asp
545 550 555 560
Gly Pro Thr Ala Lys Lys Leu Lys Thr Glu Gln Gly Gly Ala His Phe
565 570 575
Ser Val Ser Ser Leu Ala Glu Gly Ser Val Thr Ser Val Gly Ser Val
580 585 590
Asn Pro Ala Glu Asn Phe Arg Val Leu Val Lys Gln Lys Lys Ala Ser
595 600 605
Phe Glu Glu Ala Ser Asn Gln Leu Ile Asn His Ile Glu Gln Phe Leu

```
    610                 615                 620
Asp Thr Asn Glu Thr Pro Tyr Phe Met Lys Ser Ile Asp Cys Ile Arg
625                 630                 635                 640
Ala Phe Arg Glu Glu Ala Ile Lys Phe Ser Glu Glu Gln Arg Phe Asn
                645                 650                 655
Asn Phe Leu Lys Ala Leu Gln Glu Lys Val Glu Ile Lys Gln Leu Asn
            660                 665                 670
His Phe Trp Glu Ile Val Val Gln Asp Gly Ile Thr Leu Ile Thr Lys
        675                 680                 685
Glu Glu Ala Ser Gly Ser Ser Val Thr Ala Glu Glu Ala Lys Lys Phe
    690                 695                 700
Leu Ala Pro Lys Asp Lys Pro Ser Gly Asp Thr Ala Ala Val Phe Glu
705                 710                 715                 720
Glu Gly Gly Asp Val Asp Asp Leu Leu Asp Met Ile
                725                 730
```

<210> 11
<211> 6582
<212> DNA
<213> Homo sapiens

<220>
<221> CDS
<222> (125)...(3256)

<400> 11

```
agagggcaag gagagagcag agaacacact ttgccttctc tttggtattg agtaatatca 60
accaaattgc agacatctca acactttggc caggcagcct gctgagcaag gtacctcagc 120
cagc atg gca gcc tct ttc cca ccc acc ttg gga ctc agt tct gcc cca  169
     Met Ala Ala Ser Phe Pro Pro Thr Leu Gly Leu Ser Ser Ala Pro
      1               5                  10                  15

gat gaa att cag cac cca cat att aaa ttt tca gaa tgg aaa ttt aag   217
Asp Glu Ile Gln His Pro His Ile Lys Phe Ser Glu Trp Lys Phe Lys
                20                  25                  30

ctg ttc cgg gtg aga tcc ttt gaa aag aca cct gaa gaa gct caa aag   265
Leu Phe Arg Val Arg Ser Phe Glu Lys Thr Pro Glu Glu Ala Gln Lys
            35                  40                  45

gaa aag aag gat tcc ttt gag ggg aaa ccc tct ctg gag caa tct cca   313
Glu Lys Lys Asp Ser Phe Glu Gly Lys Pro Ser Leu Glu Gln Ser Pro
        50                  55                  60

gca gtc ctg gac aag gct gat ggt cag aag cca gtc cca act cag cca   361
Ala Val Leu Asp Lys Ala Asp Gly Gln Lys Pro Val Pro Thr Gln Pro
    65                  70                  75

ttg tta aaa gcc cac cct aag ttt tca aag aaa ttt cac gac aac gag   409
Leu Leu Lys Ala His Pro Lys Phe Ser Lys Lys Phe His Asp Asn Glu
80                  85                  90                  95

aaa gca aga ggc aaa gcg atc cat caa gcc aac ctt cga cat ctc tgc   457
Lys Ala Arg Gly Lys Ala Ile His Gln Ala Asn Leu Arg His Leu Cys
                100                 105                 110

cgc atc tgt ggg aat tct ttt aga gct gat gag cac aac agg aga tat   505
Arg Ile Cys Gly Asn Ser Phe Arg Ala Asp Glu His Asn Arg Arg Tyr
            115                 120                 125

cca gtc cat ggt cct gtg gat ggt aaa acc cta ggc ctt tta cga aag   553
Pro Val His Gly Pro Val Asp Gly Lys Thr Leu Gly Leu Leu Arg Lys
        130                 135                 140

aag gaa aag aga gct act tcc tgg ccg gac ctc att gcc aag gtt ttc   601
Lys Glu Lys Arg Ala Thr Ser Trp Pro Asp Leu Ile Ala Lys Val Phe
```

```
    145                 150                 155

cgg atc gat gtg aag gca gat gtt gac tcg atc cac ccc act gag ttc      649
Arg Ile Asp Val Lys Ala Asp Val Asp Ser Ile His Pro Thr Glu Phe
160                 165                 170                 175

tgc cat aac tgc tgg agc atc atg cac agg aag ttt agc agt gcc cca      697
Cys His Asn Cys Trp Ser Ile Met His Arg Lys Phe Ser Ser Ala Pro
                180                 185                 190

tgt gag gtt tac ttc ccg agg aac gtg acc atg gag tgg cac ccc cac      745
Cys Glu Val Tyr Phe Pro Arg Asn Val Thr Met Glu Trp His Pro His
            195                 200                 205

aca cca tcc tgt gac atc tgc aac act gcc cgt cgg gga ctc aag agg      793
Thr Pro Ser Cys Asp Ile Cys Asn Thr Ala Arg Arg Gly Leu Lys Arg
        210                 215                 220

aag agt ctt cag cca aac ttg cag ctc agc aaa aaa ctc aaa act gtg      841
Lys Ser Leu Gln Pro Asn Leu Gln Leu Ser Lys Lys Leu Lys Thr Val
    225                 230                 235

ctt gac caa gca aga caa gcc cgt cag cgc aag aga aga gct cag gca      889
Leu Asp Gln Ala Arg Gln Ala Arg Gln Arg Lys Arg Arg Ala Gln Ala
240                 245                 250                 255

agg atc agc agc aag gat gtc atg aag aag atc gcc aac tgc agt aag      937
Arg Ile Ser Ser Lys Asp Val Met Lys Lys Ile Ala Asn Cys Ser Lys
                260                 265                 270

ata cat ctt agt acc aag ctc ctt gca gtg gac ttc cca gag cac ttt      985
Ile His Leu Ser Thr Lys Leu Leu Ala Val Asp Phe Pro Glu His Phe
            275                 280                 285

gtg aaa tcc atc tcc tgc cag atc tgt gaa cac att ctg gct gac cct      1033
Val Lys Ser Ile Ser Cys Gln Ile Cys Glu His Ile Leu Ala Asp Pro
        290                 295                 300

gtg gag acc aac tgt aag cat gtc ttt tgc cgg gtc tgc att ctc aga      1081
Val Glu Thr Asn Cys Lys His Val Phe Cys Arg Val Cys Ile Leu Arg
    305                 310                 315

tgc ctc aaa gtc atg ggc agc tat tgt ccc tct tgc cga tat cca tgc      1129
Cys Leu Lys Val Met Gly Ser Tyr Cys Pro Ser Cys Arg Tyr Pro Cys
320                 325                 330                 335

ttc cct act gac ctg gag agt cca gtg aag tcc ttt ctg agc gtc ttg      1177
Phe Pro Thr Asp Leu Glu Ser Pro Val Lys Ser Phe Leu Ser Val Leu
                340                 345                 350

aat tcc ctg atg gtg aaa tgt cca gca aaa gag tgc aat gag gag gtc      1225
Asn Ser Leu Met Val Lys Cys Pro Ala Lys Glu Cys Asn Glu Glu Val
            355                 360                 365

agt ttg gaa aaa tat aat cac cac atc tca agt cac aag gaa tca aaa      1273
Ser Leu Glu Lys Tyr Asn His His Ile Ser Ser His Lys Glu Ser Lys
        370                 375                 380

gag att ttt gtg cac att aat aaa ggg ggc cgg ccc cgc caa cat ctt      1321
Glu Ile Phe Val His Ile Asn Lys Gly Gly Arg Pro Arg Gln His Leu
    385                 390                 395

ctg tcg ctg act cgg aga gct cag aag cac cgg ctg agg gag ctc aag      1369
Leu Ser Leu Thr Arg Arg Ala Gln Lys His Arg Leu Arg Glu Leu Lys
400                 405                 410                 415

ctg caa gtc aaa gcc ttt gct gac aaa gaa gaa ggt gga gat gtg aag      1417
```

```
Leu Gln Val Lys Ala Phe Ala Asp Lys Glu Glu Gly Gly Asp Val Lys
                420                 425                 430

tcc gtg tgc atg acc ttg ttc ctg ctg gct ctg agg gcg agg aat gag      1465
Ser Val Cys Met Thr Leu Phe Leu Leu Ala Leu Arg Ala Arg Asn Glu
            435                 440                 445

cac agg caa gct gat gag ctg gag gcc atc atg cag gga aag ggc tct      1513
His Arg Gln Ala Asp Glu Leu Glu Ala Ile Met Gln Gly Lys Gly Ser
        450                 455                 460

ggc ctg cag cca gct gtt tgc ttg gcc atc cgt gtc aac acc ttc ctc      1561
Gly Leu Gln Pro Ala Val Cys Leu Ala Ile Arg Val Asn Thr Phe Leu
    465                 470                 475

agc tgc agt cag tac cac aag atg tac agg act gtg aaa gcc atc aca      1609
Ser Cys Ser Gln Tyr His Lys Met Tyr Arg Thr Val Lys Ala Ile Thr
480                 485                 490                 495

ggg aga cag att ttt cag cct ttg cat gcc ctt cgg aat gct gag aag      1657
Gly Arg Gln Ile Phe Gln Pro Leu His Ala Leu Arg Asn Ala Glu Lys
                500                 505                 510

gta ctt ctg cca ggc tac cac cac ttt gag tgg cag cca cct ctg aag      1705
Val Leu Leu Pro Gly Tyr His His Phe Glu Trp Gln Pro Pro Leu Lys
            515                 520                 525

aat gtg tct tcc agc act gat gtt ggc att att gat ggg ctg tct gga      1753
Asn Val Ser Ser Ser Thr Asp Val Gly Ile Ile Asp Gly Leu Ser Gly
        530                 535                 540

cta tca tcc tct gtg gat gat tac cca gtg gac acc att gca aag agg      1801
Leu Ser Ser Ser Val Asp Asp Tyr Pro Val Asp Thr Ile Ala Lys Arg
    545                 550                 555

ttc cgc tat gat tca gct ttg gtg tct gct ttg atg gac atg gaa gaa      1849
Phe Arg Tyr Asp Ser Ala Leu Val Ser Ala Leu Met Asp Met Glu Glu
560                 565                 570                 575

gac atc ttg gaa ggc atg aga tcc caa gac ctt gat gat tac ctg aat      1897
Asp Ile Leu Glu Gly Met Arg Ser Gln Asp Leu Asp Asp Tyr Leu Asn
                580                 585                 590

ggc ccc ttc act gtg gtg gtg aag gag tct tgt gat gga atg gga gac      1945
Gly Pro Phe Thr Val Val Val Lys Glu Ser Cys Asp Gly Met Gly Asp
            595                 600                 605

gtg agt gag aag cat ggg agt ggg cct gta gtt cca gaa aag gca gtc      1993
Val Ser Glu Lys His Gly Ser Gly Pro Val Val Pro Glu Lys Ala Val
        610                 615                 620

cgt ttt tca ttc aca atc atg aaa att act att gcc cac agc tct cag      2041
Arg Phe Ser Phe Thr Ile Met Lys Ile Thr Ile Ala His Ser Ser Gln
    625                 630                 635

aat gtg aaa gta ttt gaa gaa gcc aaa cct aac tct gaa ctg tgt tgc      2089
Asn Val Lys Val Phe Glu Glu Ala Lys Pro Asn Ser Glu Leu Cys Cys
640                 645                 650                 655

aag cca ttg tgc ctt atg ctg gca gat gag tct gac cac gag acg ctg      2137
Lys Pro Leu Cys Leu Met Leu Ala Asp Glu Ser Asp His Glu Thr Leu
                660                 665                 670

act gcc atc ctg agt cct ctc att gct gag agg gag gcc atg aag agc      2185
Thr Ala Ile Leu Ser Pro Leu Ile Ala Glu Arg Glu Ala Met Lys Ser
            675                 680                 685
```

```
agt gaa tta atg ctt gag ctg gga ggc att ctc cgg act ttc aag ttc    2233
Ser Glu Leu Met Leu Glu Leu Gly Gly Ile Leu Arg Thr Phe Lys Phe
        690                 695                 700

atc ttc agg ggc acc ggc tat gat gaa aaa ctt gtg cgg gaa gtg gaa    2281
Ile Phe Arg Gly Thr Gly Tyr Asp Glu Lys Leu Val Arg Glu Val Glu
    705                 710                 715

ggc ctc gag gct tct ggc tca gtc tac att tgt act ctt tgt gat gcc    2329
Gly Leu Glu Ala Ser Gly Ser Val Tyr Ile Cys Thr Leu Cys Asp Ala
720                 725                 730                 735

acc cgt ctg gaa gcc tct caa aat ctt gtc ttc cac tct ata acc aga    2377
Thr Arg Leu Glu Ala Ser Gln Asn Leu Val Phe His Ser Ile Thr Arg
                740                 745                 750

agc cat gct gag aac ctg gaa cgt tat gag gtc tgg cgt tcc aac cct    2425
Ser His Ala Glu Asn Leu Glu Arg Tyr Glu Val Trp Arg Ser Asn Pro
            755                 760                 765

tac cat gag tct gtg gaa gaa ctg cgg gat cgg gtg aaa ggg gtc tca    2473
Tyr His Glu Ser Val Glu Glu Leu Arg Asp Arg Val Lys Gly Val Ser
        770                 775                 780

gct aaa cct ttc att gag aca gtc cct tcc ata gat gca ctc cac tgt    2521
Ala Lys Pro Phe Ile Glu Thr Val Pro Ser Ile Asp Ala Leu His Cys
    785                 790                 795

gac att ggc aat gca gct gag ttc tac aag atc ttc cag cta gag ata    2569
Asp Ile Gly Asn Ala Ala Glu Phe Tyr Lys Ile Phe Gln Leu Glu Ile
800                 805                 810                 815

ggg gaa gtg tat aag aat ccc aat gct tcc aaa gag gaa agg aaa agg    2617
Gly Glu Val Tyr Lys Asn Pro Asn Ala Ser Lys Glu Glu Arg Lys Arg
                820                 825                 830

tgg cag gcc aca ctg gac aag cat ctc cgg aag aag atg aac ctc aaa    2665
Trp Gln Ala Thr Leu Asp Lys His Leu Arg Lys Lys Met Asn Leu Lys
            835                 840                 845

cca atc atg agg atg aat ggc aac ttt gcc agg aag ctc atg acc aaa    2713
Pro Ile Met Arg Met Asn Gly Asn Phe Ala Arg Lys Leu Met Thr Lys
        850                 855                 860

gag act gtg gat gca gtt tgt gag tta att cct tcc gag gag agg cac    2761
Glu Thr Val Asp Ala Val Cys Glu Leu Ile Pro Ser Glu Glu Arg His
    865                 870                 875

gag gct ctg agg gag ctg atg gat ctt tac ctg aag atg aaa cca gta    2809
Glu Ala Leu Arg Glu Leu Met Asp Leu Tyr Leu Lys Met Lys Pro Val
880                 885                 890                 895

tgg cga tca tca tgc cct gct aaa gag tgc cca gaa tcc ctc tgc cag    2857
Trp Arg Ser Ser Cys Pro Ala Lys Glu Cys Pro Glu Ser Leu Cys Gln
                900                 905                 910

tac agt ttc aat tca cag cgt ttt gct gag ctc ctt tct acg aag ttc    2905
Tyr Ser Phe Asn Ser Gln Arg Phe Ala Glu Leu Leu Ser Thr Lys Phe
            915                 920                 925

aag tat agg tat gag gga aaa atc acc aat tat ttt cac aaa acc ctg    2953
Lys Tyr Arg Tyr Glu Gly Lys Ile Thr Asn Tyr Phe His Lys Thr Leu
        930                 935                 940

gcc cat gtt cct gaa att att gag agg gat ggc tcc att ggg gca tgg    3001
Ala His Val Pro Glu Ile Ile Glu Arg Asp Gly Ser Ile Gly Ala Trp
    945                 950                 955
```

```
gca agt gag gga aat gag tct ggt aac aaa ctg ttt agg cgc ttc cgg    3049
Ala Ser Glu Gly Asn Glu Ser Gly Asn Lys Leu Phe Arg Arg Phe Arg
960                 965                 970                 975

aaa atg aat gcc agg cag tcc aaa tgc tat gag atg gaa gat gtc ctg    3097
Lys Met Asn Ala Arg Gln Ser Lys Cys Tyr Glu Met Glu Asp Val Leu
                980                 985                 990

aaa cac cac tgg ttg tac acc tcc aaa tac ctc cag aag ttt atg aat    3145
Lys His His Trp Leu Tyr Thr Ser Lys Tyr Leu Gln Lys Phe Met Asn
            995                 1000                1005

gct cat aat gca tta aaa acc tct ggg ttt acc atg aac cct cag gca    3193
Ala His Asn Ala Leu Lys Thr Ser Gly Phe Thr Met Asn Pro Gln Ala
        1010                1015                1020

agc tta ggg gac cca tta ggc ata gag gac tct ctg gaa agc caa gat    3241
Ser Leu Gly Asp Pro Leu Gly Ile Glu Asp Ser Leu Glu Ser Gln Asp
    1025                1030                1035

tca atg gaa ttt taa gtagggcaac cacttatgag ttggtttttg caattgagtt    3296
Ser Met Glu Phe  *
1040

tccctctggg ttgcattgag ggcttctcct agcacccttt actgctgtgt atggggcttc 3356
accatccaag aggtggtagg ttggagtaag atgctacaga tgctctcaag tcaggaatag 3416
aaactgatga gctgattgct tgaggctttt agtgagttcc gaaaagcaac aggaaaaatc 3476
agttatctga aagctcagta actcagaaca ggagtaactg caggggacca gagatgagca 3536
aagatctgtg tgtgttgggg agctgtcatg taaatcaaag ccaaggttgt caaagaacag 3596
ccagtgaggc caggaaagaa attggtcttg tggttttcat tttttcccc cttgattgat 3656
tatattttgt attgagatat gataagtgcc ttctatttca tttttgaata attcttcatt 3716
tttataattt tacatatctt ggcttgctat ataagattca aaagagcttt ttaaattttt 3776
ctaataatat cttacatttg tacagcatga tgacctttac aaagtgctct caatgcattt 3836
acccattcgt tatataaata tgttacatca ggacaacttt gagaaaatca gtcctttttt 3896
atgtttaaat tatgtatcta ttgtaacctt cagagtttag gaggtcatct gctgtcatgg 3956
atttttcaat aatgaattta gaatacacct gttagctaca gttagttatt aaatcttctg 4016
ataatatatg tttacttagc tatcagaagc caagtatgat tctttatttt tactttttca 4076
tttcaagaaa tttagagttt ccaaatttag agcttctgca tacagtctta aagccacaga 4136
ggcttgtaaa aatataggtt agcttgatgt ctaaaaatat atttcatgtc ttactgaaac 4196
attttgccag actttctcca aatgaaacct gaatcaattt ttctaaatct aggtttcata 4256
gagtcctctc ctctgcaatg tgttattctt tctataatga tcagtttact ttcagtggat 4316
tcagaattgt gtagcaggat aaccttgtat ttttccatcc gctaagttta gatggagtcc 4376
aaacgcagta cagcagaaga gttaacattt acacagtgct tttaccact gtggaatgtt 4436
ttcacactca tttttcctta caacaattct gaggagtagg tgttgttatt atctccattt 4496
gatgggggtt taaatgattt gctcaaagtc atttaggggt aataaatact tggcttggaa 4556
atttaacaca gtccttttgt ctccaaagcc cttcttcttt ccaccacaaa ttaatcacta 4616
tgtttataag gtagtatcag aatttttta ggattcacaa ctaatcacta tagcacatga 4676
ccttgggatt acatttttat ggggcagggg taagcaagtt tttaaatcat ttgtgtgctc 4736
tggctctttt gatagaagaa agcaacacaa aagctccaaa gggcccccta accctcttgt 4796
ggctccagtt atttggaaac tatgatctgc atccttagga atctgggatt tgccagttgc 4856
tggcaatgta gagcaggcat ggaattttat atgctagtga gtcataatga tatgttagtg 4916
ttaattagtt ttttcttcct ttgattttat tggccataat tgctactctt catacacagt 4976
atatcaaaga gcttgataat ttagttgtca aaagtgcatc ggcgacatta tctttaattg 5036
tatgtatttg gtgcttcttc agggattgaa ctcagtatct ttcattaaaa aacacagcag 5096
ttttccttgc tttttatatg cagaatatca aagtcatttc taatttagtt gtcaaaaaca 5156
tatacatatt ttaacattag tttttttgaa aactcttggt tttgtttttt tggaaatgag 5216
tgggccacta agccacactt tcccttcatc ctgcttaatc cttccagcat gtctctgcac 5276
taataaacag ctaaattcac ataatcatcc tatttactga agcatggtca tgctggttta 5336
tagatttttt acccatttct actctttttc tctattggtg gcactgtaaa tactttccag 5396
tattaaatta tccttttcta acactgtagg aactattttg aatgcatgtg actaagagca 5456
tgatttatag cacaaccttt ccaataatcc cttaatcaga tcacattttg ataaaccctg 5516
ggaacatctg gctgcaggaa tttcaatatg tagaaacgct gcctatggtt ttttgccctt 5576
actgttgaga ctgcaatatc ctagacccta gttttatact agagttttat ttttagcaat 5636
gcctattgca agtgcaatta tatactccag ggaaattcac cacactgaat cgagcatttg 5696
tgtgtgtatg tgtgaagtat atactgggac ttcagaagtg caatgtattt ttctcctgtg 5756
aaacctgaat ctacaagttt tcctgccaag ccactcaggt gcattgcagg gaccagtgat 5816
```

```
aatggctgat gaaaattgat gattggtcag tgaggtcaaa aggagccttg ggattaataa 5876
acatgcactg agaagcaaga ggaggagaaa aagatgtctt tttcttccag gtgaactgga 5936
atttagtttt gcctcagatt tttttcccac aagatacaga agaagataaa gatttttttg 5996
gttgagagtg tgggtcttgc attacatcaa acagagttca aattccacac agataagagg 6056
caggatatat aagcgccagt ggtagttggg aggaataaac cattatttgg atgcaggtgg 6116
tttttgattg caaatatgtg tgtgtcttca gtgattgtat gacagatgat gtattctttt 6176
gatgttaaaa gattttaagt aagagtagat acattgtacc cattttacat tttcttattt 6236
taactacagt aatctacata aatatacctc agaaatcatt tttggtgatt atttttttgtt 6296
ttgtagaatt gcacttcagt ttattttctt acaaataacc ttacattttg tttaatggct 6356
tccaagagcc tttttttttt ttgtatttca gagaaaattc aggtaccagg atgcaatgga 6416
tttatttgat tcaggggacc tgtgtttcca tgtcaaatgt tttcaaataa aatgaaatat 6476
gagtttcaat actttttata ttttaatatt tccattcatt aatattatgg ttattgtcag 6536
caattttatg tttgaatatt tgaaataaaa gtttaagatt tgaaaa                6582
```

<210> 12

<211> 1043

<212> PRT

<213> Homo sapiens

<400> 12

```
Met Ala Ala Ser Phe Pro Pro Thr Leu Gly Leu Ser Ser Ala Pro Asp
 1               5                  10                  15
Glu Ile Gln His Pro His Ile Lys Phe Ser Glu Trp Lys Phe Lys Leu
            20                  25                  30
Phe Arg Val Arg Ser Phe Glu Lys Thr Pro Glu Glu Ala Gln Lys Glu
        35                  40                  45
Lys Lys Asp Ser Phe Glu Gly Lys Pro Ser Leu Glu Gln Ser Pro Ala
    50                  55                  60
Val Leu Asp Lys Ala Asp Gly Gln Lys Pro Val Pro Thr Gln Pro Leu
65                  70                  75                  80
Leu Lys Ala His Pro Lys Phe Ser Lys Lys Phe His Asp Asn Glu Lys
                85                  90                  95
Ala Arg Gly Lys Ala Ile His Gln Ala Asn Leu Arg His Leu Cys Arg
            100                 105                 110
Ile Cys Gly Asn Ser Phe Arg Ala Asp Glu His Asn Arg Arg Tyr Pro
        115                 120                 125
Val His Gly Pro Val Asp Gly Lys Thr Leu Gly Leu Leu Arg Lys Lys
    130                 135                 140
Glu Lys Arg Ala Thr Ser Trp Pro Asp Leu Ile Ala Lys Val Phe Arg
145                 150                 155                 160
Ile Asp Val Lys Ala Asp Val Asp Ser Ile His Pro Thr Glu Phe Cys
                165                 170                 175
His Asn Cys Trp Ser Ile Met His Arg Lys Phe Ser Ser Ala Pro Cys
            180                 185                 190
Glu Val Tyr Phe Pro Arg Asn Val Thr Met Glu Trp His Pro His Thr
        195                 200                 205
Pro Ser Cys Asp Ile Cys Asn Thr Ala Arg Arg Gly Leu Lys Arg Lys
    210                 215                 220
Ser Leu Gln Pro Asn Leu Gln Leu Ser Lys Lys Leu Lys Thr Val Leu
225                 230                 235                 240
Asp Gln Ala Arg Gln Ala Arg Gln Arg Lys Arg Arg Ala Gln Ala Arg
                245                 250                 255
Ile Ser Ser Lys Asp Val Met Lys Lys Ile Ala Asn Cys Ser Lys Ile
            260                 265                 270
His Leu Ser Thr Lys Leu Leu Ala Val Asp Phe Pro Glu His Phe Val
        275                 280                 285
Lys Ser Ile Ser Cys Gln Ile Cys Glu His Ile Leu Ala Asp Pro Val
    290                 295                 300
Glu Thr Asn Cys Lys His Val Phe Cys Arg Val Cys Ile Leu Arg Cys
305                 310                 315                 320
Leu Lys Val Met Gly Ser Tyr Cys Pro Ser Cys Arg Tyr Pro Cys Phe
                325                 330                 335
Pro Thr Asp Leu Glu Ser Pro Val Lys Ser Phe Leu Ser Val Leu Asn
            340                 345                 350
Ser Leu Met Val Lys Cys Pro Ala Lys Glu Cys Asn Glu Glu Val Ser
        355                 360                 365
Leu Glu Lys Tyr Asn His His Ile Ser Ser His Lys Glu Ser Lys Glu
```

```
    370                 375                 380
Ile Phe Val His Ile Asn Lys Gly Gly Arg Pro Arg Gln His Leu Leu
385                 390                 395                 400
Ser Leu Thr Arg Arg Ala Gln Lys His Arg Leu Arg Glu Leu Lys Leu
                405                 410                 415
Gln Val Lys Ala Phe Ala Asp Lys Glu Glu Gly Gly Asp Val Lys Ser
            420                 425                 430
Val Cys Met Thr Leu Phe Leu Leu Ala Leu Arg Ala Arg Asn Glu His
        435                 440                 445
Arg Gln Ala Asp Glu Leu Glu Ala Ile Met Gln Gly Lys Gly Ser Gly
    450                 455                 460
Leu Gln Pro Ala Val Cys Leu Ala Ile Arg Val Asn Thr Phe Leu Ser
465                 470                 475                 480
Cys Ser Gln Tyr His Lys Met Tyr Arg Thr Val Lys Ala Ile Thr Gly
                485                 490                 495
Arg Gln Ile Phe Gln Pro Leu His Ala Leu Arg Asn Ala Glu Lys Val
            500                 505                 510
Leu Leu Pro Gly Tyr His His Phe Glu Trp Gln Pro Pro Leu Lys Asn
        515                 520                 525
Val Ser Ser Ser Thr Asp Val Gly Ile Ile Asp Gly Leu Ser Gly Leu
    530                 535                 540
Ser Ser Ser Val Asp Asp Tyr Pro Val Asp Thr Ile Ala Lys Arg Phe
545                 550                 555                 560
Arg Tyr Asp Ser Ala Leu Val Ser Ala Leu Met Asp Met Glu Glu Asp
                565                 570                 575
Ile Leu Glu Gly Met Arg Ser Gln Asp Leu Asp Asp Tyr Leu Asn Gly
            580                 585                 590
Pro Phe Thr Val Val Val Lys Glu Ser Cys Asp Gly Met Gly Asp Val
        595                 600                 605
Ser Glu Lys His Gly Ser Gly Pro Val Val Pro Glu Lys Ala Val Arg
    610                 615                 620
Phe Ser Phe Thr Ile Met Lys Ile Thr Ile Ala His Ser Ser Gln Asn
625                 630                 635                 640
Val Lys Val Phe Glu Glu Ala Lys Pro Asn Ser Glu Leu Cys Cys Lys
                645                 650                 655
Pro Leu Cys Leu Met Leu Ala Asp Glu Ser Asp His Glu Thr Leu Thr
            660                 665                 670
Ala Ile Leu Ser Pro Leu Ile Ala Glu Arg Glu Ala Met Lys Ser Ser
        675                 680                 685
Glu Leu Met Leu Glu Leu Gly Gly Ile Leu Arg Thr Phe Lys Phe Ile
    690                 695                 700
Phe Arg Gly Thr Gly Tyr Asp Glu Lys Leu Val Arg Glu Val Glu Gly
705                 710                 715                 720
Leu Glu Ala Ser Gly Ser Val Tyr Ile Cys Thr Leu Cys Asp Ala Thr
                725                 730                 735
Arg Leu Glu Ala Ser Gln Asn Leu Val Phe His Ser Ile Thr Arg Ser
            740                 745                 750
His Ala Glu Asn Leu Glu Arg Tyr Glu Val Trp Arg Ser Asn Pro Tyr
        755                 760                 765
His Glu Ser Val Glu Glu Leu Arg Asp Arg Val Lys Gly Val Ser Ala
    770                 775                 780
Lys Pro Phe Ile Glu Thr Val Pro Ser Ile Asp Ala Leu His Cys Asp
785                 790                 795                 800
Ile Gly Asn Ala Ala Glu Phe Tyr Lys Ile Phe Gln Leu Glu Ile Gly
                805                 810                 815
Glu Val Tyr Lys Asn Pro Asn Ala Ser Lys Glu Glu Arg Lys Arg Trp
            820                 825                 830
Gln Ala Thr Leu Asp Lys His Leu Arg Lys Lys Met Asn Leu Lys Pro
        835                 840                 845
Ile Met Arg Met Asn Gly Asn Phe Ala Arg Lys Leu Met Thr Lys Glu
    850                 855                 860
Thr Val Asp Ala Val Cys Glu Leu Ile Pro Ser Glu Glu Arg His Glu
865                 870                 875                 880
Ala Leu Arg Glu Leu Met Asp Leu Tyr Leu Lys Met Lys Pro Val Trp
                885                 890                 895
Arg Ser Ser Cys Pro Ala Lys Glu Cys Pro Glu Ser Leu Cys Gln Tyr
            900                 905                 910
```

```
Ser Phe Asn Ser Gln Arg Phe Ala Glu Leu Leu Ser Thr Lys Phe Lys
        915                 920                 925
Tyr Arg Tyr Glu Gly Lys Ile Thr Asn Tyr Phe His Lys Thr Leu Ala
    930                 935                 940
His Val Pro Glu Ile Ile Glu Arg Asp Gly Ser Ile Gly Ala Trp Ala
945                 950                 955                 960
Ser Glu Gly Asn Glu Ser Gly Asn Lys Leu Phe Arg Arg Phe Arg Lys
                965                 970                 975
Met Asn Ala Arg Gln Ser Lys Cys Tyr Glu Met Glu Asp Val Leu Lys
            980                 985                 990
His His Trp Leu Tyr Thr Ser Lys Tyr Leu Gln Lys Phe Met Asn Ala
        995                 1000                1005
His Asn Ala Leu Lys Thr Ser Gly Phe Thr Met Asn Pro Gln Ala Ser
    1010                1015                1020
Leu Gly Asp Pro Leu Gly Ile Glu Asp Ser Leu Glu Ser Gln Asp Ser
1025                1030                1035                1040
Met Glu Phe
```

<210> 13
<211> 967
<212> DNA
<213> Homo sapiens

<220>
<221> CDS
<222> (30)...(743)

<400> 13

```
gcagaagtct ctctcagtca ggacacagc atg gac atg agg gtc cct gct cag   53
                                Met Asp Met Arg Val Pro Ala Gln
                                 1               5

ctc ctg gga ctc ctg ctg ctc tgg ctc cca gat acc aga tgt gac atc  101
Leu Leu Gly Leu Leu Leu Leu Trp Leu Pro Asp Thr Arg Cys Asp Ile
     10                  15                  20

cag atg acc cag tct cca tcc tcc ctg tct gca tct gta gga gac aga  149
Gln Met Thr Gln Ser Pro Ser Ser Leu Ser Ala Ser Val Gly Asp Arg
 25                  30                  35                  40

gtc acc atc act tgc cgg gcg aat cag gac att aag aat tat gta gcc  197
Val Thr Ile Thr Cys Arg Ala Asn Gln Asp Ile Lys Asn Tyr Val Ala
                 45                  50                  55

tgg tat cag cag aaa cca ggg aaa gtt cct aag gtc ctg atc tat gct  245
Trp Tyr Gln Gln Lys Pro Gly Lys Val Pro Lys Val Leu Ile Tyr Ala
             60                  65                  70

gca tcc act ttg caa tcg ggg gtc cca tct cgg ttc agt ggc tct gga  293
Ala Ser Thr Leu Gln Ser Gly Val Pro Ser Arg Phe Ser Gly Ser Gly
         75                  80                  85

tct ggg aca gat ttc act ctc acc atc acc agc ctg cag cct gaa gat  341
Ser Gly Thr Asp Phe Thr Leu Thr Ile Thr Ser Leu Gln Pro Glu Asp
     90                  95                 100

gtt gca act tat tac tgt caa aca tat acc agt gcc ccc cct tgg acg  389
Val Ala Thr Tyr Tyr Cys Gln Thr Tyr Thr Ser Ala Pro Pro Trp Thr
105                 110                 115                 120

ttc ggc caa ggg acc aag gtg gag atc aat cga act gtg gct gca cca  437
Phe Gly Gln Gly Thr Lys Val Glu Ile Asn Arg Thr Val Ala Ala Pro
                125                 130                 135

tct gtc ttc atc ttc ccg cca tct gat gag cag ttg aaa tct gga act  485
```

```
Ser Val Phe Ile Phe Pro Pro Ser Asp Glu Gln Leu Lys Ser Gly Thr
            140                 145                 150

gcc tct gtt gtg tgc ctg ctg aat aac ttc tat ccc aga gag gcc aaa     533
Ala Ser Val Val Cys Leu Leu Asn Asn Phe Tyr Pro Arg Glu Ala Lys
        155                 160                 165

gta cag tgg aag gtg gat aac gcc ctc caa tcg ggt aac tcc cag gag     581
Val Gln Trp Lys Val Asp Asn Ala Leu Gln Ser Gly Asn Ser Gln Glu
    170                 175                 180

agt gtc aca gag cag gac agc aag gac agc acc tac agc ctc agc agc     629
Ser Val Thr Glu Gln Asp Ser Lys Asp Ser Thr Tyr Ser Leu Ser Ser
185                 190                 195                 200

acc ctg acg ctg agc aaa gca gac tac gag aaa cac aaa gtc tac gcc     677
Thr Leu Thr Leu Ser Lys Ala Asp Tyr Glu Lys His Lys Val Tyr Ala
                205                 210                 215

tgc gaa gtc acc cat cag ggc ctg agc tcg ccc gtc aca aag agc ttc     725
Cys Glu Val Thr His Gln Gly Leu Ser Ser Pro Val Thr Lys Ser Phe
            220                 225                 230

aac agg gga gag tgt tag agggagaagt gcccccacct gctcctcagt            773
Asn Arg Gly Glu Cys  *
        235

tccagcctga ccccctccca tcctttggcc tctgaccctt tttccacagg ggacctaccc 833
ctattgcggt cctccagctc atctttcacc tcacccccct cctcctcctt ggctttaatt 893
atgctaatgt tggaggagaa tgaataaata aagtgaatct ttgcaaaaaa aaaaaaaaaa 953
aaaaaaaaaa aaaa                                                   967
```

<210> 14
<211> 237
<212> PRT
<213> Homo sapiens

<400> 14

```
Met Asp Met Arg Val Pro Ala Gln Leu Leu Gly Leu Leu Leu Leu Trp
 1               5                  10                  15
Leu Pro Asp Thr Arg Cys Asp Ile Gln Met Thr Gln Ser Pro Ser Ser
            20                  25                  30
Leu Ser Ala Ser Val Gly Asp Arg Val Thr Ile Thr Cys Arg Ala Asn
        35                  40                  45
Gln Asp Ile Lys Asn Tyr Val Ala Trp Tyr Gln Gln Lys Pro Gly Lys
    50                  55                  60
Val Pro Lys Val Leu Ile Tyr Ala Ala Ser Thr Leu Gln Ser Gly Val
65                  70                  75                  80
Pro Ser Arg Phe Ser Gly Ser Gly Ser Gly Thr Asp Phe Thr Leu Thr
                85                  90                  95
Ile Thr Ser Leu Gln Pro Glu Asp Val Ala Thr Tyr Tyr Cys Gln Thr
            100                 105                 110
Tyr Thr Ser Ala Pro Pro Trp Thr Phe Gly Gln Gly Thr Lys Val Glu
        115                 120                 125
Ile Asn Arg Thr Val Ala Ala Pro Ser Val Phe Ile Phe Pro Pro Ser
    130                 135                 140
Asp Glu Gln Leu Lys Ser Gly Thr Ala Ser Val Val Cys Leu Leu Asn
145                 150                 155                 160
Asn Phe Tyr Pro Arg Glu Ala Lys Val Gln Trp Lys Val Asp Asn Ala
                165                 170                 175
Leu Gln Ser Gly Asn Ser Gln Glu Ser Val Thr Glu Gln Asp Ser Lys
            180                 185                 190
Asp Ser Thr Tyr Ser Leu Ser Ser Thr Leu Thr Leu Ser Lys Ala Asp
        195                 200                 205
Tyr Glu Lys His Lys Val Tyr Ala Cys Glu Val Thr His Gln Gly Leu
    210                 215                 220
Ser Ser Pro Val Thr Lys Ser Phe Asn Arg Gly Glu Cys

        225                 230                 235
```

<210> 15
<211> 899
<212> DNA
<213> Homo sapiens

<220>
<221> CDS
<222> (322)...(741)

<400> 15

```
agatgcccct ctgggagaga tccccagggg tgacagccat ggaccctgga agggcctggg 60
ctagggacag ggaccagagc cagtccaggg agaggacaga gccaatggac tggggtgtac 120
tgtaacagcc ctgctggcga gagggaccag ggcaccgtcc tccagggagc ccatgctgca 180
agtcgggcca gaggtgcccc tgaacctgaa ggccaatgag acccaagaca ggccaagtgg 240
gttgtgagac ccctgaggag ctgggccctg gtcccaggca gcgctggccc ctgctgctgc 300
tgggtctggc catggtcgcc c atg gcc tgc tgc gcc caa tgg ttg cac cgc   351
                        Met Ala Cys Cys Ala Gln Trp Leu His Arg
                         1               5                  10

aaa gcg ggg acc cag acc ctg gag cct cag ttg gaa gca gcc gat cca   399
Lys Ala Gly Thr Gln Thr Leu Glu Pro Gln Leu Glu Ala Ala Asp Pro
                 15                  20                  25

gcc tgc gga gcc tgt ggg gca ggt cag ccc aag gct gcc ccc tcg gtc   447
Ala Cys Gly Ala Cys Gly Ala Gly Gln Pro Lys Ala Ala Pro Ser Val
             30                  35                  40

act ctg ttc ccg ccc tcc tct gag gag ctt caa gcc aac aag gcc aca   495
Thr Leu Phe Pro Pro Ser Ser Glu Glu Leu Gln Ala Asn Lys Ala Thr
         45                  50                  55

ctg gtg tgt ctc ata agt gac ttc tac ccg gga gcc gtg aca gtg gcc   543
Leu Val Cys Leu Ile Ser Asp Phe Tyr Pro Gly Ala Val Thr Val Ala
     60                  65                  70

tgg aag gca gat agc agc ccc gtc aag gcg gga gtg gag acc acc aca   591
Trp Lys Ala Asp Ser Ser Pro Val Lys Ala Gly Val Glu Thr Thr Thr
 75                  80                  85                  90

ccc tcc aaa caa agc aac aac aag tac gcg gcc agc agc tac ctg agc   639
Pro Ser Lys Gln Ser Asn Asn Lys Tyr Ala Ala Ser Ser Tyr Leu Ser
                 95                 100                 105

ctg acg cct gag cag tgg aag tcc cac aga agc tac agc tgc cag gtc   687
Leu Thr Pro Glu Gln Trp Lys Ser His Arg Ser Tyr Ser Cys Gln Val
             110                 115                 120

acg cat gaa ggg agc acc gtg gag aag aca gtg gcc cct aca gaa tgt   735
Thr His Glu Gly Ser Thr Val Glu Lys Thr Val Ala Pro Thr Glu Cys
         125                 130                 135

tca tag gttctcaacc ctcaccccca ccacgggaga ctagagctgc aggatcccag    791
Ser  *

gggaggggtc tctcctccca ccccaaggca tcaagccctt ctccctgcac tcaataaacc 851
ctcaataaat attctcattg tcaatcaaaa aaaaaaaaaa aaaaaaa               899
```

<210> 16
<211> 139
<212> PRT
<213> Homo sapiens

<400> 16

```
Met Ala Cys Cys Ala Gln Trp Leu His Arg Lys Ala Gly Thr Gln Thr
 1               5                  10                  15
Leu Glu Pro Gln Leu Glu Ala Ala Asp Pro Ala Cys Gly Ala Cys Gly
            20                  25                  30
Ala Gly Gln Pro Lys Ala Ala Pro Ser Val Thr Leu Phe Pro Pro Ser
        35                  40                  45
Ser Glu Glu Leu Gln Ala Asn Lys Ala Thr Leu Val Cys Leu Ile Ser
    50                  55                  60
Asp Phe Tyr Pro Gly Ala Val Thr Val Ala Trp Lys Ala Asp Ser Ser
65                  70                  75                  80
Pro Val Lys Ala Gly Val Glu Thr Thr Thr Pro Ser Lys Gln Ser Asn
                85                  90                  95
Asn Lys Tyr Ala Ala Ser Ser Tyr Leu Ser Leu Thr Pro Glu Gln Trp
            100                 105                 110
Lys Ser His Arg Ser Tyr Ser Cys Gln Val Thr His Glu Gly Ser Thr
        115                 120                 125
Val Glu Lys Thr Val Ala Pro Thr Glu Cys Ser
    130                 135
```

<210> 17
<211> 1869
<212> DNA
<213> Homo sapiens

<400> 17

```
tacctggttg atcctgccag tagcatatgc ttgtctcaaa gattaagcca tgcatgtcta 60
agtacgcacg gccggtacag tgaaactgcg aatggctcat taaatcagtt atggttcctt 120
tggtcgctcg ctcctctccc acttggataa ctgtggtaat tctagagcta atacatgccg 180
acgggcgctg accccccttcg cggggggggat gcgtgcattt atcagatcaa aaccaacccg 240
gtcagcccct ctccggcccc ggccgggggg cgggcgccgg cggctttggt gactctagat 300
aacctcgggc cgatcgcacg ccccccgtgg cggcgacgac ccattcgaac gtctgcccta 360
tcaactttcg atggtagtcg ccgtgcctac catggtgacc acgggtgacg gggaatcagg 420
gttcgattcc ggagagggag cctgagaaac ggctaccaca tccaaggaag gcagcaggcg 480
cgcaaattac ccactcccga cccggggagg tagtgacgaa aaataacaat acaggactct 540
ttcgaggccc tgtaattgga atgagtccac tttaaatcct ttaacgagga tccattggag 600
ggcaagtctg gtgccagcag ccgcggtaat tccagctcca atagcgtata ttaaagttgc 660
tgcagttaaa aagctcgtag ttggatcttg ggagcgggcg ggcggtccgc cgcgaggcga 720
gccaccgccc gtccccgccc cttgcctctc ggcgccccct cgatgctctt agctgagtgt 780
cccgcggggc ccgaagcgtt tactttgaaa aaattagagt gttcaaagca ggcccgagcc 840
gcctggatac cgcagctagg aataatggaa taggaccgcg gttctatttt gttggttttc 900
ggaactgagg ccatgattaa gagggacggc cgggggcatt cgtattgcgc cgctagaggt 960
gaaattcttg gaccggcgca agacggacca gagcgaaagc atttgccaag aatgttttca 1020
ttaatcaaga acgaaagtcg gaggttcgaa gacgatcaga taccgtcgta gttccgacca 1080
taaacgatgc cgaccggcga tgcggcggcg ttattcccat gacccgccgg gcagcttccg 1140
ggaaaccaaa gtctttgggt tccgggggga gtatggttgc aaagctgaaa cttaaaggaa 1200
ttgacggaag ggcaccacca ggagtggagc ctgcggctta atttgactca acacgggaaa 1260
cctcacccgg cccggacacg gacaggattg acagattgat agctctttct cgattccgtg 1320
ggtggtggtg catggccgtt cttagttggt ggagcgattt gtctggttaa ttccgataac 1380
gaacgagact ctggcatgct aactagttac gcgacccccg agcggtcggc gtcccccaac 1440
ttcttagagg gacaagtggc gttcagccac ccgagattga gcaataacag gtctgtgatg 1500
cccttagatg tccggggctg cacgcgcgct acactgactg gctcagcgtg tgcctaccct 1560
acgccggcag gcgcgggtaa cccgttgaac cccattcgtg atggggatcg ggattgcaa 1620
ttattcccca tgaacgagga attcccagta agtgcgggtc ataagcttgc gttgattaag 1680
tccctgccct ttgtacacac cgcccgtcgc tactaccgat tggatggttt agtgaggccc 1740
tcggatcggc cccgccgggg tcggcccacg gccctggcgg agcgctgaga agacggtcga 1800
acttgactat ctagaggaag taaaagtcgt aacaaggttt ccgtaggtga acctgcggaa 1860
ggatcatta                                                          1869
```

<210> 18
<211> 987
<212> DNA
<213> Homo sapiens

<220>
<221> CDS
<222> (61)...(420)

<400> 18

```
aatataagtg gaggcgtcgc gctggcgggc attcctgaag ctgacagcat tcgggccgag 60
atg tct cgc tcc gtg gcc tta gct gtg ctc gcg cta ctc tct ctt tct   108
Met Ser Arg Ser Val Ala Leu Ala Val Leu Ala Leu Leu Ser Leu Ser
 1               5                  10                  15

ggc ctg gag gct atc cag cgt act cca aag att cag gtt tac tca cgt   156
Gly Leu Glu Ala Ile Gln Arg Thr Pro Lys Ile Gln Val Tyr Ser Arg
            20                  25                  30

cat cca gca gag aat gga aag tca aat ttc ctg aat tgc tat gtg tct   204
His Pro Ala Glu Asn Gly Lys Ser Asn Phe Leu Asn Cys Tyr Val Ser
        35                  40                  45

ggg ttt cat cca tcc gac att gaa gtt gac tta ctg aag aat gga gag   252
Gly Phe His Pro Ser Asp Ile Glu Val Asp Leu Leu Lys Asn Gly Glu
    50                  55                  60

aga att gaa aaa gtg gag cat tca gac ttg tct ttc agc aag gac tgg   300
Arg Ile Glu Lys Val Glu His Ser Asp Leu Ser Phe Ser Lys Asp Trp
 65                  70                  75                  80

tct ttc tat ctc ttg tac tac act gaa ttc acc ccc act gaa aaa gat   348
Ser Phe Tyr Leu Leu Tyr Tyr Thr Glu Phe Thr Pro Thr Glu Lys Asp
                85                  90                  95

gag tat gcc tgc cgt gtg aac cat gtg act ttg tca cag ccc aag ata   396
Glu Tyr Ala Cys Arg Val Asn His Val Thr Leu Ser Gln Pro Lys Ile
            100                 105                 110

gtt aag tgg gat cga gac atg taa gcagcatcat ggaggtttga agatgccgca   450
Val Lys Trp Asp Arg Asp Met  *
        115

tttggattgg atgaattcca aattctgctt gcttgctttt taatattgat atgcttatac 510
acttacactt tatgcacaaa atgtagggtt ataataatgt taacatggac atgatcttct 570
ttataattct actttgagtg ctgtctccat gtttgatgta tctgagcagg ttgctccaca 630
ggtagctcta ggagggctgg caacttagag gtggggagca gagaattctc ttatccaaca 690
tcaacatctt ggtcagattt gaactcttca atctcttgca ctcaaagctt gttaagatag 750
ttaagcgtgc ataagttaac ttccaattta catactctgc ttagaatttg ggggaaaatt 810
tagaaatata attgacagga ttattggaaa tttgttataa tgaatgaaac attttgtcat 870
ataagattca tatttacttc ttatacattt gataaagtaa ggcatggttg tggttaatct 930
ggtttatttt tgttccacaa gttaaataaa tcataaaact tgatgtgtta tctctta    987
```

<210> 19
<211> 119
<212> PRT
<213> Homo sapiens

<400> 19

```
Met Ser Arg Ser Val Ala Leu Ala Val Leu Ala Leu Leu Ser Leu Ser
 1               5                  10                  15
Gly Leu Glu Ala Ile Gln Arg Thr Pro Lys Ile Gln Val Tyr Ser Arg
            20                  25                  30
His Pro Ala Glu Asn Gly Lys Ser Asn Phe Leu Asn Cys Tyr Val Ser
        35                  40                  45
Gly Phe His Pro Ser Asp Ile Glu Val Asp Leu Leu Lys Asn Gly Glu
    50                  55                  60
Arg Ile Glu Lys Val Glu His Ser Asp Leu Ser Phe Ser Lys Asp Trp
65                  70                  75                  80
Ser Phe Tyr Leu Leu Tyr Tyr Thr Glu Phe Thr Pro Thr Glu Lys Asp
                85                  90                  95
Glu Tyr Ala Cys Arg Val Asn His Val Thr Leu Ser Gln Pro Lys Ile
            100                 105                 110
Val Lys Trp Asp Arg Asp Met
        115
```

<210> 20
<211> 1704
<212> DNA
<213> Homo sapiens

<400> 20

```
cctccaccaa gggcccatcg gtcttccccc tggcaccctc ctccaagagc acctctgggg 60
gcacagcagc cctgggctgc ctggtcaagg actacttccc cgaaccggtg acggtgtcgt 120
ggaactcagg cgccctgacc agcggcgtgc acaccttccc ggctgtccta cagtcctcag 180
gactctactc cctcagcagc gtggtgaccg tgccctccag cagcttgggc acccagacct 240
acatctgcaa cgtgaatcac aagcccagca acaccaaggt ggacaagaaa gttggtgaga 300
ggccagcaca gggagggagg gtgtctgctg gaagccaggc tcagcgctcc tgcctggacg 360
catcccggct atgcagcccc agtccagggc agcaaggcag gccccgtctg cctcttcacc 420
cggaggcctc tgcccgcccc actcatgctc agggagaggg tcttctggct ttttccccag 480
gctctgggca ggcacaggct aggtgcccct aacccaggcc ctgcacacaa aggggcaggt 540
gctgggctca gacctgccaa gagccatatc cgggaggacc ctgcccctga cctaagccca 600
ccccaaaggc caaactctcc actccctcag ctcggacacc ttctctcctc ccagattcca 660
gtaactccca atcttctctc tgcagagccc aaatcttgtg acaaaactca cacatgccca 720
ccgtgcccag gtaagccagc ccaggcctcg ccctccagct caaggcggga caggtgccct 780
agagtagcct gcatccaggg acaggcccca gccgggtgct gacacgtcca cctccatctc 840
ttcctcagca cctgaactcc tggggggacc gtcagtcttc ctcttccccc caaaacccaa 900
ggacaccctc atgatctccc ggacccctga ggtcacatgc gtggtggtgg acgtgagcca 960
cgaagaccct gaggtcaagt tcaactggta cgtggacggc gtggaggtgc ataatgccaa 1020
gacaaagccg cgggaggagc agtacaacag cacgtaccgt gtggtcagcg tcctcaccgt 1080
cctgcaccag gactggctga atggcaagga gtacaagtgc aaggtctcca acaaagccct 1140
cccagccccc atcgagaaaa ccatctccaa agccaaaggt gggacccgtg gggtgcgagg 1200
gccacatgga cagaggccgg ctcggcccac cctctgccct gagagtgacc gctgtaccaa 1260
cctctgtccc tacagggcag ccccgagaac cacaggtgta caccctgccc ccatcccggg 1320
atgagctgac caagaaccag gtcagcctga cctgcctggt caaaggcttc tatcccagcg 1380
acatcgccgt ggagtgggag agcaatgggc agccggagaa caactacaag accacgcctc 1440
ccgtgctgga ctccgacggc tccttcttcc tctacagcaa gctcaccgtg gacaagagca 1500
ggtggcagca ggggaacgtc ttctcatgct ccgtgatgca tgaggctctg cacaaccact 1560
acacacagaa gagcctctcc ctgtctccgg gtaaatgagt gccacggccg gcaagccccc 1620
gctccccagg ctctcggggt cgcgcgagga tgcttggcac gtaccccgtg tacatacttc 1680
ccaggcaccc agcatggaaa taaa                                        1704
```

<210> 21
<211> 1527
<212> DNA
<213> Homo sapiens

<400> 21

```
catccccgac cagccccaag gtcttcccgc tgagcctctg cagcacccag ccagatggga 60
acgtggtcat cgcctgcctg gtccagggct tcttccccca ggagccactc agtgtgacct 120
ggagcgaaag cggacagggc gtgaccgcca gaaacttccc acccagccag gatgcctccg 180
gggacctgta caccacgagc agccagctga ccctgccggc cacacagtgc ctagccggca 240
agtccgtgac atgccacgtg aagcactaca cgaatcccag ccaggatgtg actgtgccct 300
gcccaggtca gagggcaggc tggggagtgg ggcggggcca ccccgtcgtg ccctgacact 360
gcgcctgcac ccgtgttccc cacagggagc cgccccttca ctcacaccag agtggaccgc 420
gggccgagcc ccaggaggtg gtggtggaca ggccaggagg ggcgaggcgg gggcatgggg 480
aagtatgtgc tgaccagctc aggccatctc tccactccag ttccctcaac tccacctacc 540
ccatctccct caactccacc taccccatct ccctcatgct gccacccccg actgtcactg 600
caccgaccgg ccctcgagga cctgctctta ggttcagaag cgaacctcac gtgcacactg 660
accggcctga gagatgcctc aggtgtcacc ttcacctgga cgccctcaag tgggaagagc 720
gctgttcaag gaccacctga gcgtgacctc tgtggctgct acagcgtgtc cagtgtcctg 780
ccgggctgtg ccgagccatg gaaccatggg aagaccttca cttgcactgc tgcctacccc 840
gagtccaaga ccccgctaac cgccaccctc tcaaaatccg gtgggtccag accctgctcg 900
gggccctgct cagtgctctg gtttgcaaag catattcctg gcctgcctcc tccctcccaa 960
tcctgggctc cagtgctcat gccaagtaca gagggaaact gaggcaggct gaggggccag 1020
gacacagccc agggtgccca ccagagcaga ggggctctct catcccctgc ccagccccct 1080
gacctggctc tctaccctcc aggaaacaca ttccggcccg aggtccacct gctgccgccg 1140
ccgtcggagg agctggccct gaacgagctg gtgacgctga cgtgcctggc acgcggcttc 1200
agccccaagg atgtgctggt tcgctggctg caggggtcac aggagctgcc ccgcgagaag 1260
tacctgactt gggcatcccg gcaggagccc agccagggca ccaccacctt cgctgtgacc 1320
agcatactgc gcgtggcagc cgaggactgg aagaaggggg acaccttctc ctgcatggtg 1380

ggccacgagg ccctgccgct ggccttcaca cagaagacca tcgaccgctt ggcgggtaaa 1440
cccacccatg tcaatgtgtc tgttgtcatg gcggaggtgg acggcacctg ctactgagcc 1500
gcccgcctgt ccccacccct gaataaa                                    1527
```

<210> 22
<211> 21
<212> DNA
<213> Artificial Sequence

<220>
<223> Forward primer

<400> 22
ctctggccct gcttattgtt g 21

<210> 23
<211> 24
<212> DNA
<213> Artificial Sequence

<220>
<223> Reverse primer

<400> 23
gaagacattt gggaaggact gact 24

<210> 24
<211> 20
<212> DNA
<213> Artificial Sequence

<220>
<223> Forward primer

<400> 24
tgggcaagtg gatctgaaca 20

<210> 25
<211> 22
<212> DNA
<213> Artificial Sequence

<220>
<223> Reverse primer

<400> 25
ctcagggaag tagcctttga ca 22

<210> 26
<211> 20
<212> DNA
<213> Artificial Sequence

<220>
<223> Foward primer

<400> 26
ggcccttcca gatctttgag 20

<210> 27
<211> 22
<212> DNA
<213> Artificial Sequence

<220>
<223> Reverse primer

<400> 27
ggatccagag ttccaggtca ct 22

<210> 28
<211> 27
<212> DNA
<213> Artificial Sequence

<220>
<223> Forward primer

<400> 28
gggcttccaa gccaacaggg caggaca 27

<210> 29
<211> 21
<212> DNA
<213> Artificial Sequence

<220>
<223> Reverse primer

<400> 29
gttgccgttg gggtgctgga c 21

<210> 30
<211> 25
<212> DNA

<213> Artificial Sequence

<220>
<223> Forward primer

<400> 30
ggctgaggtt agggttccat ctcag 25

<210> 31
<211> 26
<212> DNA
<213> Artificial Sequence

<220>
<223> Reverse primer

<400> 31
gagggagtca agaaagtcac gctgga 26

<210> 32
<211> 26
<212> DNA
<213> Artificial Sequence

<220>
<223> Forward primer

<400> 32
gttagcatgc cagagtctcg ttcgtt 26

<210> 33
<211> 17
<212> DNA
<213> Artificial Sequence

<220>
<223> Reverse primer

<400> 33
ccgttcttag ttggtgg 17

<210> 34
<211> 25
<212> DNA
<213> Artificial Sequence

<220>
<223> Forward primer

<220>
<221> misc_feature
<222> 9
<223> The nucleotide at position 9 can have a detectable label.

<400> 34
cctgaattat ttcagttaag catgt 25

<210> 35

<211> 19
<212> DNA
<213> Artificial Sequence

<220>
<223> Reverse primer

<400> 35
cctcgtctcc tgtgagaat 19

<210> 36
<211> 16
<212> DNA
<213> Artificial Sequence

<220>
<223> nucleic acid probe

<220>
<221> misc_feature
<222> 8
<223> The nucleotide at position 8 can have a detectable label.

<400> 36
ctccatcact ttctcc 16

<210> 37
<211> 16
<212> DNA
<213> Artificial Sequence

<220>
<223> Forward primer

<400> 37
caacagggca ggacac 16

<210> 38
<211> 20
<212> DNA
<213> Artificial Sequence

<220>
<223> Forward primer

<400> 38
gaggatctcc tgtccatcag 20

<210> 39
<211> 25
<212> DNA
<213> Artificial Sequence

<220>
<223> Reverse primer

<400> 39
ccaaatgtgg ccgtggtttc tgtca 25

<210> 40
<211> 18
<212> DNA
<213> Artificial Sequence

<220>
<223> Nucleic acid probe

<400> 40
tcacctctcc tactcact 18

<210> 41
<211> 25
<212> DNA
<213> Artificial Sequence

<220>
<223> Forward primer

<400> 41
ctagaaattg acacaagtgg acctt 25

<210> 42
<211> 25
<212> DNA
<213> Artificial Sequence

<220>
<223> Reverse primer

<400> 42
cccggtctcc caaataaata cattc 25

<210> 43
<211> 15
<212> DNA
<213> Artificial Sequence

<220>
<223> Nucleic acid probe

<220>
<221> misc_feature
<222> 15
<223> The nucleotide at position 15 can have a detectable label.

<400> 43
gccttcctct ctcca 15

<210> 44
<211> 24
<212> DNA
<213> Artificial Sequence

<220>
<223> Forward Primer

<400> 44

ctgtgacttt ggcttatctg atct 24

<210> 45
<211> 25
<212> DNA
<213> Artificial Sequence

<220>
<223> Reverse Primer

<400> 45
gcgtcacttt gaagaatctc ctggt 25

<210> 46
<211> 17
<212> DNA
<213> Artificial Sequence

<220>
<223> Nucleic acid probe

<400> 46
gcctgtgttc ccttgtg 17

<210> 47
<211> 23
<212> DNA
<213> Artificial Sequence

<220>
<223> Forward primer

<400> 47
ccaatgaaag gccctattgc tat 23

<210> 48
<211> 25
<212> DNA
<213> Artificial Sequence

<220>
<223> Reverse primer

<400> 48
cagtgaagac atcctcctga agagt 25

<210> 49
<211> 15
<212> DNA
<213> Artificial Sequence

<220>
<223> Nucleic acid probe

<220>
<221> misc_feature
<222> 11
<223> The nucleotide at position 11 can have a detectable label.

<400> 49
aatctggtcc aaaac 15

<210> 50
<211> 28
<212> DNA
<213> Artificial Sequence

<220>
<223> Forward primer

<220>
<221> misc_feature
<222> 2
<223> The nucleotide at position 2 can have a detectable label.

<220>
<221> misc_feature
<222> 8
<223> The nucleotide at position 8 can have a detectable label.

<220>
<221> misc_feature
<222> 13
<223> The nucleotide at position 13 can have a detectable label.

<400> 50
gacacggaca ggattgacag attgatag 28

<210> 51
<211> 26
<212> DNA
<213> Artificial Sequence

<220>
<223> Reverse primer

<220>
<221> misc_feature
<222> 4
<223> The nucleotide at position 4 can have a detectable label.

<400> 51
gttagcatgc cagagtctcg ttcgtt 26

<210> 52
<211> 17
<212> DNA
<213> Artificial Sequence

<220>
<223> Nucleic acid probe

<220>
<221> misc_feature
<222> 9
<223> The nucleotide at position 9 canhave a detectable label.

<400> 52
ccgttcttag ttggtgg 17

<210> 53
<211> 24
<212> DNA
<213> Artificial Sequence

<220>
<223> Forward primer

<400> 53
acagtcttag ggagagttta tgac 24

<210> 54
<211> 19
<212> DNA
<213> Artificial Sequence

<220>
<223> Reverse primer

<400> 54
caccacgtgt tcgtctgtg 19

<210> 55
<211> 25
<212> DNA
<213> Artificial Sequence

<220>
<223> Forward primer

<400> 55
gatattctga tagagtggcc ttcat 25

<210> 56
<211> 16
<212> DNA
<213> Artificial Sequence

<220>
<223> Forward primer

<400> 56
agcagagctg gccgta 16

<210> 57
<211> 17
<212> DNA
<213> Artificial Sequence

<220>
<223> Forward primer

<400> 57
ccagaaaggc ccagagt 17

<210> 58
<211> 480
<212> DNA
<213> Artificial Sequence

<220>
<223> Nucleotides 961381 to 961860 of GenBank Accession No. NG_001019 (Jan 10, 2006 version).

<400> 58

```
gtaagatgtt taagaaatta aacagtctta gggagagttt atgactgtat tcaaaaagtt 60
ttttaaatta gcttgttatc ccttcatgtg ataactaatc tcaaatactt tttcgatacc 120
tcagagcatt attttcataa tgactgtgtt cacaatcttt ttaggttaac tcgttttctc 180
tttgtgatta aggagaaaca ctttgatatt ctgatagagt ggccttcatt ttagtatttt 240
tcaagaccac ttttcaacta ctcactttag gataagtttt aggtaaaatg tgcatcatta 300

tcctgaatta tttcagttaa gcatgttagt tggtggcata agagaaaact caatcagata 360
gtgctgaaga caggactgtg gagacacctt agaaggacag attctgttcc gaatcaccga 420
tgcggcgtca gcaggactgg cctagcggag gctctgggag ggtggctgcc aggcccggcc 480
```

<210> 59
<211> 360
<212> DNA
<213> Artificial Sequence

<220>
<223> Nucleotides 967201 to 967560 of GenBank Accession No. NG_001019 (Jan 10, 2006 version).

<400> 59

```
tgtcctgcgg gtcctcaggg agtgcatccg ccccaaccct tttccccctc gtctcctgtg 60
agaattcccc gtcggatacg agcagcgtgg ccgttggctg cctcgcacag gacttccttc 120
ccgactccat cactttctcc tggaaataca agaacaactc tgacatcagc agcacccggg 180
gcttcccatc agtcctgaga gggggcaagt acgcagccac ctcacaggtg ctgctgcctt 240
ccaaggacgt catgcagggc acagacgaac acgtggtgtg caaagtccag caccccaacg 300
gcaacaaaga aaagaacgtg cctcttccag gtgagggccg ggcccagcca ccgggacaga 360
```

<210> 60
<211> 421
<212> DNA
<213> Artificial Sequence

<220>
<223> Nucleotides 1047841 to 1048261 of GenBank Accession No. NG_001019 (Jan 10, 2006 version).

<400> 60

```
ccttcaggca ccgatgccca cccagtgcga gacgacaggg accgtgggca ggggcttcca 60
agccaacagg gcaggacaca ccagaggctg actgaggcct ccaggacgac cgggctggga 120
gtgtgaggaa catgacggga tggggcagag ccagccatgg ggtgatgcca ggatgggcat 180
gaccgacctg agctcaggag gcagcagaga gagggaggag gagaggcccc aggtgaaccg 240
aggggcttgt ccaggccggc agcatcaccg gagcccaggg cagggtcagc agagctggcc 300
gtagggccct cctctcagcc aggaccaagg acagcaggtg agctgggagc agagcaggga 360
gggtgagtgt ggcagcagga caggagggtg gaagccaagg agcccagagg cagaggcagg 420
g                                                                 421
```